# EUROPEAN PATENT SPECIFICATION

(11) **EP 3 431 476 B1**
(45) Date of publication and mention of the grant of the patent: **04.09.2024**
(21) Application number: 17766990.0
(22) Date of filing: 16.03.2017
(51) Int. Cl.: H10K 85/60, C07D 487/04, C07D 495/04, C07D 209/56, C07D 209/82, C09K 11/06, H10K 50/11, H10K 101/10

(54) **ORGANIC ELECTRIC ELEMENT AND ELECTRONIC DEVICE THEREFROM**
ORGANISCHES ELEKTRISCHES ELEMENT UND ELEKTRONISCHE VORRICHTUNG DARAUS
ÉLÉMENT ÉLECTRIQUE ORGANIQUE ET DISPOSITIF ÉLECTRONIQUE ASSOCIÉ

(30) Priority: 16.03.2016 KR 20160031744; 30.03.2016 KR 20160038502; 30.09.2016 KR 20160126778
(43) Date of publication of application: 23.01.2019
(73) Proprietor: Duk San Neolux Co., Ltd., Chungcheongnam-do 31027 (KR)
(72) Inventor: LEE, Mun Jae, Cheonan-si Chungcheongnam-do 31204 (KR); MUN, Soung Yun, Cheonan-si Chungcheongnam-do 31027 (KR); KWON, Jae Taek, Cheonan-si Chungcheongnam-do 31044 (KR); KIM, Dae Sung, Yongin-si Gyeonggi-do 17090 (KR); PARK, Moo Jin, Cheonan-si Chungcheongnam-do 31100 (KR); LEE, Sun Hee, Hwaseong-si Gyeonggi-do 18436 (KR); HWANG, Sun Pil, Ansan-si Gyeonggi-do 15258 (KR); JUNG, Ho Young, Cheonan-si Chungcheongnam-do 31027 (KR); LEE, Bum Sung, Hwaseong-si Gyeonggi-do 18441 (KR)
(74) Representative: Gassner, Birgitta
(86) International application number: PCT/KR2017/002828
(87) International publication number: WO 2017/160089

(56) References cited:
- KR-A- 20110 066 766
- KR-A- 20120 081 539
- KR-A- 20140 018 789
- KR-A- 20150 126 755
- KR-A- 20160 003 362
- KR-A- 20160 012 895
- US-A1- 2014 299 865

## Description

### BACKGROUND

### Technical Field

The present invention relates to compound for organic electric element, organic electric element using the same, and an electronic device thereof.

### Background Art

In general, organic light emitting phenomenon refers to a phenomenon that converts electric energy into light energy by using an organic material. An organic electric element using an organic light emitting phenomenon usually has a structure including an anode, a cathode, and an organic material layer interposed there between. Here, in order to increase the efficiency and stability of the organic electronic element, the organic material layer is often composed of a multi-layered structure composed of different materials, and for example, may include a hole injection layer, a hole transport layer, an emitting layer, an electron transport layer, an electron injection layer and the like.

A material used as an organic material layer in an organic electric element may be classified into a light emitting material and a charge transport material, such as a hole injection material, a hole transport material, an electron transport material, an electron injection material and the like depending on its function.

In the case of a polycyclic compound containing a heteroatom, the difference in properties according to the material structure is so large that it is applied to various layers as a material of an organic electric element. In particular, it has characteristics of different band gaps (HOMO, LUMO), electrical characteristics, chemical properties, and physical properties depending on the number of rings, fused positions and the type and arrangement of heteroatoms, therefore application development for layers of various organic electric elements using the same has been progressed.

As a representative example thereof, in the following Patent Documents 1 to 4, the performance of the 5-membered cyclic compound in the polycyclic compound has been reported depending on the hetero type, arrangement, substituent type, fused position, and the like.
Patent Document 1: U.S. Patent No. 5843607
Patent Document 2: Japanese Laid-Open Patent Publication No. 1999-162650
Patent Document 3: Korean Published Patent Application No. 2008-0085000
Patent Document 4: US Patent Publication No. 2010-0187977
Patent Document 5: Korean Published Patent Application No. 2011-0018340
Patent Document 6: Korean Published Patent 2009-0057711
Patent Document 7: US Published Patent Application No. 2014/299865A1
Patent Document 8: Korean Published Patent Application No. 2016-0012895A

Patent Documents 1 and 2 disclose an embodiment in which the indolecarbazole core in which the hetero atom in the 5-membered cyclic compound is composed only of nitrogen (N) is used, and an aryl group substituted or unsubstituted in N of indolocarbazole is used. However, in the prior invention 1, there exists only a simple aryl group substituted or unsubstituted with an alkyl group, an amino group, an alkoxy group, or the like as a substituent, so that the effect of the substituents of the polycyclic compounds was very poor to prove, and only the use as a hole transport material is described, and the use thereof as a phosphorescent host material is not described.

Patent Documents 3 and 4 disclose a compound in which pyridine, pyrimidine, triazine or the like containing an aryl group and N is substituted for an indolecarbazole core having a hetero atom N in the same 5-membered cyclic compound as in the above Patent Documents 1 and 2, however only the use examples for phosphorescent green host materials are described, and the performance for other heterocyclic compounds substituted for indolecarbazole core is not described.

In Patent Document 5, Nitrogen (N), oxygen (O), sulfur (S), carbon and the like are described as heteroatom in the 5-membered cyclic compound, however there are only examples using the same heteroatom in the performance measurement data, the performance characteristics of a 5-membered cyclic compound containing a different heteroatom could not be confirmed. Therefore, the patent document does not disclose solutions to low charge carrier mobility and low oxidation stability of a 5-membered cyclic compound containing same heteroatom.

When the 5-membered cyclic compound molecules are generally laminated, as the adjacent π- electrons increase, they have a strong electrical interaction, and this is closely related to the charge carrier mobility, particularly, the same 5-membered cyclic compound of N-N type has an edge-to-face morphology as an order of arrangement of molecules when molecules are laminated, otherwise a different 5-membered cyclic compound with different heteroatoms has an antiparallel cofacial π-stacking structure in which the packing structure of the molecules is opposite to each other, so that the arrangement order of the molecules becomes face-to-face morphology. It is reported that the steric effect of the substituent substituted on the asymmetrically arranged hetero atom N as the cause of this laminated structure causes relatively high carrier mobility and high oxidation stability *(*Org. Lett. 2008, 10, 1199).

In Patent Document 6, an example of using as a fluorescent host material for various polycyclic compounds having seven or more membered cyclic compounds has been reported.

In Patent Document 7 an organic element comprising two electrodes and a phosphorescent light emitting layer comprising a first and a second host and a phosphorescent dopant material which contains a metal complex is disclosed. In Patent Document 8 an organic electric element comprising a first electrode; a second electrode; and an organic layer between the first electrode and the second electrode is described.

As described above, the fused positions, the number of rings, the arrangement of heteroatoms, and characteristic change by type of the polycyclic compounds have not yet been sufficiently developed.

Particularly, in a phosphorescent organic electric element using a phosphorescent dopant material, the LUMO and HOMO levels of the host material have a great influence on the efficiency and life span of the organic electric element, this is because the charge balance control in the emitting layer, the quenching of the dopant, and the reduction in efficiency and life span due to light emission at the interface of the hole transport layer can be prevented, depending on whether electron and hole injection in the emitting layer can be efficiently controlled.

For fluorescent and phosphorescent host materials, recently we have been studying the increase of efficiency and life span of organic electric elements using TADF (thermal activated delayed fluorescent), exciplex, etc., particularly, and many studies have been carried out to identify the energy transfer method from the host material to the dopant material.

Although there are various methods for identifying the energy transfer in the emitting layer for TADF (thermally activated delayed fluorescent) and exciplex, it can be easily confirmed by the PL lifetime (TRTP) measurement method.

The TRTP (Time Resolved Transient PL) measurement method is a method of observing a decay time over time after irradiating the host thin film with a pulsed light source, and therefore it is possible to identify the energy transfer method by observing the energy transfer and the lag time. The TRTP measurement can distinguish between fluorescence and phosphorescence, an energy transfer method in a mixed host material, an exciplex energy transfer method, and a TADF energy transfer method.

There are various factors affecting the efficiency and life span depending on the manner in which the energy is transferred from the host material to the dopant material, and the energy transfer method differs depending on the material, so that the development of stable and efficient host material for organic electric element has not yet been sufficiently developed. Therefore, development of new materials is continuously required, and especially development of a host material for an emitting layer is urgently required.

### DETAILED DESCRIPTION OF THE INVENTION

### Summary

The present invention has been proposed in order to solve the problems of the phosphorescent host material, and an object of the present invention is, by controlling the HOMO level of a host material of a phosphorescent emitting organic electric element including a phosphorescent dopant, to provide a compound capable of controlling charge balance and of improving efficiency and life span in an emitting layer, and an organic electric element using the same and an electronic device thereof.

### Technical Solution

In order to control the efficient hole injection in the emitting layer of the phosphorescent emitting organic electric element, the present invention can contain a mixture of a specific second host material in combination with a specific first host material as a main component so that the energy barrier of the emitting layer and the adjacent layer can be reduced, and provide high efficiency and long life span of the organic electric element by maximizing the charge balance in the emitting layer.

The present invention provides an organic electronic element characterized by comprising a first electrode, a second electrode, and an organic material layer formed between the first electrode and the second electrode, wherein the organic material layer comprises an emitting layer, wherein the emitting layer comprises a first host compound represented by Formula 1 and a second host compound represented by Formula 2.

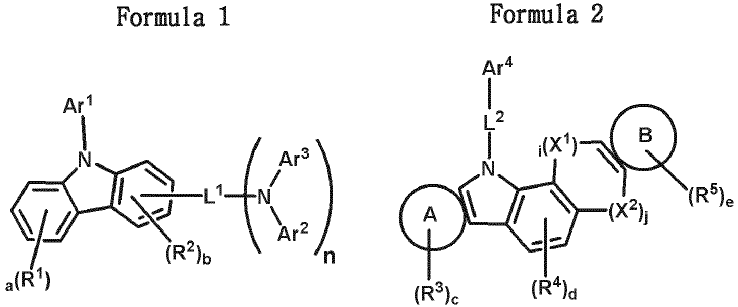

The present invention also provides organic electric elements and electronic devices using the compounds represented by the Formulas.

### Effects of the Invention

By using the mixture according to the present invention as a phosphorescent host material, it is possible to achieve a high luminous efficiency and a low driving voltage of an organic electric element, and the life span of the device can be greatly improved.

### BRIEF DESCRIPTION OF THE DRAWINGS

Fig. 1 is an illustration of an organic electroluminescent device according to the present invention.

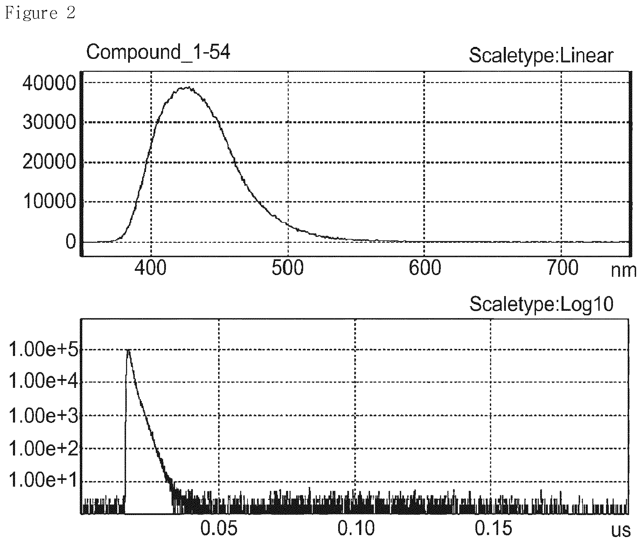

Fig. 2 shows the PL lifetime results of compounds 1-54 according to the present invention.

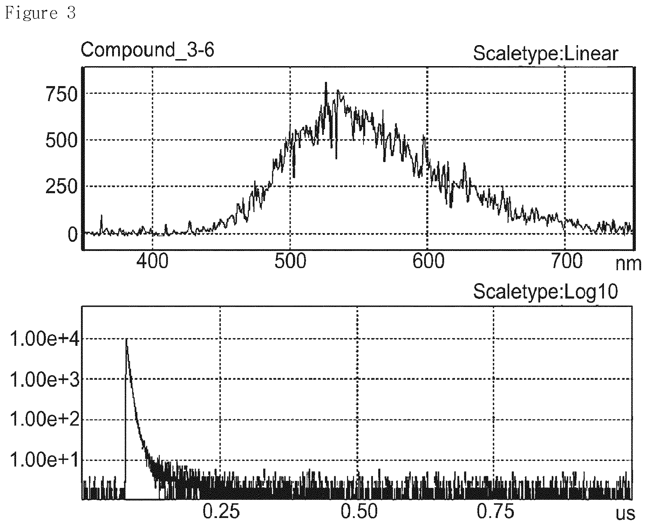

Figure 3 shows the PL lifetime results of compounds 3-6 according to the present invention.

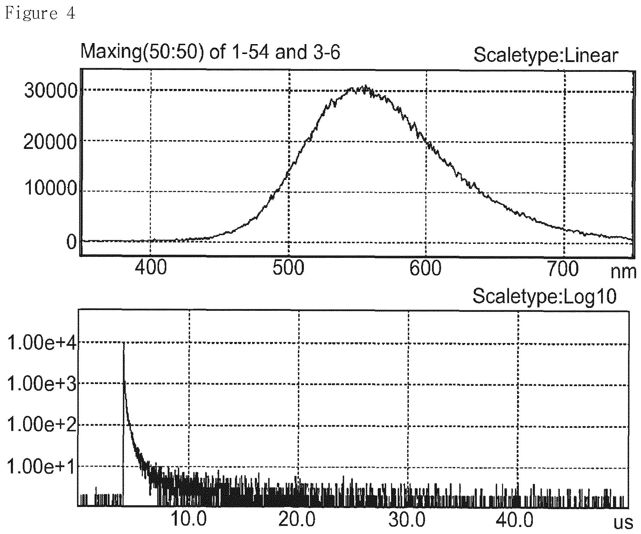

FIG. 4 shows the PL lifetime results when compound 1-54 and compound 3-6 according to the present invention were mixed.

| | | | |
|---|---|---|---|
| 100: | organic electric element, | 110: | substrate |
| 120: | the first electrode (anode), | 130: | the hole injection layer |
| 140: | the hole transport layer, | 141: | a buffer layer |
| 150: | the emitting layer, | 151: | the emitting auxiliary layer |
| 160: | the electron transport layer, | 170: | the electron injection layer |
| 180: | the second electrode (cathode) | | |

### DETAILED DESCRIPTION

Hereinafter, some embodiments of the present invention will be described in detail. Further, in the following description of the present invention, a detailed description of known functions and configurations incorporated herein will be omitted when it may make the subject matter of the present invention rather unclear.

In addition, terms, such as first, second, A, B, (a), (b) or the like may be used herein when describing components of the present invention. Each of these terminologies is not used to define an essence, order or sequence of a corresponding component but used merely to distinguish the corresponding component from other component(s). It should be noted that if a component is described as being "connected", "coupled", or "connected" to another component, the component may be directly connected or connected to the other component, but another component may be "connected ", " coupled" or "connected" between each component.

As used in the specification and the accompanying claims, unless otherwise stated, the following is the meaning of the term as follows.

Unless otherwise stated, the term "halo" or "halogen" , as used herein, includes fluorine, bromine, chlorine, or iodine.

Unless otherwise stated, the term "alkyl" or "alkyl group" , as used herein, has a single bond of 1 to 60 carbon atoms, and means saturated aliphatic functional radicals including a linear alkyl group, a branched chain alkyl group, a cycloalkyl group (alicyclic), an cycloalkyl group substituted with a alkyl or an alkyl group substituted with a cycloalkyl. Unless otherwise stated, the term "haloalkyl" or "halogen alkyl" , as used herein, includes an alkyl group substituted with a halogen.

Unless otherwise stated, the term "heteroalkyl" , as used herein, means alkyl substituted one or more of carbon atoms consisting of an alkyl with hetero atom.

Unless otherwise stated, the term "alkenyl" or "alkynyl" , as used herein, has double or triple bonds of 2 to 60 carbon atoms, but is not limited thereto, and includes a linear or a branched chain group.

Unless otherwise stated, the term "cycloalkyl" , as used herein, means alkyl forming a ring having 3 to 60 carbon atoms, but is not limited thereto. Unless otherwise stated, the term "alkoxyl group" , "alkoxy group" or "alkyloxy group" , as used herein, means an oxygen radical attached to an alkyl group, but is not limited thereto, and has 1 to 60 carbon atoms. Unless otherwise stated, the term "alkenoxyl group" , "alkenoxy group" , "alkenyloxy group" or "alkenyloxy group" , as used herein, means an oxygen radical attached to an alkenyl group, but is not limited thereto, and has 2 to 60 carbon atoms.

Unless otherwise stated, the term "aryloxyl group" or "aryloxy group" , as used herein, means an oxygen radical attached to an aryl group, but is not limited thereto, and has 6 to 60 carbon atoms.

Unless otherwise stated, the term "aryl group" or "arylene group" , as used herein, has 6 to 60 carbon atoms, but is not limited thereto. Herein, the aryl group or arylene group means a monocyclic and polycyclic aromatic group, and may also be formed in conjunction with an adjacent group. Examples of "aryl group" may include a phenyl group, a biphenyl group, a fluorene group, or a spirofluorene group.

The prefix "aryl" or "ar" means a radical substituted with an aryl group. For example, an arylalkyl may be an alkyl substituted with an aryl, and an arylalkenyl may be an alkenyl substituted with aryl, and a radical substituted with an aryl has a number of carbon atoms as defined herein. Also, when prefixes are named subsequently, it means that substituents are listed in the order described first. For example, an arylalkoxy means an alkoxy substituted with an aryl, an alkoxylcarbonyl means a carbonyl substituted with an alkoxyl, and an arylcarbonylalkenyl also means an alkenyl substituted with an arylcarbonyl, wherein the arylcarbonyl may be a carbonyl substituted with an aryl.

Unless otherwise stated, the term "heteroalkyl" , as used herein, means alkyl containing one or more of hetero atoms. Unless otherwise stated, the term "heteroaryl group" or "heteroarylene group" , as used herein, means a C2 to C60 aryl containing one or more of hetero atoms or arylene group, but is not limited thereto, and includes at least one of monocyclic and polycyclic rings, and may also be formed in conjunction with an adjacent group.

Unless otherwise stated, the term "heterocyclic group" , as used herein, contains one or more heteroatoms, but is not limited thereto, has 2 to 60 carbon atoms, includes any one of monocyclic and polycyclic rings, and may include heteroaliphatic ring and/or heteroaromatic ring. Also, the heterocyclic group may also be formed in conjunction with an adjacent group. Unless otherwise stated, the term "heteroatom" , as used herein, represents at least one of N, O, S, P, or Si.

Also, the term "heterocyclic group" may include a ring containing SO₂ instead of carbon consisting of cycle. For example, "heterocyclic group" includes compound below.

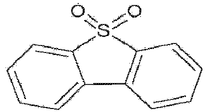

Unless otherwise stated, the term "aliphatic" , as used herein, means an aliphatic hydrocarbon having 1 to 60 carbon atoms, and the term "aliphatic ring" , as used herein, means an aliphatic hydrocarbon ring having 3 to 60 carbon atoms.

Unless otherwise stated, the term "ring" , as used herein, means an aliphatic ring having 3 to 60 carbon atoms, or an aromatic ring having 6 to 60 carbon atoms, or a hetero ring having 2 to 60 carbon atoms, or a fused ring formed by the combination of them, and includes a saturated or unsaturated ring.

Other hetero compounds or hetero radicals other than the above-mentioned hetero compounds include, but are not limited thereto, one or more heteroatoms.

Unless otherwise stated, the term "carbonyl" , as used herein, is represented by -COR' , wherein R' may be hydrogen, an alkyl having 1 to 20 carbon atoms, an aryl having 6 to 30 carbon atoms, a cycloalkyl having 3 to 30 carbon atoms, an alkenyl having 2 to 20 carbon atoms, an alkynyl having 2 to 20 carbon atoms, or the combination of these.

Unless otherwise stated, the term "ether" , as used herein, is represented by -R-O-R' , wherein R or R' may be independently hydrogen, an alkyl having 1 to 20 carbon atoms, an aryl having 6 to 30 carbon atoms, a cycloalkyl having 3 to 30 carbon atoms, an alkenyl having 2 to 20 carbon atoms, an alkynyl having 2 to 20 carbon atoms, or the combination of these.

Unless otherwise stated, the term "substituted or unsubstituted" , as used herein, means that substitution is substituted by at least one substituent selected from the group consisting of, but is not limited thereto, deuterium, halogen, an amino group, a nitrile group, a nitro group, a C₁-C₂₀ alkyl group, a C₁-C₂₀ alkoxyl group, a C₁-C₂₀ alkylamine group, a C₁-C₂₀ alkylthiophene group, a C₆-C₂₀ arylthiophene group, a C₂-C₂₀ alkenyl group, a C₂-C₂₀ alkynyl group, a C₃-C₂₀ cycloalkyl group, a C₆-C₂₀ aryl group, a C₆-C₂₀ aryl group substituted by deuterium, a C₈-C₂₀ arylalkenyl group, a silane group, a boron group, a germanium group, and a C₂-C₂₀ heterocyclic group.

Unless otherwise expressly stated, the Formula used in the present invention, as used herein, is applied in the same manner as the substituent definition according to the definition of the exponent of the following Formula, 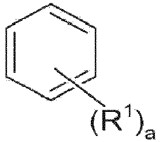 wherein, when a is an integer of zero, the substituent R¹ is absent, when a is an integer of 1, the sole substituent R¹ is linked to any one of the carbon constituting the benzene ring, when a is an integer of 2 or 3, they are respectively combined as follows, in which R¹ may be the same as or different from each other, and when a is an integer of 4 to 6, and it is bonded to the carbon of the benzene ring in a similar manner, whereas the indication of hydrogen bonded to the carbon forming the benzene ring is omitted.

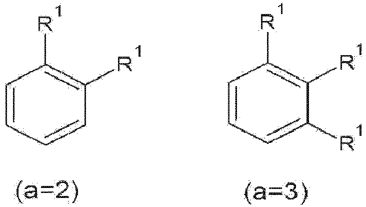

Unless otherwise expressly stated, the terms "ortho", "meta", and "para" used in the present invention refer to the substitution positions of all substituents, and the ortho position indicates the position of the substituent immediately adjacent to the compound, for example, when benzene is used, it means 1 or 2 position, and the meta position is the next substitution position of the neighbor substitution position, when benzene as an example stands for 1 or 3 position, and the para position is the next substitution position of the meta position, which means 1 and 4 position when benzene is taken as an example. A more detailed example of the substitution position is as follows, and it can be confirmed that the ortho-, and meta-position are substituted by non-linear type and para- positions are substituted by linear type.

Example of ortho- position:

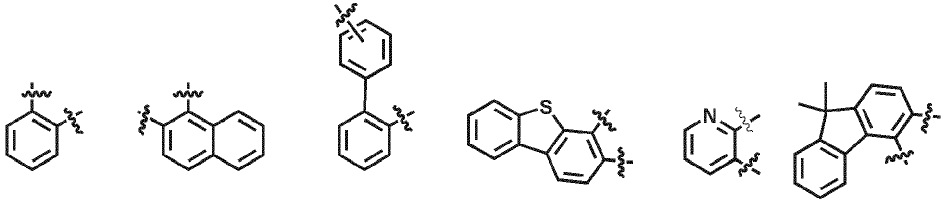

Example of meta- position:

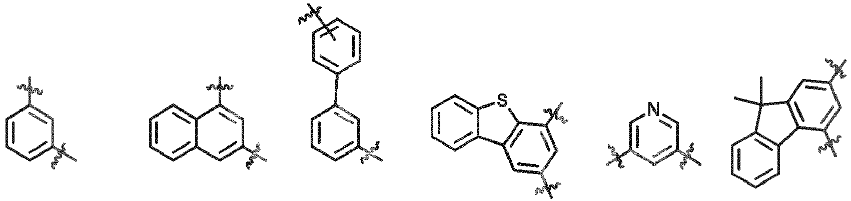

Example of para- position:

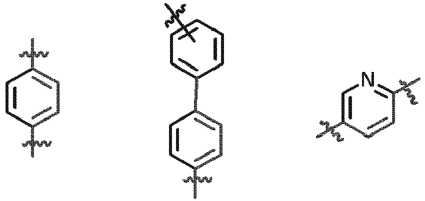

Hereinafter, a compound according to an aspect of the present invention and an organic electric element comprising the same will be described.

The present invention provides an organic electronic element characterized by comprising a first electrode, a second electrode, and an organic material layer formed between the first electrode and the second electrode, wherein the organic material layer comprises an emitting layer, wherein the emitting layer comprises a first host compound represented by Formula 1 as the phosphorescent emitting layer and a second host compound represented by Formula 2, 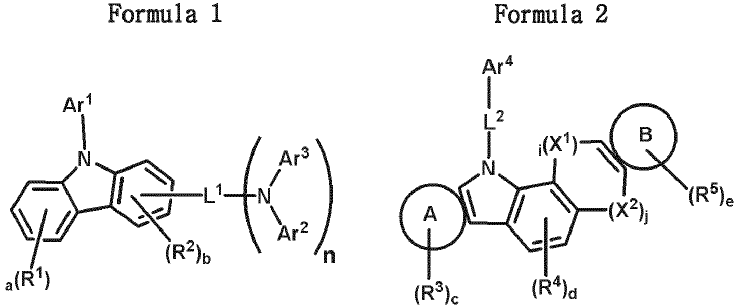 wherein
1) Ar¹, Ar², Ar³, and Ar⁴ are each independently selected from the group consisting of a C₆-C₆₀ aryl group; a fluorenyl group; a C₂-C₆₀ heterocyclic group including at least one hetero atom of O, N, S, Si and P; a fused ring group of a C₃-C₆₀ aliphatic ring and a C₆-C₆₀ aromatic ring, a C₁-C₅₀ alkyl group; a C₂-C₂₀ alkenyl group; a C₂-C₂₀ alkynyl group; a C₁-C₃₀ alkoxyl group; a C₆-C₃₀ aryloxy group;
2) a is an integer of 0 to 4, and b is an integer of 0 to 3, and c and e are integer of 0 to 10, and d is an integer of 0 to 2,
3) R1, R², R³, R⁴ and R⁵ may be the same or different, and are each independently selected from the group consisting of deuterium; halogen; a C₆-C₆₀ aryl group; a fluorenyl group; a C₂-C₆₀ heterocyclic group including at least one heteroatom of O, N, S, Si or P; a fused ring group of a C₃-C₆₀ aliphatic ring and a C₆-C₆₀ aromatic ring: a C₁-C₅₀ alkyl group; a C₂-C₂₀ alkenyl group; a C₂-C₂₀ alkynyl group; a C₁-C₃₀ alkoxyl group; a C₆-C₃₀ aryloxy groups; or in case a, b, c, and e are two or more, and d is 2, and are each in plural and are the same or different, and a plurality of R¹ or a plurality of R² or a plurality of R³ or a plurality of R⁴ or a plurality of R⁵ may be bonded too each other to form a ring.
4) L¹ and L² may be independently selected from the group consisting of a single bond, a C₆-C₆₀ arylene group, and a fluorenylene group; a fused ring group of a C₃-C₆₀ aliphatic ring and a C₆-C₆₀ aromatic ring; and a C₂-C₆₀ heterocyclic group,
5) A and B may be independently a C₆-C₆₀ aryl group or a C₂-C₂₀ heterocyclic group, when both A and B are a substituted or unsubstituted C₆ aryl group (phenyl group), d is 2, and R⁴s are bonded to each other to form a ring to form an aromatic or hetero ring,
6)i and j are independently 0 or 1, however i + j is 1 or more, and when i or j is 0, it means a direct bond,
7) X¹ and X² are independently NR', O, S, or CR'R",
   R' and R" are independently hydrogen; a C₆-C₆₀ aryl group; a fluorenyl group; a C₃-C₆₀ heterocyclic group: or a C₁-C₅₀ alkyl group; R' and R" may be bonded to each other to form a spiro,
8) n is an integer of 1 or 2, when n is 2, two Ar² are the same or different, and two Ar³ are each the same or different, and
   wherein any aryl group, fluorenyl group, arylene group, heterocyclic group, fused ring group, alkyl group, alkenyl group, alkoxy group and aryloxy group may be substituted with one or more substituents selected from the group consisting of deuterium; halogen; C₁-C₂₀ alkyl group; C₂-C₂₀ alkenyl group; C₂-C₂₀ alkynyl group; C₆-C₂₀ aryl group; C₆-C₂₀ aryl group substituted with deuterium; a fluorenyl group; C₂-C₂₀ heterocyclic group; and also, these substituents may combine each other and form a ring, wherein the term 'ring' means C₃-C₆₀ aliphatic ring or C₆-C₆₀ aromatic ring or a C₂-C₆₀ heterocyclic ring or a fused ring formed by the combination of them, and includes a saturated or unsaturated ring.

The present invention also provides a compound represented by the Formula 2 In a specific aspect of the invention, the first host compound represented by Formula 1 includes a compound represented by Formula 3 or Formula 4 below 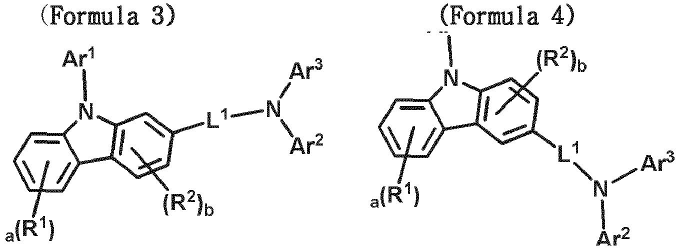 wherein R¹, R², L¹, Ar¹ , Ar², Ar³, a and b are the same as defined above. In the Formula 1 of the present invention, L¹ and L² are selected from the group consisting of the following Formulas (A-1) to (A-12), 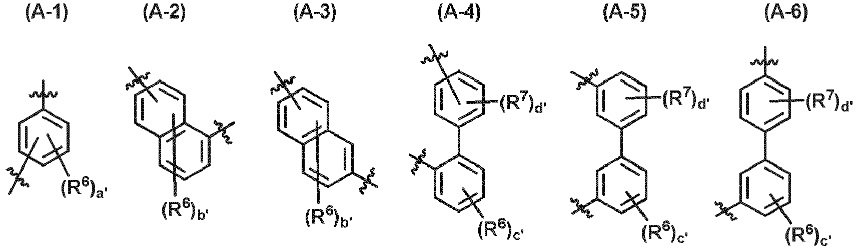 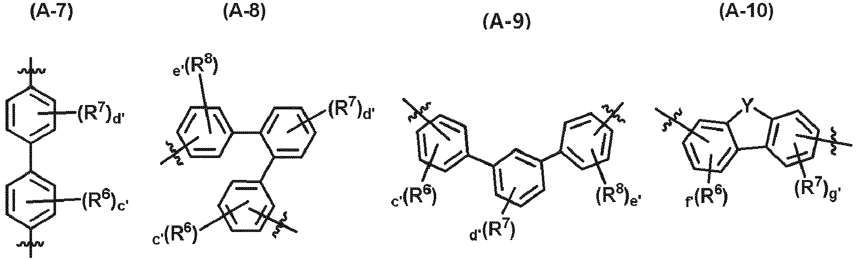 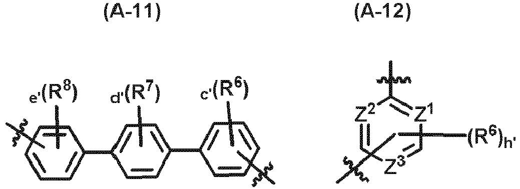 wherein
1) a', c', d' and e' are integer of 0 to 4, and b' is an integer of 0 to 5, and f' and g' are integer of 0 to 3, and h' is an integer of 0 to 1,
2) R⁶, R⁷ and R⁸ are the same or different, and are each independently selected from the group consisting of deuterium; halogen; a C₆-C₆₀ aryl group;
   a fluorenyl group; a C₂-C₆₀ heterocyclic group including at least one heteroatom of 0, N, S, Si or P; a fused ring group of a C₃-C₆₀ aliphatic ring and a C₆-C₆₀ aromatic ring, a C₁-C₅₀ alkyl group; a C₂-C₂₀ alkenyl group; a C₂-C₂₀ alkynyl group; a C₁-C₃₀ alkoxyl group; a C₆-C₃₀ aryl oxy group;
   or when f 'and g' are two or more, they are the same as or different from each other, and plurality of R⁶ or plurality of R⁷ or plurality of R⁸ may be bonded to each other to form an aromatic or a heteroaromatic ring,
   two adjacent R⁶ and R⁷, or R⁷ and R⁸ may be bonded to form an aromatic or heteroaromatic ring,
3) Y is NR', 0, S or CR'R"
4) R' and R" are the same as defined above,
5) Z¹, Z² and Z³ are independently of each other CR 'or N and at least one is N.

Also, the present invention provides a compound wherein the first host compound represented by the Formula 1 is represented by any one of the following Formulas 5 to 8, 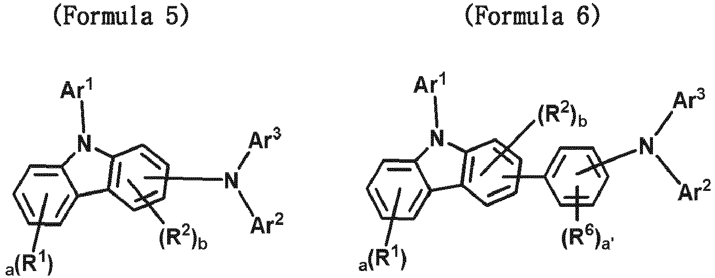 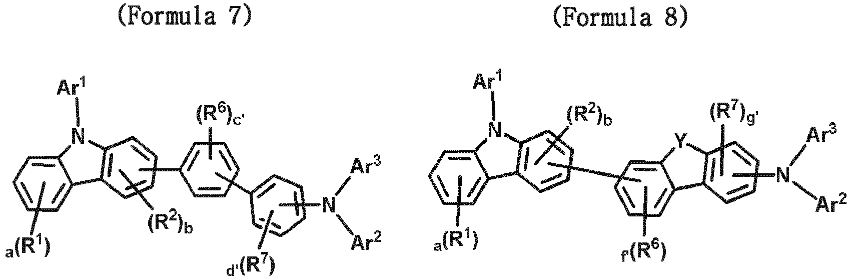 wherein
1) R¹, R², Ar\. Ar², Ar³, a, b, R' and R" are the same as defined above,
2) R⁶ and R⁷ are the same or different, and are each independently selected from the group consisting of deuterium; halogen; a C₆-C₆₀ aryl group; a fluorenyl group; a C₂-C₆₀ heterocyclic group including at least one heteroatom of O, N, S, Si or P; a fused ring group of a C₃-C₆₀ aliphatic ring and a C₆-C₆₀ aromatic ring, a C₁-C₅₀ alkyl group; a C₂-C₂₀ alkenyl group; a C₂-C₂₀ alkynyl group; a C₁-C₃₀ alkoxyl group; a C₆-C₃₀ aryloxy group; or in case f' and g' are two or more, each as plurality are the same as or different from each other, and a plurality of R⁶ or a plurality of R⁷ or a plurality of R⁸ or adjacent R⁶ and R⁷, or adjacent R⁷ and R⁸ may be bonded to each other to form an aromatic or a heteroaromatic ring,
3) a', c' and d' are integer of 0 to 4, and f' and g' are integer of 0 to 3,
4) Y is NR', O, S or CR'R".

The present invention also provides an organic electric element wherein the first host compound represented by Formula 1 is represented by any one of Formulas 9 to 20 below, 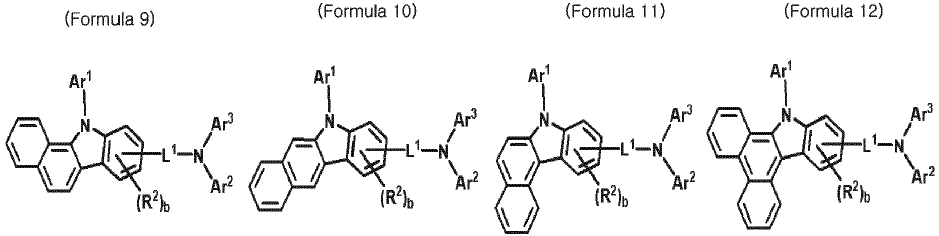 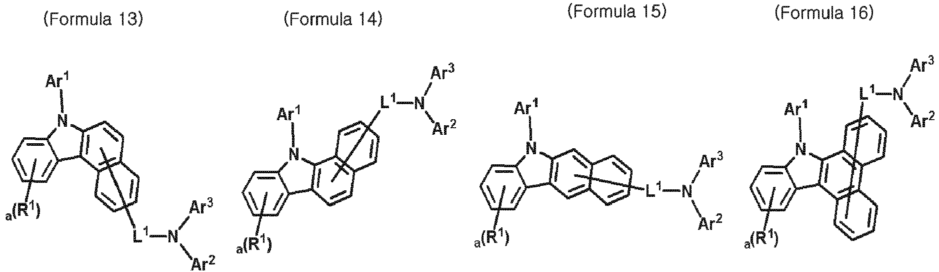 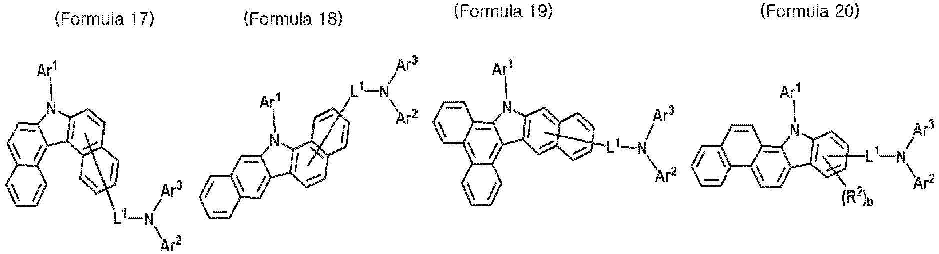 wherein R¹, R², L¹, Ar¹ , Ar², Ar³, a and b are the same as defined above. Also, the first host compound represented by Formula 1 includes a compound represented by Formula 21 to Formula 22 below, 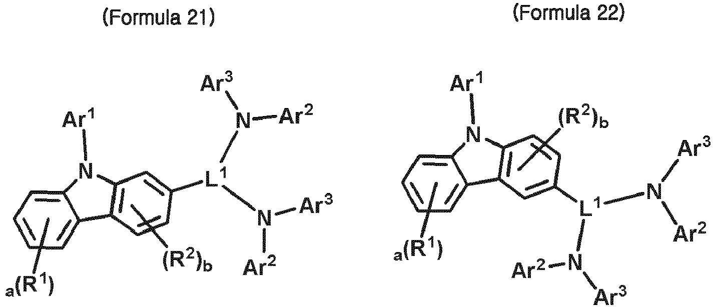 wherein R¹, R², L1, Ar¹, Ar², Ar³, a and b are the same as defined above.

The present invention also provides an organic electric element wherein n is 1 in Formula 1 and n is 2 in Formula 2.

The present invention provides an organic electric element wherein the first host compound represented by Formula 1 comprises a compound represented by Formula 23 below, 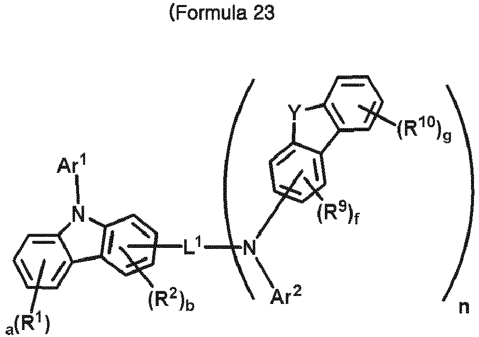 wherein
1) R¹, R², L¹, Ar¹, Ar², a. b and n are the same as defined above,
2) f is an integer of 0 to 3, and g is an integer of 0 to 4,
3) R⁹ and R¹⁰ are the same or different, and are each independently selected from the group consisting of deuterium; halogen; a C₆-C₆₀ aryl group; a fluorenyl group; a C₂-C₆₀ heterocyclic group including at least one heteroatom of O, N, S, Si or P; a fused ring group of a C₃-C₆₀ aliphatic ring and a C₆-C₆₀ aromatic ring, a C₁-C₅₀ alkyl group; a C₂-C₂₀ alkenyl group; a C₂-C₂₀ alkynyl group; a C₁-C₃₀ alkoxyl group; a C₆-C₃₀ aryloxy group; or in case f and g are two or more, each as plurality are the same as or different from each other, and a plurality of R⁹ or a plurality of R¹⁰ or adjacent R⁹ and R¹⁰ may be bonded to each other to form an aromatic or a heteroaromatic ring,
4) Y is NR', O, S or CR'R",
5) R' and R" are the same as defined above.

The present invention provides an organic electric element wherein the second host compound represented by Formula 2 comprises a compound represented by Formula 24 or Formula 25 below, 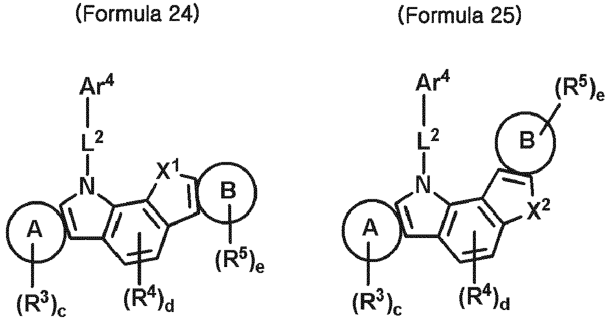 wherein R³, R⁴, R⁵, Ar⁴, L², c, d, e, A, B, X¹ and X² are the same as defined above.

The present invention also provides an organic electric element comprising a compound wherein A and B in Formula 2 are selected from the group consisting of the following Formulas (B-1) to (B-7), 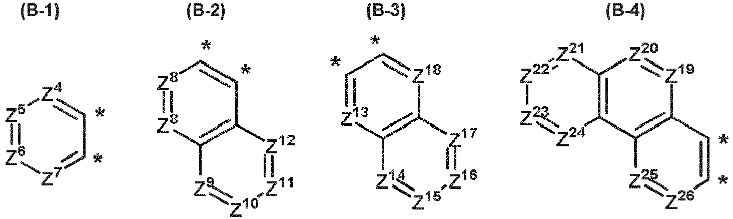 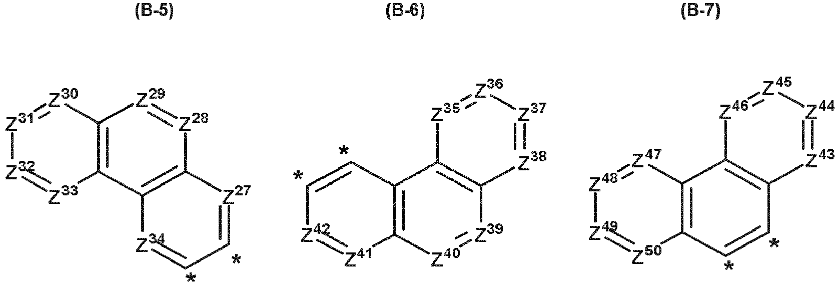 wherein
1) Z⁴ to Z⁵⁰ are CR' or N,
2) R' is the same as defined above,
3) * indicates the position to be condensed.

As a specific example of the present invention, R⁴ in the Formula 2 includes a compound necessarily forming an aromatic ring or a heteroaromatic ring.

As another example, the present invention provides a compound wherein the second host compound represented by Formula 2 includes a compound represented by any one of the following Formulas 26 to 45, 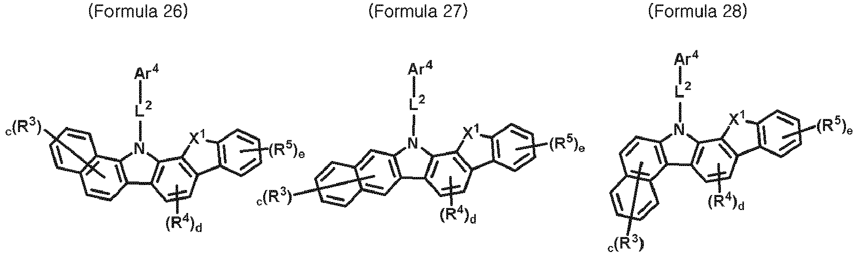 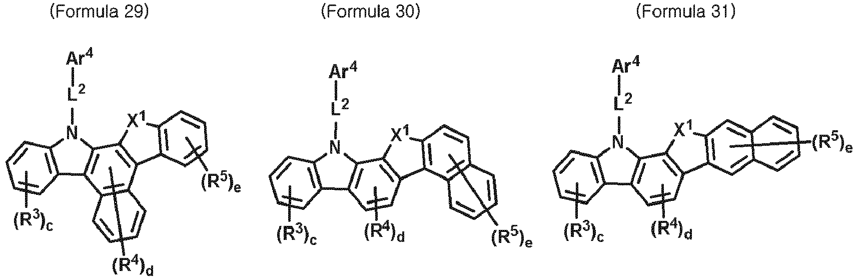 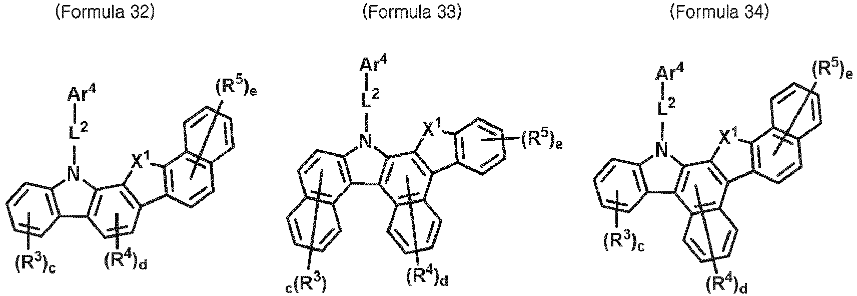 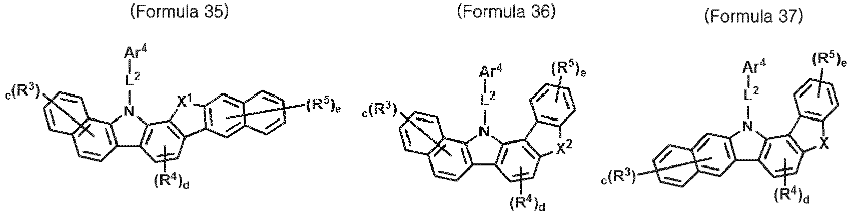 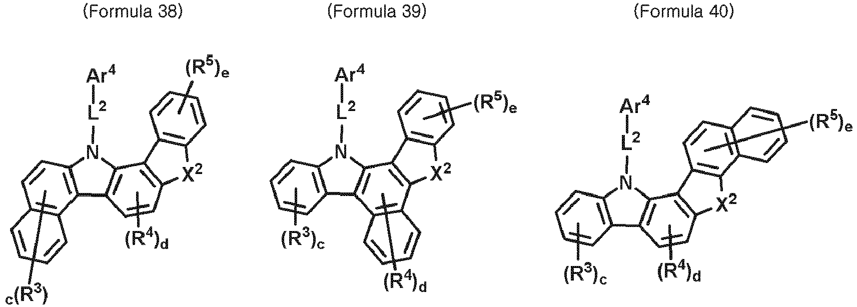 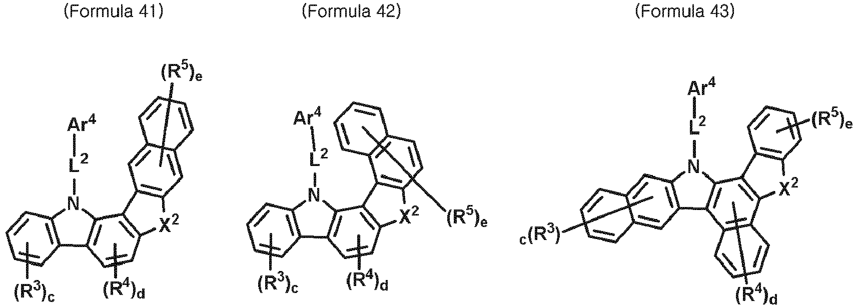 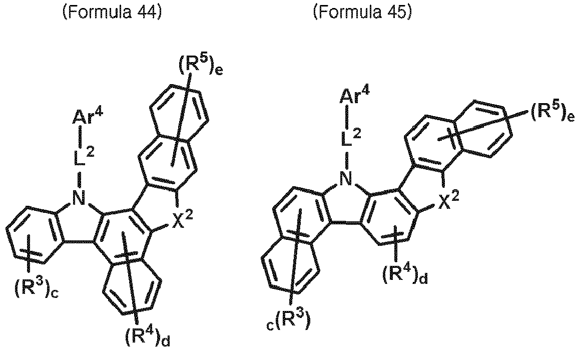 wherein
Ar⁴, X¹. X², R³, R⁴, R⁵, L², c, d, and e are the same as defined above.

In the present invention, the second host compound represented by Formula 2 comprises any one of compounds represented by Formulas 46 to 53 below, 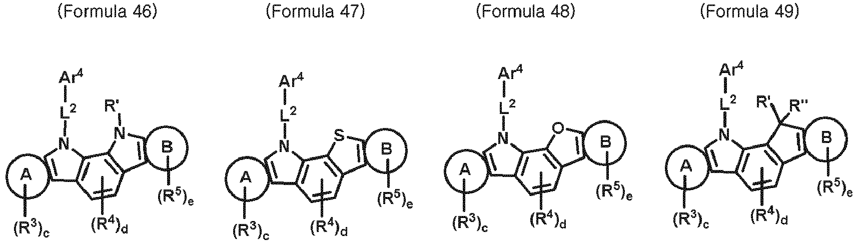 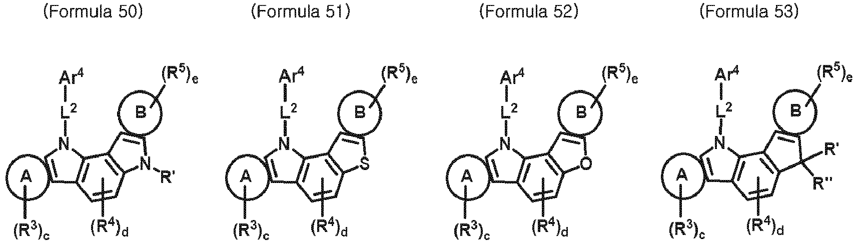 wherein
R³, R⁴, R⁵, Ar⁴, L², c, d, e, A, B, R' and R" are the same as defined above. As a specific example of the present invention, the first host compound represented by Formula 1 is any one of the following Compounds 1-1 to 1-68 and Compounds 2-1 to 2-68.

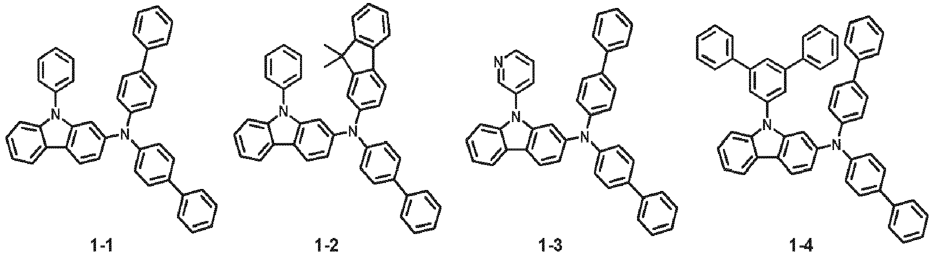

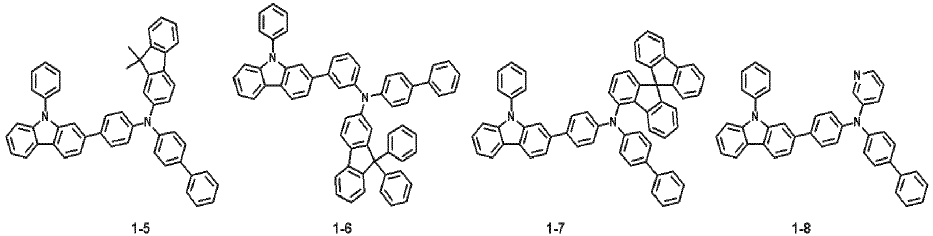

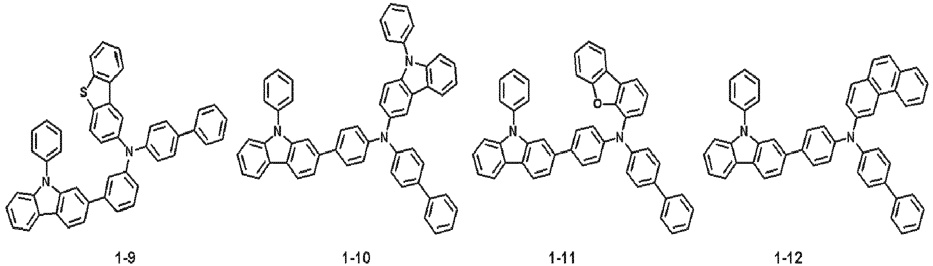

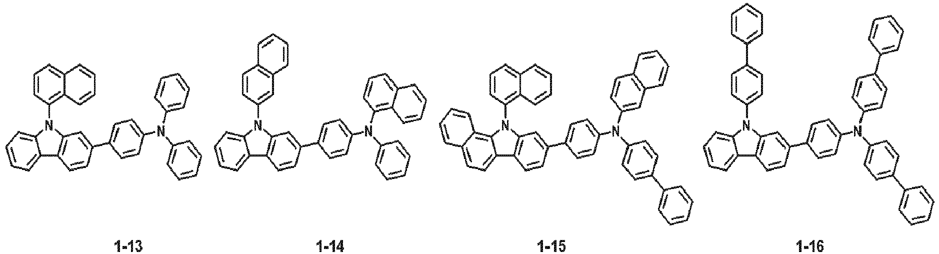

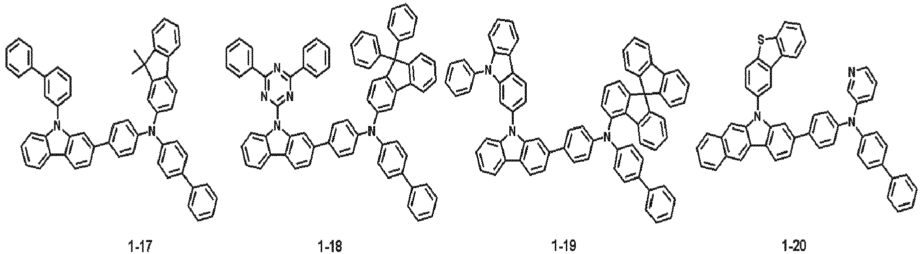

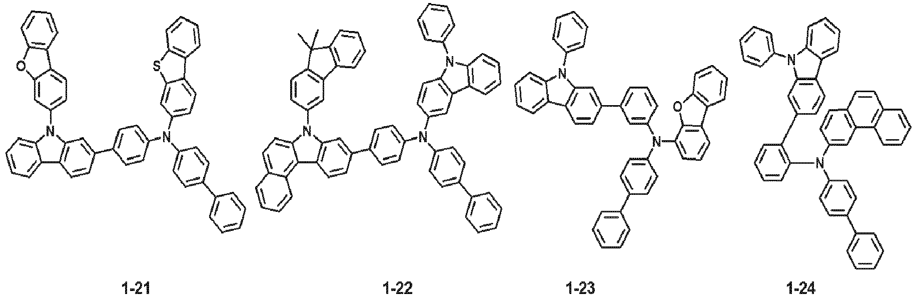

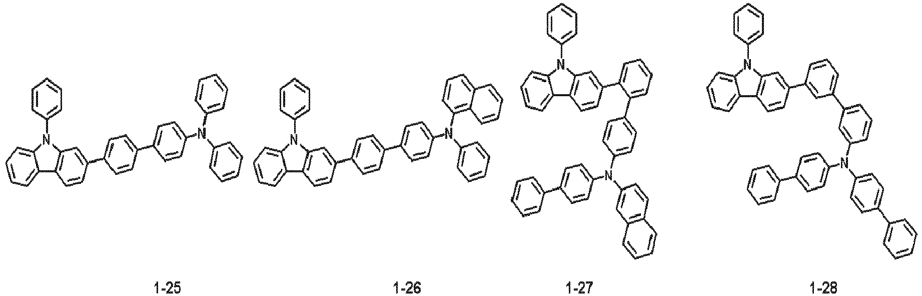

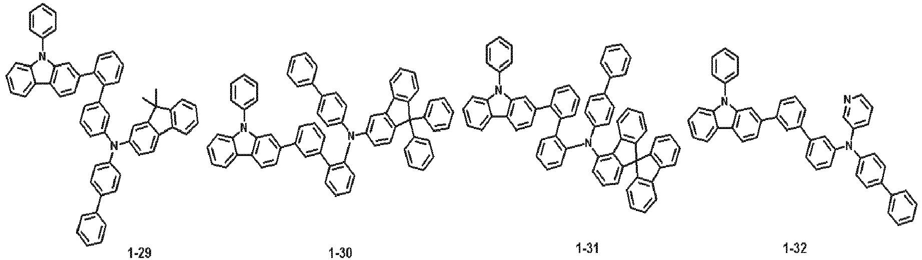

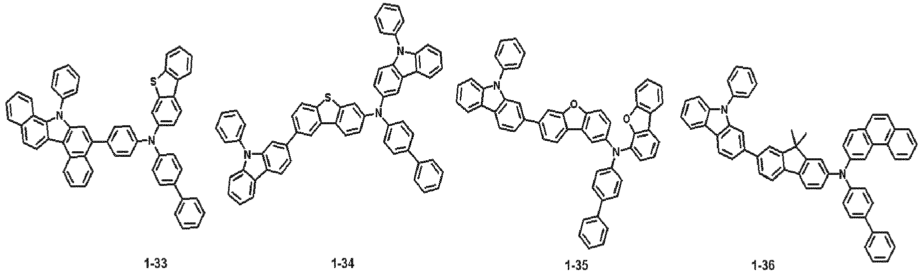

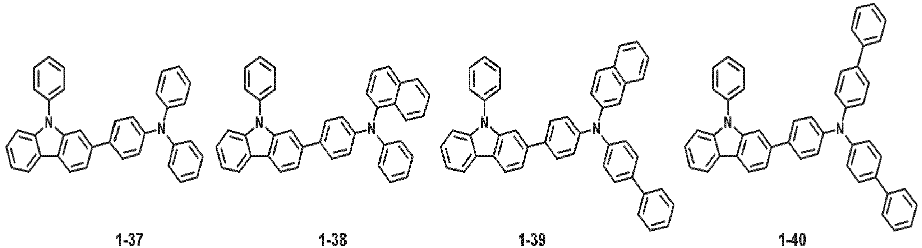

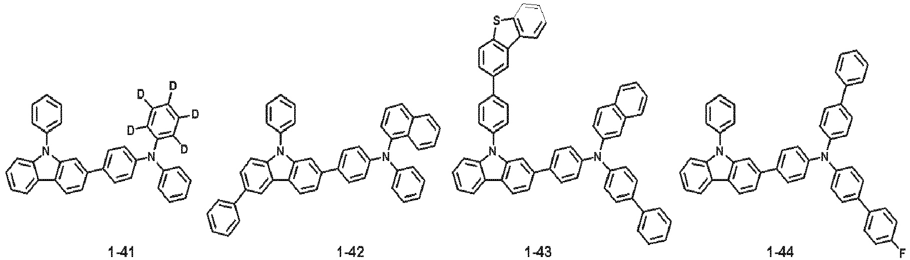

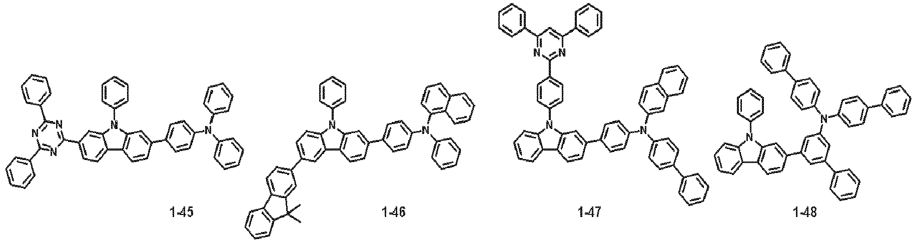

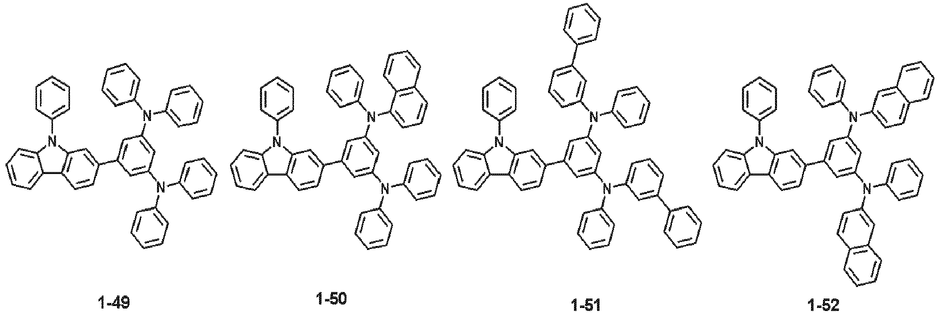

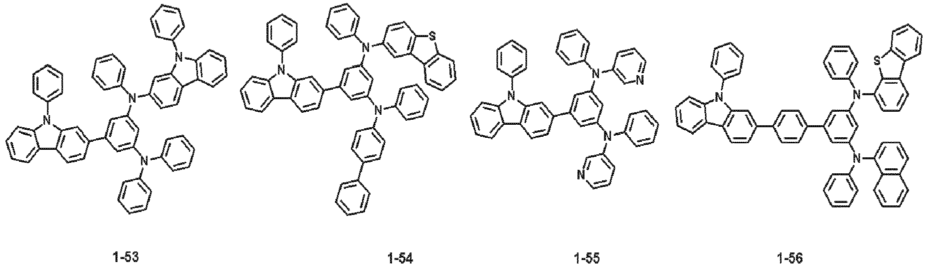

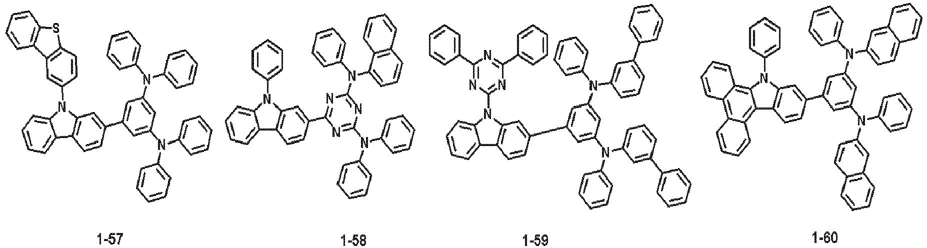

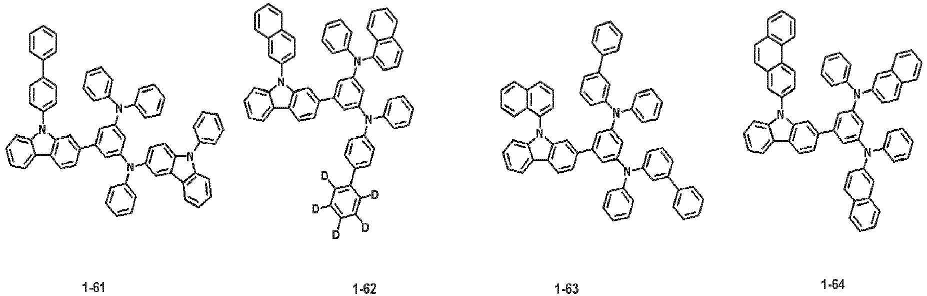

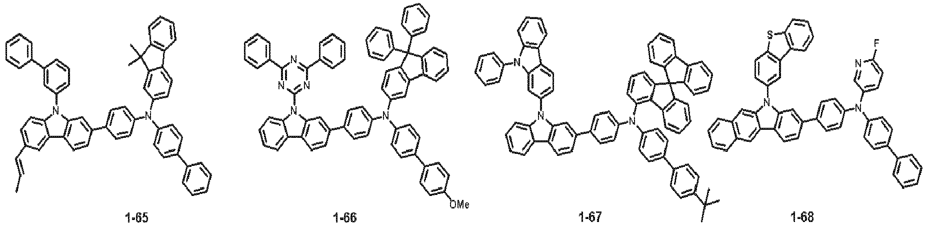

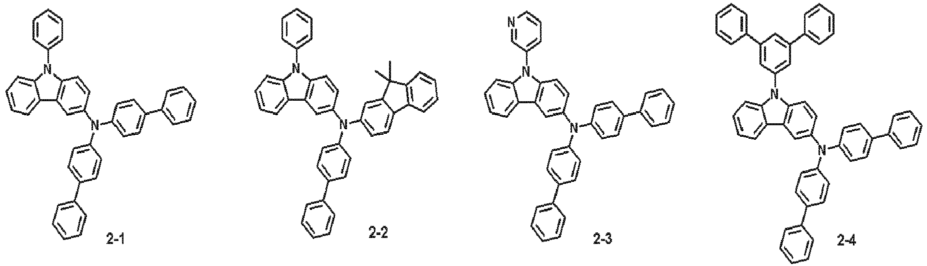

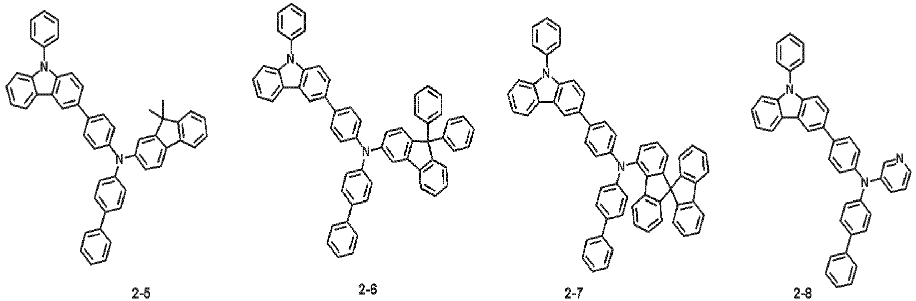

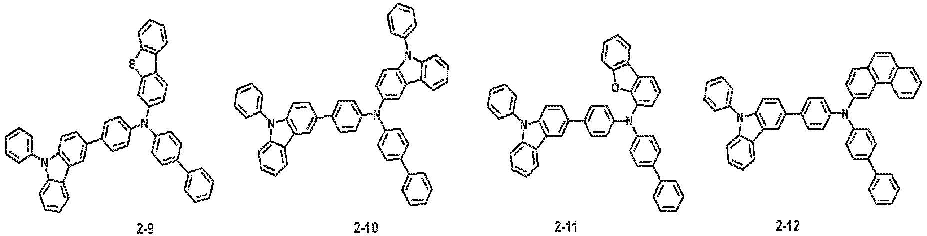

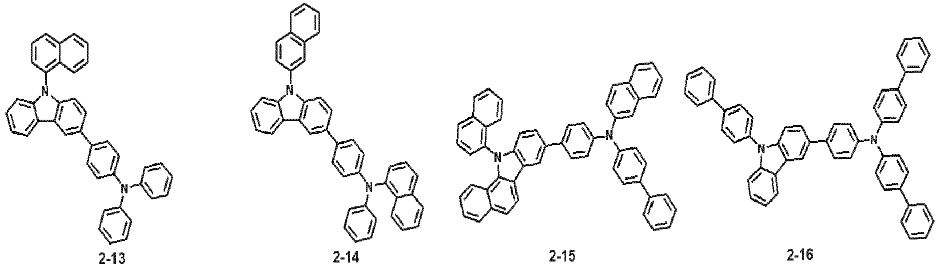

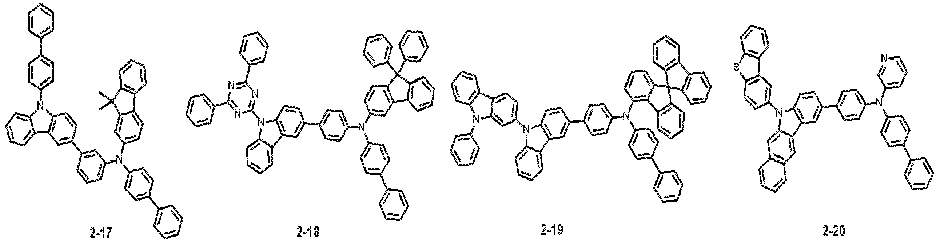

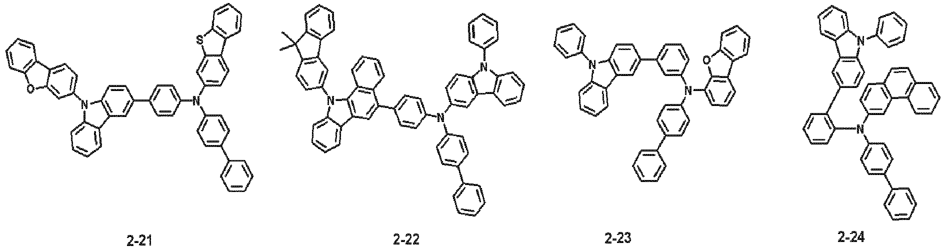

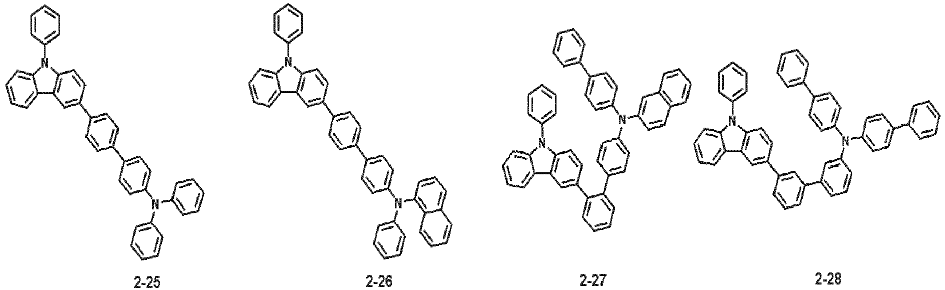

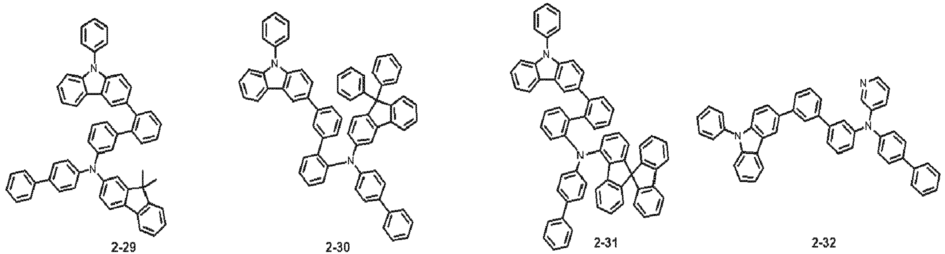

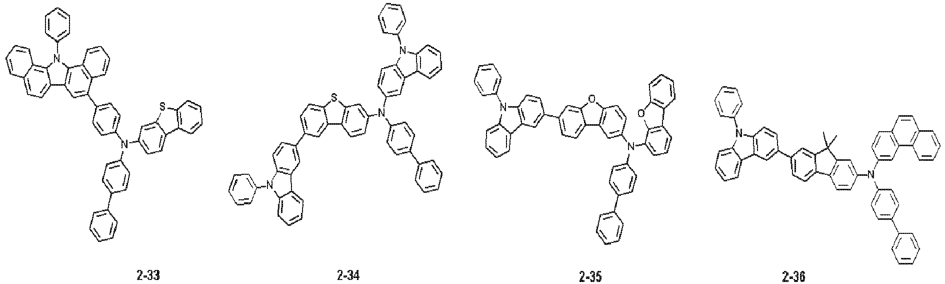

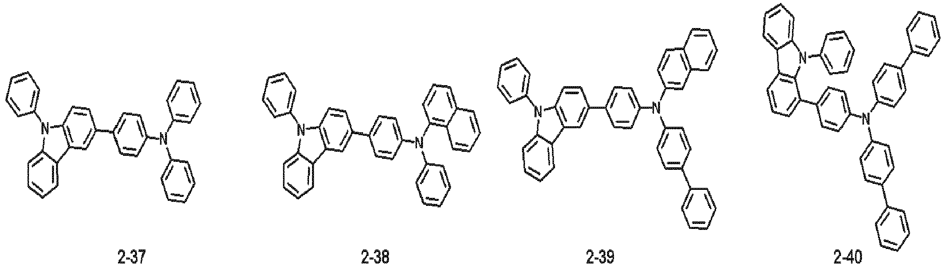

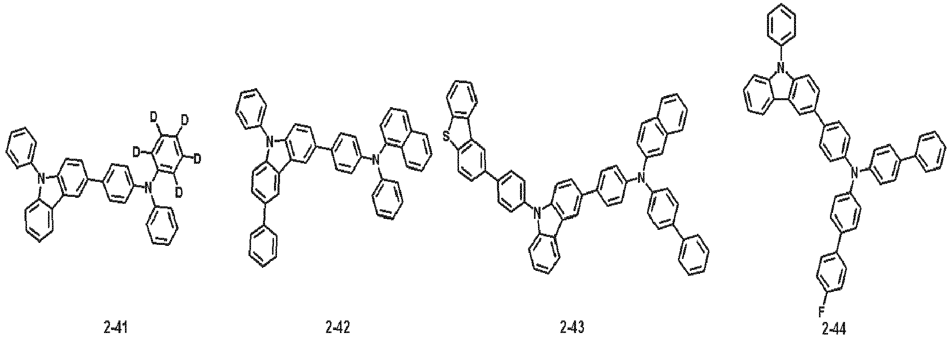

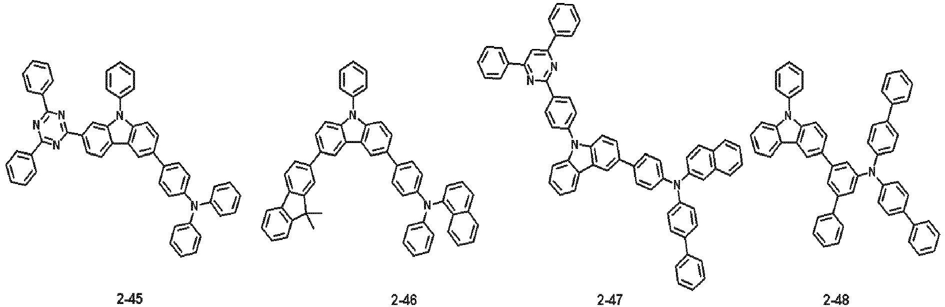

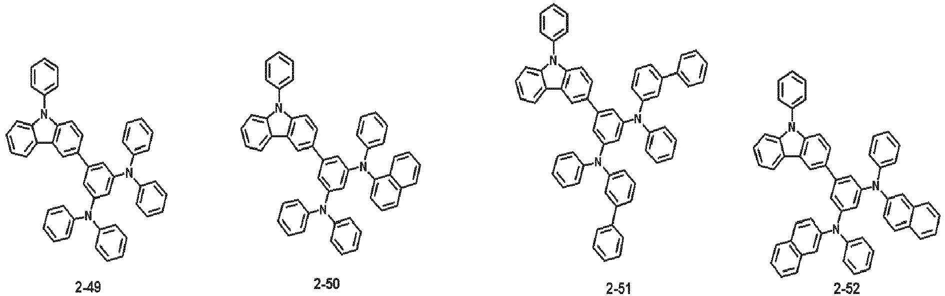

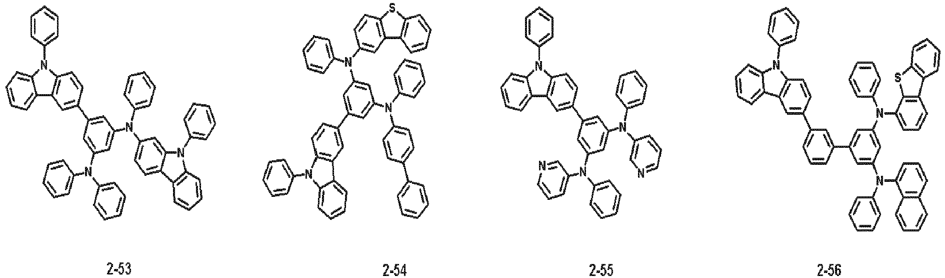

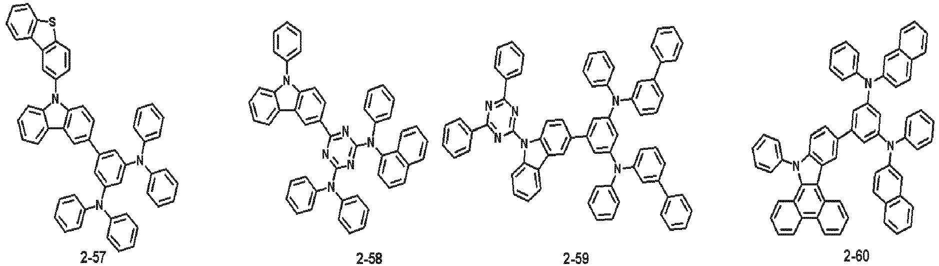

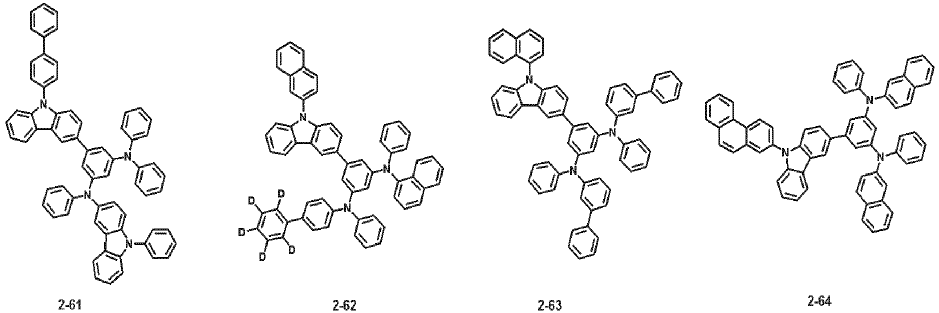

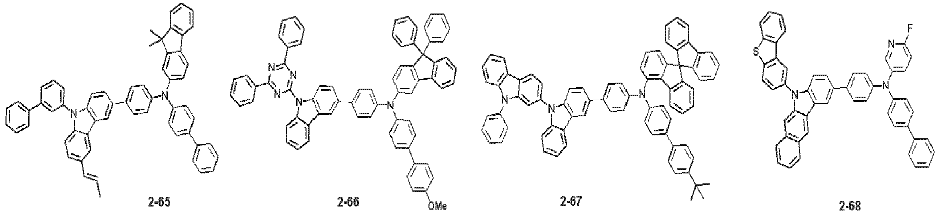

Further, in the present invention, the second host compound represented by Formula 2 includes any one of the following Compounds 3-1 to 3-88.

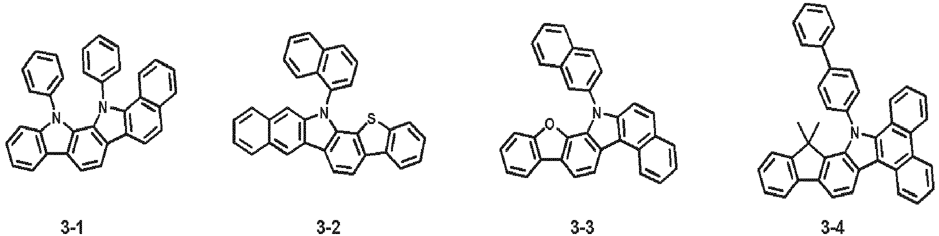

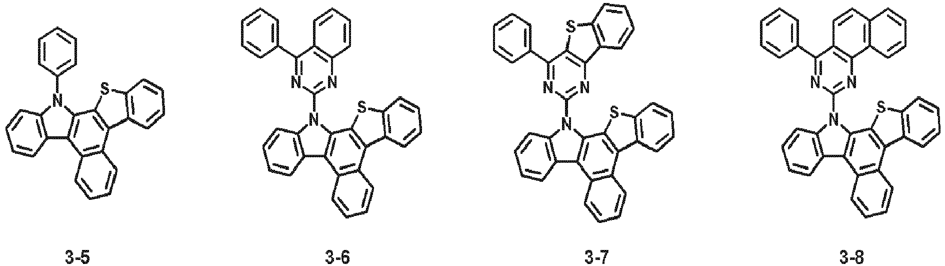

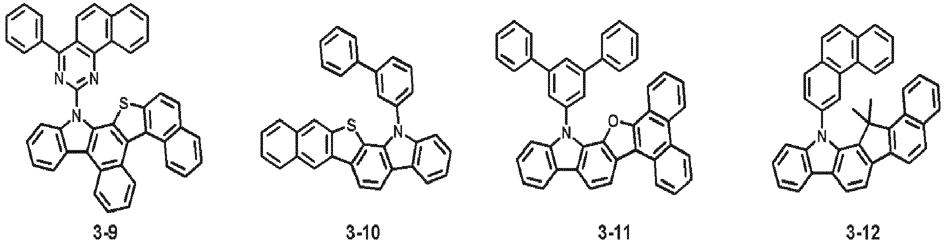

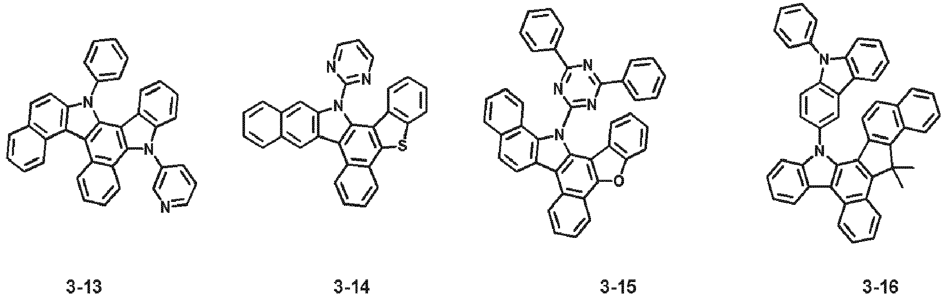

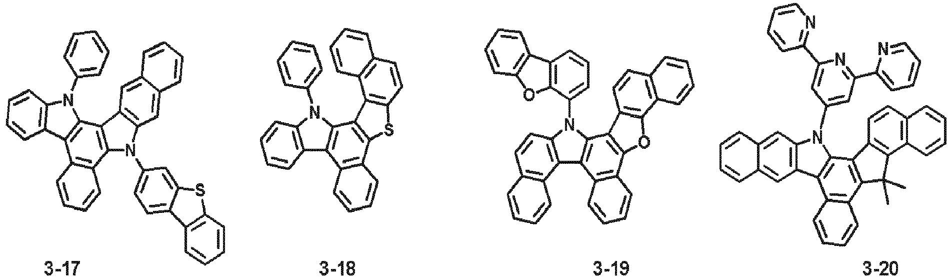

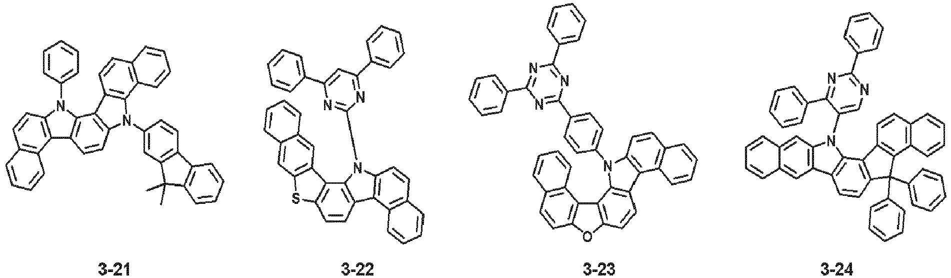

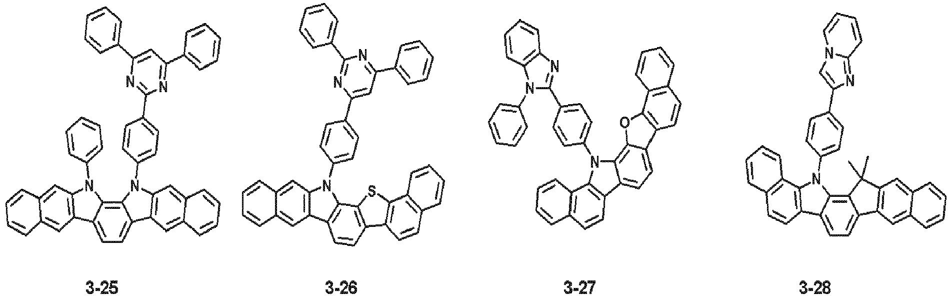

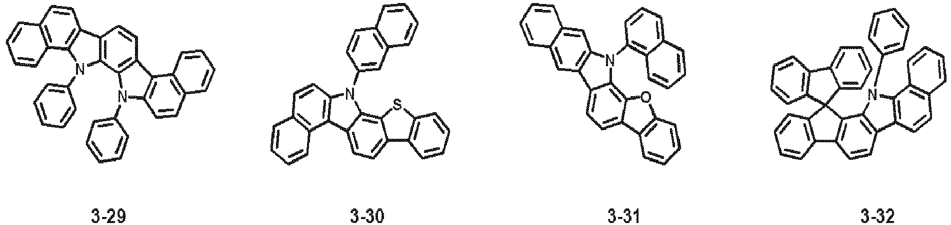

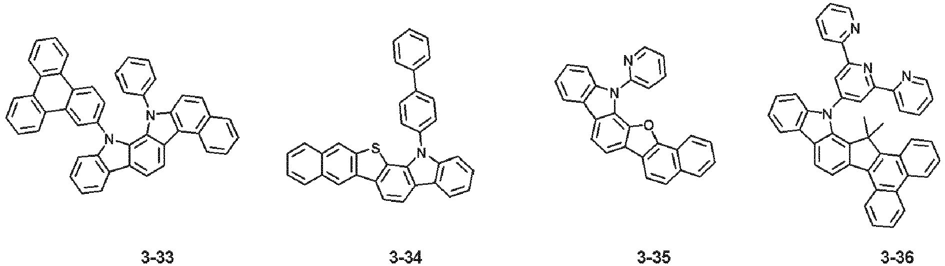

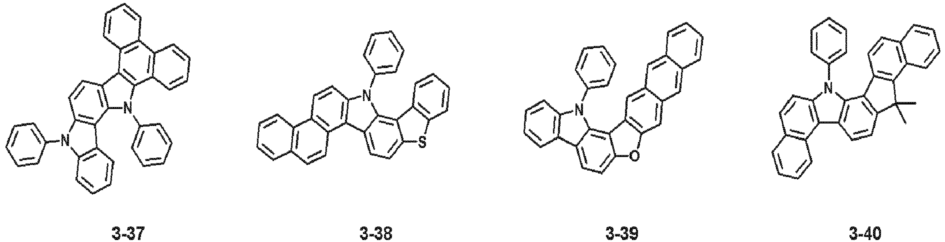

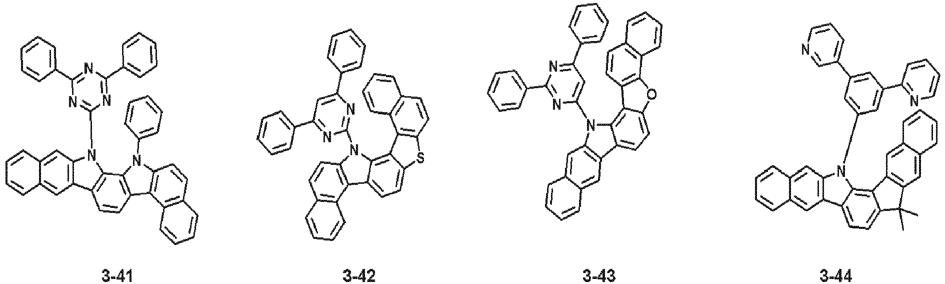

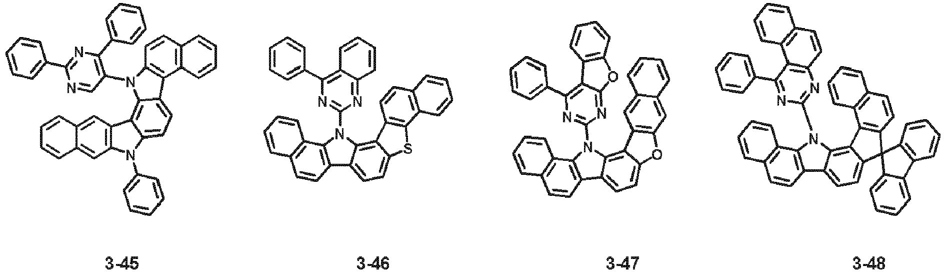

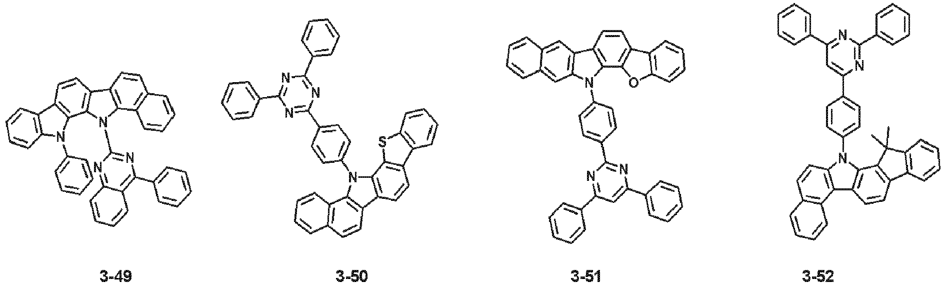

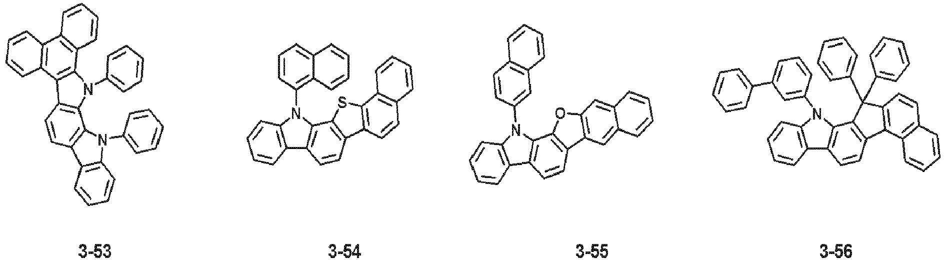

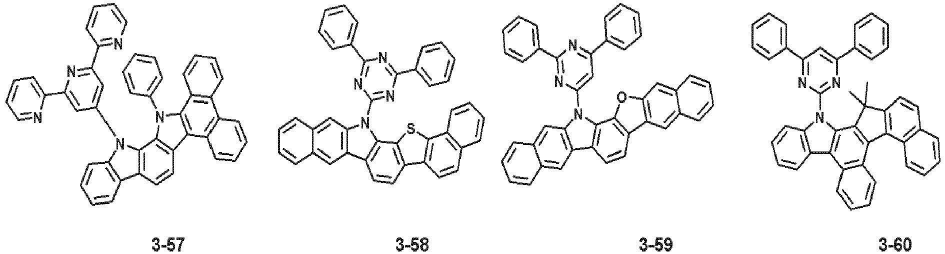

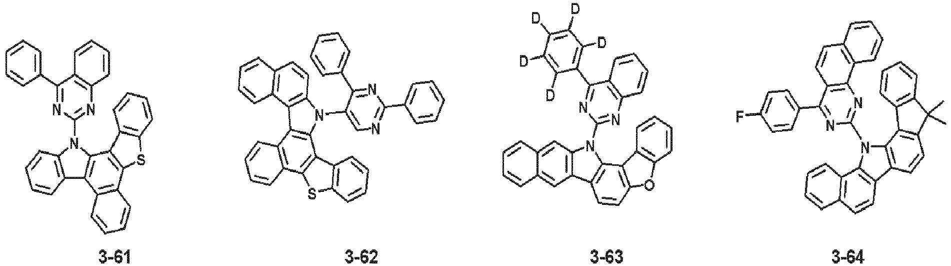

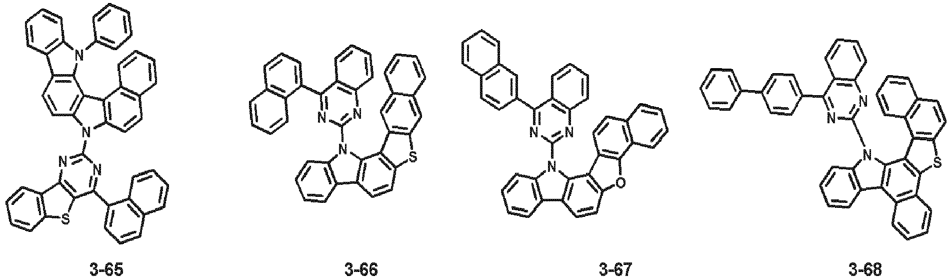

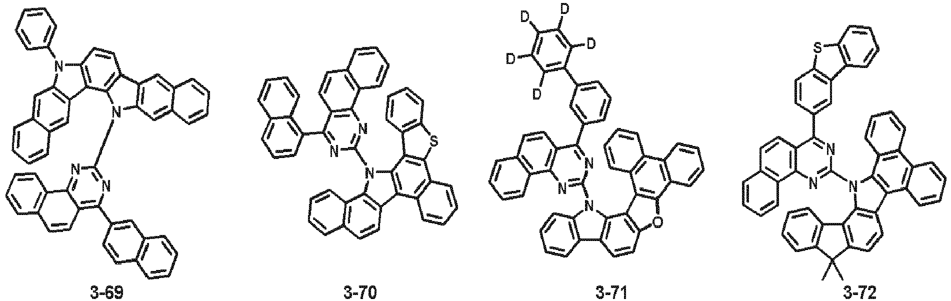

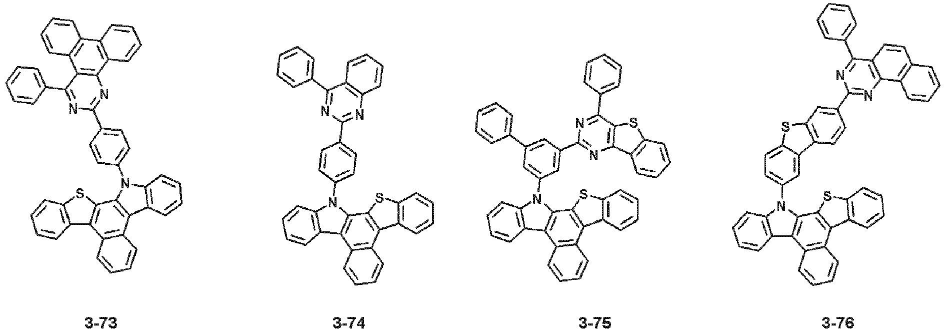

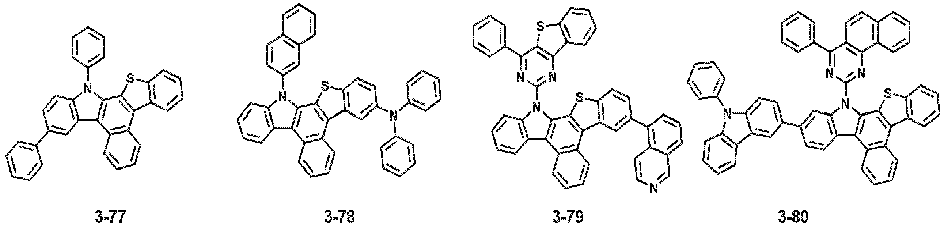

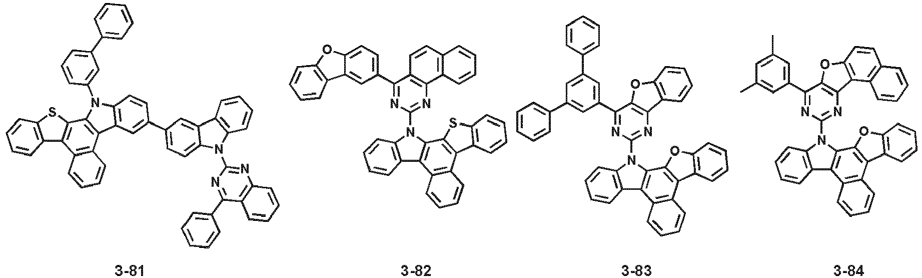

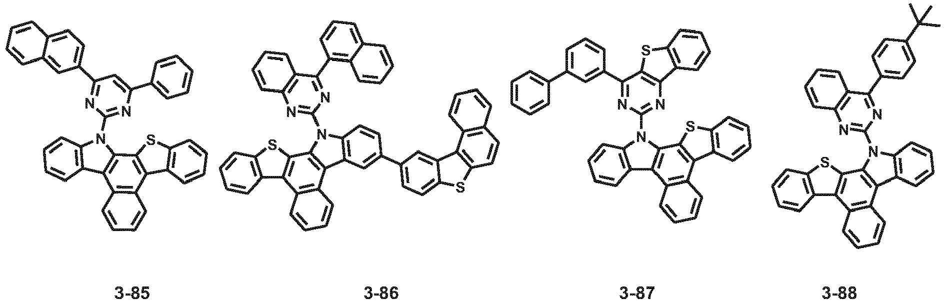

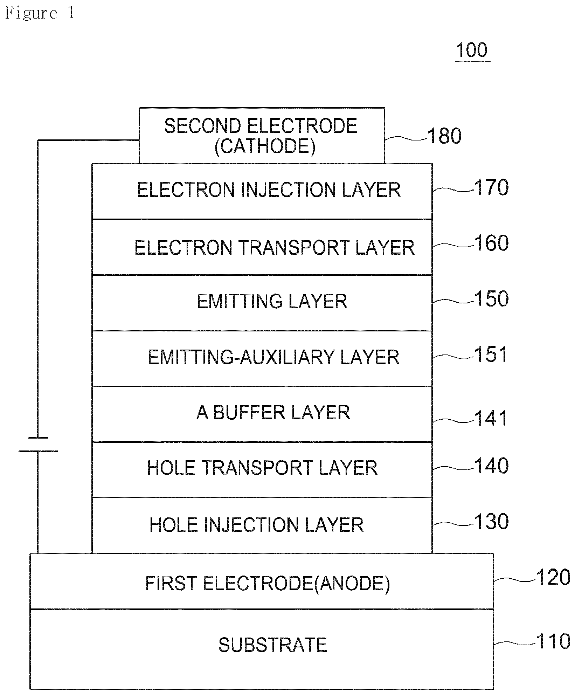

Referring to FIG. 1, the organic electric element(100) according to the present invention includes a first electrode(120) formed on a substrate(110), a second electrode(180), and an organic material layer including the compound represented by Formula 1 between the first electrode(120) and the second electrode(180). Here, the first electrode(120) may be an anode (positive electrode), and the second electrode(180) may be a cathode (negative electrode). In the case of an inverted organic electric element, the first electrode may be a cathode, and the second electrode may be an anode.

The organic material layer may include a hole injection layer(130), a hole transport layer(140), an emitting layer(150), an electron transport layer(160), and an electron injection layer(170) formed in sequence on the first electrode(120). Here, the remaining layers except the emitting layer(150) may not be formed. The organic material layer may further include a hole blocking layer, an electron blocking layer, an emitting-auxiliary layer(151), an electron transport auxiliary layer, a buffer layer(141), etc., and the electron transport layer(160) and the like may serve as a hole blocking layer.

Although not shown, the organic electric element according to the present invention may further include a protective layer formed on at least one side of the first and second electrodes, which is a side opposite to the organic material layer.

Otherwise, even if the same core is used, the band gap, the electrical characteristics, the interface characteristics, and the like may vary depending on which substituent is bonded at which position, therefore the choice of core and the combination of sub-substituents associated therewith is also very important, and in particular, when the optimal combination of energy levels and T1 values and unique properties of materials(mobility, interfacial characteristics, etc.) of each organic material layer is achieved, a long life span and high efficiency can be achieved at the same time.

The organic electroluminescent device according to an embodiment of the present invention may be manufactured using a PVD (physical vapor deposition) method. For example, a metal or a metal oxide having conductivity or an alloy thereof is deposited on a substrate to form a cathode, and the organic material layer including the hole injection layer(130), the hole transport layer(140), the emitting layer(150), the electron transport layer(160), and the electron injection layer(170) is formed thereon, and then depositing a material usable as a cathode thereon can manufacture an organic electroluminescent device according to an embodiment of the present invention.

In addition, an emission auxiliary layer(151) may be further formed between the hole transport layer(140) and the emitting layer(150), and an electron transport auxiliary layer may be further formed between the emitting layer(150) and the electron transport layer (160).

The present invention may further include a light efficiency enhancing layer formed on at least one of the opposite side to the organic material layer among one side of the first electrode, or one of the opposite side to the organic material layer among one side of the second electrode.

Also, the present invention provides the organic electric element wherein the organic material layer is formed by one of a spin coating process, a nozzle printing process, an inkjet printing process, a slot coating process, a dip coating process or a roll-to-roll process, and since the organic material layer according to the present invention can be formed by various methods, the scope of the present invention is not limited by the method of forming the organic material layer.

As another specific example, the present invention provides an organic electric element wherein the emitting layer in the organic material layer is a phosphorescent light emitting layer.

The compounds represented by Formula 1 and by Formula 2 are mixed in a ratio of any one of 1: 9 to 9: 1 to be included in the emitting layer of the organic material layer.

The compounds represented by Formula 1 and by Formula 2 are mixed in a ratio of any one of 1: 9 to 5: 5 to be included in the emitting layer of the organic material layer.

More preferably, the mixing ratio of the compound represented by the Formula 1 and by the Formula 2 is 2: 8 or 3: 7 to be included in the emitting layer of the organic material layer.

The organic electric element according to an embodiment of the present invention may be a front emission type, a back emission type, or a both-sided emission type, depending on the material used.

WOLED (White Organic Light Emitting Device) has advantages of high resolution realization and excellent fairness, and can be manufactured using conventional LCD color filter technology. Various structures for a white organic light emitting device mainly used as a backlight device have been proposed and patented. Representatively, there are side-by-side arrangement of the radiation part of the red (R), green (G) and blue (B), a stacking method in which R, G, and B emitting layers are laminated on top and bottom, electroluminescence by the blue (B) organic emitting layer and, by using the light from this, a color conversion material (CCM) method using a photoluminescence of an inorganic phosphor, etc., and the present invention may be applied to such WOLED.

The present invention also provides an electronic device comprising a display device including the organic electric element; and a control unit for driving the display device.

According to another aspect, the present invention provides an electronic device characterized in that the organic electric element is at least one of an OLED, an organic solar cell, an organic photo conductor, an organic transistor and an element for monochromic or white illumination. Here, the electronic device may be a wired/wireless communication terminal which is currently used or will be used in the future, and covers all kinds of electronic devices including a mobile communication terminal such as a cellular phone, a personal digital assistant (PDA), an electronic dictionary, a point-to-multipoint (PMP), a remote controller, a navigation unit, a game player, various kinds of TVs, and various kinds of computers.

Hereinafter, Synthesis Examples of the compound represented by Formula 1 according to the present invention and preparation examples of the organic electric element will be described in detail by way of example, but are not limited to the following examples of the invention.

### Synthesis Example 1

The final products 1 represented by Formula 1 of the present invention can be synthesized by reaction between Sub 1 and Sub 2 as illustrated in the following Reaction Scheme 1.

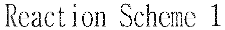

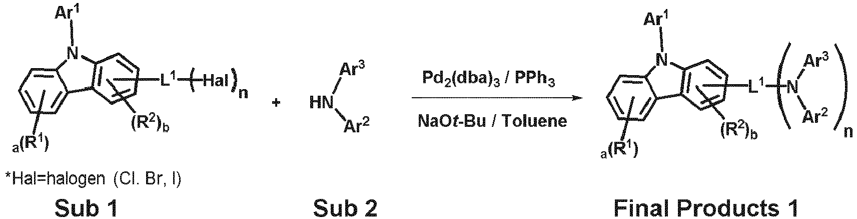

### Synthesis Examples of Sub 1

When L¹ in Sub 1 of Reaction Scheme 1 is not a single bond, it can be synthesized by the reaction path of the following Reaction Scheme 2, but is not limited thereto.

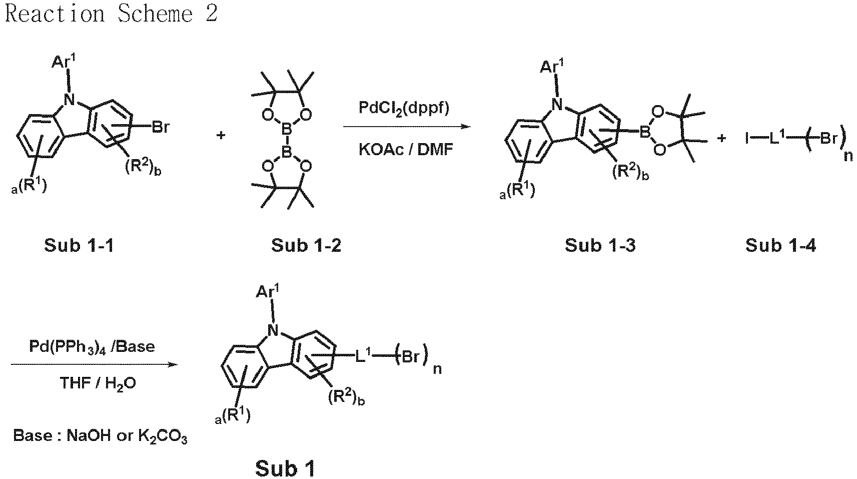

### Synthesis Examples of Sub 1-3(1)

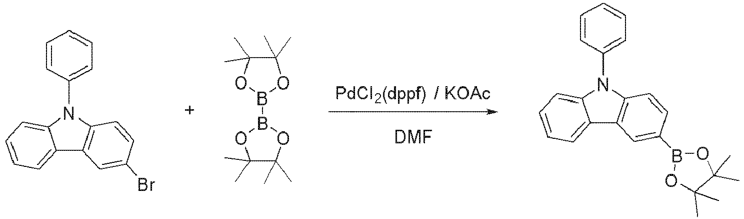

3-Bromo-9-phenyl-9H-carbazole (45.1 g, 140 mmol) was dissolved in DMF 980mL, bispinacolborate (39.1 g, 154 mmol), PdCl₂(dppf)catalyst(3.43 g, 4.2 mmol), KOAc (41.3 g, 420 mmol) were added in order and stirred for 24 hours and then after synthesizing the borate compound, the obtained compound was separated over a silicagel column and recrystallization to give 35.2g of the borate compound (yield: 68%).

### Synthesis Examples of Sub 1-3(2)

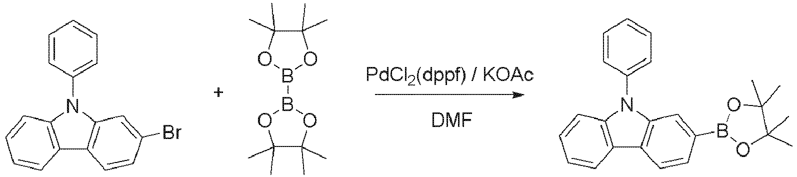

2-Bromo-9-phenyl-9H-carbazole (76.78 g, 238.3 mmol), bis(pinacolato)diboron (66.57 g, 262.1 mmol), Pd(dppf)Cl₂ (5.84 g, 7.1 mmol), KOAc (70.16 g, 714.9 mmol) were carried out in the same manner as in Sub 1-3(1) to obtain 73.92 g (yield: 84%) of the product Sub 1-3(2).

### Synthesis Examples of Sub 1(10)

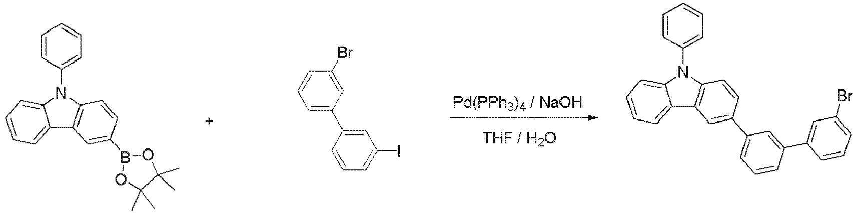

9-Phenyl-3-(4,4,5,5-tetramethyl-1,3,2-dioxaborolan-2-yl)-9H-carbazole (29.5 g, 80 mmol) were dissolved in THF, 3-bromo-3'-iodo-1,1'-biphenyl (30.16 g, 84 mmol), Pd(PPh₃)₄ (2.8 g, 2.4mmol), NaOH (9.6 g, 240mmol), 180 mL of water were added and were refluxed with stirring. After the reaction was completed, the reaction mixture was extracted with ether and water. The organic layer was dried over MgSO₄ and concentrated. The resulting organic material was separated by silicagel column chromatography and recrystallization to obtain 26.56 g of the product (yield: 70%).

### Synthesis Examples of Sub 1(3)

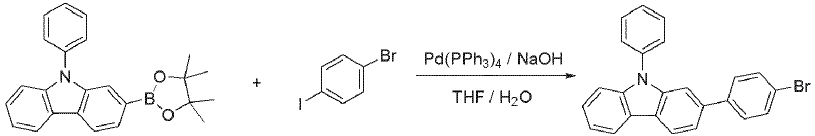

9-phenyl-2-(4,4,5,5-tetramethyl-1,3,2-dioxaborolan-2-yl)-9H-carbazole (29.5 g, 80 mmol), THF 360 mL, 1-bromo-4-iodobenzene (23.8 g, 84 mmol), Pd(PPh₃)₄ (2.8 g, 2.4mmol), NaOH (9.6 g, 240mmol), and 180 mL of water were carried out in the same manner as in Sub 1(10) to obtain 22.9 g of the product Sub 1(3) (yield: 72%).

### Synthesis Examples of Sub 1(5)

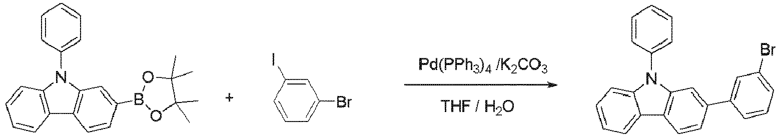

9-Phenyl-2-(4,4,5,5-tetramethyl-1,3,2-dioxaborolan-2-yl)-9H-carbazole (73.92 g, 200.2 mmol) were dissolved in THF 880 mL in a round bottom flask, 1-bromo-2-iodobenzene (85.0 g, 300.3 mmol), Pd(PPh₃)₄ (11.6 g, 10 mmol), K₂CO₃ (83 g, 600.6 mmol), and 440 mL of water were carried out in the same manner as in Sub 1(10) to obtain 55.8 g of the product Sub 1(5) (yield: 70%).

### Synthesis Examples of Sub 1(15)

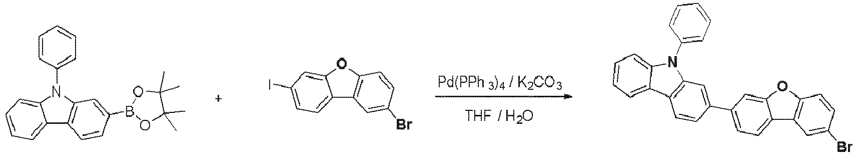

9-Phenyl-2-(4,4,5,5-tetramethyl-1,3,2-dioxaborolan-2-yl)-9H-carbazole (73.92 g, 200.2 mmol) were dissolved in THF 880 mL in a round bottom flask, 2-bromo-7-iododibenzo[b,d]furan (112.0 g, 300.3 mmol), Pd(PPh₃)₄ (11.6 g, 10 mmol), K₂CO₃ (83 g, 600.6 mmol), and 440 mL of water were carried out in the same manner as in Sub 1(10) to obtain 72.4 g of the product Sub 1(15) (yield: 74%).

### Synthesis Examples of Sub 1(22)

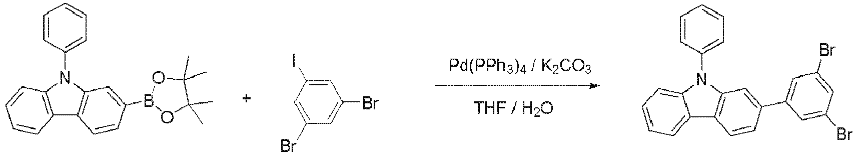

9-Phenyl-2-(4,4,5,5-tetramethyl-1,3,2-dioxaborolan-2-yl)-9H-carbazole (73.92 g, 200.2 mmol) were dissolved in THF 880 mL in a round bottom flask, 1,3-dibromo-5-iodobenzene (108.65 g, 300.3 mmol), Pd(PPh₃)₄ (11.6 g, 10 mmol), K₂CO₃(83g, 600.6mmol), and 440 mL of water were carried out in the same manner as in Sub 1(10) to obtain 69.7 g of the product Sub 1(22) (yield: 73%).

Examples of Sub 1 are as follows, but are not limited thereto.

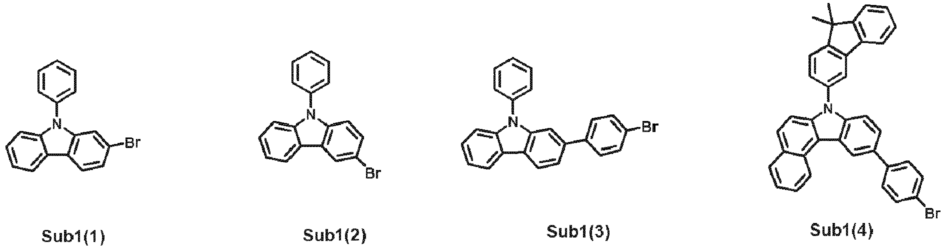

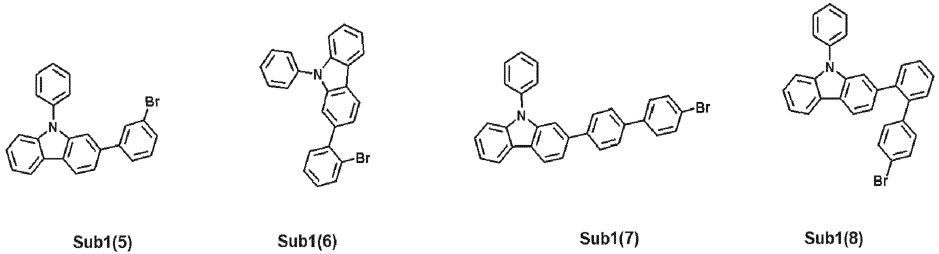

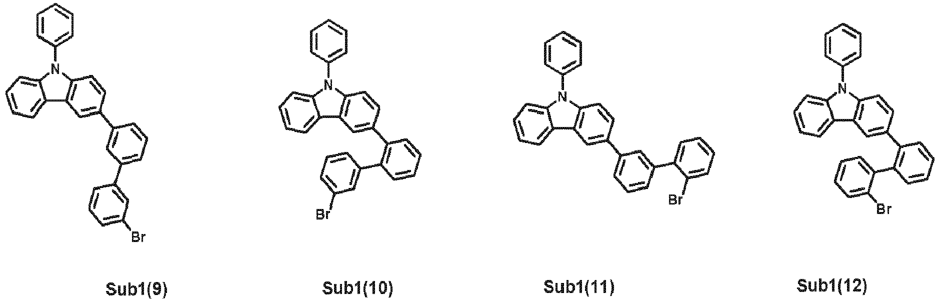

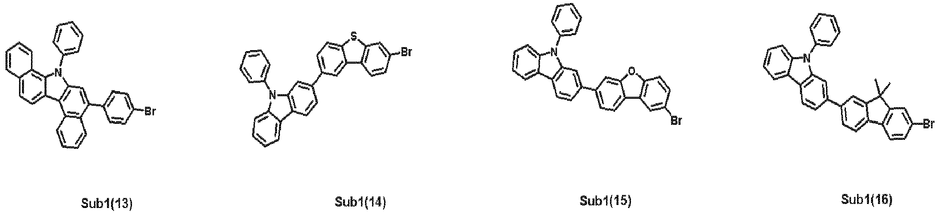

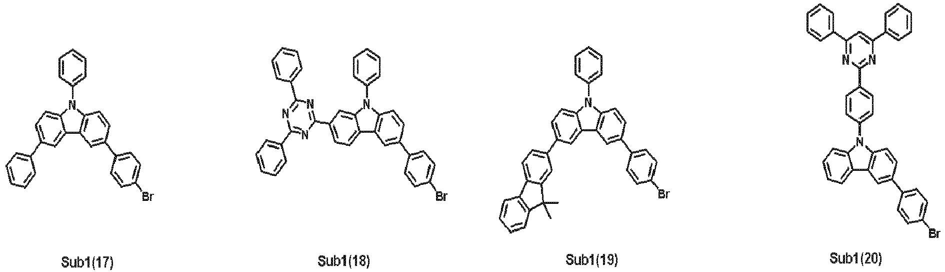

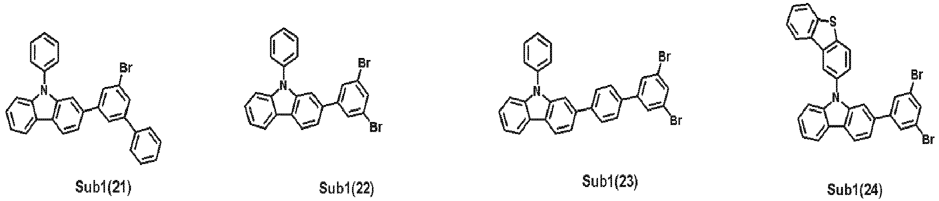

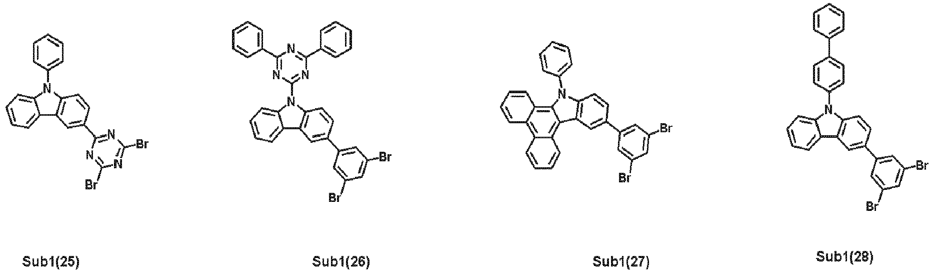

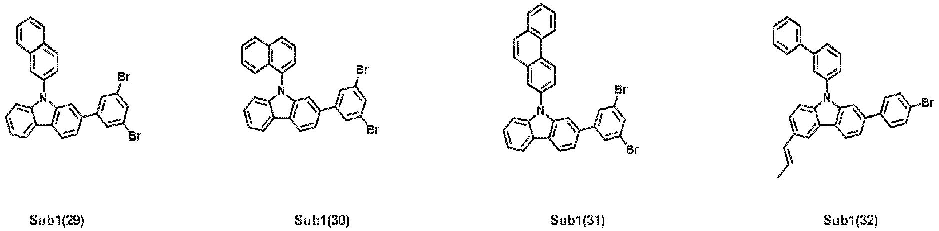

**Table 1**

| Compound | FD-MS | Compound | FD-MS |
|---|---|---|---|
| Sub 1(1) | m/z=321.02(C₁₈H₁₂BrN=322.21) | Sub 1(2) | m/z=321.02(C₁₈H₁₂BrN=322.21) |
| Sub 1(3) | m/z=397.05(C₂₄H₁₆BrN=398.30) | Sub 1(4) | m/z=563.12(C₃₇H₂₆BrN=564.53) |
| Sub 1(5) | m/z=397.05(C₂₄H₁₆BrN=398.30) | Sub 1(6) | m/z=397.05(C₂₄H₁₆BrN=398.30) |
| Sub 1(7) | m/z=473.08CC₃₀H₂₀BrN=474.40) | Sub 1(8) | m/z=473.08(C₃₀H₂₀BrN=474.40) |
| Sub 1(9) | m/z=473.08CC₃₀H₂₀BrN=474.40) | Sub 1(10) | m/z=473.08(C₃₀H₂₀BrN=474.40) |
| Sub 1(11) | m/z=473.08CC₃₀H₂₀BrN=474.40) | Sub 1(12) | m/z=473.08(C₃₀H₂₀BrN=474.40) |
| Sub 1(13) | m/z=497.08(C₃₂H₂₀BrN=498.42) | Sub 1(14) | m/z=503.03(C₃₀H₁₈BrNS=504.45) |
| Sub 1(15) | m/z=487.06CC₃₀H₁₈BrNO=488.38) | Sub 1(16) | m/z=513.11(C₃₃H₂₄BrN=514.47) |
| Sub 1(17) | m/z=473.08(C₃₀H₂₀BrN=474.40) | Sub 1(18) | m/z=628.13(C₃₉H₂₅BrN₄=629.56) |
| Sub 1(19) | m/z=589.14(C₃₉H₂₈BrN=590.56) | Sub 1(20) | m/z=627.13(C₄₀H₂₆BrN₃=628.57) |
| Sub 1(21) | m/z=473.08CC₃₀H₂₀BrN=474.40) | Sub 1(22) | m/z=474.96(C₂₄H₁₅Br₂N=477.20) |
| Sub 1(23) | m/z=550.99(C₃₀H₁₉Br₂N=553.30) | Sub 1(24) | m/z=580.94(C₃₀H₁₇Br₂N=580.34) |
| Sub 1(25) | m/z=477.94(C₂₁H₁₂Br₂N₄=480.16) | Sub 1(26) | m/z=630.01(C₃₃H₂₀Br₂N₄=632.36) |
| Sub 1(27) | m/z=574.99(C₃₂H₁₉Br₂N=577.32) | Sub 1(28) | m/z=550.99(C₃₀H₁₉Br₂N=553.30) |
| Sub 1(29) | m/z=524.97(C₂₈H₁₇Br₂N=527.26) | Sub 1(30) | m/z=524.97(C₂₈H₁₇Br₂N=527.26) |
| Sub 1(31) | m/z=574.99(C₃₂H₁₉Br₂N=577.32) | Sub 1(32) | m/z=513.11(C₃₃H₂₄BrN=514.47) |

### Synthesis Examples of Sub 2

Sub 2 of reaction scheme 1 can be synthesized by the reaction path of reaction scheme 3 below, but is not limited thereto.

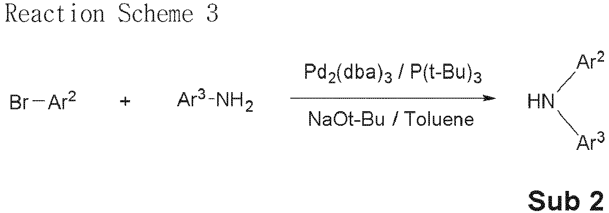

### Synthesis Examples of Sub 2-1

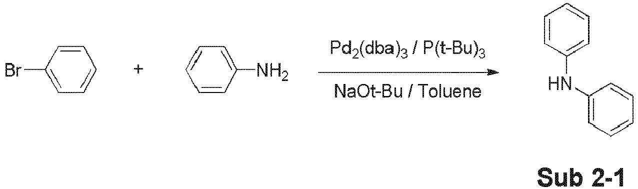

Bromobenzene (37.1 g, 236.2 mmol) was added to a round bottom flask and dissolved in toluene (2200 mL), aniline (20 g, 214.8 mmol), Pd₂(dba)₃ (9.83 g, 10.7 mmol), P(t-Bu)₃ (4.34g, 21.5mmol), NaOt-Bu (62 g, 644.3 mmol) were added in the order and stirred at 100 ° C. After the reaction was completed, the reaction mixture was extracted with ether and water. The organic layer was dried over MgSO₄ and concentrated. The resulting compound was separated by silicagel column chromatography and recrystallized to obtain 28 g of the product (yield: 77%).

### Synthesis Examples of Sub 2-13

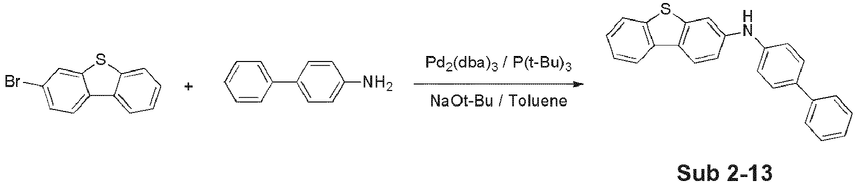

3-Bromodibenzo[b,d]thiophene (42.8 g, 162.5 mmol), toluene(1550 mL), [1,1'-biphenyl]-4-amine (25 g, 147.7 mmol), Pd₂(dba)₃ (6.76 g, 7.4 mmol), P(t-Bu)₃ (3g, 14.8mmol), NaOt-Bu (42.6 g, 443.2 mmol) were added, the same procedure as described in the synthesis method of the 2-1 was carried out to obtain 37.9g of the product (yield: 73%).

Examples of Sub 2 include, but are not limited to, the followings.

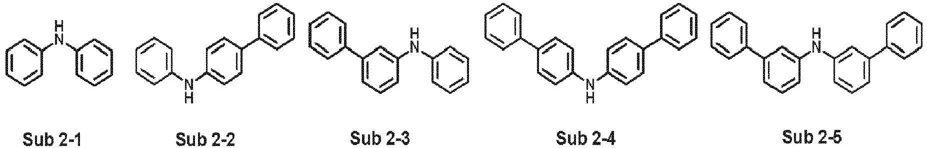

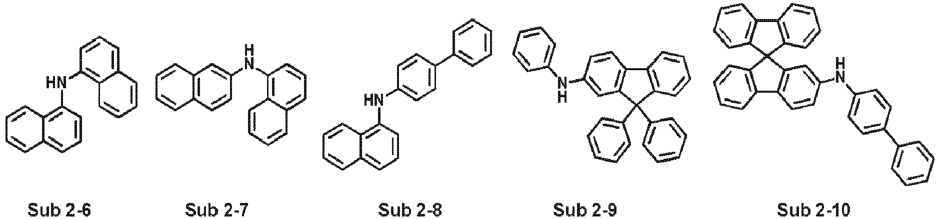

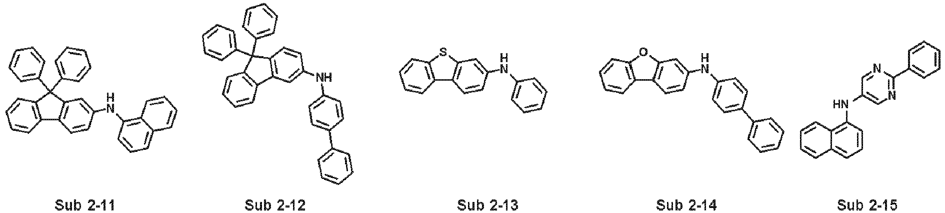

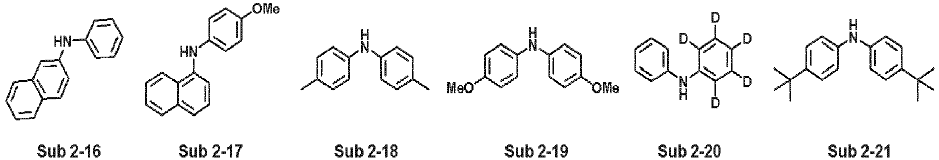

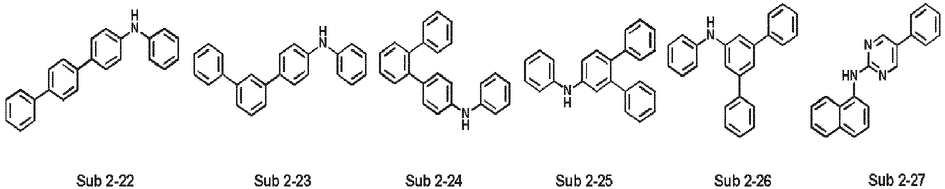

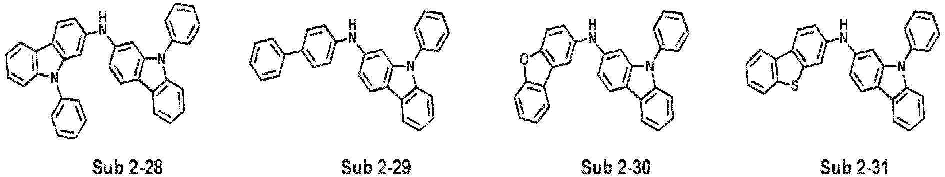

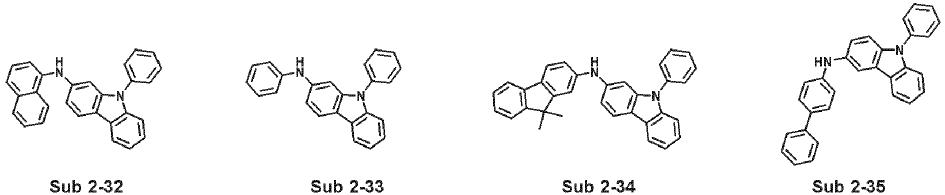

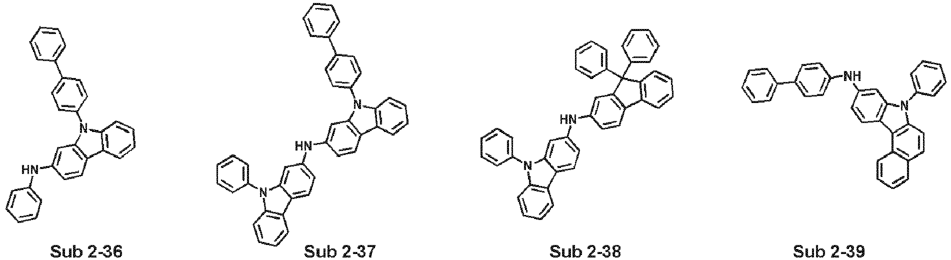

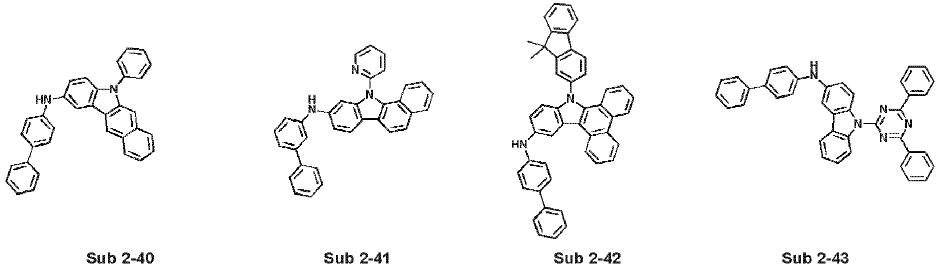

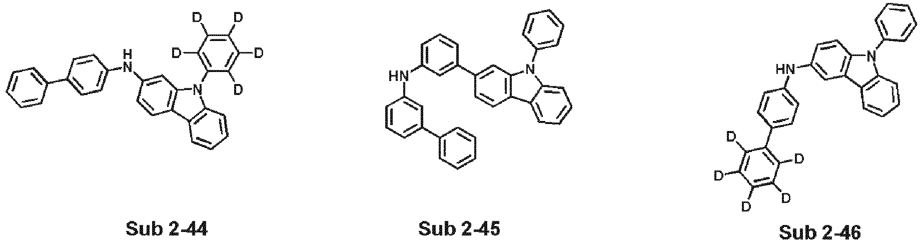

**Table 2**

| compound | FD-MS | compound | FD-MS |
|---|---|---|---|
| Sub 2-1 | m/z=169.09(C₁₂H₁₁N=169.22) | Sub 2-2 | m/z=245.12C₁₈H₁₅N=245.32) |
| Sub 2-3 | m/z=245.12(C₁₈H₁₅N=245.32) | Sub 2-4 | m/z=321.15(C₂₄H₁₉N=321.41) |
| Sub 2-5 | m/z=321.15(C₂₄H₁₉N=321.41) | Sub 2-6 | m/z=269.12C₂₀H₁₅N=269.34) |
| Sub 2-7 | m/z=269.12CC₂₀H₁₅N=269.34) | Sub 2-8 | m/z=295.14(C₂₂H₁₇N=295.38) |
| Sub 2-9 | m/z=409.18(C₃₁H₂₃N=409.52) | Sub 2-10 | m/z=483.20C₃₇H₅N=483.60) |
| Sub 2-11 | m/z=459.20(C₃₅H₂₅N=459.58) | Sub 2-12 | m/z=485.21(C₃₇H₂₇N=485.62) |
| Sub 2-13 | m/z=275.08(C₁₈H₁₃NS=275.37) | Sub 2-14 | m/z=335.13(C₂₄H₁₇NO=335.40) |
| Sub 2-15 | m/z=297.13(C₂₀H₁₅N₃=297.35) | Sub 2-16 | m/z=219.10(C₁₆H₁₃N=219.28) |
| Sub 2-17 | m/z=249.12CC₁₇H₁₅NO=249.31) | Sub 2-18 | m/z=197.12(C₁₄H₁₅N=197.28) |
| Sub 2-19 | m/z=229.11(C₁₄H₁₅NO₂=229.27) | Sub 2-20 | m/z=174.12CC₁₂H₆D₅N=174.25) |
| Sub 2-21 | m/z=281.21(C₂₀H₂₇N=281.44) | Sub 2-22 | m/z=321.15(C₂₄H₁₉N=321.41) |
| Sub 2-23 | m/z=321.15(C₂₄H₁₉N=321.41) | Sub 2-24 | m/z=321.15(C₂₄H₁₉N=321.41) |
| Sub 2-25 | m/z=321.15(C₂₄H₁₉N=321.41) | Sub 2-26 | m/z=321.15(C₂₄H₁₉N=321.41) |
| Sub 2-27 | m/z=297.13(C₂₀H₁₅N₃=297.35) | Sub 2-28 | m/z=499.20(C₃₆H₂₅N₃=499.60) |
| Sub 2-29 | m/z=499.20(C₃₆H₂₂N₂=410.51) | Sub 2-30 | m/z=424.16(C₃₀H₂₀N₂O=424.49) |
| Sub 2-31 | m/z=440.13(C₃₀H₂₀N₂S=440.56) | Sub 2-32 | m/z=384.16(C₂₈H₂₀N₂=384.47) |
| Sub 2-33 | m/z=334.15(C₂₄H₁₈N₂=334.41) | Sub 2-34 | m/z=450.21(C₃₃H₂₆N₂=450.57) |
| Sub 2-35 | m/z=410.18(C₃₀H₂₂N₂=410.51) | Sub 2-36 | m/z=410.18(C₃₀H₂₂N₂=410.51) |
| Sub 2-37 | m/z=575.24(C₄₂H₂₉N₃=575.70) | Sub 2-38 | m/z=574.24(C₄₃H₃₀N₂=574.71) |
| Sub 2-39 | m/z=460.19(C₃₄H₂₄N₂=460.57) | Sub 2-40 | m/z=460.19(C₃₄H₂₄N₂=460.57) |
| Sub 2-41 | m/z=461.19(C₃₃H₂₃N₃=461.56) | Sub 2-42 | m/z=626.27(C₄₇H₃₄N₂=626.79) |
| Sub 2-43 | m/z=565.23(C₃₉H₂₇N_{S}=565.67) | Sub 2-44 | m/z=415.21(C₃₀H₁₇D₅N₂=415.54) |
| Sub 2-45 | m/z=486.21(C₃₆H₂₆N₂=486.61) | Sub 2-46 | m/z=415.21(C₃₀H₁₇D₅N₂=415.54) |

### Synthesis Examples of Final products 1

### Synthesis Examples of 1-37

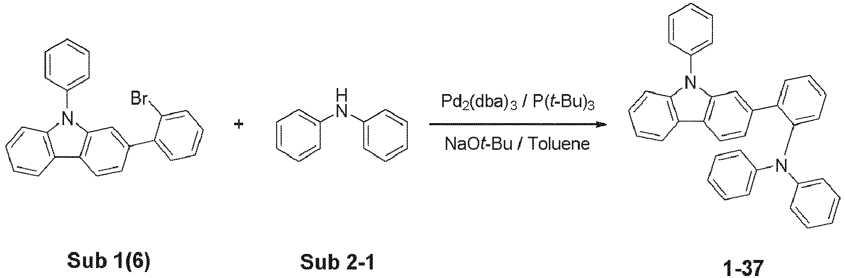

Sub 2-1 (8.0 g, 47.3 mmol) was added in a round bottom flask and dissolved in toluene (500 mL), Sub 1(6) (20.7 g, 52.0 mmol), Pd₂(dba)₃ (2.4 g, 2.6 mmol), P(t-Bu)₃ (1.05g, 5.2mmol), NaOt-Bu (13.6 g, 141.8 mmol) were added and stirred at 100 ° C. After the reaction was completed, the reaction mixture was extracted with CH₂Cl₂ and water. The organic layer was dried over MgSO₄ and concentrated. The resulting compound was separated by silicagel column chromatography and recrystallized to obtain 16.1 g of the product 1-37 (yield:70%).

### Synthesis Examples of 1-10

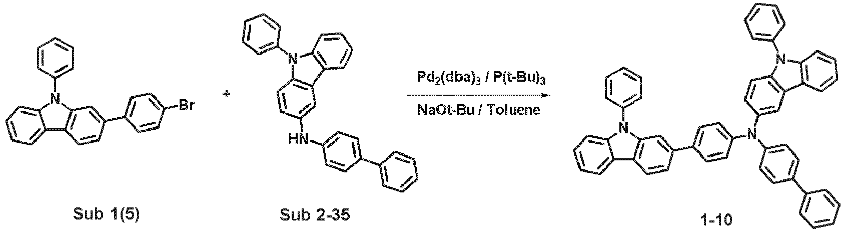

Sub 2-35 (19.4 g, 47.3 mmol), toluene (500 mL), Sub 1(5) (20.7 g, 52.0 mmol), Pd₂(dba)₃ (2.4 g, 2.6 mmol), P(t-Bu)₃ (1.05g, 5.2mmol), NaOt-Bu (13.6 g, 141.8 mmol) were added, the same procedure as described in the synthesis method of the 1-37 was carried out to obtain 24.1g of the product 1-10 (yield:70%).

### Synthesis Examples of 1-54

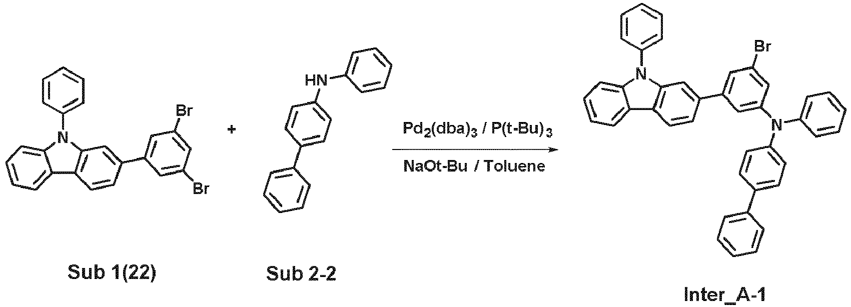

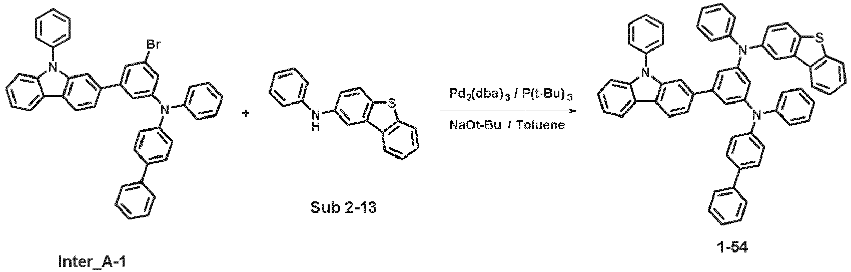

### Synthesis method of Inter_A-1

Sub 2-2 (11.6 g, 47.3 mmol), toluene (500 mL), Sub 1(22) (24.8 g, 52.0 mmol), Pd₂(dba)₃ (2.4 g, 2.6 mmol), P(t-Bu)₃ (1.05g, 5.2mmol), NaOt-Bu (13.6 g, 141.8 mmol) were added, the same procedure as described in the synthesis method of the 1-37 was carried out to obtain 22.8g of the product Inter_A-1 (yield:75%).

### Synthesis method of 1-54

Sub 2-13 (8 g, 29.05 mmol), 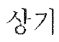 Inter_A-1 (20.5 g, 32 mmol), toluene (305 mL), Pd₂(dba)₃ (1.5 g, 1.6 mmol), P(t-Bu)₃ (0.65g, 3.2mmol), NaOt-Bu (8.4 g, 87.2 mmol) were added, the same procedure as described in the synthesis method of the 1-37 was carried out to obtain 18g of the product 1-54 (yield:74%).

### Synthesis Examples of 2-5

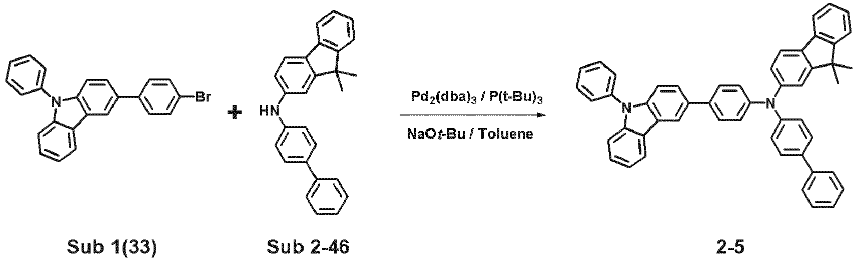

Sub 2-46 (7.2 g, 20 mmol), Sub 1(33) (8.73 g, 22 mmol), Pd₂(dba)₃ (1 g, 1.1 mmol), P(t-Bu)₃ (0.4g, 2.2mmol), NaOt-Bu (5.74 g, 60 mmol), toluene (210 mL) were added, the same procedure as described in the synthesis method of the 1-37 was carried out to obtain 11.5 g of the product 2-5 (yield:85%).

### Synthesis Examples of 2-18

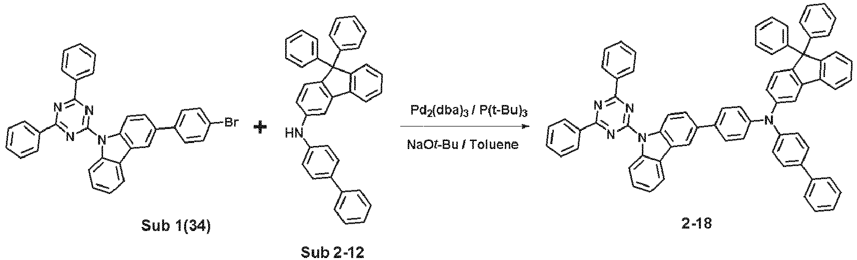

Sub 2-12 (9.7 g, 20 mmol), Sub 1(34) (12.2 g, 22 mmol), Pd₂(dba)₃ (1.0 g, 1.1 mmol), P(t-Bu)₃ (0.4g, 2.2mmol), NaOt-Bu (5.8 g, 60 mmol), toluene (210 mL) were added, the same procedure as described in the synthesis method of the 1-37 was carried out to obtain 15.5 g of the product 2-18 (yield:81%).

### Synthesis Examples of 2-60

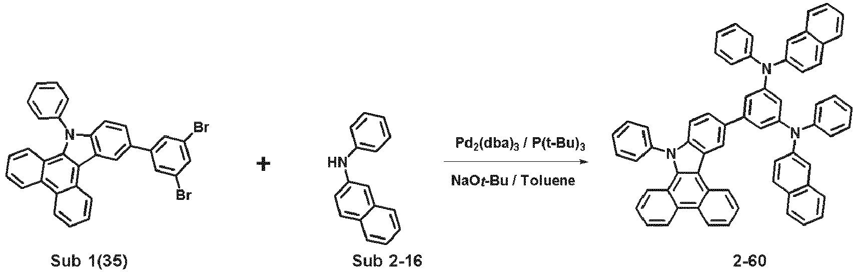

Sub 1(35) (13.9 g, 24.1 mmol), Sub 2-16 (6.3 g, 28.9 mmol), Pd₂(dba)₃ (2.2 g, 2.4 mmol), P(t-Bu)₃ (1g,4 .8mmol), NaOt-Bu (8.3 g, 86.7 mmol), toluene (260 mL) were added, the same procedure as described in the synthesis method of the 1-37 was carried out to obtain 16.5 g of the product 2-60 (yield:80%).

**Table 3**

| compound | FD-MS | compound | FD-MS |
|---|---|---|---|
| 1-1 | m/z=562.24(C₄₂H₃₀N₂=562.72) | 1-2 | m/z=602.27(C₄₅H₃₄N₂=602.78) |
| 1-3 | m/z=563.24(C₄₁H₂₉N₃=563.70) | 1-4 | m/z=714.30(C₅₄H₃₈N₂=714.91) |
| 1-5 | m/z=678.30(C₅₁H₃₈N₂=678.88) | 1-6 | m/z=802.33(C₆₁H₄₂N₂=803.02) |
| 1-7 | m/z=800.32(C₆₁H₄₀N₂=801.01) | 1-8 | m/z=563.24(C₄₁H₂₉N₃=563.70) |
| 1-9 | m/z=668.23(C₄₈H₃₂N₂S=668.86) | 1-10 | m/z=727.30(C₅₄H₃₇N₃=727.91) |
| 1-11 | m/z=652.25(C₄₈H₃₂N₂O=652.80) | 1-12 | m/z=662.27(C₅₀H₃₄N₂=662.84) |
| 1-13 | m/z=536.23(C₄₀H₂₈N₂=536.68) | 1-14 | m/z=586.24(C₄₄H₃₀N₂=586.74) |
| 1-15 | m/z=712.29(C₅₄H₃₆N₂=712.90) | 1-16 | m/z=714.30(C₅₄H₃₈N₂=714.91) |
| 1-17 | m/z=754.33(C₅₇H₄₂N₂=754.98) | 1-18 | m/z=957.38(C₇₀H₄₇N₅=958.18) |
| 1-19 | m/z=965.38(C₇₃H₄₇N₃=966.20) | 1-20 | m/z=719.24(C₅₁H₃₃N₃S=719.91) |
| 1-21 | m/z=758.24(C₅₄H₃₄N₂OS=758.94) | 1-22 | m/z=893.38(C₆₇H₄₇N₃=894.13) |
| 1-23 | m/z=652.25(C₄₈H₃₂N₂O=652.80) | 1-24 | m/z=662.27(C₅₀H₃₄N₂=662.84) |
| 1-25 | m/z=562.24(C₄₂H₃₀N₂=562.72) | 1-26 | m/z=612.26(C₄₆H₃₂N₂=612.78) |
| 1-27 | m/z=688.29(C₅₂H₃₆N₂=688.87) | 1-28 | m/z=714.30(C₅₄H₃₈N₂=714.91) |
| 1-29 | m/z=754.33 (C₅₇H₄₂N₂=754.98) | 1-30 | m/z=878.37(C₆₇H₄₆N₂=879.12) |
| 1-31 | m/z=876.35(C₆₇H₄₄N₂=877.10) | 1-32 | m/z=639.27(C₄₇H₃₃N₃=369.80) |
| 1-33 | m/z=768.26(C₅₆H₃₆N₂S=768.98) | 1-34 | m/z=833.29(C₆₀H₃₉N₃S=834.05) |
| 1-35 | m/z=742.26 (C₅₄H₃₄N₂Oₛ=7 42.88) | 1-36 | m/z=778.333(C₅₉H₄₂N₂=779.00) |
| 1-37 | m/z=486.21(C₃₆H₂₆N₂=486.62) | 1-38 | m/z=536.23(C₄₀H₂₈N₂=536.68) |
| 1-39 | m/z=612.26(C₄₆H₃₂N₂=612.78) | 1-40 | m/z=638.27(C₄₈H₃₄N₂=638.81) |
| 1-41 | m/z=491.24(C₃₆H₂₁D₅N₂=491.65) | 1-42 | m/z=612.26(C₄₆H₃₂N₂=612.78) |
| 1-43 | m/z=794.28(C₅₈H₃₈N₂S=795.02) | 1-44 | m/z=656.26(C₄₈H₃₃FN₂=656.80) |
| 1-45 | m/z=717.29(C₅₁H₃₅N₅=717.88) | 1-46 | m/z=728.32(C₅₅H₄₀N₂=728.94) |
| 1-47 | m/z=842.34(C₆₂H₄₂N₄=843.05) | 1-48 | m/z=714.30(C₅₄H₃₈N₂=714.91) |
| 1-49 | m/z=653.28(C₄₈H₃₅N₃=653.81) | 1-50 | m/z=703.30(C₅₂H₃₇N₃=703.87) |
| 1-51 | m/z=805.35(C₆₀H₄₃N₃=806.00) | 1-52 | m/z=753.31(C₅₆H₃₉N₃=753.93) |
| 1-53 | m/z=818.34(C₆₀H₄₂N₄=819.00) | 1-54 | m/z=835.30(C₆₀H₄₁N₃S=836.05) |
| 1-55 | m/z=655.27(C₄₆H₃₃N₅=655.79) | 1-56 | m/z=885.32(C₆₄H₄₃N₃S=886.11) |
| 1-57 | m/z=759.27(C₅₄H₃₇N₃S=759.96) | 1-58 | m/z=706.28(C₄₉H₃₄N₆=706.83) |
| 1-59 | m/z=960.39(C₆₉H₄₈N₆=961.16) | 1-60 | m/z=853.35(C₆₄H₄₃N₃=854.05) |
| 1-61 | m/z=894.37(C₆₆H₄₆N₄=895.10) | 1-62 | m/z=834.38(C₆₂H₃₈D₅N₃=835.06) |
| 1-63 | m/z=855.36(C₆₄H₄₅N₃=856.06) | 1-64 | m/z=853.35(C₆₄H₄₃N₃=854.05) |
| 1-65 | m/z=794.37(C₆₀H₄₆N₂=795.04) | 1-66 | m/z=987.39(C₇₁H₄₉N₅0=988.21) |
| 1-67 | m/z=1021.44(C₇₇H₅₅N₃=1022.31) | 1-68 | m/z=737.23(C₅₁H₃₂FN₃S=737.90) |
| 2-1 | m/z=562.24(C₄₂H₃₀N₂=562.72) | 2-2 | m/z=602.27(C₄₅H₃₄N₂=602.78) |
| 2-3 | m/z=563.24(C₄₁H₂₉N₃=563.70) | 2-4 | m/z=714.30(C₅₄H₃₈N₂=714.91) |
| 2-5 | m/z=678.30(C₅₁H₃₈N₂=678.88) | 2-6 | m/z=802.33(C₆₁H₄₂N₂=803.02) |
| 2-7 | m/z=800.32(C₆₁H₄₀N₂=801.01) | 2-8 | m/z=563.24(C₄₁H₂₉N₃=563.70) |
| 2-9 | m/z=668.23(C₄₈H₃₂N₂S=668.86) | 2-10 | m/z=727.30(C₅₄H₃₇N₃=727.91) |
| 2-11 | m/z=652.25(C₄₈H₃₂N₂0=652.80) | 2-12 | m/z=662 . 27 (C₅₀H₃₄N₂=662.84) |
| 2-13 | m/z=536.23(C₄₀H₂₈N₂=536.68) | 2-14 | m/z=586 . 24(C₄₄H₃₀N₂=586.74) |
| 2-15 | m/z=712.29(C₅₄H₃₆N₂=712.90) | 2-16 | m/z=714.30(C₅₄H₃₈N₂=714.91) |
| 2-17 | m/z=754.33(C₅₇H₄₂N₂=754.98) | 2-18 | m/z=957.38(C₇₀H₄₇N₅=958.18) |
| 2-19 | m/z=965.38(C₇₃H₄₇N₃=966.20) | 2-20 | m/z=719.24(C₅₁H₃₃N₃S=719.91) |
| 2-21 | m/z=758 . 24(C₅₄H₃₄N₂0S=758. 94) | 2-22 | m/z=893.38(C₆₇H₄₇N₃=894.13) |
| 2-23 | m/z=652.25(C₄₈H₃₂N₂0=652.80) | 2-24 | m/z=662.27(C₅₀H₃₄N₂=662.84) |
| 2-25 | m/z=562.24(C₄₂H₃₀N₂=562.72) | 2-26 | m/z=612.26(C₄₆H₃₂N₂=612.78) |
| 2-27 | m/z=688.29(C₅₂H₃₆N₂=688.87) | 2-28 | m/z=714.30(C₅₄H₃₈N₂=714.91) |
| 2-29 | m/z=754.33(C₅₇H₄₂N₂=754.98) | 2-30 | m/z=878.37(C₆₇H₄₆N₂=879.12) |
| 2-31 | m/z=876.35(C₆₇H₄₄N₂=877.10) | 2-32 | m/z=639.27(C₄₇H₃₃N₃=369. 80) |
| 2-33 | m/z=768.26(C₅₆H₃₆N₂S=768.98) | 2-34 | m/z=833.29(C₆oH₃₉N₃S=834.05) |
| 2-35 | m/z=742.26(C₅₄H₃₄N₂0ₛ=742.88) | 2-36 | m/z=778.333(C₅₉H₄₂N₂=779.00) |
| 2-37 | m/z=486.21(C₃₆H₂₆N₂=486.62) | 2-38 | m/z=536.23(C₄₀H₂₈N₂=536.68) |
| 2-39 | m/z=612.26(C₄₆H₃₂N₂=612.78) | 2-40 | m/z=638.27(C₄₈H₃₄N₂=638.81) |
| 2-41 | m/z=491.24(C₃₆H₂₁D₅N₂=491.65) | 2-42 | m/z=612.26(C₄₆H₃₂N₂=612.78) |
| 2-43 | m/ z=794.28 (C₅₈H₃₈N₂S=795. 02) | 2-44 | m/ z=656.26(C₄₈H₃₃FN₂=656.80) |
| 2-45 | m/z=717.29(C₅₁H₃₅N₅=717.88) | 2-46 | m/z=728.32(C₅₅H₄₀N₂=728.94) |
| 2-47 | m/z=842.34(C₆₂H₄₂N₄=843.05) | 2-48 | m/z=714.30(C₅₄H₃₈N₂=714.91) |
| 2-49 | m/z=653.28(C₄₈H₃₅N₃=653.81) | 2-50 | m/z=703.30(C₅₂H₃₇N₃=703. 87) |
| 2-51 | m/z=805.35(C₆₀H₄₃N₃=806.00) | 2-52 | m/z=753.31(C₅₆H₃₉N₃=753.93) |
| 2-53 | m/z=818.34(C₆₀H₄₂N₄=819.00) | 2-54 | m/z=835.30(C₆₀H₄₁N₃S=836.05) |
| 2-55 | m/z=655.27(C₄₆H₃₃N₅=655.79) | 2-56 | m/z=885.32(C₆₄H₄₃N₃S=886.11) |
| 2-57 | m/z=759.27(C₅₄H₃₇N₃S=759. 96) | 2-58 | m/z=706.28(C₄₉H₃₄N₆=706. 83) |
| 2-59 | m/z=960.39(C₆₉H₄₈N₆=961.16) | 2-60 | m/z=853.35(C₆₄H₄₃N₃=854.05) |
| 2-61 | m/z=894.37(C₆₆H₄₆N₄=895.10) | 2-62 | m/z=834.38(C₆₂H₃₈D₅N₃=835.06) |
| 2-63 | m/z=855.36(C₆₄H₄₅N₃=856.06) | 2-64 | m/z=853.35(C₆₄H₄₃N₃=854.05) |
| 2-65 | m/z=794.37(C₆₀H₄₆N₂=795.04) | 2-66 | m/z=987.39(C₇₁H₄₉N₅0=988. 21) |
| 2-67 | m/z=1021.44(C₇₇H₅₅N₃=1022.31) | 2-68 | m/z=737.23(C₅₁H₃₂FN₃S=737.90) |

### Synthesis Examples 2

The final product 2 represented by Formula 2 of the present invention is prepared by reacting Sub 3 and Sub 4 as shown in Reaction Scheme 4 below.

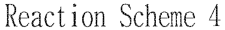

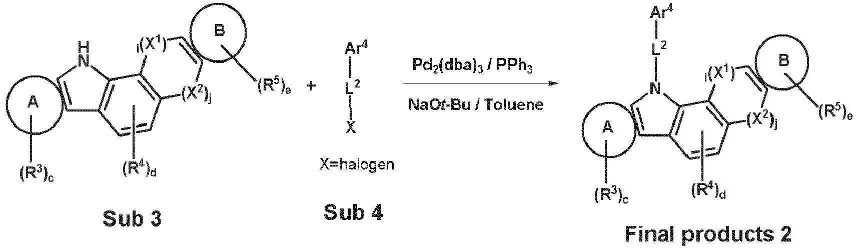

### Synthesis example of Sub 3

Sub 3 of Reaction Scheme 4 can be synthesized by the reaction path of Reaction Scheme 5 below, but is not limited thereto.

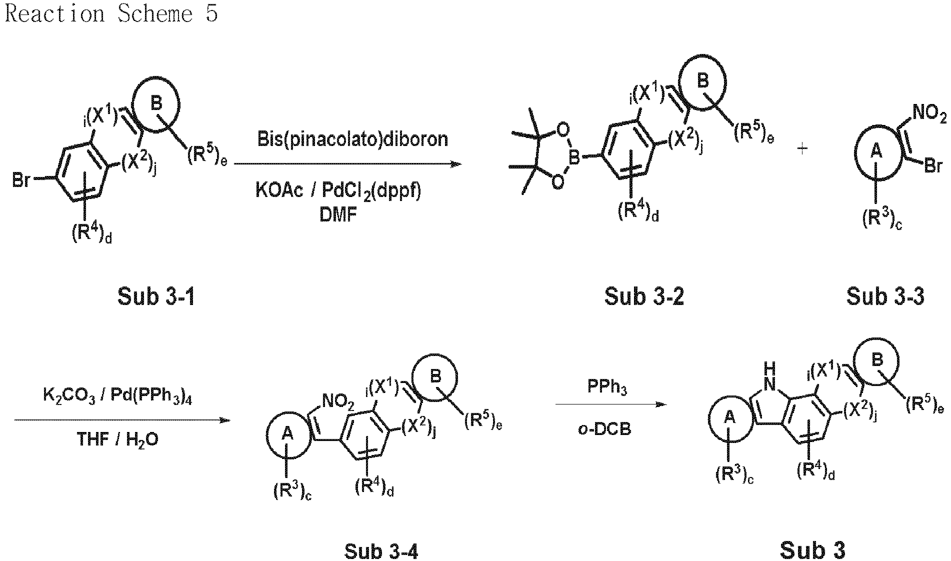

### Synthesis Examples of Sub 3(1)

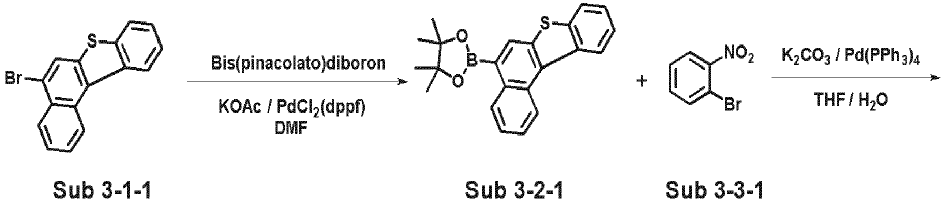

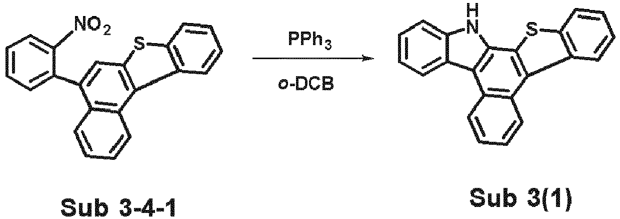

### Synthesis method of Sub 3-2-1

After 5-bromobenzo[*b*]naphtha[1,2-d]thiophene (50 g, 155 mmol), bis(pinacolato)diboron (43.4 g, 171 mmol), KOAc (46 g, 466 mmol), PdCl₂(dppf) (3.8 g, 4.7 mmol) were dissolved in DMF (980 mL), and refluxed at 120 ° C for 12 hours. When the reaction was completed, the temperature of the reaction was cooled to room temperature, extracted with CH₂Cl₂ and wiped with water. The organic layer was dried over MgSO₄ and concentrated. The resulting compound was recrystallized by CH₂Cl₂ and methanol solvent to obtain Sub 3-2-1 (45 g, 80%).

### Synthesis method of Sub 3-4-1

Sub 3-2-1 (40 g, 111 mmol), bromo-2-nitrobenzene (26.91 g, 133 mmol), K₂CO₃ (46.03 g, 333 mmol), Pd(PPh₃)₄ (7.7 g, 6.66 mmol) were added in a round bottom flask and THF (490 mL) and water (245 mL) were added to dissolve and refluxed at 80 ° C for 12 hours. When the reaction was completed, the temperature of the reaction was cooled to room temperature, extracted with CH₂Cl₂ and wiped with water. The organic layer was dried over MgSO₄ and concentrated. The resulting compound was separated by silicagel column chromatography to obtain Sub 3-4-1 (27.6 g, 70%).

### Synthesis method of Sub 3(1)

Sub 3-4-1 (20 g, 56.3 mmol) and triphenylphosphine (37 g, 141 mmol) were dissolved in o-dichlorobenzene (235 mL) and refluxed for 24 hours. When the reaction was completed, the solvent was removed using reduced pressure distillation. The resulting compound was separated by silicagel column chromatography and recrystallized to obtain Sub 3(1) (13.6g, 75%).

### Synthesis example of Sub 3(2)

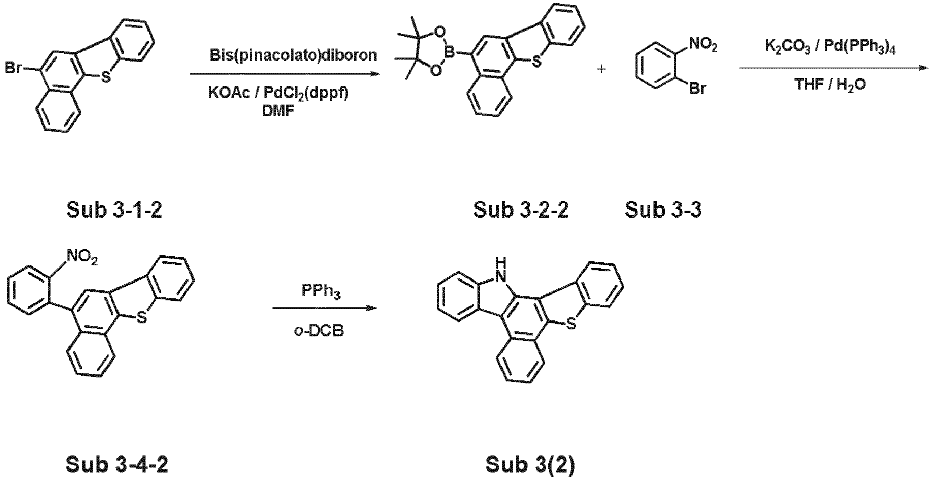

### Synthesis method of Sub 3-2-2

5-bromobenzo[b]naphtho[2,1-d]thiophene (50 g, 160 mmol), bis(pinacolato)diboron (44.6 g, 176 mmol), KOAc (47 g, 479 mmol), PdCl₂(dppf) (3.91 g, 4.8 mmol), DMF (1L) were carried out in the same manner as in Sub 3-2-1 to give the product Sub 3-2-2 (45 g, 78%).

### Synthesis method of Sub 3-4-2

Sub 3-2-2 (40 g, 111 mmol), bromo-2-nitrobenzene (26.91 g, 133 mmol), K₂CO₃ (46.03 g, 333 mmol), Pd(PPh₃)₄ (7.7 g, 6.7 mmol), THF (490 mL), and water (245 mL) were carried out in the same manner as in Sub 3-4-1 to give the product Sub 3-4-2 (25.6 g, 65%).

### Synthesis method of Sub 3(2)

Sub 3-4-2 (20 g, 56.3 mmol), triphenylphosphine (44.28 g, 0.17 mol), σ dichlorobenzene (235 mL) were carried out in the same manner as in Sub 3(1) to give the product Sub 3(2) (12 g, 66%).

### Synthesis example of Sub 3(7)

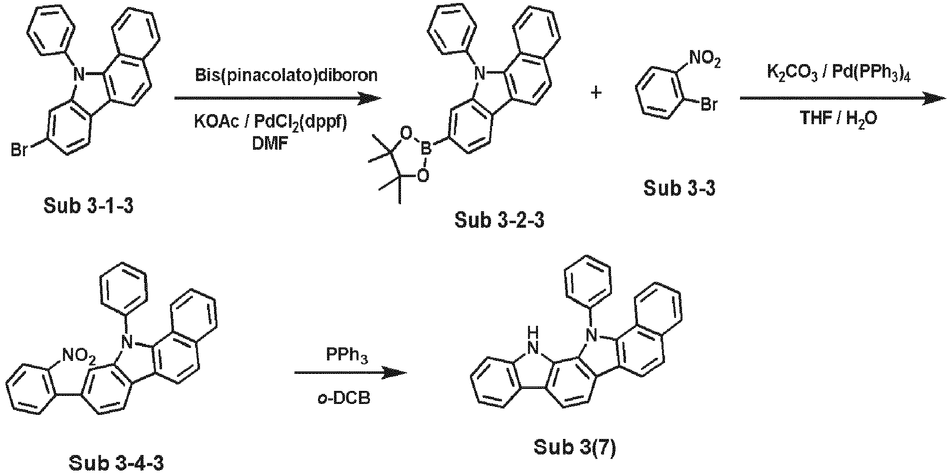

### Synthesis method of Sub 3-2-3

9-bromo-11-phenyl-11H-benzo[a]carbazole (50 g, 134 mmol), bis(pinacolato)diboron (37.5 g, 148 mmol), KOAc (40 g, 403 mmol), PdCl₂(dppf) (3.3 g, 4.0 mmol) were carried out in the same manner as in Sub 3-2-1 to give the product Sub 3-2-3 (45.1 g, 80 %).

### Synthesis method of Sub 3-4-3

Sub 3-2-3 (46.1 g, 111 mmol), Sub 3-3 (26.7 g, 131 mmol), K₂CO₃ (45.6g, 330mmol), Pd(PPh₃)₄ (7.63 g, 6.6 mmol) were carried out in the same manner as in Sub 3-4-1 to give the product Sub 3-4-3 (29.6 g, 65 %).

### Synthesis method of Sub 3(7)

Sub 3-4-3 (20.7 g, 50 mmol), triphenylphosphine (32.7 g, 125 mmol), σ dichlorobenzene (205 mL) were carried out in the same manner as in Sub 3(1) to give the product Sub 3(7) (13.0 g, 68 %).

### Synthesis example of Sub 3(13)

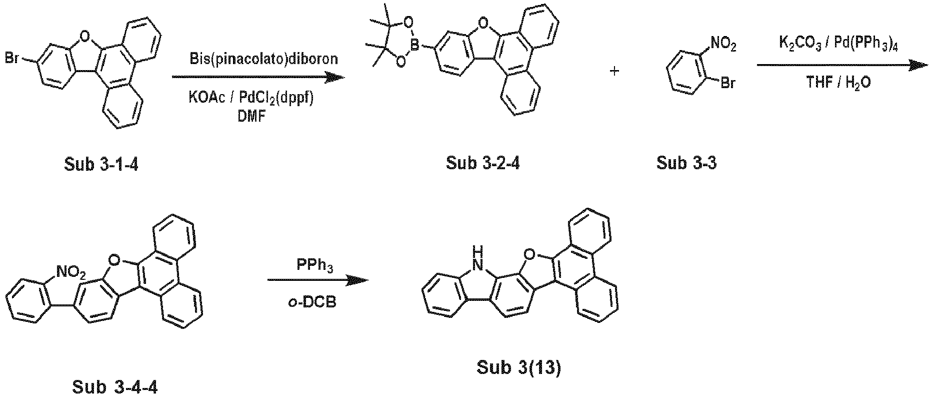

### Synthesis method of Sub 3-2-4

Sub 3-1-4 (55.6 g, 160 mmol), bis(pinacolato)diboron (44.7 g, 176 mmol), KOAc (47.1 g, 480 mmol), PdCl₂(dppf) (3.92 g, 4.8 mmol) were carried out in the same manner as in Sub 3-2-1 to give the product Sub 3-2-4 (47.9 g, 76 %).

### Synthesis method of Sub 3-4-4

Sub 3-2-4 (43.4 g, 110 mmol), Sub 3-3 (26.7 g, 132 mmol), K₂CO₃ (45.6 g, 330 mmol), Pd(PPh₃)₄ (7.63 g, 6.6 mmol) were carried out in the same manner as in Sub 3-4-1 to give the product Sub 3-4-4 (27.4 g, 64 %).

### Synthesis method of Sub 3(13)

Sub 3-4-4 (19.5 g, 50.1 mmol), triphenylphosphine (32.8 g, 125.2 mmol), σ dichlorobenzene (205 mL) were carried out in the same manner as in Sub 3(1) to give the product Sub 3(13) (11.5 g, 64 %).

### Synthesis example of Sub 3(26)

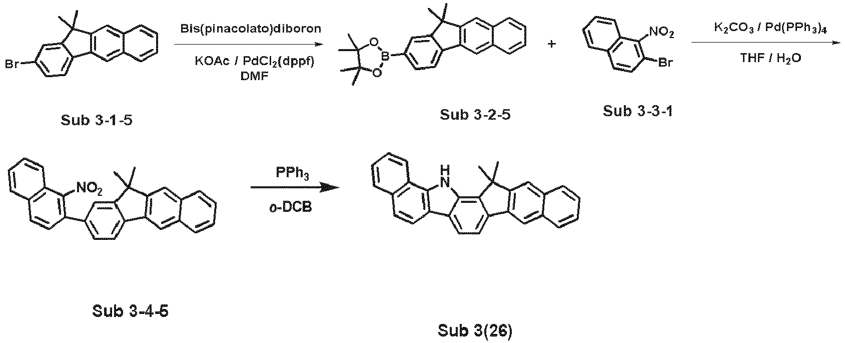

### Synthesis method of Sub 3-2-5

Sub 3-1-5 (51.7 g, 160 mmol), bis(pinacolato)diboron (44.7 g, 176 mmol), KOAc (47.1 g, 480 mmol), PdCl₂(dppf) (3.92 g, 4.8 mmol) were carried out in the same manner as in Sub 3-2-1 to give the product Sub 3-2-5 (46.2 g, 78 %).

### Synthesis method of Sub 3-4-5

Sub 3-2-5 (40.7 g, 110 mmol), Sub 3-3-1 (33.2 g, 132 mmol), K₂CO₃ (45.6 g, 330 mmol), Pd(PPh₃)₄ (7.62 g, 6.6 mmol) were carried out in the same manner as in Sub 3-4-1 to give the product Sub 3-4-5 (30.6 g, 67 %).

### Synthesis method of Sub 3(26)

Sub 3-4-5 (20.8 g, 50.1 mmol), triphenylphosphine (32.8 g, 125 mmol), σ dichlorobenzene (205 mL) were carried out in the same manner as in Sub 3(1) to give the product Sub 3(26) (11.9 g, 62 %).

### Synthesis example of Sub 3(39)

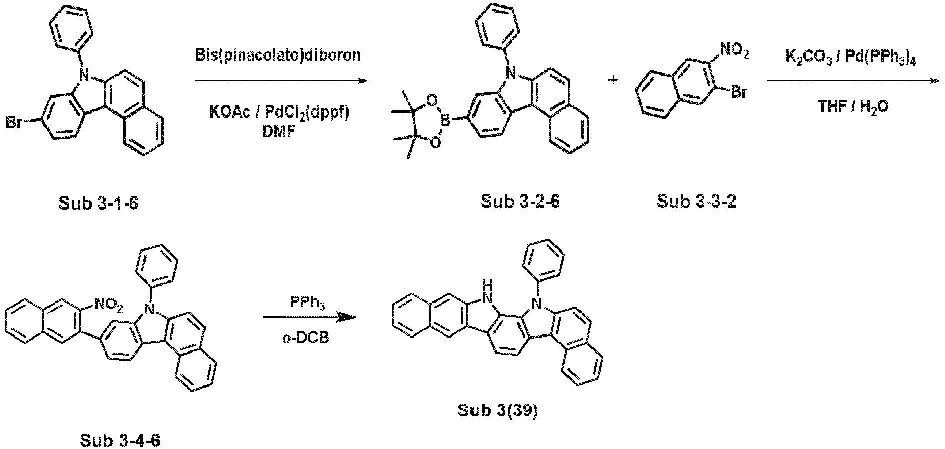

### Synthesis method of Sub 3-2-6

Sub 3-1-6 (59.6 g, 160 mmol), bis(pinacolato)diboron (44.7 g, 176 mmol), KOAc (47.1 g, 480 mmol), PdCl₂(dppf) (3.92 g, 4.8 mmol) were carried out in the same manner as in Sub 3-2-1 to give the product Sub 3-2-6 (50.4 g, 75 %).

### Synthesis method of Sub 3-4-6

Sub 3-2-6 (46.1 g, 110 mmol), Sub 3-3-2 (33.3 g, 132 mmol), K₂CO₃ (45.6 g, 330 mmol), Pd(PPh₃)₄ (7.63 g, 6.6 mmol) were carried out in the same manner as in Sub 3-4-1 to give the product Sub 3-4-6 (32.2 g, 63 %).

### Synthesis method of Sub 3(39)

Sub 3-4-6 (23.2 g, 50 mmol), triphenylphosphine (32.7 g, 125 mmol), σ dichlorobenzene (205 mL) were carried out in the same manner as in Sub 3(1) to give the product Sub 3(39) (14.1 g, 65 %).

### Synthesis example of Sub 3(45)

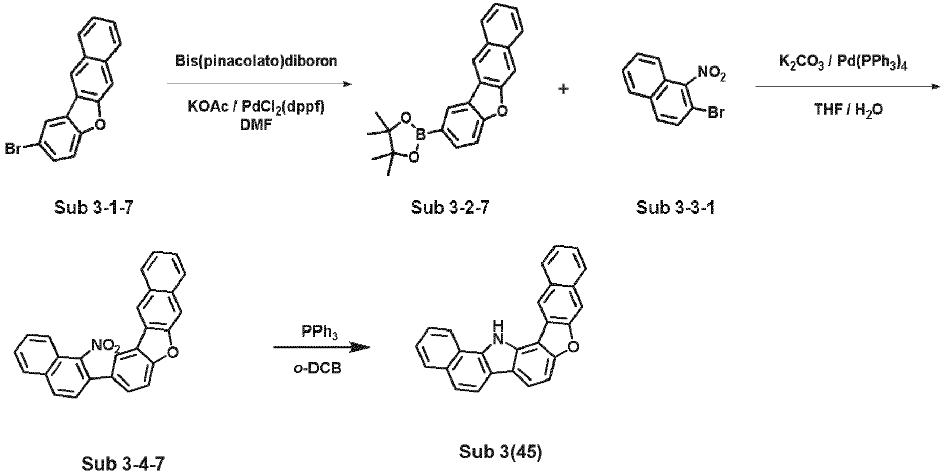

### Synthesis method of Sub 3-2-7

Sub 3-1-7 (47.5 g, 160 mmol), bis(pinacolato)diboron (44.7 g, 176 mmol), KOAc (47.1 g, 480 mmol), PdCl₂(dppf) (3.92 g, 4.8 mmol) were carried out in the same manner as in Sub 3-2-1 to give the product Sub 3-2-7 (43.0 g, 78 %).

### Synthesis method of Sub 3-4-7

Sub 3-2-7 (37.9 g, 110 mmol), Sub 3-3-1 (33.3 g, 132 mmol), K₂CO₃ (45.7 g, 330 mmol), Pd(PPh₃)₄ (7.64 g, 6.6 mmol) were carried out in the same manner as in Sub 3-4-1 to give the product Sub 3-4-7 (27.4 g, 64 %).

### Synthesis method of Sub 3(45)

Sub 3-4-7 (19.5 g, 50.1 mmol), triphenylphosphine (32.8 g, 125 mmol), σ dichlorobenzene (205 mL) were carried out in the same manner as in Sub 3(1) to give the product Sub 3(45) (12.0 g, 67 %).

### Synthesis example of Sub 3(66)

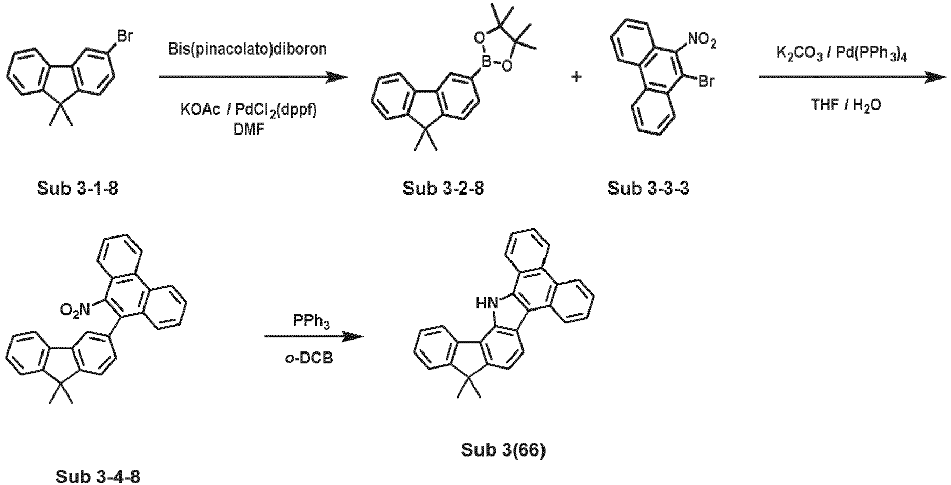

### Synthesis method of Sub 3-2-8

Sub 3-1-8 (43.7 g, 160 mmol), bis(pinacolato)diboron (44.7 g, 176 mmol), KOAc (47.1 g, 480 mmol), PdCl₂(dppf) (3.9 g, 4.8 mmol) were carried out in the same manner as in Sub 3-2-1 to give the product Sub 3-2-8 (38.4 g, 75 %).

### Synthesis method of Sub 3-4-8

Sub 3-2-8 (35.2 g, 110 mmol), Sub 3-3-3 (39.9 g, 132 mmol), K₂CO₃ (45.6 g, 330 mmol), Pd(PPh₃)₄ (7.6 g, 6.6 mmol) were carried out in the same manner as in Sub 3-4-1 to give the product Sub 3-4-8 (31.5 g, 69 %).

### Synthesis method of Sub 3(66)

Sub 3-4-8 (20.8 g, 50 mmol), triphenylphosphine (32.8 g, 125 mmol), σ dichlorobenzene (205 mL) were carried out in the same manner as in Sub 3(1) to give the product Sub 3(66) (12.5 g, 65 %).

Examples of Sub 3 include, but are not limited to, the followings.

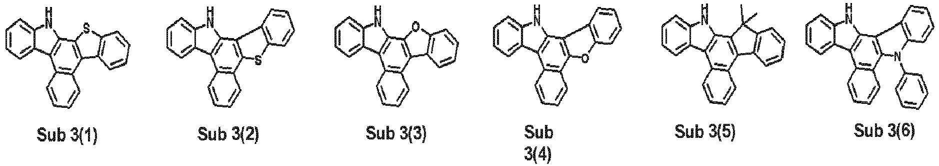

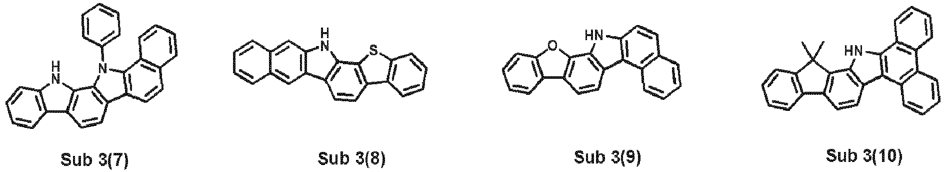

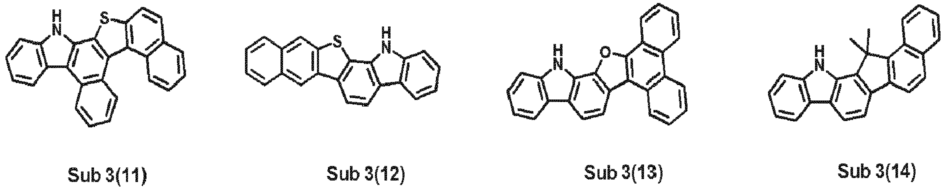

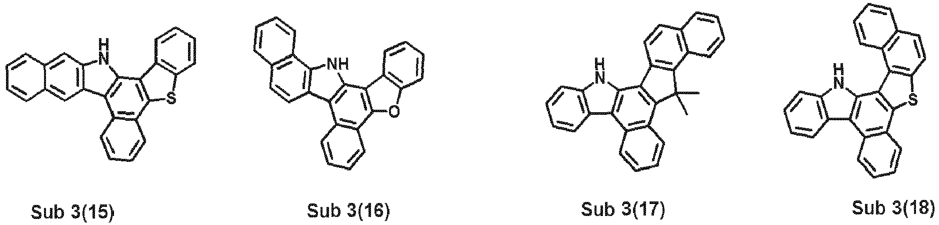

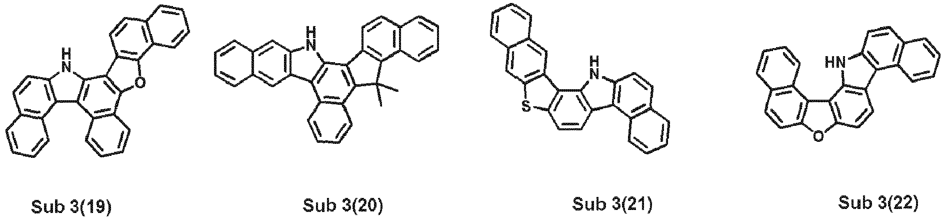

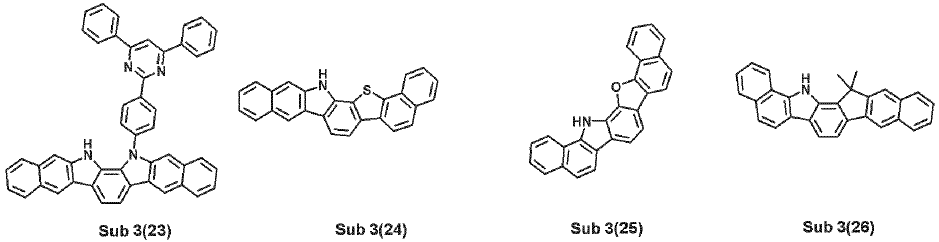

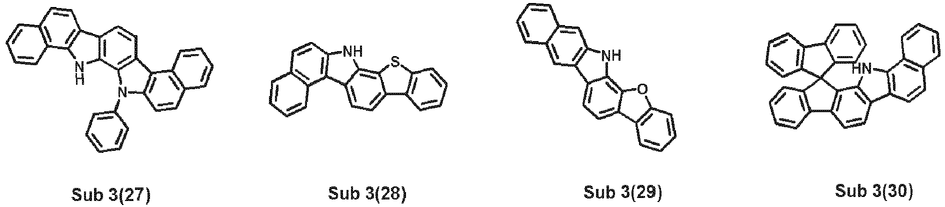

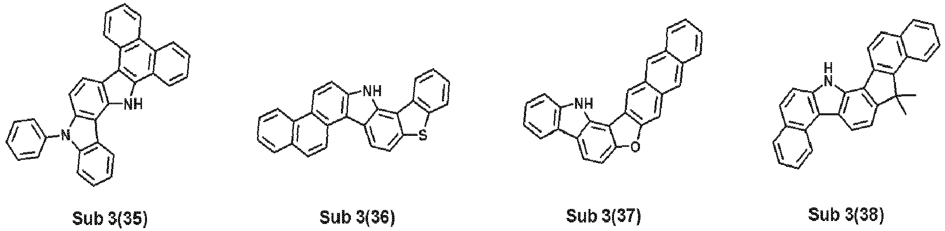

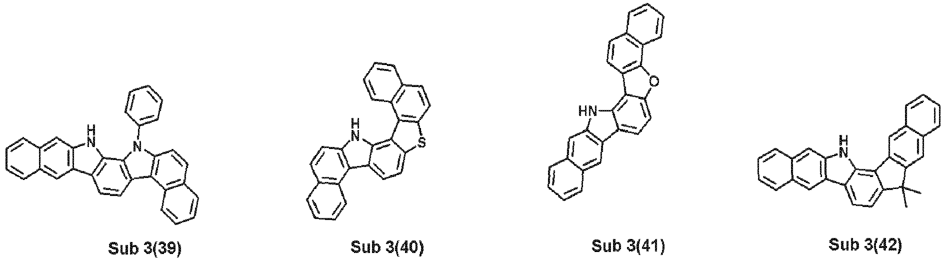

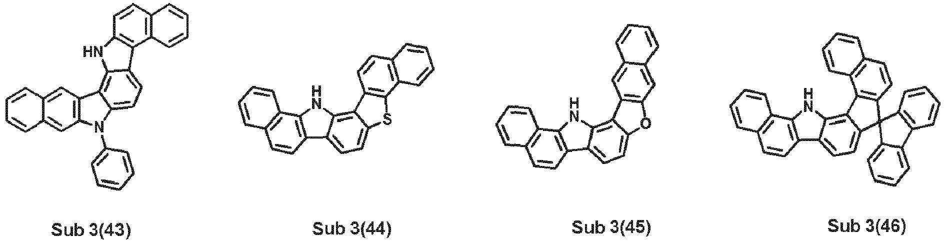

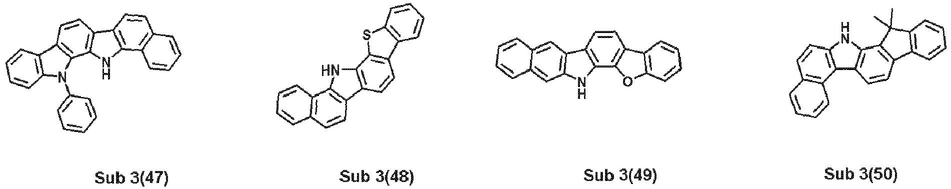

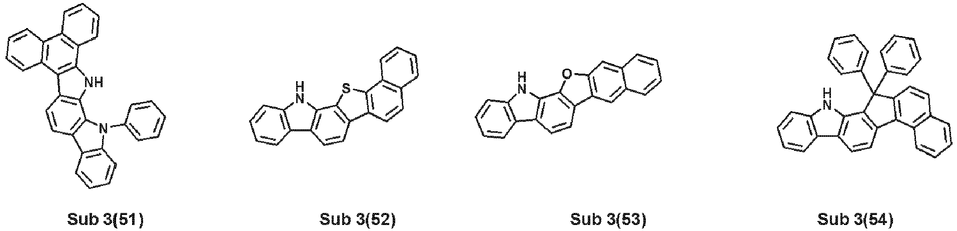

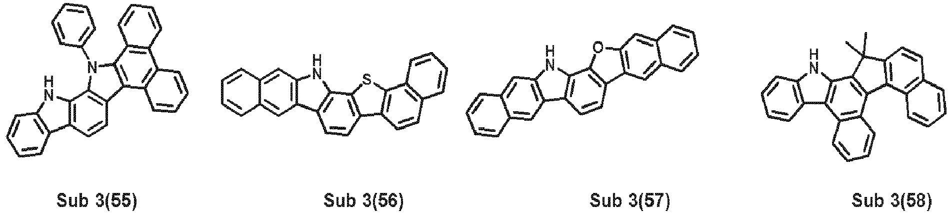

**Table 4**

| compound | FD-MS | compound | FD-MS |
|---|---|---|---|
| Sub 3(1) | m/z=323.08(C₂₂H₁₃NS=323.41) | Sub 3(2) | m/z=323.08(C₂₂H₁₃NS=323.41) |
| Sub 3(3) | m/z=307.10(C₂₂H₁₃NO=307.34) | Sub 3(4) | m/z=307.10(C₂₂H₁₃NO=307.34) |
| Sub 3(5) | m/z=333.15(C₂₅H₁₉N=333.43) | Sub 3(6) | m/z=382.15(C₂₈H₁₈N₂=382.46) |
| Sub 3(7) | m/z=382.15(C₂₈H₁₈N₂=382.47) | Sub 3(8) | m/z=323.08(C₂₂H₁₃NS=323.41) |
| Sub 3(9) | m/z=307.10(C₂₂H₁₃NO=307.35) | Sub 3(10) | m/z=383.17(C₂₉H₂₁N=383.49) |
| Sub 3(11) | m/z=373.09(C₂₆H₁₅NS=373.47) | Sub 3(12) | m/z=323.08 (C₂₂H₁₃NS=323.41) |
| Sub 3(13) | m/z=357.12(C₂₆H₁₅NO=357.41) | Sub 3(14) | m/z=333.15(C₂₅H₁₉N=333.43) |
| Sub 3(15) | m/z=373.09(C₂₆H₁₅NS=373.47) | Sub 3(16) | m/z=357.12(C₂₆H₁₅NO=357.41) |
| Sub 3(17) | m/z=383.17(C₂₉H₂₁N=383.49) | Sub 3(18) | m/z=373.09(C₂₆H₁₅NS=373.47) |
| Sub 3(19) | m/z=407.13(C₃₀H₁₇NO=407.47) | Sub 3(20) | m/z=433.18(C₃₃H₂₃N=433.55) |
| Sub 3(21) | m/z=373.09(C₂₆H₁₅NS=373.47) | Sub 3(22) | m/z=357.12(C₂₆H₁₅NO=357.41) |
| Sub 3(23) | m/z=662.25(C₄₈H₃₀N₄=662.80) | Sub 3(24) | m/z=373.09(C₂₆H₁₅NS=373.47) |
| Sub 3(25) | m/z=357.12(C₂₆H₁₅NO=357.41) | Sub 3(26) | m/z=383.17(C₂₉H₂₁N=383.49) |
| Sub 3(27) | m/z=432.16(C₃₂H₂₀N₂=432.53) | Sub 3(28) | m/z=323.08(C₂₂H₁₃NS=323.41) |
| Sub 3(29) | m/z=307.10(C₂₂H₁₃NO=307.35) | Sub 3(30) | m/z=455.17(C₃₅H₂₁N=455.56) |
| Sub 3(31) | m/z=432.16(C₃₂H₂₀N₂=432.53) | Sub 3(32) | m/z=323.08(C₂₂H₁₃NS=323.41) |
| Sub 3(33) | m/z=307.10(C₂₂H₁₃NO=307.35) | Sub 3(34) | m/z=383.17(C₂₉H₂₁N=383.49) |
| Sub 3(35) | m/z=432.16(C₃₂H₂₀N₂=439.53) | Sub 3(36) | m/z=373.09(C₂₆H₁₅NS=373.47) |
| Sub 3(37) | m/z=357.12(C₂₆H₁₅NO=357.41) | Sub 3(38) | m/z=383.17(C₂₉H₂₁N=383.49) |
| Sub 3(39) | m/z=432.16(C₃₂H₂₀N₂=432.53) | Sub 3(40) | m/z=373.09(C₂₆H₁₅NS=373.47) |
| Sub 3(41) | m/z=357.12(C₂₆H₁₅NO=357.41) | Sub 3(42) | m/z=383.17(C₂₉H₂₁N=383.49) |
| Sub 3(43) | m/z=432.16(C₃₂H₂₀N₂=432.53) | Sub 3(44) | m/z=373.09(C₂₆H₁₅NS=373.47) |
| Sub 3(45) | m/z=357.12(C₂₆H₁₅NO=357.41) | Sub 3(46) | m/z=505.18(C₃₉H₂₃N=505.62) |
| Sub 3(47) | m/z=382.15(C₂₈H₁₈N₂=382.47) | Sub 3(48) | m/z=323.08(C₂₂H₁₃NS=323.41) |
| Sub 3(49) | m/z=307.10(C₂₂H₁₃NO=307.35) | Sub 3(50) | m/z=333.15(C₂₅H₁₉N=333.43) |
| Sub 3(51) | m/z=432.16(C₃₂H₂₀N₂=432.53) | Sub 3(52) | m/z=323.08(C₂₂H₁₃NS=323.41) |
| Sub 3(53) | m/z=307.10(C₂₂H₁₃NO=307.35) | Sub 3(54) | m/z=457.18(C₃₅H₂₃N=457.58) |
| Sub 3(55) | m/z= 432.16(C₃₂H₂₀N₂=432.53) | Sub 3(56) | m/z=373.09(C₂₆H₁₅NS=373.47) |
| Sub 3(57) | m/z=357.12(C₂₆H₁₅NO=357.41) | Sub 3(58) | m/z=383.17(C₂₉H₂₁N=383.49) |
| Sub 3(59) | m/z=616.17(C₄₂H₂₄N₄S=616.74) | Sub 3(60) | m/z=323.08(C₂₂H₁₃NS=323.41) |
| Sub 3(61) | m/z=307.10(C₂₂H₁₃NO=307.35) | Sub 3(62) | m/z=373.09(C₂₆H₁₅NS=373.47) |
| Sub 3(63) | m/z=432.16(C₃₂H₂₀N₂=432.53) | Sub 3(64) | m/z=373.09(C₂₆H₁₅NS=373.47) |
| Sub 3(65) | m/z=357.12(C₂₆H₁₅NO=357.41) | Sub 3(66) | m/z=383.17(C₂₉H₂₁N=383.49) |

### Examples of Sub 4

Examples of Sub 4 include, but are not limited to, the followings.

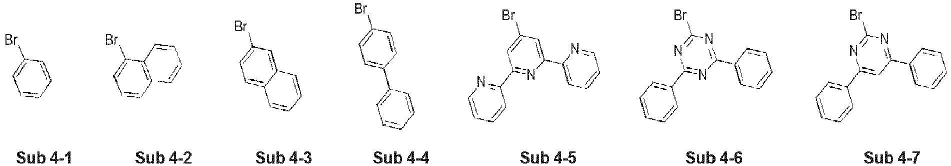

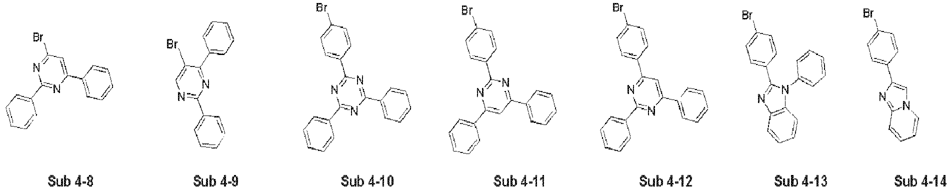

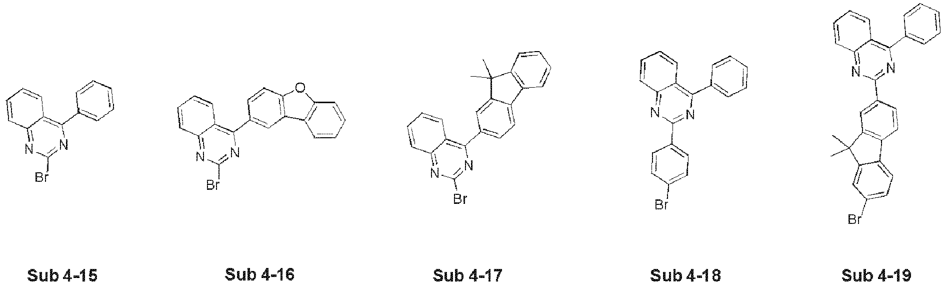

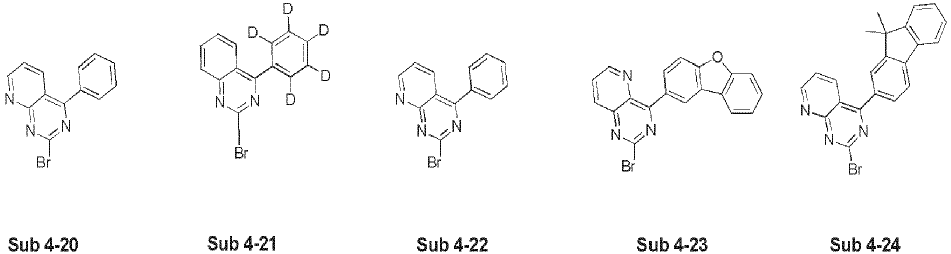

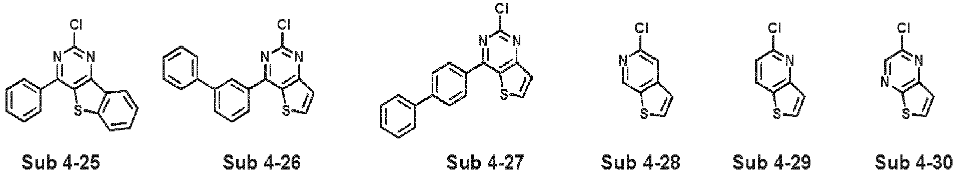

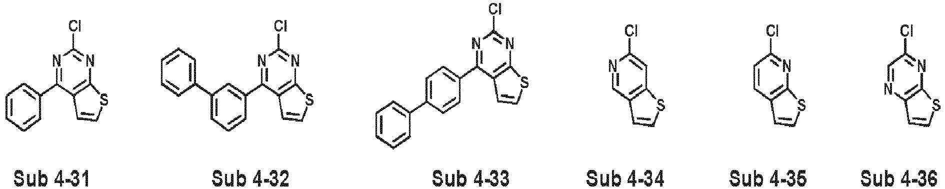

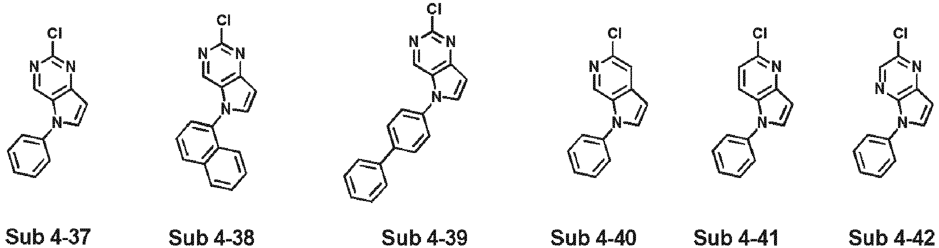

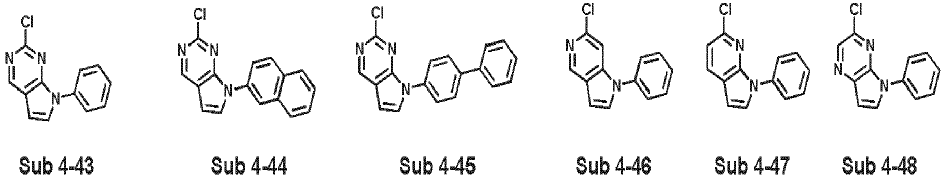

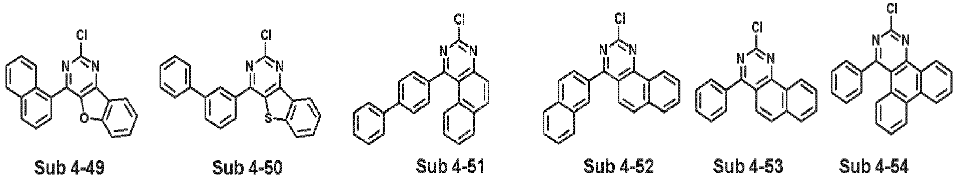

**Table 5**

| compound | FD-MS | compound | FD-MS |
|---|---|---|---|
| Sub 4-1 | m/z=155.96(C₆H₅Br =157.01) | Sub 4-2 | m/z=205.97(C₁₀H₇Br =207.07) |
| Sub 4-3 | m/z=205.97(C₁₀H₇Br =207.07) | Sub 4-4 | m/z=231.99(C₁₂H₉Br =233.10) |
| Sub 4-5 | m/z=309.02(C₁₇H₁₂BrN =310.19) | Sub 4-6 | m/z=311.01(C₁₅H₁₀BrN₃ =312.16) |
| Sub 4-7 | m/z=310.01(C₁₆H₁₁BrN₂ =311.18) | Sub 4-8 | m/z=310.01(C₁₆H₁₁BrN₂ =311.18) |
| Sub 4-9 | m/z=310.01(C₁₆H₁₁BrN₂ =311.18) | Sub 4-10 | m/z=387.04(C₂₁H1₄BrN₃ =388.26) |
| Sub 4-11 | m/z=386.04(C₂₂H₁₅BrN₂ =387.27) | Sub 4-12 | m/z=386.04(C₂₂H₁₅BrN₂ =387.27) |
| Sub 4-13 | m/z=348.03(C₁₉H₁₃BrN₂ =349.22) | Sub 4-14 | m/z=271.99(C₁₃H₉BrN₂ =273.13) |
| Sub 4-15 | m/z=283.99(C₁₄H₉BrN₂=285. 14) | Sub 4-16 | m/z=374.01(C₂₀H₁₁BrN₂0=375.22) |
| Sub 4-17 | m/z=400.06(C₂₃H₁₇BrN₂=401.30) | Sub 4-18 | m/z=360.03(C₂₀H₁₃BrN₂=361.23) |
| Sub 4-19 | m/z=476.09(C₂₉H₂₁BrN₂=477.39) | Sub 4-20 | m/z=284.99(C₁₃H₈BrN₃=286.13) |
| Sub 4-21 | m/z=289.03(C₁₄H₄D₅BrN₂=290.2) | Sub 4-22 | m/z=284.99(C₁₃H₈BrN₃=286.13) |
| Sub 4-23 | m/z=375.00(C₁₉H₁₀BrN₃0=376.2) | Sub 4-24 | m/z=401.05(C₂₂H₁₆BrN₃=402.29) |
| Sub 4-25 | m/z=296.02(C₁₆H₉ClN₂S=296.77) | Sub 4-26 | m/z=322.03(C₁₈HnClN₂S =322.81) |
| Sub 4-27 | m/z=322.03(C₁₈H₁₁ClN₂S=322.81) | Sub 4-28 | m/z=168.98(C₇H₄ClNS=169.63) |
| Sub 4-29 | m/z=168.98(C₇H₄ClNS=169.63)) | Sub 4-30 | m/z=169.97(C₆H₃ClN₂S=170.62) |
| Sub 4-31 | m/z=246.00 (C₁₂H₇ClN₂S=246.72) | Sub 4-32 | m/z=322.03(C₁aH₁₁ClN₂S=322.81) |
| Sub 4-33 | m/z=322.03(C₁₈H₁₁ClN₂S=322.81) | Sub 4-34 | m/z=168.98(C₇H₄ClNS=169.63) |
| Sub 4-35 | m/z=168.98(C₇H₄ClNS=169.63)) | Sub 4-36 | m/z=169.97(C₆H₃ClN₂S=170.62) |
| Sub 4-37 | m/z=229.04(C₁₂H₈ClN₃=229.67) | Sub 4-38 | m/z=279.06(C₁₆H₁₀ClN₃=279.72) |
| Sub 4-39 | m/z=305.07(C₁₈H₁₂ClN₃=305.76) | Sub 4-40 | m/z=228.05(C₁₃H₉ClN₂=228.68) |
| Sub 4-41 | m/z=228.05(C₁₃H₉ClN₂=228.68) | Sub 4-42 | m/z=229.04(C₁₂H₈ClN₃=229.67) |
| Sub 4-43 | m/z=229.04(C₁₂H₈ClN₃=229.67) | Sub 4-44 | m/z=279.06CC₁₆H₁₀ClN₃=279.72) |
| Sub 4-45 | m/z=305.07(C₁₈H₁₂ClN₃=305.76) | Sub 4-46 | m/z=228.05(C₁₃H₉ClN₂=228.68) |
| Sub 4-47 | m/z=228.05(C₁₃H₉ClN₂=228.68) | Sub 4-48 | m/z=229.04(C₁₂H₈ClN₃=229.67) |
| Sub 4-49 | m/z=330. 1(C₂₀H₁₁ClN₂0=330.77) | Sub 4-50 | m/z=372.05(C₂₂Hₗ₃ClN₂S=372.87) |
| Sub 4-51 | m/z=366.09(C₂₄H₁₅ClN₂=366.85) | Sub 4-52 | m/z=340.08(C₂₂H₁₃ClN₂=340.81) |
| Sub 4-53 | m/z=290.06(C1₈H₁₁ClN₂=290.75) | Sub 4-54 | m/z=340.08(C₂₂H₁₃ClN₂=340.81) |

### Synthesis Examples of Final products 2

### Synthesis example of 3-6

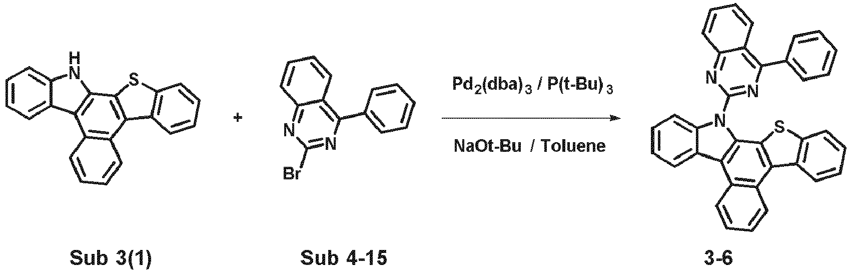

After Sub 3(1) (15.3 g, 47.3 mmol) was added in a round bottom flask and dissolved in toluene (500 mL), Sub 4-15 (14.8 g, 52.0 mmol), Pd₂(dba)₃ (2.4 g, 2.6 mmol), P(t-Bu)₃ (1.1g, 5.2mmol), NaOt-Bu (15 g, 156.1 mmol) were added and stirred at 100 ° C. After the reaction was completed, the reaction mixture was extracted with CH₂Cl₂ and water. The organic layer was dried over MgSO₄ and concentrated. The resulting compound was separated by silicagel column chromatography and recrystallized to obtain 17.0 g of the product (yield:68%).

### Synthesis example of 3-1

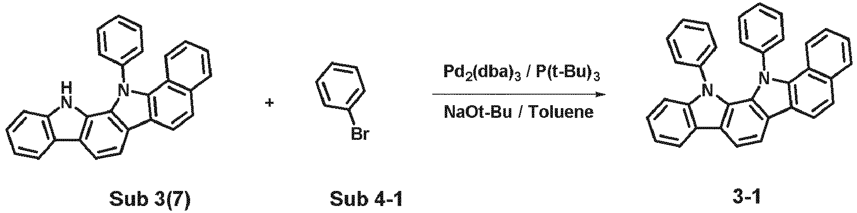

Sub 3(7) (18.1 g, 47.3 mmol), toluene (500 mL), Sub 4-1 (8.2 g, 52.0 mmol), Pd₂(dba)₃ (2.0 g, 2.2 mmol), P(t-Bu)₃ (0.9g, 4.4mmol), NaOt-Bu (12.7 g, 132 mmol) were added, the same procedure as described in the synthesis method of the 3-6 was carried out to obtain 15.6 g of the final product. (yield:72 %).

### Synthesis example of 3-7

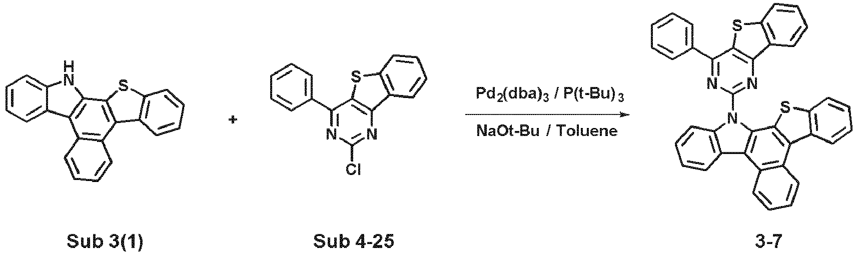

Sub 3(1) (15.3 g, 47.3 mmol), toluene (500 mL), Sub 4-25 (15.4 g, 52.0 mmol), Pd₂(dba)₃ (2.4 g, 2.6 mmol), P(*t*-Bu)₃ (1.05g, 5.2mmol), NaOt-Bu (15 g, 156 mmol) were added, the same procedure as described in the synthesis method of the 3-6 was carried out to obtain 19.3 g of the final product (yield:70 %).

### Synthesis example of 3-8

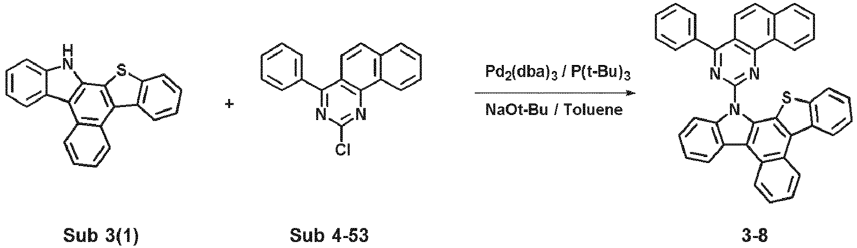

Sub 3(1) (15.3 g, 47.3 mmol), toluene (500 mL), Sub 4-53 (15.1 g, 52 mmol), Pd₂(dba)₃ (2.4 g, 2.6 mmol), P(*t*-Bu)₃ (1.05g, 5.2mmol), NaOt-Bu (15 g, 156 mmol) were added, the same procedure as described in the synthesis method of the 3-6 was carried out to obtain 19.4 g of the final product. (yield:71 %).

### Synthesis example of 3-11

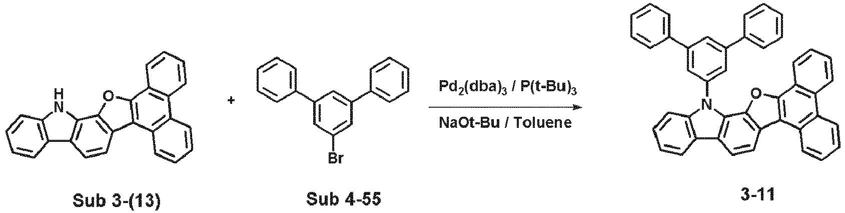

Sub 3(13) (16.9 g, 47.3 mmol), toluene (500 mL), Sub 4-55 (16.1 g, 52.0 mmol), Pd₂(dba)₃ (2.4 g, 2.6 mmol), P(*t*-Bu)₃ (1.05g, 5.2mmol), NaOt-Bu (15 g, 156 mmol) were added, the same procedure as described in the synthesis method of the 3-6 was carried out to obtain 20.2 g of the final product.
(yield:73 %).

### Synthesis example of 3-16

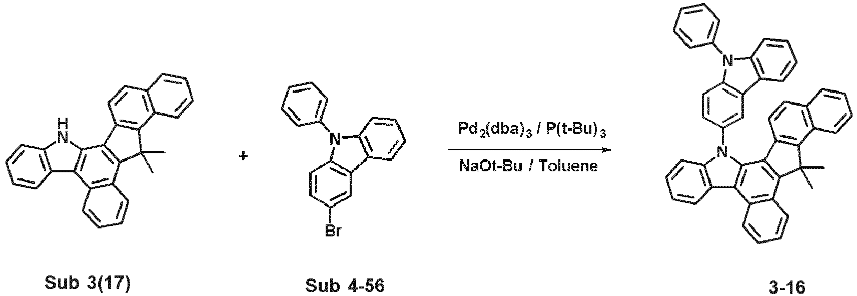

Sub 3(17) (18.1 g, 47.2 mmol), toluene (500 mL), Sub 4-56 (16.7 g, 52.0 mmol), Pd₂(dba)₃ (2.4 g, 2.6 mmol), P(*t*-Bu)₃ (1.05g, 5.2mmol), NaOt-Bu (15 g, 156 mmol) were added, the same procedure as described in the synthesis method of the 3-6 was carried out to obtain 19.8 g of the final product.
(yield:67 %).

### Synthesis example of 3-17

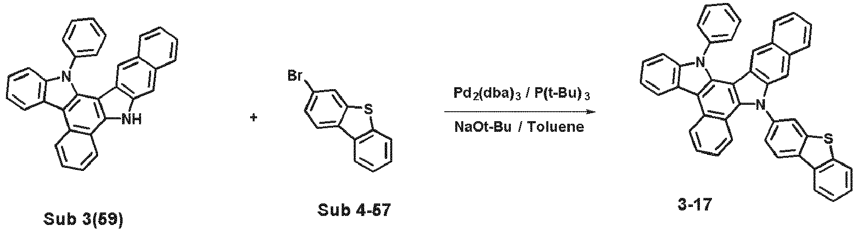

Sub 3(59) (20.5 g, 47.4 mmol), toluene (500 mL), Sub 4-57 (13.7 g, 52.0 mmol), Pd₂(dba)₃ (2.4 g, 2.6 mmol), P(*t*-Bu)₃ (1.05g, 5.2mmol), NaOt-Bu (15 g, 156 mmol) were added, the same procedure as described in the synthesis method of the 3-6 was carried out to obtain 20.1 g of the final product.
(yield:69 %)

### Synthesis example of 3-47

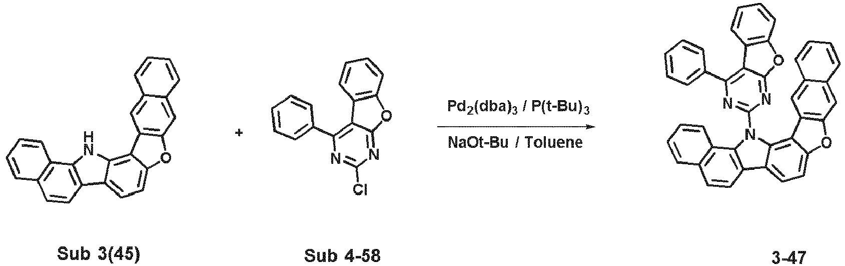

Sub 3(45) (16.9 g, 47.3 mmol), toluene (500 mL), Sub 4-58 (14.6 g, 52.0 mmol), Pd₂(dba)₃ (2.4 g, 2.6 mmol), P(*t*-Bu)₃ (1.05g, 5.2mmol), NaOt-Bu (15 g, 156 mmol) were added, the same procedure as described in the synthesis method of the 3-6 was carried out to obtain 20.5 g of the final product.
(yield:72 %).

### Synthesis example of 3-52

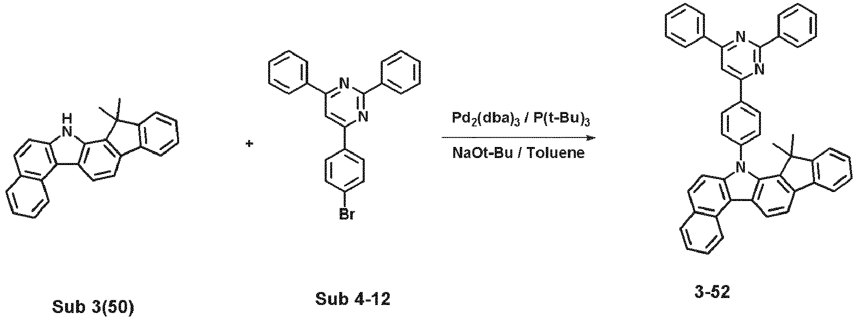

Sub 3(50) (15.8 g, 47.4 mmol), toluene (500 mL), Sub 4-12 (20.2 g, 52.0 mmol), Pd₂(dba)₃ (2.4 g, 2.6 mmol), P(*t*-Bu)₃ (1.05g, 5.2mmol), NaOt-Bu (15 g, 156 mmol) were added, the same procedure as described in the synthesis method of the 3-6 was carried out to obtain 20.0 g of the final product.
(yield:66 %).

### Synthesis example of 3-70

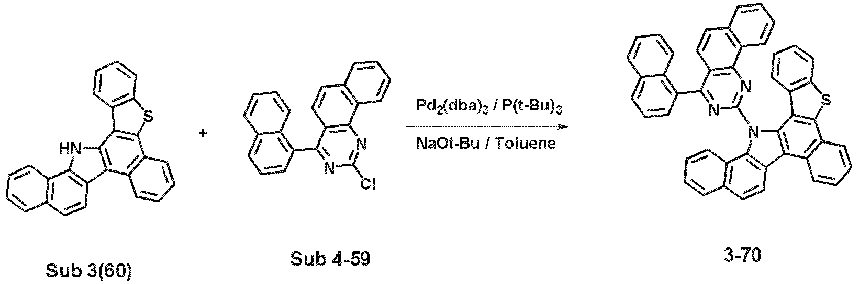

Sub 3(60) (17.7 g, 47.4 mmol), toluene (500 mL), Sub 4-59 (17.8 g, 52.0 mmol), Pd₂(dba)₃ (2.4 g, 2.6 mmol), P(*t*-Bu)₃ (1.05g, 5.2mmol), NaOt-Bu (15 g, 156 mmol) were added, the same procedure as described in the synthesis method of the 3-6 was carried out to obtain 22.8 g of the final product.
(yield:71 %).

**Table 6**

| compound | FD-MS | compound | FD-MS |
|---|---|---|---|
| 3-1 | m/z=458.18(C₃₄H₂₂N₂=458.56) | 3-2 | m/z=449.12(C₃₂H₁₉NS=449.57) |
| 3-3 | m/z=433.15(C₃₂H₁₉NO=433.51) | 3-4 | m/z=535.23(C₄₁H₂₉N=535.69) |
| 3-5 | m/z=399.11(C₂₈H₁₇NS=399.51) | 3-6 | m/z=527.15(C₃₆H₂₁N₃S=527.65) |
| 3-7 | m/z=583.12(C₃₈H₂₁N₃S₂=583.73) | 3-8 | m/z=577.16(C₄₀H₂₃N₃S=577.71) |
| 3-9 | m/z=627.18(C₄₄H₂₅N₃S=627.77) | 3-10 | m/z=475.14(C₃₄H₂₁NS=475.61) |
| 3-11 | m/z=585.21(C₄₄H₂₇NO=585.71) | 3-12 | m/z=509.21(C₃₉H₂₇N=509.65) |
| 3-13 | m/z=509.19(C₃₇H₂₃N₃=509.61) | 3-14 | m/z=451..11(C₃₀H₁₇N₃S=451.55) |
| 3-15 | m/z=588.20(C₄₁H₂₄N₄O=588.67) | 3-16 | m/z=624.26(C₄₇H₃₂N₂=624.79) |
| 3-17 | m/z=614.18(C₄₄H₂₆N₂S=614.77) | 3-18 | m/z=449.12(C₃₂H₁₉NS=449.57) |
| 3-19 | m/z=573.17(C₄₂H₂₃NO₂=573.65) | 3-20 | m/z=664.26(C₄₈H₃₂N₄=664.81) |
| 3-21 | m/z=624.26(C₄₇H₃₂N₂=624.79) | 3-22 | m/z=603.18(C₄₂H₂₅N₃S=603.74) |
| 3-23 | m/z=664.23(C₄₇H₂₈N₄O=664.77) | 3-24 | m/z=737.28(C₅₅H₃₅N₃=737.91) |
| 3-25 | m/z=738.28(C₅₄H₃₄N₄=738.89) | 3-26 | m/z= 679.21(C₄₈H₂₉N₃S=679.84) |
| 3-27 | m/z=625.22(C₄₅H₂₇N₃O=625.73) | 3-28 | m/z=575.24(C₄₂H₂₉N₃=575.72) |
| 3-29 | m/z=508.19(C₃₈H₂₄N₂=508.62) | 3-30 | m/z=449.12(C₃₂H₁₉NS=449.57) |
| 3-31 | m/z=433.15(C₃₂H₁₉NO=433.51) | 3-32 | m/z=531.20(C₄₁H₂₅N=531.66) |
| 3-33 | m/z=608.23(C₄₆H₂₈N₂=608.74) | 3-34 | m/z=475.14(C₃₄H₂₁NS=475.61) |
| 3-35 | m/z=384.13(C₂₇H₁₆N₂O=384.44) | 3-36 | m/z=614.25(C₄₄H₃₀N₄=614.75) |
| 3-37 | m/z=508.19(C₃₈H₂₄N₂=508.62) | 3-38 | m/z=449.12(C₃₂H₁₉NS=449.57) |
| 3-39 | m/z=433.15(C₃₂H₁₉NO=433.51) | 3-40 | m/z=459.20(C₃₅H₂₅N=459.59) |
| 3-41 | m/z=663.24(C₄₇H₂₉N₅=663.78) | 3-42 | m/z=603.18(C₄₂H₂₅N₃S=603.74) |
| 3-43 | m/z=587.20(C₄₂H₂₅N₃O=587.68) | 3-44 | m/z=613.25(C₄₅H₃₁N₃=613.76) |
| 3-45 | m/z=662.25(C₄₈H₃₀N₄=662.80) | 3-46 | m/z=577.16(C₄₀H₂₃N₃S=577.71) |
| 3-47 | m/z=601.18(C₄₂H₂₃N₃O₂=601.67) | 3-48 | m/z=759.27(C₅₇H₃₃N₃=759.91) |
| 3-49 | m/z=589.22(C₄₂H₂₆N₄=586.70) | 3-50 | m/z=630.19(C₄₃H₂₆N₄S=630.77) |
| 3-51 | m/z=613.22(C₄₄H₂₇N₃O=613.72) | 3-52 | m/z=639.27(C₄₇H₃₃N₃=639.80) |
| 3-53 | m/z=508.19(C₃₈H₂₄N₂=508.62) | 3-54 | m/z=449.12(C₃₂H₁₉NS=449.57) |
| 3-55 | m/z=433.15(C₃₂H₁₉NO=433.51) | 3-56 | m/z=609.25(C₄₇H₃₁N=609.77) |
| 3-57 | m/z=663.24(C₄₇H₂₉N₅=663.78) | 3-58 | m/z=604.17(C₄₁H₂₄N₄S=604.73) |
| 3-59 | m/z=587.20(C₄₂H₂₅N₃O=587.68) | 3-60 | m/z613.25(C₄₅H₃₁N₃=613.76) |
| 3-61 | m/z=527.15(C₃₆H₂₄N₃S=527.65) | 3-62 | m/z=603.18(C₄₂H₂₅N₃S=603.74) |
| 3-63 | m/z=511.17(C₃₆H₂₁N₃O=511.58) | 3-64 | m/z=587.24(C₄₃H₂₉N₃=587.73) |
| 3-65 | m/z=692.20(C₄₈H₂₈N₄S=692.84) | 3-66 | m/z=577.16(C₄₀H₂₃N₃S=577.71) |
| 3-67 | m/z=561.18(C₄₀H₂₃N₃O=561.64) | 3-68 | m/z=653.19(C₄₆H₂₇N₃S=653.80) |
| 3-69 | m/z=736.26(C₅₄H₃₂N₄=736.88) | 3-70 | m/z=677.19(C₄₈H₂₇N₃S=677.83) |
| 3-71 | m/z=687.23(C₅₀H₂₉N₃O=687.80) | 3-72 | m/z=743.24(C₅₃H₃₃N₃S=743.93) |
| 3-73 | m/z=703.21(C₅₀H₂₉N₃S=703.86) | 3-74 | m/z=603.18(C₄₂H₂₅N₃S=603.74) |
| 3-75 | m/z=735.18(C₅₀H₂₉N₃S₂=735.92) | 3-76 | m/z=759.18(C₅₂H₂₉N₃S₂=759.95) |
| 3-77 | m/z=475.14(C₃₄H₂₁NS=475.61) | 3-78 | m/z=616.20(C₄₄H₂₈N₂S=616.78) |
| 3-79 | m/z=710.16(C₄₇H₂₆N₄S₂=710.87) | 3-80 | m/z=818.25(C₅₈H₃₄N₄S=819.00) |
| 3-81 | m/z=844.27(C₆₀H₃₆N₄S=845.04) | 3-82 | m/z=667.17(C₄₆H₂₅N₃OS=667.79) |
| 3-83 | m/z=703.80(C₅₀H₂₉N₃O₂=703.80) | 3-84 | m/z=629.21(C₄₄H₂₇N₃O₂=629.72) |

Otherwise, the synthesis examples of the present invention represented by the Formulas 1 and 2 have been described, but these are all based on the Buchwald-Hartwig cross coupling reaction, Suzuki cross- coupling reaction, Intramolecular acid-induced cyclization reaction (J. mater. Chem.1999, 9, 2095), Pd(II)-catalyzed oxidative cyclization reaction (Org. Lett.2011, 13, 5504), Grignard reaction, Cyclic Dehydration reaction and PPh3-mediated reductive cyclization reaction (J. Org. Chem. 2005, 70, 5014.), and those skilled in the art will readily understand that the above reaction proceeds even when, besides the substituent specified in the specific synthesis example, other substituents(R¹ to R⁵, Ar¹ to Ar⁴, L¹ to L², X¹ to X²) defined in the Formulas 1 and 2 are bonded.

For example, Sub 1 + Sub 2 -> Final Products 1 reaction in Reaction Scheme 1, the synthetic reaction of Sub 2 in Reaction Scheme 3, Sub 3 + Sub 4 -> Final Products 2 reaction in Reaction Scheme 4 are all based on the Buchwald-Hartwig cross coupling reaction, and Sub 1-3 + Sub 1-4 -> Sub 1 reaction in Reaction Scheme 2 and Sub 3-2 + Sub 3-3 -> Sub 3-4 in Reaction Scheme 5 are all based on the Suzuki cross-coupling reaction, and Sub 3-4 -> Sub 3 in Reaction Scheme 5 is based on the PPh3-mediated reductive cyclization reaction (J. Org. Chem. 2005, 70, 5014.) The above reactions will proceed even if a substituent not specifically mentioned is bonded.

### Evaluation of Manufacture of Organic Electric element

Example 1) Manufacture and evaluation of Red organic light emitting diode First, on an ITO layer(anode) formed on a glass substrate, N¹-(naphthalen-2-yl)-N⁴,N⁴-bis(4-(naphthalen-2-yl(phenyl)amino)phenyl)-N¹-phenyl benzene-1,4-diamine(hereinafter will be abbreviated as 2-TNATA) was vacuum-deposited to form a hole injection layer with a thickness of 60 nm, and N,N' -bis(1-naphthalenyl)-N,N' -bis-phenyl-(1,1' -biphenyl)-4,4' -diamine (hereinafter will be abbreviated as NPB) was vacuum-deposited to form a hole transport layer with a thickness of 60 nm. On the hole transport layer, a mixture of the compounds represented by the Formulas 1 and 2 as a host in a ratio of 3: 7 was used as a host, and as a dopant, a light emitting layer with a thickness of 30 nm was deposited on the hole transport layer by doping (piq)₂Ir(acac) [bis-(1-phenylisoquinolyl)iridium(III)acetylacetonate] with a weight of 95: 5. (1,1' -bisphenyl)-4-olato)bis(2-methyl-8-quinolinolato)aluminum (hereinafter abbreviated as BAlq) was vacuum deposited as a hole blocking layer to a thickness of 10 nm, and tris(8-quinolinol)aluminum (hereinafter abbreviated as Alq3) was deposited to a thickness of 40 nm as an electron transport layer. After that, an alkali metal halide, LiF was vacuum deposited as an electron injection layer to a thickness of 0.2 nm, and Al was deposited to a thickness of 150 nm to form a cathode to manufacture an OLED.

To the OLEDs which were manufactured by examples and comparative examples, a forward bias direct current voltage was applied, and electroluminescent(EL) properties were measured using PR-650 of Photoresearch Co., and T95 life was measured using a life measuring apparatus manufactured by McScience Inc. with a reference luminance of 2500 cd/m². In the following table, the manufacture of a device and the results of evaluation are shown.

### Comparative Examples 1 to 3

An organic electroluminescent device was manufactured in the same manner as in Example 1, except that the compound represented by Formula 2 was used as a host alone.

### Comparative Example 4

An organic electroluminescent device was manufactured in the same manner as in Example 1, except that the comparative compound 1 was used as a host alone.

### Comparative Example 5

An organic electroluminescent device was manufactured in the same manner as in Example 1, except that the comparative compound 2 was used as a host alone.

### Comparative Example 6

An organic electroluminescent device was manufactured in the same manner as in Example 1, except that the comparative compound 3 was used as a host alone.

### Comparative Example 7

An organic electroluminescent device was manufactured in the same manner as in Example 1, except that the comparative compound 4 was used as a host alone.

### Comparative Example 8

An organic electroluminescent device was manufactured in the same manner as in Example 1, except that comparative compound 1 and comparative compound 2 were mixed and used as a host.

### Comparative Example 9

An organic electroluminescent device was manufactured in the same manner as in Example 1, except that comparative compound 3 and comparative compound 4 were mixed and used as a host.

### Comparative Example 10

An organic electroluminescent device was manufactured in the same manner as in Example 1, except that the compound represented by the Formula 1 and the comparative compound 4 were mixed and used as a host.

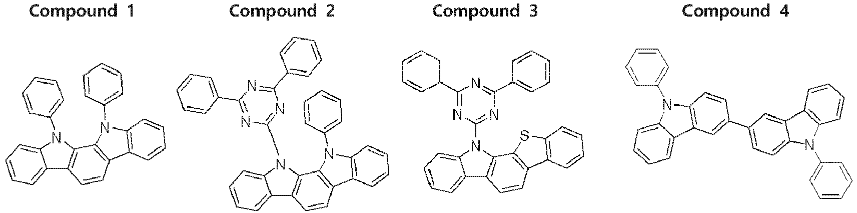

**Table 7**

| | First host | Second host | Voltage | Current Density | Brightness (cd/m²) | Efficie ncy | Lifetime T(95) |
|---|---|---|---|---|---|---|---|
| Comparative example(1) | - | Compound (3-6) | 6.1 | 15.7 | 2500.0 | 15.9 | 108.9 |
| comparative example(2) | - | Compound (3-61) | 6.3 | 16.2 | 2500.0 | 15.5 | 104.2 |
| comparative example(3) | - | Compound (3-74) | 6.4 | 16.2 | 2500.0 | 15.4 | 103.2 |
| comparative example(4) | - | Comparative compound 1- | 6.9 | 18.6 | 2500.0 | 13.4 | 84.3 |
| comparative example(5) | - | Comparative compound 2 | 6.8 | 18.3 | 2500.0 | 13.7 | 83.3 |
| comparative example(6) | - | Comparative compound 3 | 6.7 | 17.5 | 2500.0 | 14.3 | 87.4 |
| comparative example(7) | - | Comparative compound 4 | 6.9 | 18.4 | 2500.0 | 13.6 | 82.9 |
| comparative example(8) | Comparative compound 1 | Comparative compound 2 | 5.9 | 13.0 | 2500.0 | 19.3 | 103.8 |
| comparative example(9) | Comparative compound 3 | Comparative compound 4 | 5.6 | 10.5 | 2500.0 | 23.9 | 108.9 |
| comparative example(10) | compound (2-5) | Comparative compound 3 | 5.4 | 10.0 | 2500.0 | 25.0 | 111.2 |
| example(1) | Compound (1-1) | Compound (3-6) | 4.5 | 6.8 | 2500.0 | 36.6 | 138.3 |
| example(2) | compound (1-6) | Compound (3-6) | 4.6 | 7.1 | 2500.0 | 35.1 | 136.9 |
| example(3) | compound (1-9) | Compound (3-6) | 4.5 | 6.9 | 2500.0 | 36.1 | 136.0 |
| example(4) | compound (1-28) | Compound (3-6) | 4.6 | 7.1 | 2500.0 | 35.2 | 135.5 |
| example(5) | compound (1-54) | Compound (3-6) | 4.6 | 6.9 | 2500.0 | 36.0 | 137.5 |
| example(6) | compound (2-5) | Compound (3-6) | 4.6 | 6.8 | 2500.0 | 36.7 | 135.3 |
| example(7) | compound (2-17) | Compound (3-6) | 4.6 | 7.0 | 2500.0 | 35.5 | 137.0 |
| example(8) | compound (2-40) | compound(3-6) | 4.3 | 7.0 | 2500.0 | 36.0 | 135.2 |
| example(9) | compound (2-41) | compound(3-6) | 4.6 | 6.8 | 2500.0 | 36.6 | 136.6 |
| example(10) | compound (2-49) | compound(3-6) | 4.7 | 6.8 | 2500.0 | 36.8 | 138.1 |
| example(11) | compound (1-1) | compound(3-7) | 5.0 | 7.9 | 2500.0 | 31.7 | 128.3 |
| example(12) | compound (1-6) | compound(3-7) | 4.9 | 8.3 | 2500.0 | 30.3 | 128.4 |
| example(13) | compound (1-9) | compound(3-7) | 4.9 | 8.0 | 2500.0 | 31.1 | 127.3 |
| example(14) | compound (1-28) | compound(3-7) | 4.9 | 8.6 | 2500.0 | 29.0 | 131.1 |
| example(15) | Compound (1-54) | compound(3-7) | 4.8 | 8.7 | 2500.0 | 28.8 | 127.2 |
| example(16) | compound (2-5) | compound(3-7) | 5.0 | 7.9 | 2500.0 | 31.5 | 133.2 |
| example(17) | compound (2-17) | compound(3-7) | 5.0 | 8.1 | 2500.0 | 30.7 | 131.8 |
| example(18) | compound (2-40) | compound(3-7) | 4.8 | 8.5 | 2500.0 | 29.4 | 132.2 |
| example(19) | compound (2-41) | compound(3-7) | 4.9 | 8.6 | 2500.0 | 28.9 | 128.4 |
| example(20) | compound (2-49) | compound(3-7) | 4.9 | 8.7 | 2500.0 | 28.6 | 128.9 |
| example(21) | compound (1-1) | compound(3-8) | 5.0 | 7.8 | 2500.0 | 31.9 | 131.2 |
| example(22) | compound (1-6) | compound(3-8) | 5.0 | 7.8 | 2500.0 | 31.9 | 131.7 |
| example(23) | compound (1-9) | compound(3-8) | 4.9 | 8.4 | 2500.0 | 29.7 | 131.2 |
| example(24) | compound (1-28) | compound(3-8) | 4.9 | 8.4 | 2500.0 | 29.9 | 131.9 |
| example(25) | compound (1-54) | compound(3-8) | 4.9 | 8.4 | 2500.0 | 29.7 | 133.9 |
| example(26) | Compound (2-5) | compound(3-8) | 4.8 | 7.9 | 2500.0 | 31.7 | 131.3 |
| example(27) | compound (2-17) | compound(3-8) | 5.0 | 8.5 | 2500.0 | 29.3 | 125.7 |
| example(28) | Compound (2-40) | compound(3-8) | 4.9 | 8.0 | 2500.0 | 31.1 | 134.5 |
| example(29) | compound (2-41) | compound(3-8) | 4.9 | 8.1 | 2500.0 | 31.0 | 128.7 |
| example(30) | compound (2-49) | compound(3-8) | 5.0 | 8.1 | 2500.0 | 30.9 | 129.5 |
| example(31) | compound (1-1) | compound(3-9) | 4.9 | 8.0 | 2500.0 | 31.2 | 128.1 |
| example(32) | compound (1-6) | Compound (3-9) | 4.8 | 8.3 | 2500.0 | 30.2 | 130.9 |
| example(33) | Compound (1-9) | Compound (3-9) | 4.9 | 8.2 | 2500.0 | 30.3 | 126.2 |
| example(34) | Compound (1-28) | Compound (3-9) | 4.9 | 7.9 | 2500.0 | 31.8 | 130.3 |
| example(35) | compound (1-54) | Compound (3-9) | 4.8 | 8.0 | 2500.0 | 31.2 | 131.1 |
| example(36) | Compound (2-5) | Compound (3-9) | 4.8 | 8.9 | 2500.0 | 28.0 | 131.1 |
| example(37) | Compound (2-17) | Compound (3-9) | 4.8 | 8.6 | 2500.0 | 29.2 | 133.3 |
| example(38) | Compound (2-40) | Compound (3-9) | 4.8 | 8.6 | 2500.0 | 29.2 | 129.7 |
| example(39) | compound (2-41) | Compound (3-9) | 4.9 | 8.1 | 2500.0 | 30.7 | 134.8 |
| example(40) | compound (2-49) | Compound (3-9) | 5.0 | 8.9 | 2500.0 | 28.1 | 133.9 |
| example(41) | compound (1-1) | Compound (3-15) | 4.8 | 8.0 | 2500.0 | 31.3 | 127.3 |
| example(42) | compound (1-6) | Compound (3-15) | 4.8 | 8.7 | 2500.0 | 28.8 | 134.7 |
| example(43) | compound (1-9) | Compound (3-15) | 4.9 | 8.7 | 2500.0 | 28.9 | 125.2 |
| example(44) | compound (1-28) | Compound (3-15) | 4.9 | 8.6 | 2500.0 | 29.0 | 130.9 |
| example(45) | Compound (1-54) | Compound (3-15) | 4.8 | 8.0 | 2500.0 | 31.3 | 133.9 |
| example(46) | Compound (2-5) | Compound (3-15) | 5.0 | 8.3 | 2500.0 | 30.3 | 125.0 |
| example(47) | Compound (2-17) | Compound (3-15) | 4.9 | 8.8 | 2500.0 | 28.4 | 132.2 |
| example(48) | Compound (2-40) | Compound (3-15) | 4.8 | 8.8 | 2500.0 | 28.4 | 126.1 |
| example(49) | compound (2-41) | Compound (3-15) | 4.9 | 8.6 | 2500.0 | 29.2 | 125.0 |
| example(50) | compound (2-49) | Compound (3-15) | 5.0 | 8.1 | 2500.0 | 31.0 | 125.6 |
| example(51) | compound (1-1) | Compound (3-37) | 4.9 | 8.7 | 2500.0 | 28.8 | 134.2 |
| example(52) | compound (1-6) | Compound (3-37) | 4.8 | 8.9 | 2500.0 | 28.1 | 125.7 |
| example(53) | compound (1-9) | Compound (3-37) | 4.8 | 8.0 | 2500.0 | 31.2 | 127.5 |
| example(54) | Compound (1-28) | Compound (3-37) | 4.9 | 7.8 | 2500.0 | 31.9 | 131.2 |
| example(55) | compound (1-54) | Compound (3-37) | 4.9 | 8.9 | 2500.0 | 28.2 | 126.7 |
| example(56) | compound (2-5) | Compound (3-37) | 4.9 | 8.4 | 2500.0 | 29.9 | 127.2 |
| example(57) | compound (2-17) | Compound (3-37) | 4.8 | 8.4 | 2500.0 | 29.9 | 126.1 |
| example(58) | Compound (2-40) | Compound (3-37) | 4.8 | 8.1 | 2500.0 | 30.7 | 133.4 |
| example(59) | Compound (2-41) | Compound (3-37) | 5.0 | 7.9 | 2500.0 | 31.7 | 135.0 |
| example(60) | compound (2-49) | Compound (3-37) | 4.8 | 7.9 | 2500.0 | 31.5 | 125.0 |
| example(61) | Compound (1-1) | Compound (3-46) | 4.8 | 7.9 | 2500.0 | 31.6 | 130.3 |
| example(62) | compound (1-6) | Compound (3-46) | 4.8 | 8.3 | 2500.0 | 30.1 | 133.6 |
| example(63) | compound (1-9) | Compound (3-46) | 5.0 | 7.8 | 2500.0 | 31.9 | 128.4 |
| example(64) | Compound (1-28) | compound(3-46) | 4.9 | 7.8 | 2500.0 | 32.0 | 126.5 |
| example(65) | compound (1-54) | Compound (3-46) | 5.0 | 8.0 | 2500.0 | 31.2 | 125.3 |
| example(66) | compound (2-5) | Compound (3-46) | 5.0 | 8.2 | 2500.0 | 30.4 | 126.4 |
| example(67) | compound (2-17) | Compound (3-46) | 4.9 | 8.3 | 2500.0 | 30.2 | 130.4 |
| example(68) | compound (2-40) | Compound (3-46) | 4.7 | 8.6 | 2500.0 | 29.2 | 131.8 |
| example(69) | compound (2-41) | Compound (3-46) | 4.9 | 8.7 | 2500.0 | 28.8 | 133.6 |
| example(70) | Compound (2-49) | Compound (3-46) | 4.9 | 8.2 | 2500.0 | 30.6 | 133.3 |
| example(71) | compound (1-1) | Compound (3-50) | 4.9 | 8.6 | 2500.0 | 29.2 | 125.8 |
| example(72) | Compound (1-6) | Compound (3-50) | 5.0 | 8.8 | 2500.0 | 28.5 | 134.5 |
| example(73) | compound (1-9) | Compound (3-50) | 5.0 | 8.2 | 2500.0 | 30.4 | 132.2 |
| example(74) | compound (1-28) | Compound (3-50) | 4.9 | 8.9 | 2500.0 | 28.1 | 126.0 |
| example(75) | compound (1-54) | Compound (3-50) | 5.0 | 7.8 | 2500.0 | 31.9 | 129.3 |
| example(76) | compound (2-5) | Compound (3-50) | 5.0 | 8.6 | 2500.0 | 29.0 | 132.3 |
| example(77) | compound (2-17) | Compound (3-50) | 5.0 | 8.2 | 2500.0 | 30.3 | 128.6 |
| example(78) | compound (2-40) | Compound (3-50) | 4.8 | 8.8 | 2500.0 | 28.5 | 134.9 |
| example(79) | compound (2-41) | Compound (3-50) | 4.8 | 8.9 | 2500.0 | 28.1 | 133.1 |
| example(80) | Compound (2-49) | Compound (3-50) | 4.9 | 8.4 | 2500.0 | 29.8 | 130.1 |
| example(81) | compound (1-1) | Compound (3-61) | 4.6 | 7.5 | 2500.0 | 33.3 | 144.5 |
| example(82) | compound (1-6) | Compound (3-61) | 4.7 | 7.5 | 2500.0 | 33.3 | 143.4 |
| example(83) | Compound (1-9) | Compound (3-61) | 4.6 | 7.1 | 2500.0 | 35.0 | 141.7 |
| example(84) | compound (1-28) | Compound (3-61) | 4.5 | 7.2 | 2500.0 | 35.0 | 142.0 |
| example(85) | compound (1-54) | Compound (3-61) | 4.5 | 7.4 | 2500.0 | 33.9 | 142.7 |
| example(86) | Compound (2-5) | compound(3-61) | 4.6 | 7.4 | 2500.0 | 33.8 | 141.2 |
| example(87) | Compound (2-17) | Compound (3-61) | 4.6 | 7.2 | 2500.0 | 34.7 | 144.5 |
| example(88) | Compound (2-40) | Compound (3-61) | 4.4 | 7.2 | 2500.0 | 34.5 | 142.0 |
| example(89) | compound (2-41) | Compound (3-61) | 4.6 | 7.2 | 2500.0 | 34.8 | 144.2 |
| example(90) | compound (2-49) | Compound (3-61) | 4.5 | 7.5 | 2500.0 | 33.3 | 144.7 |
| example(91) | Compound (1-1) | Compound (3-74) | 4.8 | 8.0 | 2500.0 | 31.3 | 130.9 |
| example(92) | Compound (1-6) | Compound (3-74) | 4.9 | 8.1 | 2500.0 | 31.0 | 131.3 |
| example(93) | compound (1-9) | Compound (3-74) | 4.8 | 8.1 | 2500.0 | 31.0 | 129.3 |
| example(94) | compound (1-28) | Compound (3-74) | 4.8 | 8.5 | 2500.0 | 29.5 | 131.2 |
| example(95) | Compound (1-54) | Compound (3-74) | 4.8 | 8.9 | 2500.0 | 28.2 | 130.2 |
| example(96) | compound (2-5) | Compound (3-74) | 4.9 | 8.5 | 2500.0 | 29.4 | 125.8 |
| example(97) | compound (2-17) | Compound (3-74) | 4.8 | 8.2 | 2500.0 | 30.6 | 132.5 |
| example(98) | compound (2-40) | Compound (3-74) | 4.8 | 8.3 | 2500.0 | 30.3 | 127.3 |
| example(99) | Compound (2-41) | Compound (3-74) | 4.9 | 7.8 | 2500.0 | 31.9 | 133.0 |
| example(100) | Compound (2-49) | Compound (3-74) | 4.8 | 8.0 | 2500.0 | 31.4 | 131.9 |

| | | | | | | | |
|---|---|---|---|---|---|---|---|
| TRTP (Time resolved transient PL) measurement experiment | | | | | | | |

**Table 8**

| | First host | Second host | wavelength (nm) | time(ns) |
|---|---|---|---|---|
| Comparative example | 1-54 | - | 423 | 1.64 |
| Comparative example | - | 3-6 | 526 | 9.26 |
| example | 1-54 | 3-6 | 556 | 580 |

As can be seen from the results of Table 7, when the organic electric element material of the present invention represented by the Formulas 1 and 2 is mixed and used as a phosphorescent host(Examples 1 to 100), it was confirmed that the driving voltage, efficiency, and life span were significantly improved as compared with the element(comparative examples 1 to 7) using a single material.

More specifically, in Comparative Examples 1 to 7, the compounds of the present invention represented by the Formula 2 and comparative compounds 1 to 4 are used alone as a phosphorescent host, wherein Comparative Examples 1 to 3 using the compounds (3-6, 3-61, and 3-74) of the present invention had higher efficiency and longer life span than Comparative Examples 4 to 7 using the comparative compound.

Also, Comparative Example 8 and Comparative Example 9 in which Comparative Compound 1 and Comparative Compound 2 or Comparative Compound 3 and Comparative Compound 4 were mixed and used as a phosphorescent host were found to exhibit higher efficiency than Comparative Examples 1 to 7 using the single substance.

Comparing Comparative Example 8 with Comparative Example 9, it was confirmed that Comparative Example 9 using a mixture containing a polycyclic compound having a different heteroatom (N, S) among the 5-membered compounds had higher efficiency than Comparative Example 8 mixed a 5-membered heterocyclic compound having the same nitrogen atom.

Among the compounds of the present invention, comparing Comparative Example 10 using a mixture of Compound 2-5 corresponding to Formula 1 and Comparative Compound 3, and Comparative Example 9 using a mixture of Comparative Compound 3 and Comparative Compound 4, when the comparative compound 3 was commonly used and the biscarbazole substance (Comparative Example 9) and Compound 2-5 corresponding to the present invention compound 1 were used as the host material, it was confirmed that Comparative Example 10 using the compound 2-5 exhibited a higher efficiency and a relatively longer life span.

And it was confirmed that Example 1 to 100 using the mixture of the compound of the Formula 1 and the Formula 2 as a host exhibited remarkably high efficiency and long life span and a low driving voltage than the Comparative Example 1 to 10.

On the basis of the above experimental results, the inventors of the present invention have found that, in the case of a mixture of the substance of the Formula 1 and the substance of the Formula 2, they have novel characteristics other than those for the respective materials, and have measured the PL lifetime using the substance (1-54) of Formula 1, the substance (3-6) of Formula 2, and the mixture (1-54 + 3-6) of the present invention as shown in the Table 8.

As can be seen from the results of the Table 8, it was confirmed that a new PL wavelength (556 nm) was formed when the compounds of Formulas 1 (1-54) and 2 (3-6) were mixed, and the decreasing and disappearing time of the newly formed PL wavelength increased from 62.6 times (3-6 standard) to 360 times (1-54 standard) compared to the reduction and disappearance times of substances 1-54 and 3-6, respectively. It is considered when mixed with the compound of the present invention, not only electrons and holes are moved through the energy level of each substance, but also the efficiency and life span are increased by electron, hole transport or energy transfer by a new region(exciplex) having a new energy level formed due to mixing. As a result, when the mixture of the present invention is used, the mixed thin film is an important example showing exciplex energy transfer and light emitting process.

The reason why the combination of the present invention is superior to Comparative Examples 8 to 10 in which a comparative compound is used as a phosphorescent host is that the high T1 and high LUMO energy values improve the electron blocking ability and allow more holes to be moved to the emitting layer more quickly and easily when a compound represented by the general Formula 1 having a strong hole property is mixed with a polycyclic compound represented by the Formula 2, which is characterized not only by electron but also by hole stability and high T1. As a result, the charge balance in the emitting layer of holes and electrons is increased, so that light emission is well performed inside the emitting layer rather than at the interface of the hole transport layer, and therefore the deterioration in the HTL interface is also reduced, thereby maximizing the driving voltage, efficiency and life span of the device. That is, it is considered that the combination of the Formula 1 and the Formula 2 is electro-chemically synergistic to improve the performance of the entire device.

### Example 2) Manufacture and evaluation of Red organic light emitting diode by Mixing Ratio

**Table 9**

| | First host | Second host | Mixing ratio (first host : second host) | Voltage | Current Density | Brightness (cd/m²) | Efficien cy | Lifeti me T(95) |
|---|---|---|---|---|---|---|---|---|
| example (101) | compound (1-54) | Compound (3-6) | 2:8 | 4.5 | 6.7 | 2500.0 | 37.5 | 141.4 |
| example (102) | compound (1-54) | Compound (3-6) | 3:7 | 4.6 | 6.9 | 2500.0 | 36.0 | 137.5 |
| example (103) | compound (1-54) | Compound (3-6) | 4:6 | 4.5 | 7.3 | 2500.0 | 34.4 | 132.5 |
| example (104) | compound (1-54) | Compound (3-6) | 5:5 | 4.7 | 8.2 | 2500.0 | 30.6 | 123.8 |
| example (105) | compound (2-5) | Compound (3-61) | 2:8 | 4.5 | 7.3 | 2500.0 | 34.1 | 143.5 |
| example (106) | compound (2-5) | Compound (3-61) | 3:7 | 4.6 | 7.4 | 2500.0 | 33.8 | 141.2 |
| example (107) | compound (2-5) | Compound (3-61) | 4:6 | 4.7 | 8.2 | 2500.0 | 30.5 | 135.8 |
| example (108) | compound (2-5) | Compound (3-61) | 5:5 | 5.1 | 8.5 | 2500.0 | 29.4 | 125.1 |

As shown in Table 9, the mixture of the compound of the present invention was measured by fabricating the device in (2:8, 3:7, 4:6, 5:5).

To explain the results in detail, in the result of the mixture of the compound 1-54 and the compound 3-6, the results of the driving voltage, the efficiency and the life span were similarly excellent at 2: 8 and 3: 7, but as the ratio of the first host increases, such as 4: 6 and 5: 5, the results of the driving voltage, the efficiency and the life span are gradually decreased, this was also the same in the result of the mixture of the compound 2-5 and the compound 3-61. This can be explained by the fact that the charge balance in the emitting layer is maximized when an appropriate amount of the compound represented by the Formula 1 having strong hole properties such as 2: 8 and 3: 7 is mixed.

Although exemplary embodiments of the present invention have been described for illustrative purposes, those skilled in the art will appreciate that various modifications, additions and substitutions are possible, without departing from the scope and spirit of the invention as disclosed in the accompanying claims. Therefore, the embodiment disclosed in the present invention is intended to illustrate the scope of the technical idea of the present invention, and the scope of the present invention is not limited by the embodiment. The scope of the present invention shall be construed on the basis of the accompanying claims, and it shall be construed that all of the technical ideas included within the scope of the claims belong to the present invention.

## Claims

1. An organic electric element (100) comprising:
a first electrode (120), second electrode (180), and an organic material layer formed between the first electrode and the second electrode, wherein the organic material layer comprises an emitting layer (150), wherein the emitting layer comprises a first host compound represented by Formula 1 as the phosphorescent light emitting layer and a second host compound represented by Formula 2, 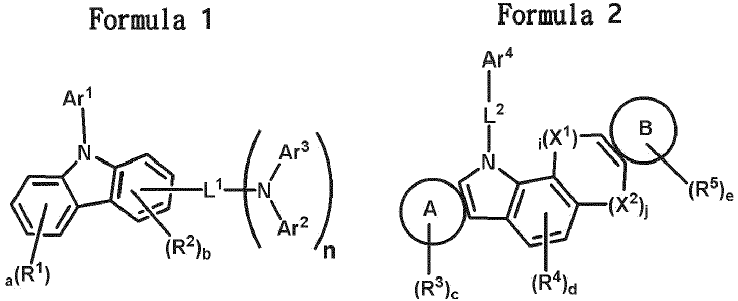 wherein
1) Ar¹, Ar², Ar³, and Ar⁴ are each independently selected from the group consisting of a C₆-C₆₀ aryl group; a fluorenyl group; a C₂-C₆₀ heterocyclic group including at least one hetero atom of O, N, S, Si and P; a fused ring group of a C₃-C₆₀ aliphatic ring and a C₆-C₆₀ aromatic ring: a C₁-C₅₀ alkyl group; a C₂-C₂₀ alkenyl group; a C₂-C₂₀ alkynyl group; a C₁-C₃₀ alkoxyl group; a C₆-C₃₀ aryl oxy group; ,
2) a is an integer of 0 to 4, and b is an integer of 0 to 3, and c and e are integer of 0 to 10, and d is an integer of 0 to 2,
3) R¹, R², R³, R⁴ and R⁵ may be the same or different, and are each independently selected from the group consisting of deuterium; halogen; a C₆-C₆₀ aryl group; a fluorenyl group; a C₂-C₆₀ heterocyclic group including at least one heteroatom of O, N, S, Si or P; a fused ring group of a C₃-C₆₀ aliphatic ring and a C₆-C₆₀ aromatic ring: a C₁-C₅₀ alkyl group; a C₂-C₂₀ alkenyl group; a C₂-C₂₀ alkynyl group; a C₁-C₃₀ alkoxyl group; a C₆-C₃₀ aryloxy group;; or in case a, b, c, and e are 2 or more, and d is 2, R¹, R², R³, R⁴ and R⁵ are each in plural and are the same or different, and a plurality of R¹ or a plurality of R² or a plurality of R³ or a plurality of R⁴ or a plurality of R⁵ may be bonded to each other to form a ring.
4) L¹ and L² are each independently selected from the group consisting of a single bond, a C₆-C₆₀ arylene group, and a fluorenylene group; a fused ring group of a C₃-C₆₀ aliphatic ring and a C₆-C₆₀ aromatic ring; and a C₂-C₆₀ heterocyclic group,
5) A and B are each independently a C₆-C₆₀ aryl group or a C₂-C₂₀ heterocyclic group, wherein both A and B are a substituted or unsubstituted C₆ aryl group (phenyl group), d is 2, and R⁴s are bonded to each other to form a ring to form an aromatic or hetero ring,
6) i and j are independently 0 or 1, with the proviso that i + j is 1 or more, and when i or j is 0, it means a direct bond,
7) X¹ and X² are independently NR', O, S, or CR'R", wherein
R' and R" are independently hydrogen: a C₆-C₆₀ aryl group; a fluorenyl group; a C₃-C₆₀ heterocyclic group; or a C₁-C₅₀ alkyl group; wherein R' and R" may be bonded to each other to form a spiro, and
8) n is an integer of 1 or 2, when n is 2, two Ar² are the same or different, and two Ar³ are each the same or different,
and wherein, aryl group, fluorenyl group, arylene group, heterocyclic group, fused ring group, alkyl group, alkenyl group, alkoxy group and aryloxy group may be substituted with one or more substituents selected from the group consisting of deuterium: halogen; C₁-C₂₀ alkyl group; C₂-C₂₀ alkenyl group; C₂-C₂₀ alkynyl group; C₆-C₂₀ aryl group; C₆-C₂₀ aryl group substituted with deuterium; a fluorenyl group; C₂-C₂₀ heterocyclic group; wherein the substituents may combine each other and form a saturated or unsaturated ring, wherein the term 'ring' means a C₃-C₆₀ aliphatic ring or a C₆-C₆₀ aromatic ring or a C₂-C₆₀ heterocyclic ring or a fused ring formed by the combination thereof.

2. The organic electric element of claim 1, wherein the first host compound represented by the Formula 1 comprises a compound represented by Formula 3 or Formula 4, Formula 21 or Formula 22 below, 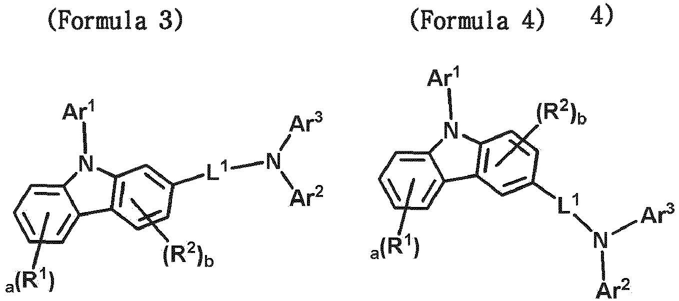 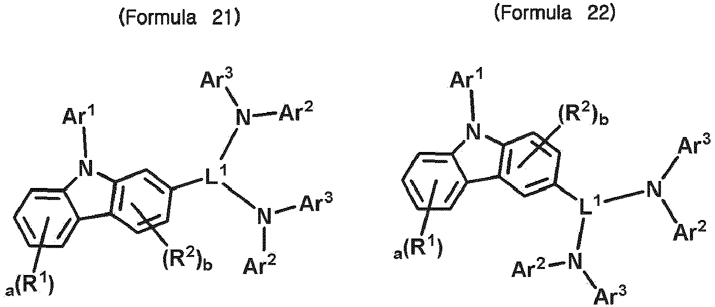 wherein R¹, R², L¹, Ar¹, Ar², Ar³, a and b are the same as defined in the claim 1.

3. The organic electric element of claim 1, wherein L¹ and L² are selected from the group consisting of the following Formulas (A-1) to (A-12), 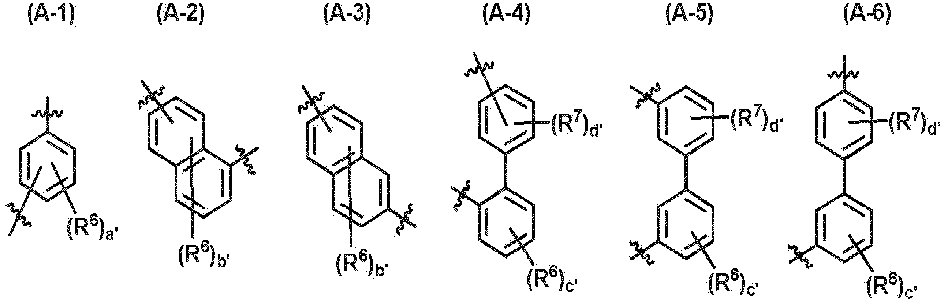 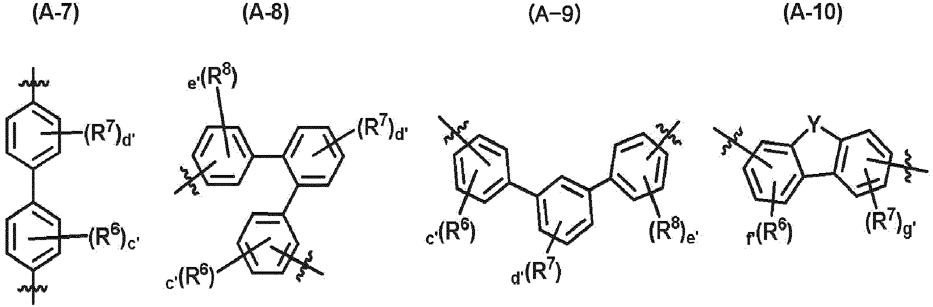 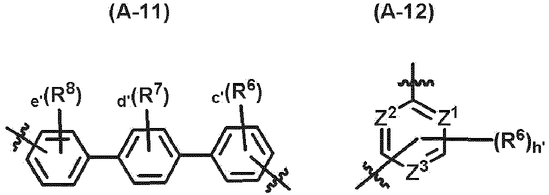 wherein
1) a', c', d' and e' are an integer of 0 to 4, and b' is an integer of 0 to 5, and f' and g' are an integer of 0 to 3, and h' is an integer of 0 to 1,
2) R⁶, R⁷ and R⁸ are the same or different, and are each independently selected from the group consisting of deuterium; halogen; a C₆-C₆₀ aryl group; a fluorenyl group; a C₂-C₆₀ heterocyclic group including at least one heteroatom of O, N, S, Si or P; a fused ring group of a C₃-C₆₀ aliphatic ring and a C₆-C₆₀ aromatic ring: a C₁-C₅₀ alkyl group; a C₂-C₂₀ alkenyl group; a C₂-C₂₀ alkynyl group; a C₁-C₃₀ alkoxyl group; a C₆-C₃₀ aryl oxy group;
or when f 'and g' are two or more, they are the same as or different from each other, and a plurality of R⁶ or a plurality of R⁷ or a plurality of R⁸ may be bonded to each other to form an aromatic or a heteroaromatic ring, two adjacent R⁶ and R⁷, or R⁷ and R⁸ may be bonded to form an aromatic or heteroaromatic ring,
3) Y is NR', O, S or CR'R"
4) R' and R" are the same as defined in the claim 1, and
5) Z¹, Z² and Z³ are independently of each other CR 'or N and at least one is N.

4. The organic electric element of claim 1, wherein the first host compound represented by the Formula 1 is represented by any one of the following Formulas 5 to 20, 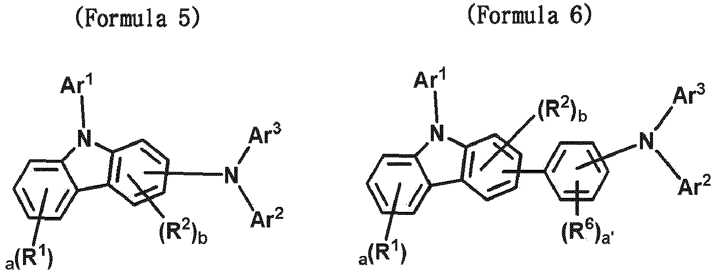 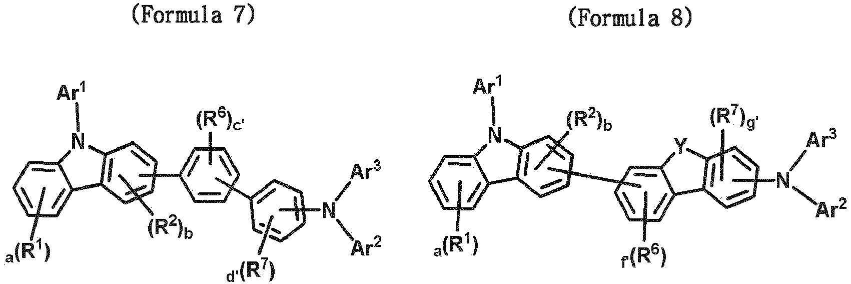 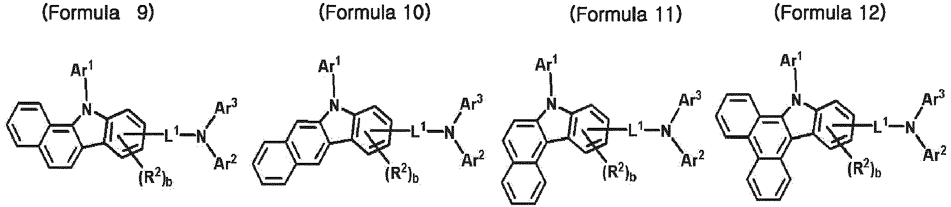 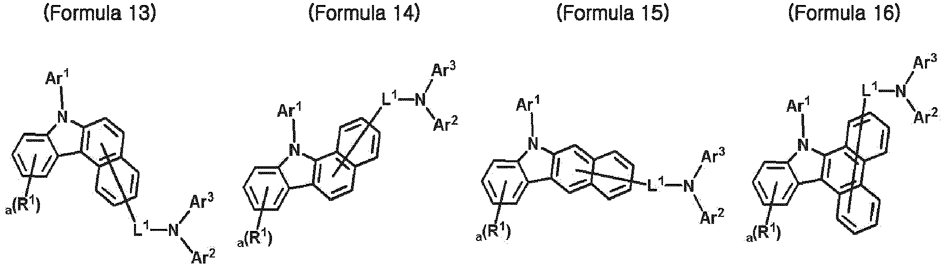 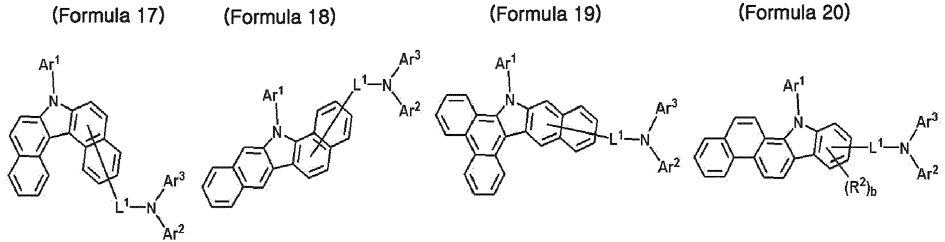 wherein
1) R¹, R², Ar¹, Ar², Ar³, a, b, R' and R" are the same as defined in the claim 1,
2) R⁶ and R⁷ are the same or different, and are each independently selected from the group consisting of deuterium; halogen; a C₆-C₆₀ aryl group; a fluorenyl group; a C₂-C₆₀ heterocyclic group including at least one heteroatom of O, N, S, Si or P; a fused ring group of a C₃-C₆₀ aliphatic ring and a C₆-C₆₀ aromatic ring: a C₁-C₅₀ alkyl group; a C₂-C₂₀ alkenyl group; a C₂-C₂₀ alkynyl group; a C₁-C₃₀ alkoxyl group; a C₆-C₃₀ aryloxy group; or in case f' and g' are 2 or more, each as plurality are the same as or different from each other, and a plurality of R⁶ or a plurality of R⁷ or a plurality of R⁸ or adjacent R⁶ and R⁷, or adjacent R⁷ and R⁸ may be bonded to each other to form an aromatic or a heteroaromatic ring,
3) a', c' and d' are each an integer of 0 to 4, and f' and g' are each an integer of 0 to 3,
4) Y is NR', O, S or CR'R".

5. The organic electric element of claim 1, wherein the first host compound represented by the Formula 1 comprises a compound represented by Formula 23 below, 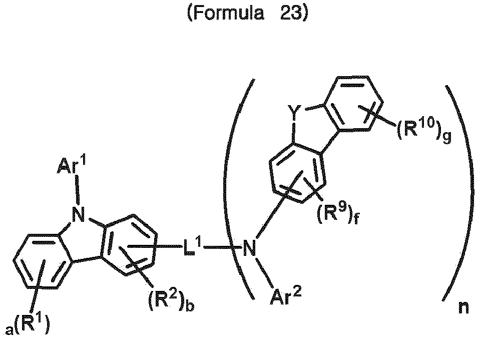 wherein
1) R¹, R², L¹, Ar¹, Ar², a. b and n are the same as defined in the claim 1,
2) f is an integer of 0 to 3, and g is an integer of 0 to 4,
3) R⁹ and R¹⁰ are the same or different, and are each independently selected from the group consisting of deuterium; halogen; a C₆-C₆₀ aryl group; a fluorenyl group; a C₂-C₆₀ heterocyclic group including at least one heteroatom of O, N, S, Si or P; a fused ring group of a C₃-C₆₀ aliphatic ring and a C₆-C₆₀ aromatic ring: a C₁-C₅₀ alkyl group; a C₂-C₂₀ alkenyl group; a C₂-C₂₀ alkynyl group; a C₁-C₃₀ alkoxyl group; a C₆-C₃₀ aryloxy group;or in case f and g are two or more, each as plurality are the same as or different from each other, and a plurality of R⁹ or a plurality of R¹⁰ or adjacent R⁹ and R¹⁰ may be bonded to each other to form an aromatic or a heteroaromatic ring,
4) Y is NR', O, S or CR'R", and
5) R' and R" are the same as defined in the claim 1.

6. The organic electric element of claim 1, wherein the second host compound represented by the Formula 2 comprises a compound represented by Formula 24 or Formula 25 below, 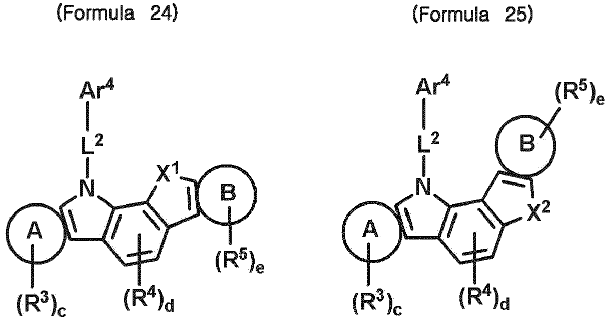 wherein R³, R⁴, R⁵, Ar⁴, L², c, d, e, A, B, X¹ and X² are the same as defined in the claim 1.

7. The organic electric element of claim 1, wherein A and B in the Formula 2 are selected from the group consisting of the following Formulas (B-1) to (B-7), 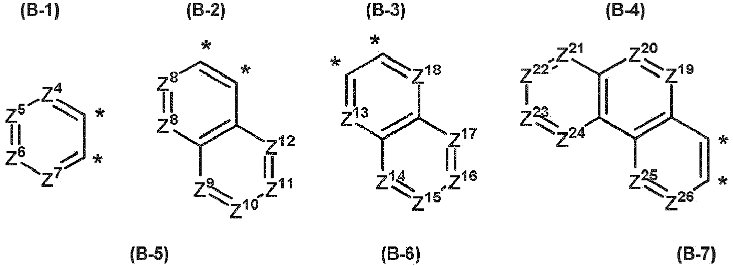 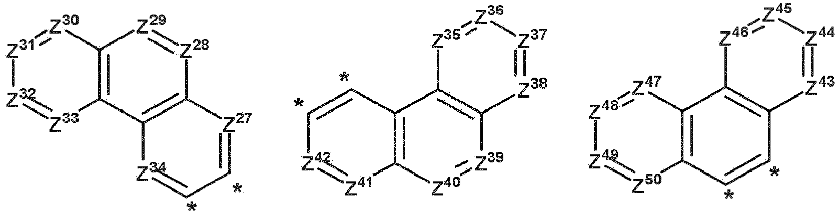 wherein
1) Z⁴ to Z⁵⁰ are CR' or N,
2) R' is the same as defined in the claim 1, and
3) _{*} indicates the position to be condensed.

8. The organic electric element of claim 1, wherein R⁴ in Formula 2 comprises a compound forming an aromatic ring or a heteroaromatic ring.

9. The organic electric element of claim 1, wherein the second host compound represented by Formula 2 comprises a compound represented by any one of the following Formulas 26 to 45 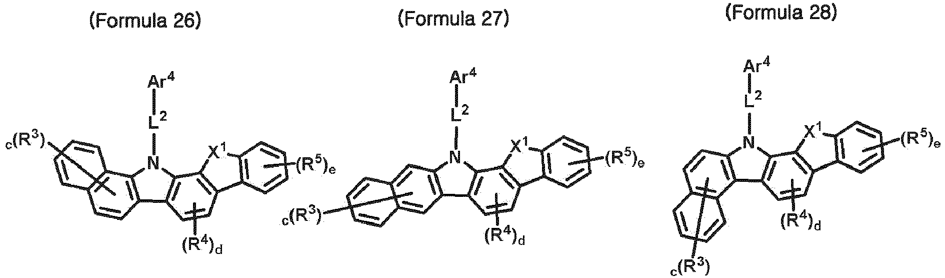 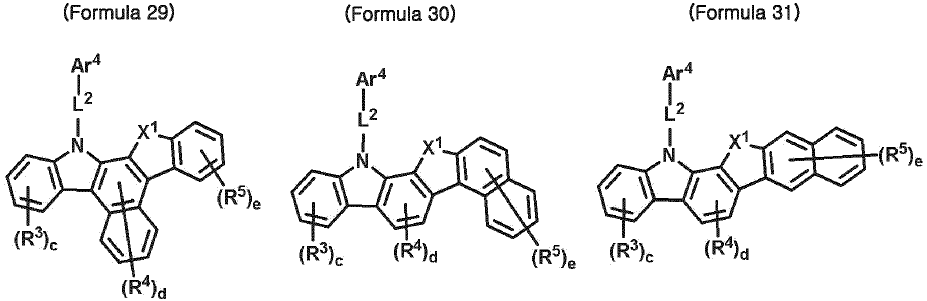 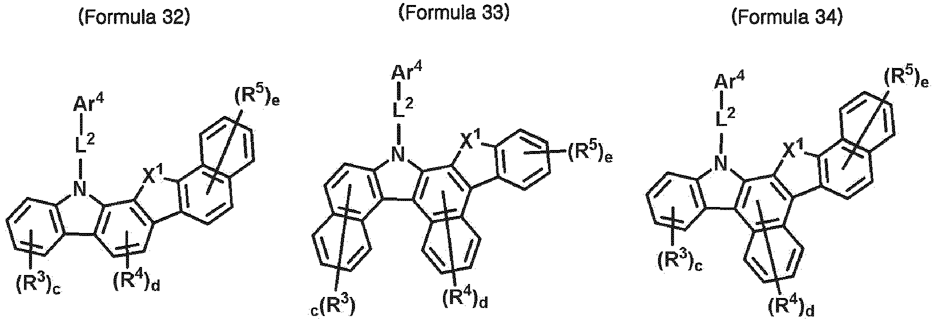 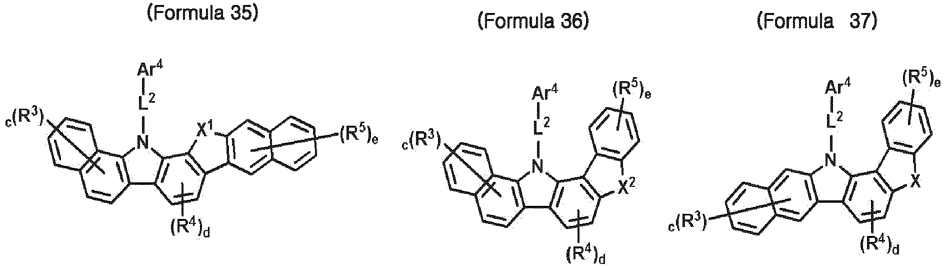 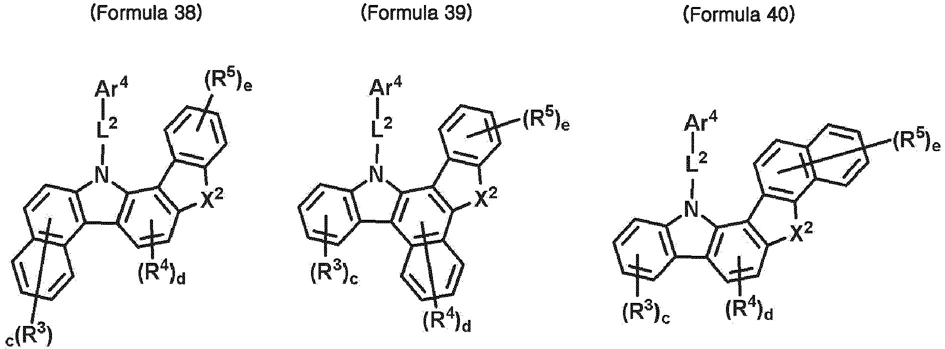 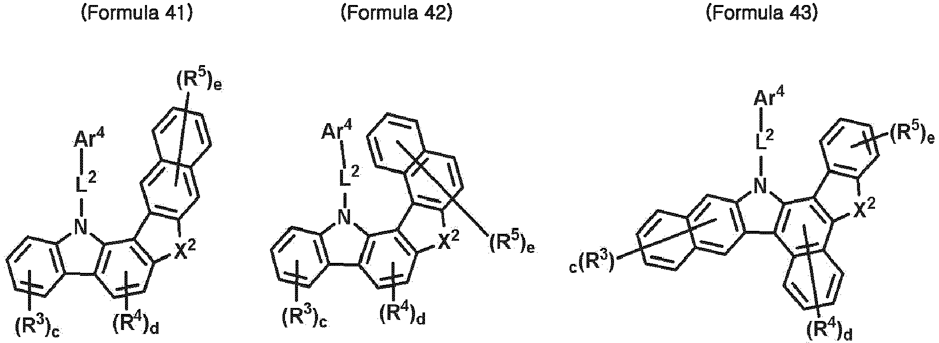 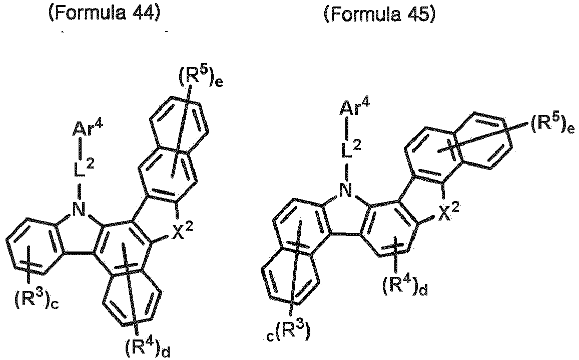 wherein
Ar⁴, X¹, X², R³, R⁴, R⁵, L², c, d, and e are the same as defined in the claim 1.

10. The organic electric element of claim 1, wherein the second host compound represented by Formula 2 comprises any one of compounds represented by Formulas 46 to 53, 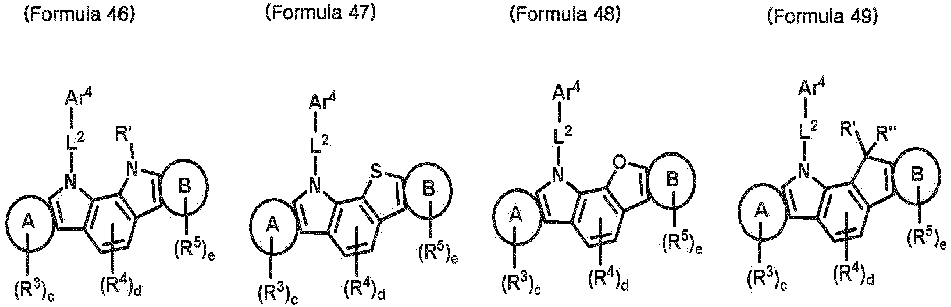 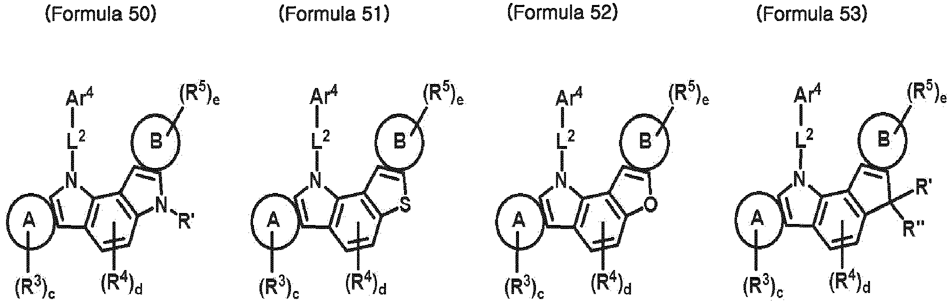 wherein
R³, R⁴, R⁵, Ar⁴, L², c, d, e, A, B, R' and R" are the same as defined in the claim 1.

11. The organic electric element of claim 1, wherein the first host compound represented by Formula 1 is any one of the following Compounds 1-1 to 1-68 and Compounds 2-1 to 2-68

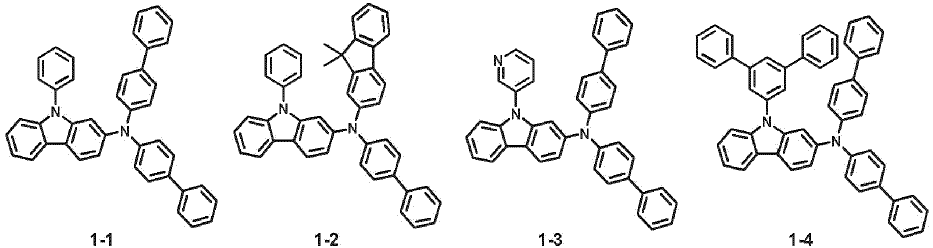

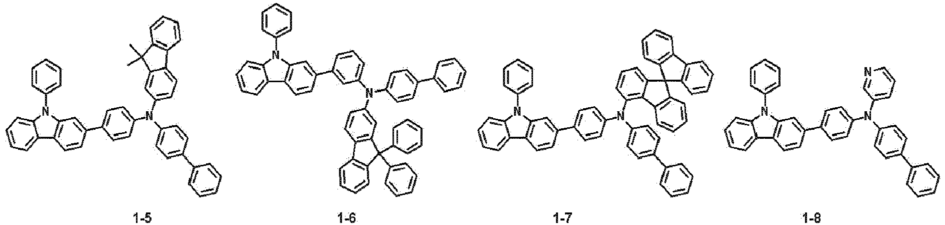

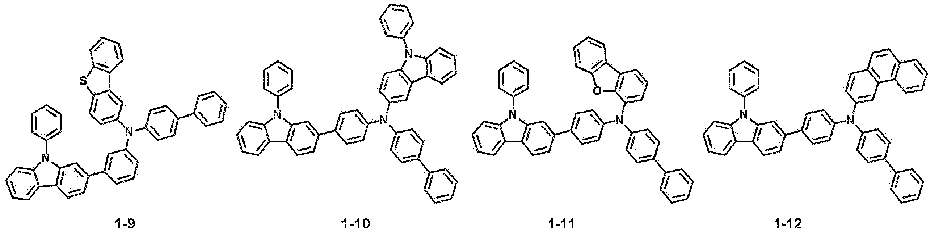

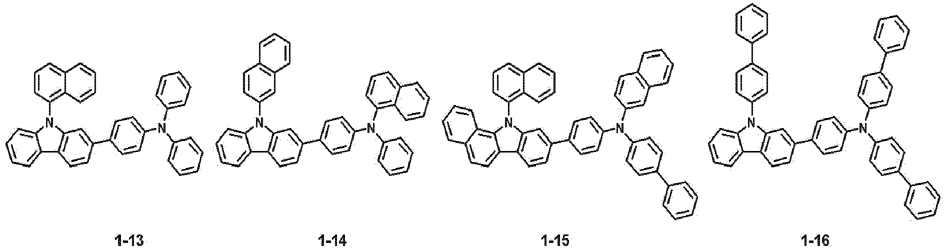

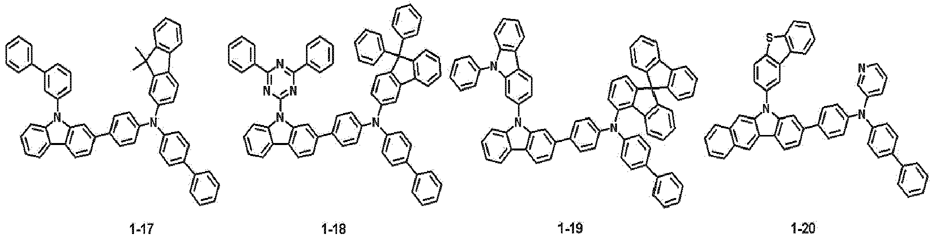

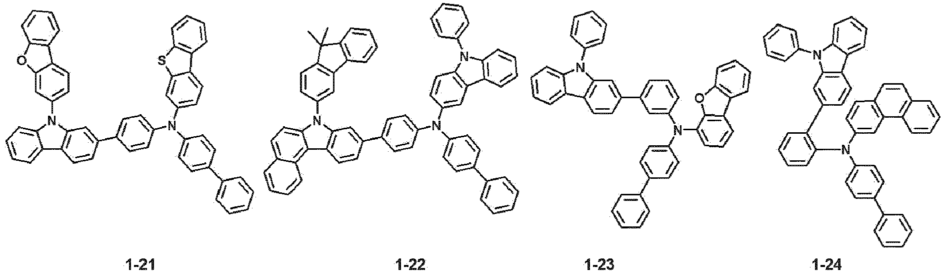

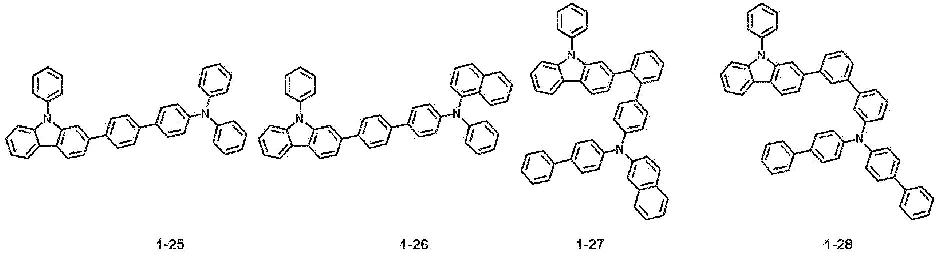

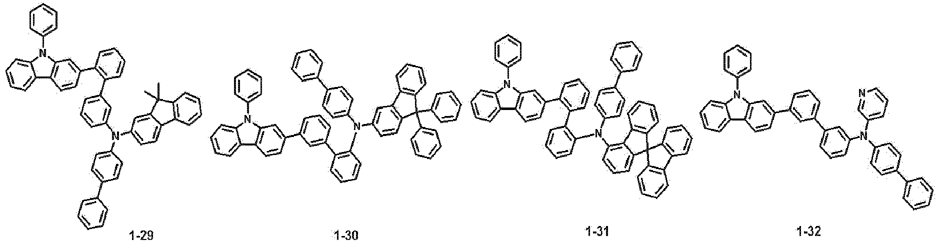

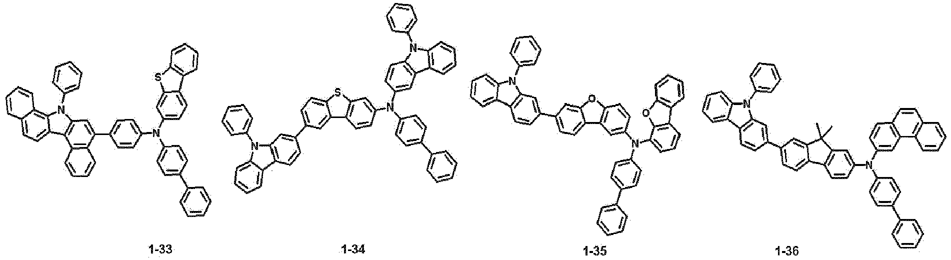

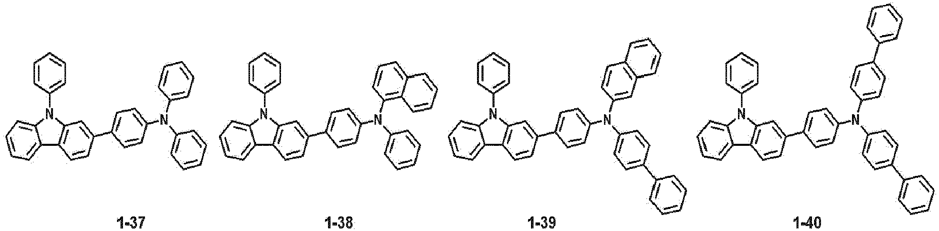

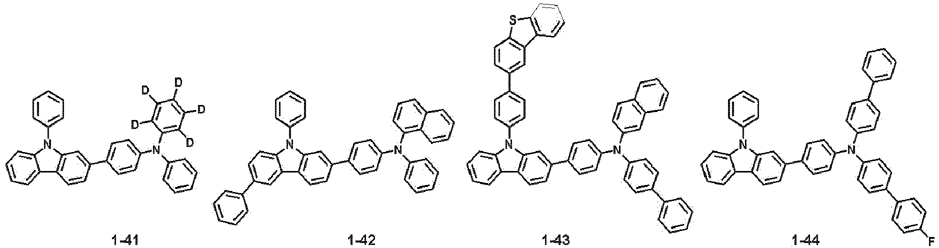

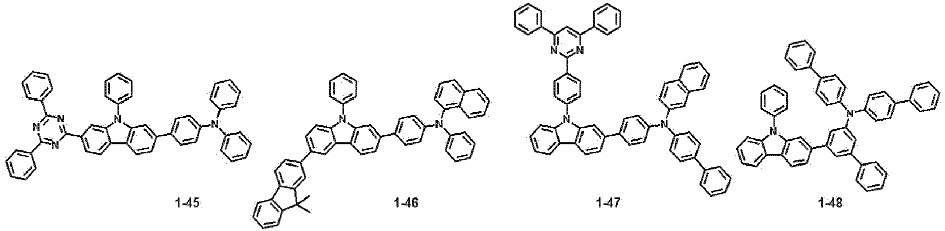

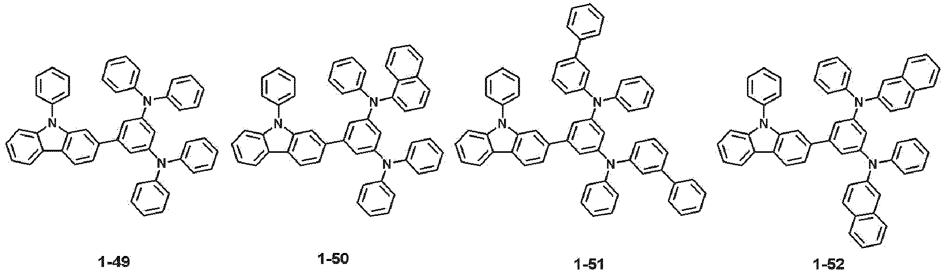

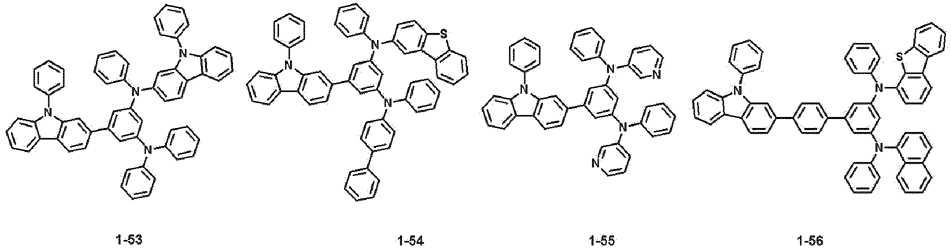

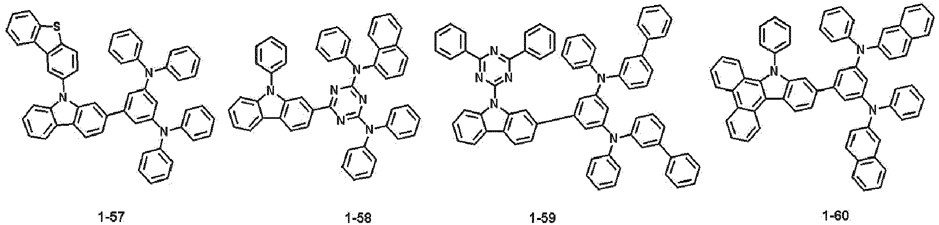

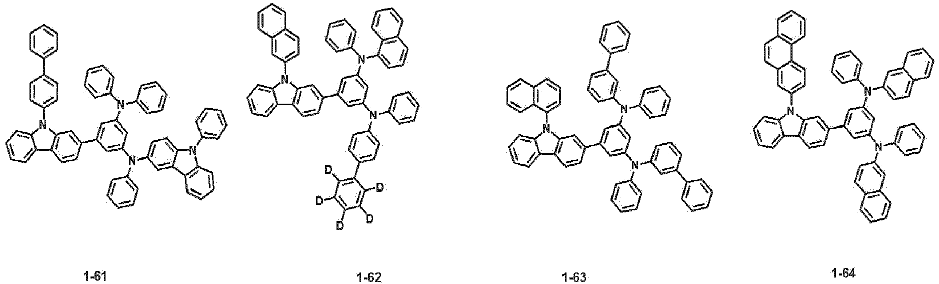

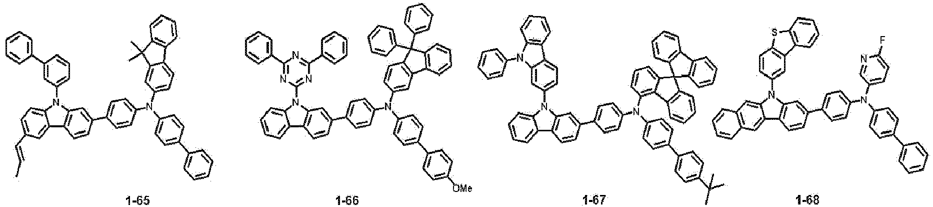

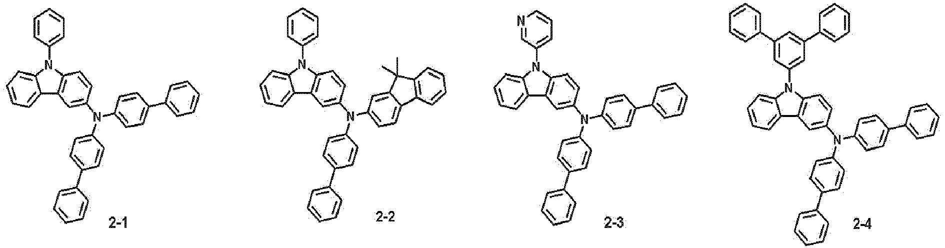

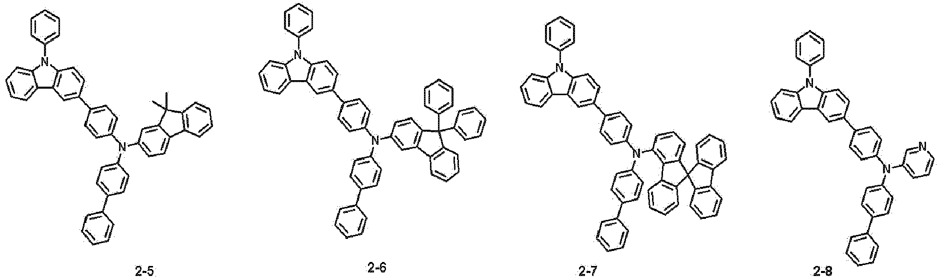

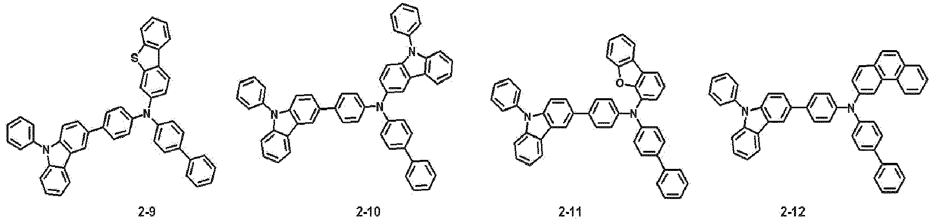

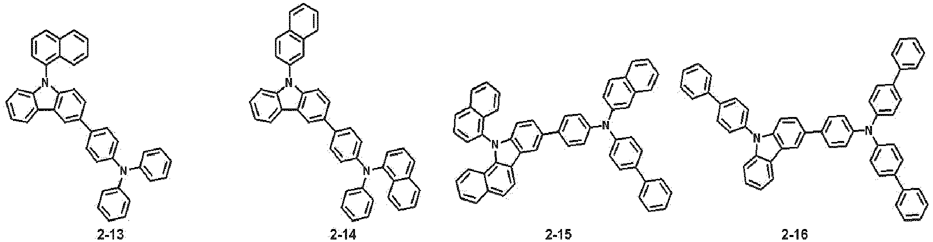

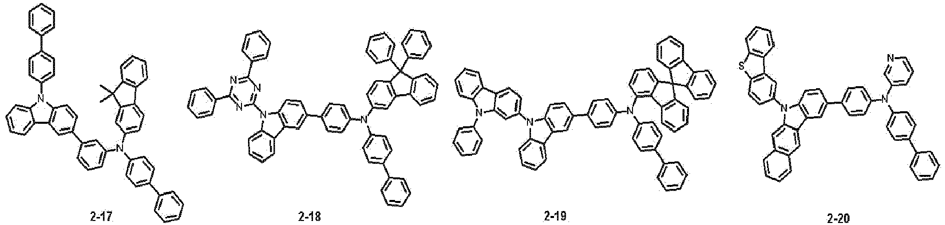

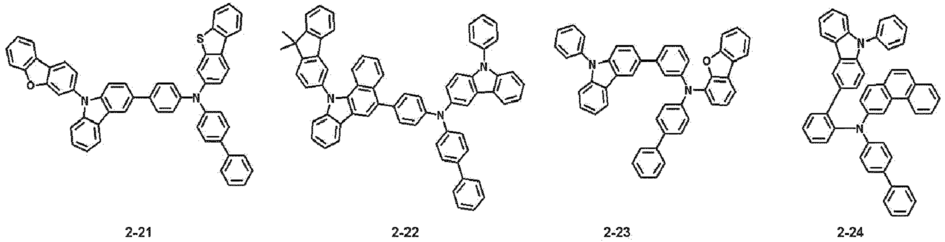

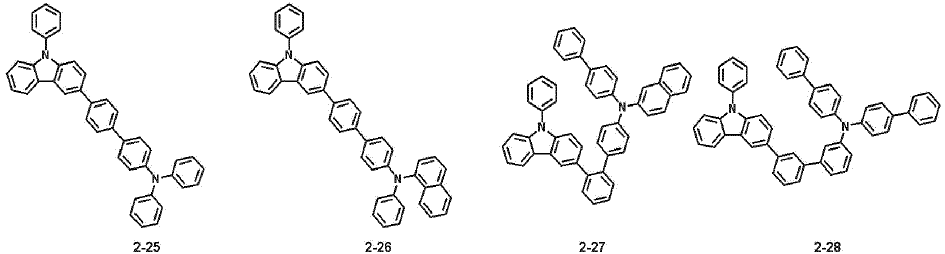

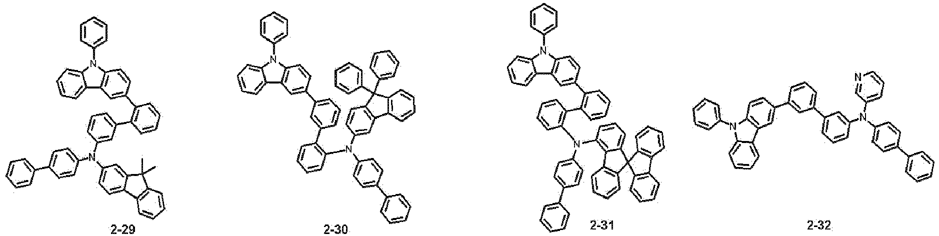

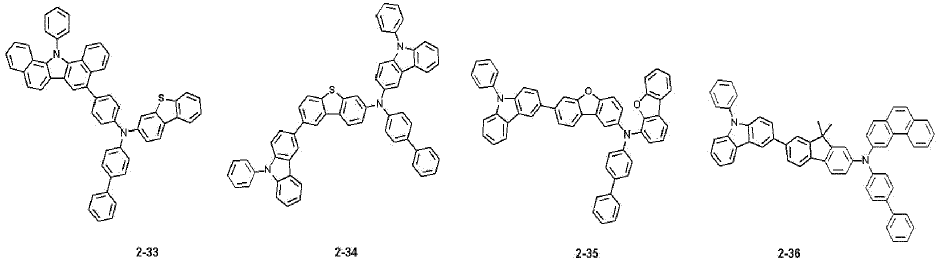

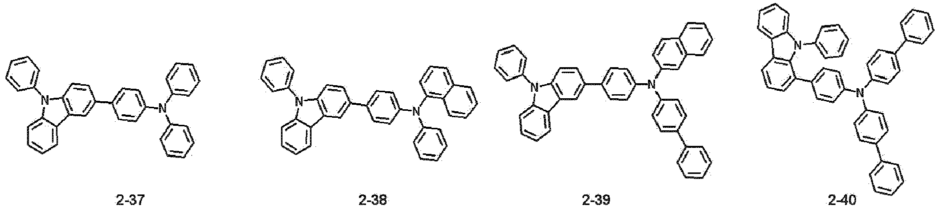

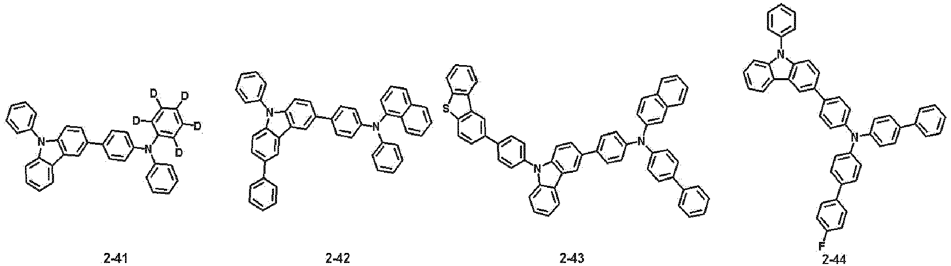

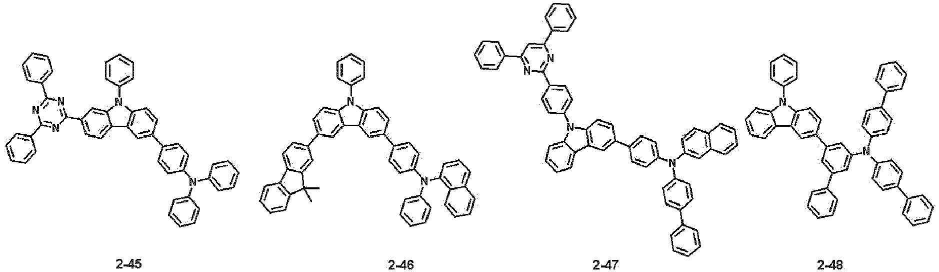

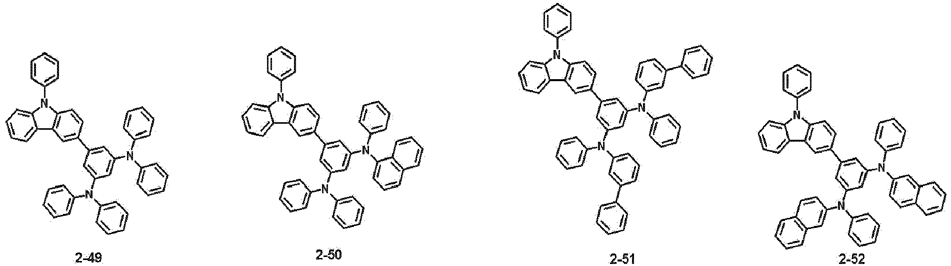

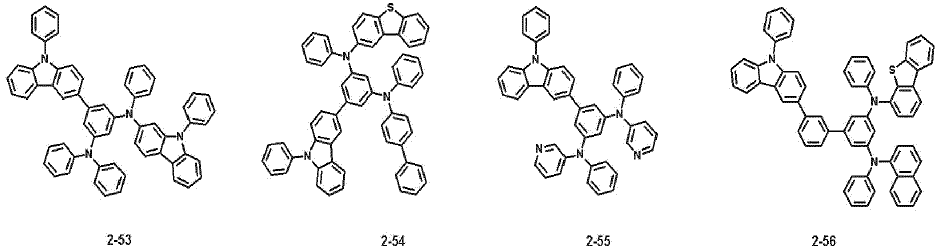

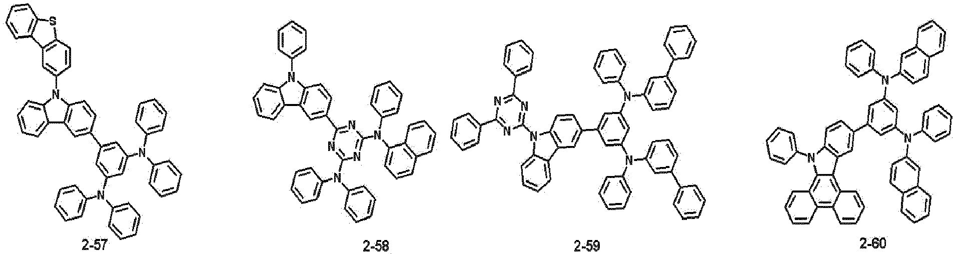

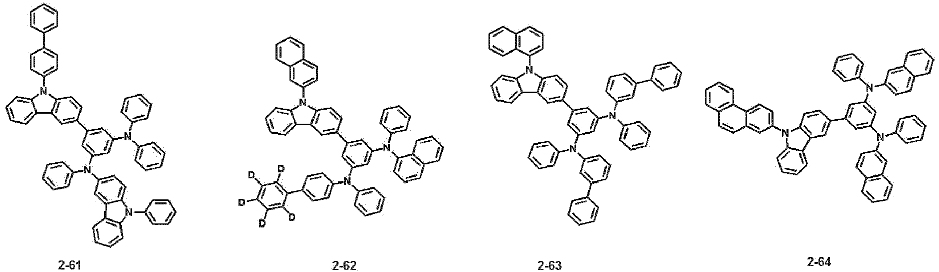

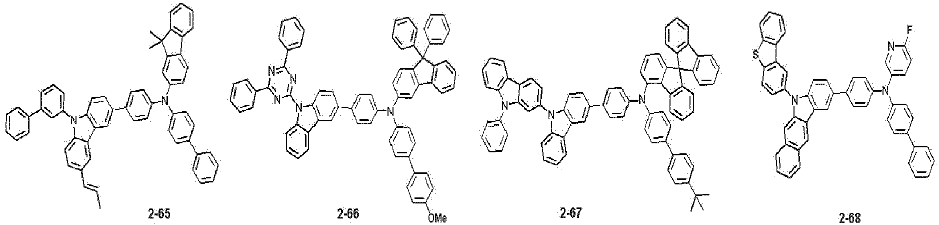

12. The organic electric element of claim 1, wherein the second host compound represented by Formula 2 comprises any one of the following Compounds 3-1 to 3-88

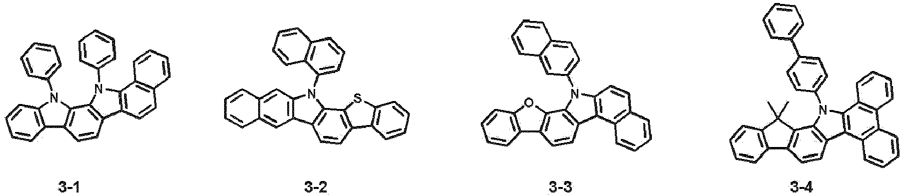

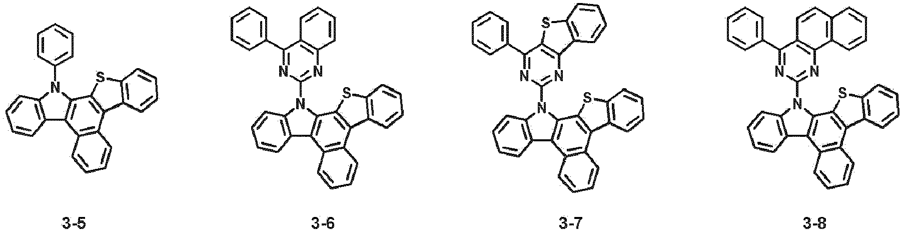

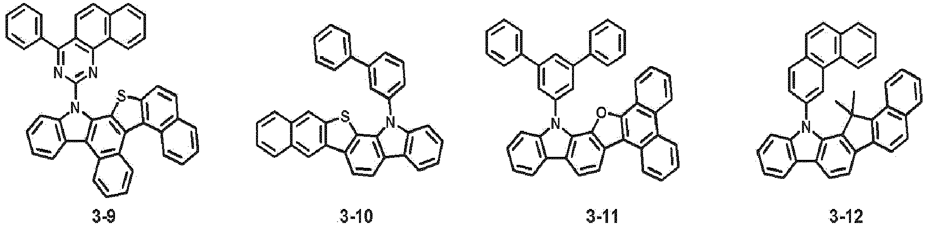

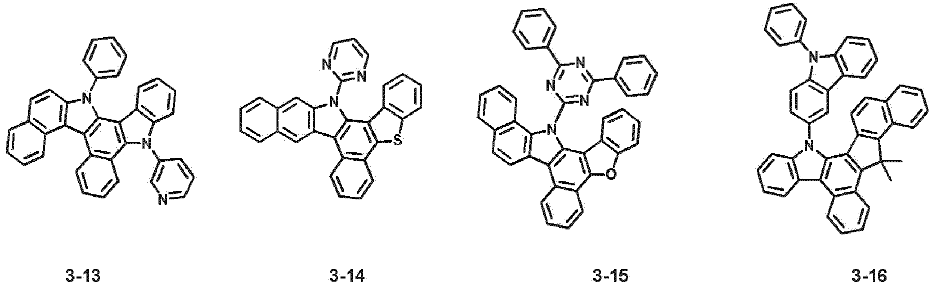

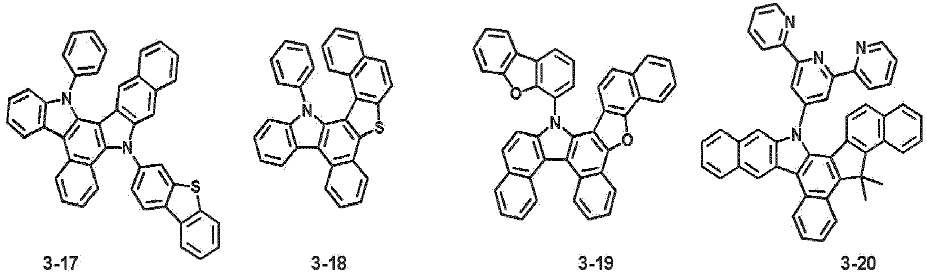

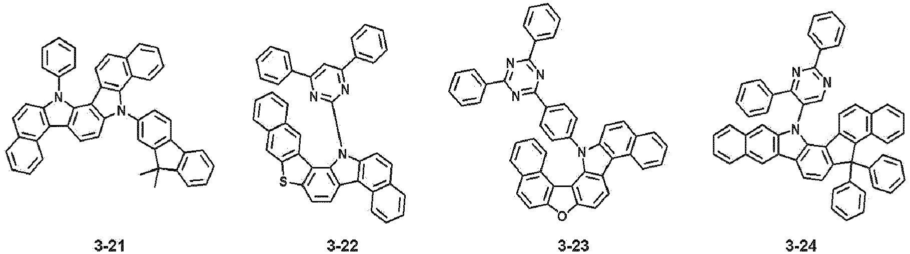

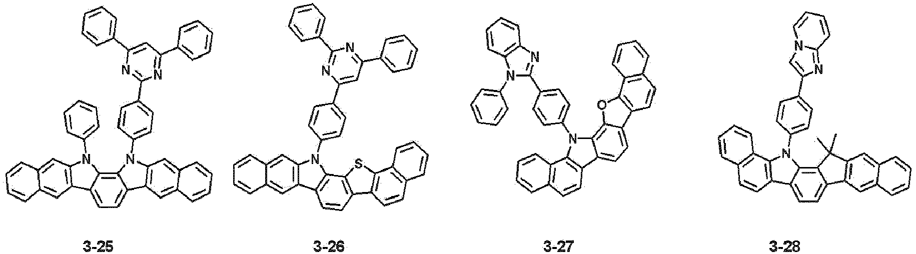

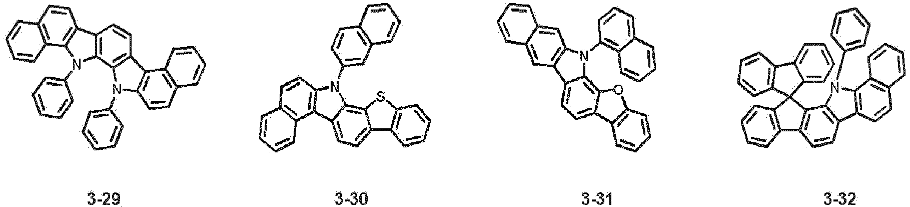

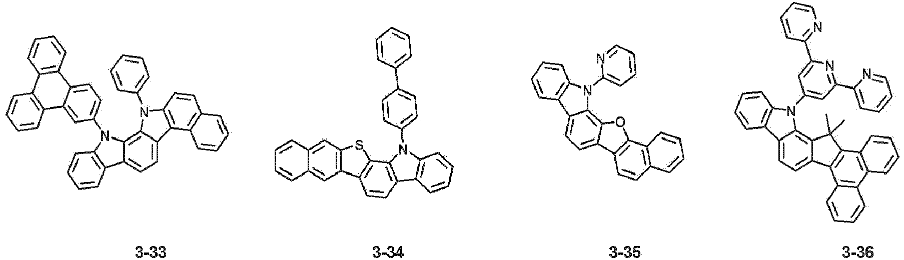

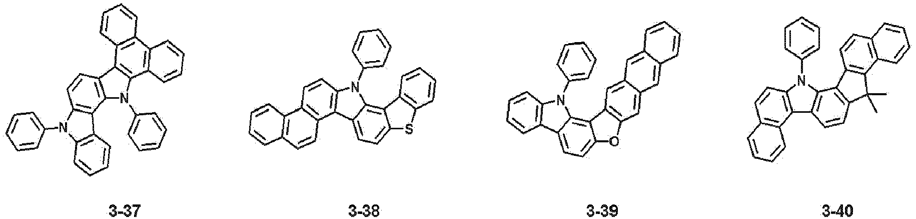

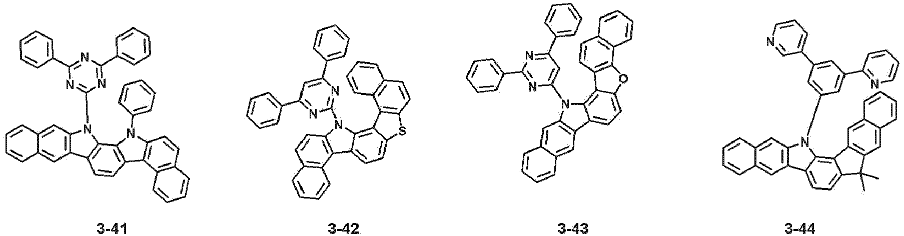

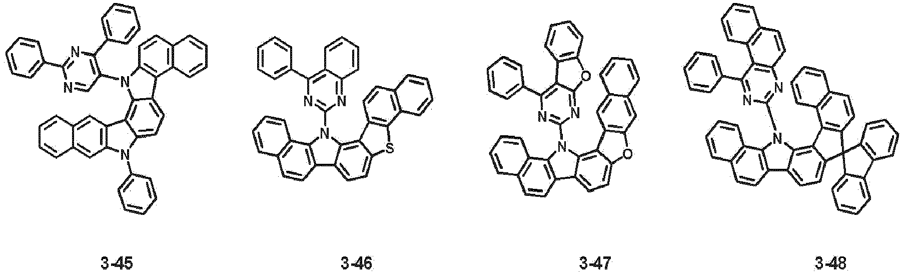

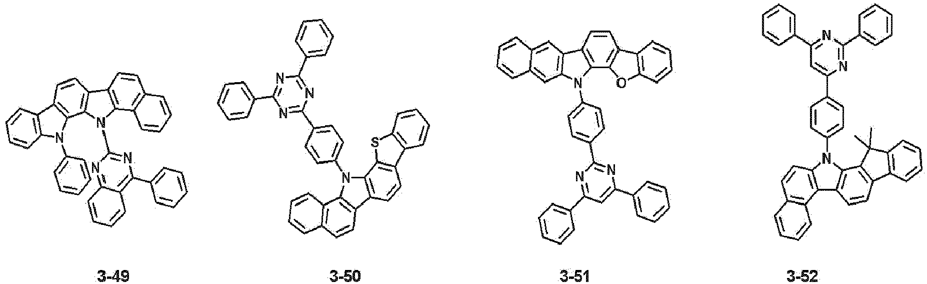

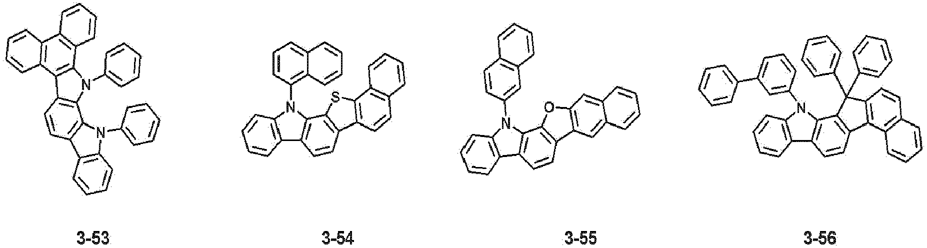

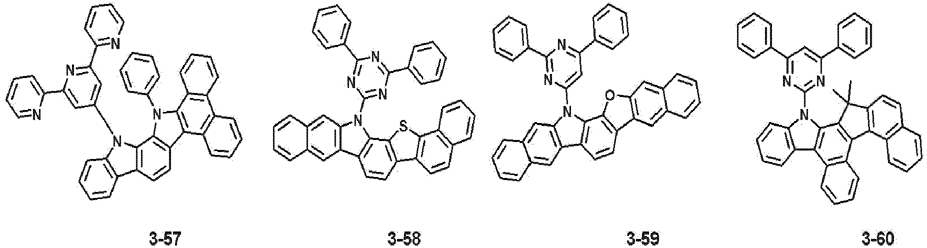

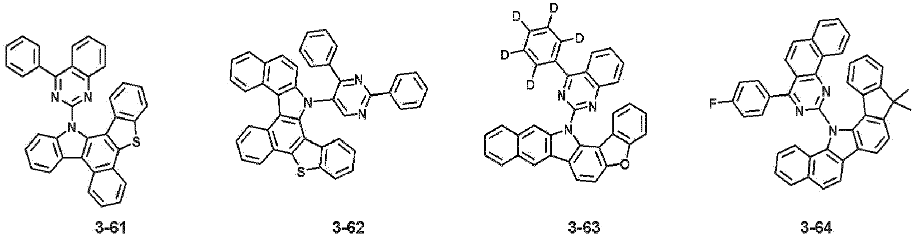

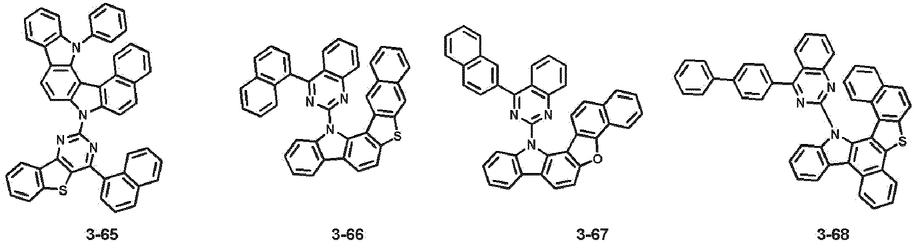

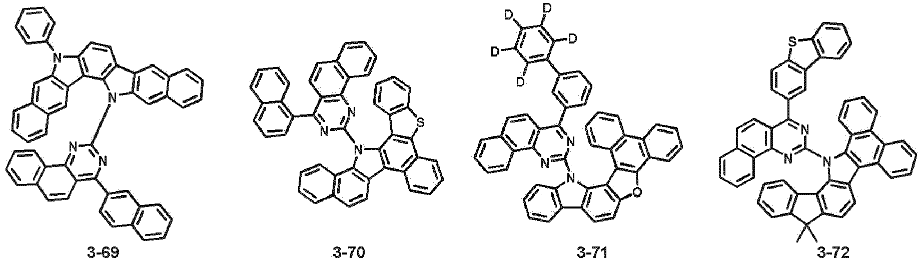

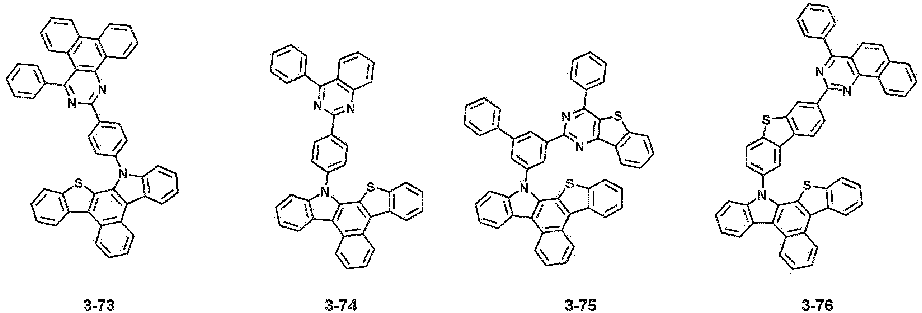

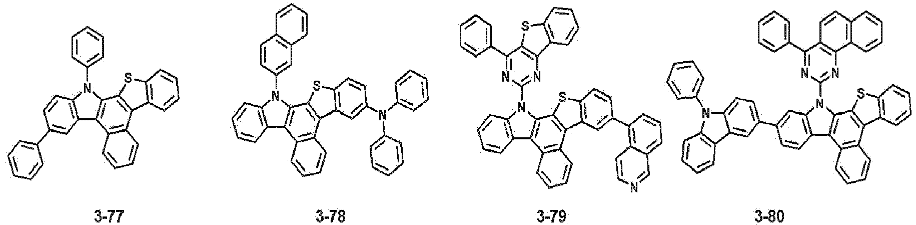

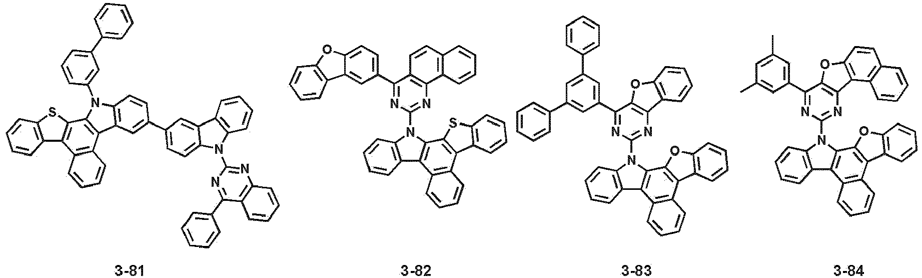

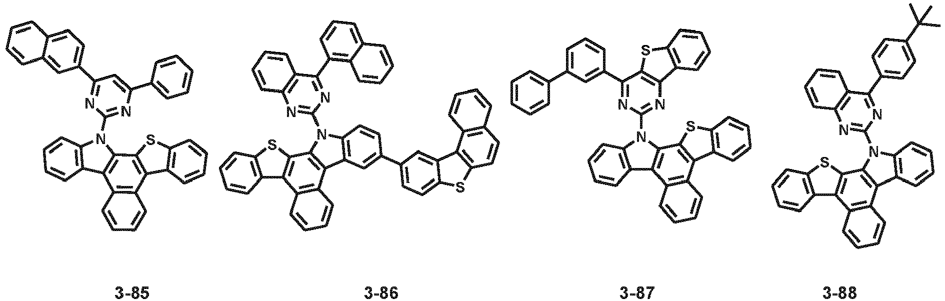

13. The organic electric element of claim 1, wherein the compounds represented by Formula 1 and by Formula 2 are mixed in a ratio of 1: 9 to 9: 1 to be included in the emitting layer.

14. The organic electric element of claim 1, wherein the compound represented by Formula 1 and by Formula 2 are mixed in a ratio of 1: 9 to 5: 5 to be included in the emitting layer.

15. The organic electric element of claim 1, wherein he compound represented by Formula 1 and by Formula 2 are mixed in a ratio of 2:8 or 3:7 to be included in the emitting layer.

16. An electronic device comprising a display device including the organic electric element of claim 1; and a control unit for driving the display device.

## Patentansprüche

1. Organisches elektrisches Element (100), umfassend:
eine erste Elektrode (120), eine zweite Elektrode (180) und eine organische Materialschicht, die zwischen der ersten Elektrode und der zweiten Elektrode ausgebildet ist, wobei die organische Materialschicht eine emittierende Schicht (150) umfasst,
wobei die emittierende Schicht eine erste Hostverbindung, die durch Formel 1 dargestellt wird, als die phosphoreszierendes Licht emittierende Schicht und eine zweite Hostverbindung, die durch Formel 2 dargestellt wird, umfasst, 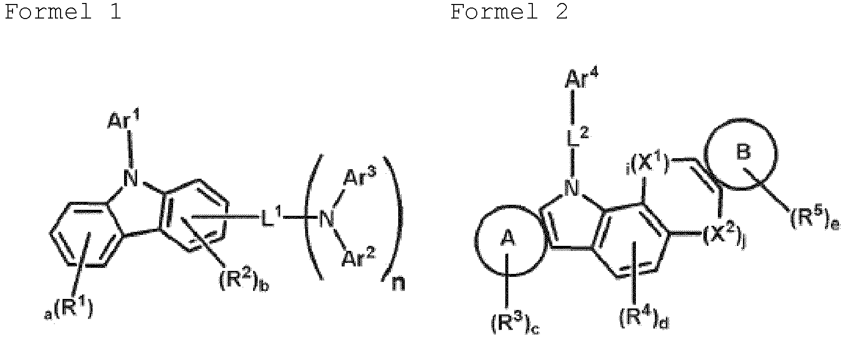 wobei
1) Ar¹, Ar², Ar³ und Ar⁴ jeweils unabhängig ausgewählt sind aus der Gruppe bestehend aus einer C₆-C₆₀-Arylgruppe; einer Fluorenyl-Gruppe; einer heterozyklischen C₂-C₆₀-Gruppe, die mindestens ein Heteroatom aus O, N, S, Si und P beinhaltet; einer kondensierten Ring-Gruppe aus einem aliphatischen C₃-C₆₀-Ring und einem aromatischen C₆-C₆₀-Ring; einer C₁-C₅₀-Alkylgruppe; einer C₂-C₂₀-Alkenylgruppe; einer C₂-C₂₀-Alkinylgruppe; einer C₁-C₃₀-Alkoxygruppe; einer C₆-C₃₀ Aryloxygruppe;
2) a eine ganze Zahl von 0 bis 4 ist, und b eine ganze Zahl von 0 bis 3 ist, und c und e eine ganze Zahl von 0 bis 10 sind, und d eine ganze Zahl von 0 bis 2 ist,
3) R¹, R², R³, R⁴ und R⁵ gleich oder unterschiedlich sein können, und jeweils unabhängig ausgewählt sind aus der Gruppe bestehend aus Deuterium; Halogen; einer C₆-C₆₀-Arylgruppe;
einer Fluorenyl-Gruppe; einer heterozyklischen C₂-C₆₀-Gruppe, die mindestens ein Heteroatom aus O, N, S, Si oder P beinhaltet; einer kondensierten Ring-Gruppe aus einem aliphatischen C₃-C₆₀-Ring und einem aromatischen C₆-C₆₀-Ring; einer C₁-C₅₀-Alkylgruppe; einer C₂-C₂₀-Alkenylgruppe; einer C₂-C₂₀-Alkinylgruppe; einer C₁-C₃₀-Alkoxygruppe; einer C₆-C₃₀-Aryloxygruppe; oder in dem Fall, dass a, b, c und e 2 oder mehr sind, und d 2 ist, R¹, R², R³, R⁴ und R⁵ jeweils in einer Vielzahl vorkommen und gleich oder unterschiedlich sind, und eine Vielzahl von R¹ oder eine Vielzahl von R² oder eine Vielzahl von R³ oder eine Vielzahl von R⁴ oder eine Vielzahl von R⁵ aneinander gebunden sein können, um einen Ring zu bilden,
4) L¹ und L² jeweils unabhängig ausgewählt sind aus der Gruppe bestehend aus einer Einfachbindung, einer C₆-C₆₀-Arylengruppe, und einer Fluorenylen-Gruppe; einer kondensierten Ring-Gruppe aus einem aliphatischen C₃-C₆₀-Ring und einem aromatischen C₆-C₆₀-Ring; und einer heterozyklischen C₂-C₆₀-Gruppe,
5) A und B jeweils unabhängig eine C₆-C₆₀-Arylgruppe oder eine heterozyklische C₂-C₂₀-Gruppe sind, wobei A und B beide eine substituierte oder unsubstituierte C₆-Arylgruppe (Phenylgruppe) sind, d 2 ist und die R⁴ aneinander gebunden sind, um einen Ring, um einen aromatischen oder Heteroring zu bilden,
6) i und j unabhängig 0 oder 1 sind, mit der Maßgabe, dass i + j 1 oder mehr ist, und wenn i oder j 0 ist, dies eine direkte Bindung bedeutet,
7) X¹ und X² unabhängig NR', O, S oder CR'R" sind, wobei R' und R" unabhängig Wasserstoff; eine C₆-C₆₀-Arylgruppe; eine Fluorenyl-Gruppe; eine heterozyklische C₃-C₆₀-Gruppe; oder eine C₁-C₅₀-Alkylgruppe sind; wobei R' und R" aneinander gebunden sein können, um eine Spiroverbindung zu bilden, und
8) n eine ganze Zahl von 1 oder 2 ist, wenn n 2 ist, zwei Ar² gleich oder unterschiedlich sind, und zwei Ar³ jeweils gleich oder unterschiedlich sind, und wobei eine Arylgruppe, Fluorenyl-Gruppe, Arylengruppe, heterozyklische Gruppe, kondensierte Ring-Gruppe, Alkylgruppe, Alkenylgruppe, Alkoxygruppe und Aryloxygruppe mit einem oder mehreren Substituenten ausgewählt aus der Gruppe bestehend aus Deuterium; Halogen; einer C₁-C₂₀-Alkylgruppe; einer C₂-C₂₀-Alkenylgruppe; einer C₂-C₂₀-Alkinylgruppe; einer C₆-C₂₀-Arylgruppe; einer mit Deuterium substituierten C₆-C₂₀-Arylgruppe; einer Fluorenyl-Gruppe; einer heterozyklischen C₂-C₂₀-Gruppe substituiert sein kann; wobei die Substituenten sich miteinander kombinieren können und einen gesättigten oder ungesättigten Ring bilden, wobei der Begriff 'Ring' einen aliphatischen C₃-C₆₀-Ring oder einen aromatischen C₆-C₆₀-Ring oder einen heterozyklischen C₂-C₆₀-Ring oder einen kondensierten Ring bedeutet, der durch die Kombination davon gebildet wird.

2. Organisches elektrisches Element nach Anspruch 1, wobei die erste Hostverbindung, die durch die Formel 1 dargestellt wird, eine Verbindung umfasst, die durch die nachstehende Formel 3 oder Formel 4, Formel 21 oder Formel 22 dargestellt wird, 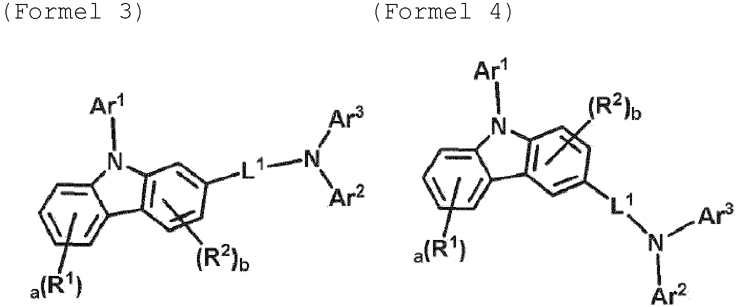 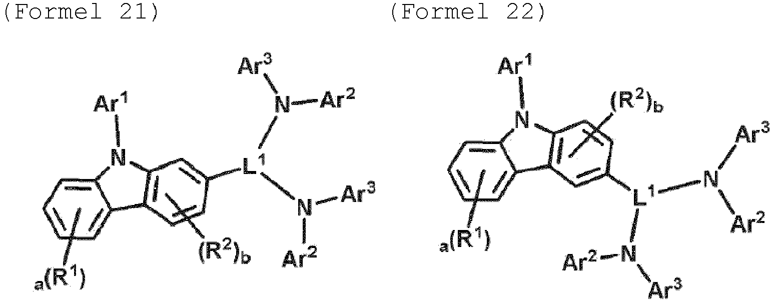 wobei R¹, R², L¹, Ar¹, Ar², Ar³, a und b gleich wie in Anspruch 1 definiert sind.

3. Organisches elektrisches Element nach Anspruch 1, wobei L¹ und L² aus der Gruppe bestehend aus den folgenden Formeln (A-1) bis (A-12) ausgewählt sind, 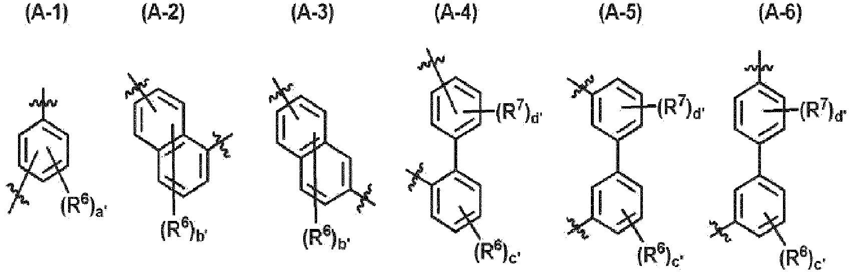 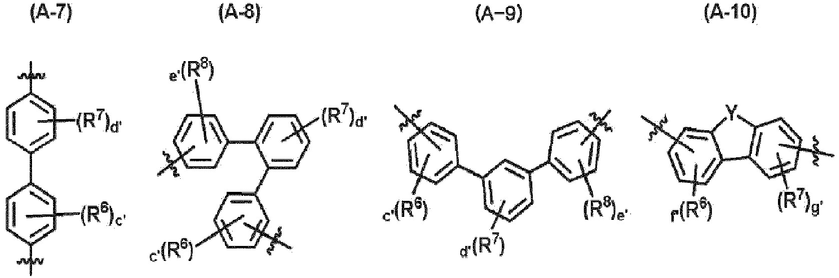 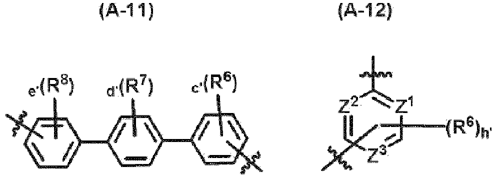 wobei
1) a', c', d' und e' eine ganze Zahl von 0 bis 4 sind, und b' eine ganze Zahl von 0 bis 5 ist, und f' und g' eine ganze Zahl von 0 bis 3 sind, und h' eine ganze Zahl von 0 bis 1 ist,
3) R⁶, R⁷ und R⁸ gleich oder unterschiedlich sind, und jeweils unabhängig ausgewählt sind aus der Gruppe bestehend aus Deuterium; Halogen; einer C₆-C₆₀-Arylgruppe; einer Fluorenyl-Gruppe; einer heterozyklischen C₂-C₆₀-Gruppe, die mindestens ein Heteroatom aus O, N, S, Si oder P beinhaltet; einer kondensierten Ring-Gruppe aus einem aliphatischen C₃-C₆₀-Ring und einem aromatischen C₆-C₆₀-Ring; einer C₁-C₅₀-Alkylgruppe; einer C₂-C₂₀-Alkenylgruppe; einer C₂-C₂₀-Alkinylgruppe; einer C₁-C₃₀-Alkoxygruppe; einer C₆-C₃₀ Aryloxygruppe;
oder wenn f' und g' zwei oder mehr sind, sie gleich oder voneinander unterschiedlich sind, und eine Vielzahl von R⁶ oder eine Vielzahl von R⁷ oder eine Vielzahl von R⁸ aneinander gebunden sein können, um einen aromatischen oder einen heteroaromatischen Ring zu bilden, zwei benachbarte R⁶ und R⁷, oder R⁷ und R⁸ verbunden sein können, um einen aromatischen oder heteroaromatischen Ring zu bilden,
3) Y NR', O, S oder CR'R" ist
4) R' und R" gleich wie in Anspruch 1 definiert sind, und
5) Z¹, Z² und Z³ unabhängig voneinander CR' oder N sind und zumindest eines davon N ist.

4. Organisches elektrisches Element nach Anspruch 1, wobei die erste Hostverbindung, die durch die Formel 1 dargestellt wird, durch eine der folgenden Formeln 5 bis 20 dargestellt wird, 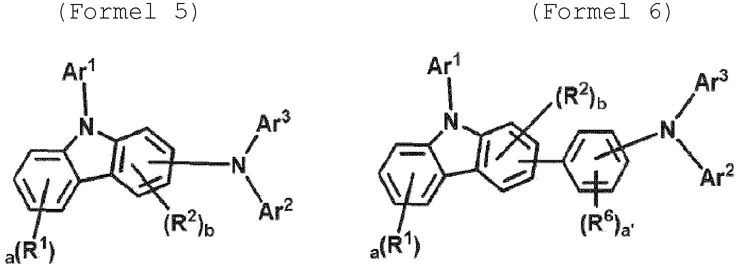 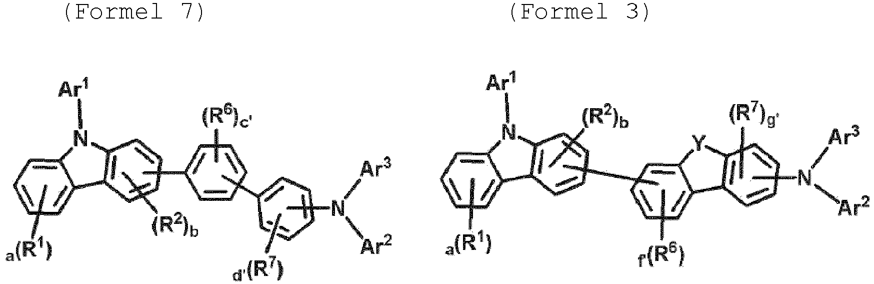 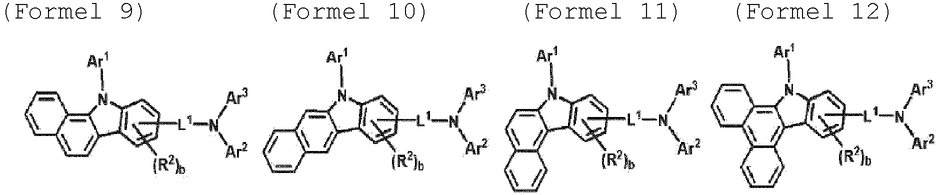 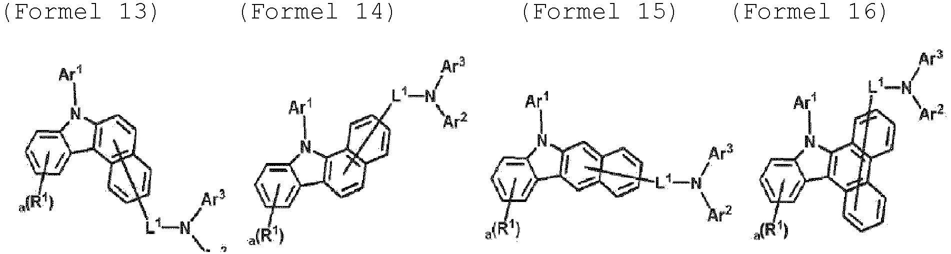 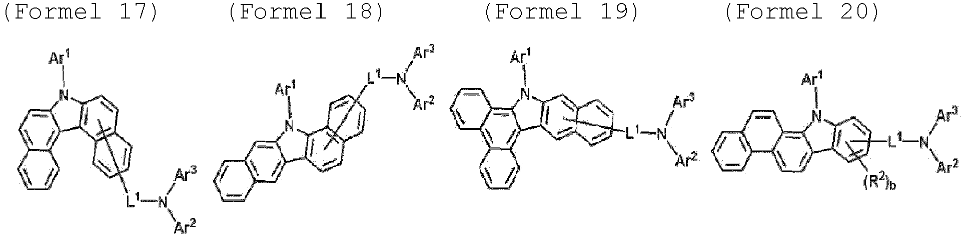 wobei
1) R¹, R², Ar¹, Ar², Ar³, a, b, R' und R" gleich wie in Anspruch 1 definiert sind,
2) R⁶ und R⁷ gleich oder unterschiedlich sind, und jeweils unabhängig ausgewählt sind aus der Gruppe bestehend aus Deuterium; Halogen; einer C₆-C₆₀-Arylgruppe; einer Fluorenyl-Gruppe; einer heterozyklischen C₂-C₆₀-Gruppe, die mindestens ein Heteroatom aus O, N, S, Si oder P beinhaltet; einer kondensierten Ring-Gruppe aus einem aliphatischen C₃-C₆₀-Ring und einem aromatischen C₆-C₆₀-Ring; einer C₁-C₅₀-Alkylgruppe; einer C₂-C₂₀-Alkenylgruppe; einer C₂-C₂₀-Alkinylgruppe; einer C₁-C₃₀-Alkoxygruppe; einer C₆-C₃₀ Aryloxygruppe; oder in dem Fall, dass f' und g' 2 oder mehr sind, jedes davon in der Vielzahl gleich oder unterschiedlich ist, und eine Vielzahl von R⁶ oder eine Vielzahl von R⁷ oder eine Vielzahl von R⁸ oder benachbarte R⁶ und R⁷ oder benachbarte R⁷ und R⁸ aneinander gebunden sein können, um einen aromatischen oder einen heteroaromatischen Ring zu bilden,
3) a', c' und d' jeweils eine ganze Zahl von 0 bis 4 sind, und f' und g' jeweils eine ganze Zahl von 0 bis 3 sind,
4) Y NR', O, S oder CR'R" ist.

5. Organisches elektrisches Element nach Anspruch 1, wobei die erste Hostverbindung, die durch die Formel 1 dargestellt wird, eine Verbindung umfasst, die durch die nachstehende Formel 23 dargestellt wird, 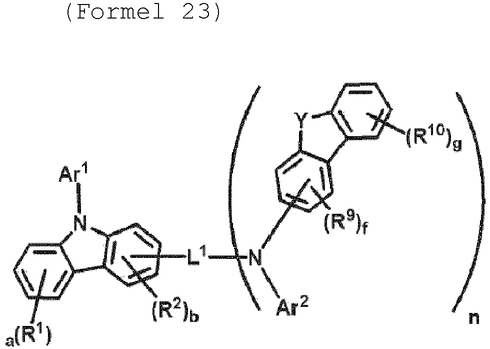 wobei
1) R¹, R², L¹, Ar¹, Ar², a, b und n gleich wie in Anspruch 1 definiert sind,
2) f eine ganze Zahl von 0 bis 3 ist, und g eine ganze Zahl von 0 bis 4 ist,
3) R⁹ und R¹⁰ gleich oder unterschiedlich sind, und jeweils unabhängig ausgewählt sind aus der Gruppe bestehend aus Deuterium; Halogen; einer C₆-C₆₀-Arylgruppe; einer Fluorenyl-Gruppe; einer heterozyklischen C₂-C₆₀-Gruppe, die mindestens ein Heteroatom aus O, N, S, Si oder P beinhaltet; einer kondensierten Ring-Gruppe aus einem aliphatischen C₃-C₆₀-Ring und einem aromatischen C₆-C₆₀-Ring; einer C₁-C₅₀-Alkylgruppe; einer C₂-C₂₀-Alkenylgruppe; einer C₂-C₂₀-Alkinylgruppe; einer C₁-C₃₀-Alkoxygruppe; einer C₆-C₃₀ Aryloxygruppe; oder in dem Fall, dass f und g zwei oder mehr sind, jedes davon in der Vielzahl gleich oder unterschiedlich ist, und eine Vielzahl von R⁹ oder eine Vielzahl von R¹⁰ oder benachbarte R⁹ und R¹⁰ aneinander gebunden sein können, um einen aromatischen oder einen heteroaromatischen Ring zu bilden,
4) Y NR', O, S oder CR'R" ist, und
5) R' und R" gleich wie in Anspruch 1 definiert sind.

6. Organisches elektrisches Element nach Anspruch 1, wobei die zweite Hostverbindung, die durch die Formel 2 dargestellt wird, eine Verbindung umfasst, die durch die nachstehende Formel 24 oder Formel 25 dargestellt wird, 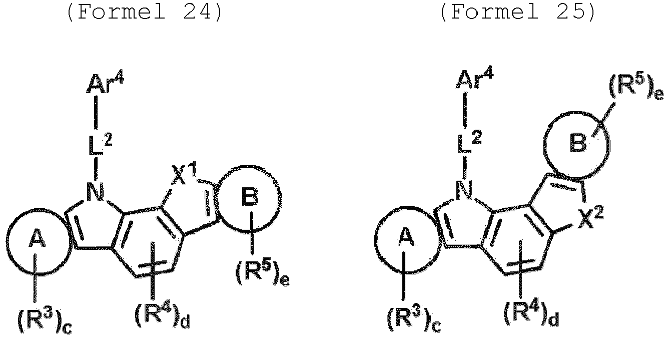 wobei R³, R⁴, R⁵, Ar⁴, L², c, d, e, A, B, X¹ und X² gleich wie in Anspruch 1 definiert sind.

7. Organisches elektrisches Element nach Anspruch 1, wobei A und B in der Formel 2 aus der Gruppe bestehend aus den folgenden Formeln (B-1) bis (B-7) ausgewählt sind, 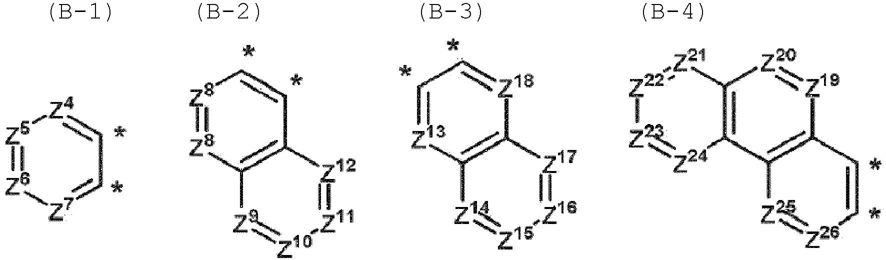 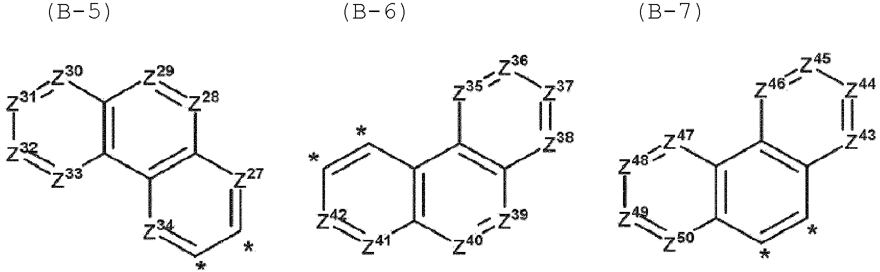 wobei
1) Z⁴ bis Z⁵⁰ CR' oder N sind,
2) R' gleich wie in Anspruch 1 definiert ist, und
3) * die zu kondensierende Position angibt.

8. Organisches elektrisches Element nach Anspruch 1, wobei R⁴ in Formel 2 eine Verbindung umfasst, die einen aromatischen Ring oder einen heteroaromatischen Ring bildet.

9. Organisches elektrisches Element nach Anspruch 1, wobei die zweite Hostverbindung, die durch Formel 2 dargestellt wird, eine Verbindung umfasst, die durch eine der folgenden Formeln 26 bis 45 dargestellt wird, 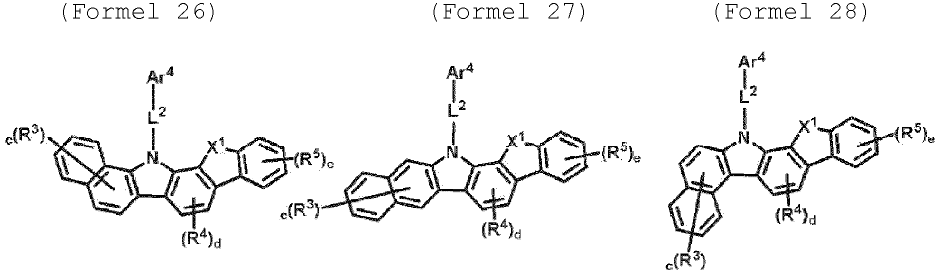 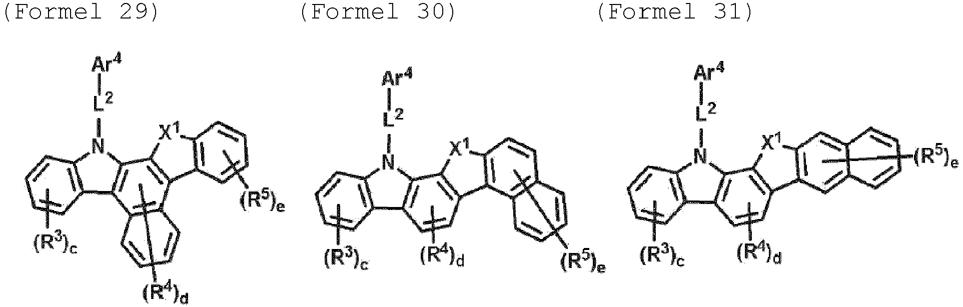 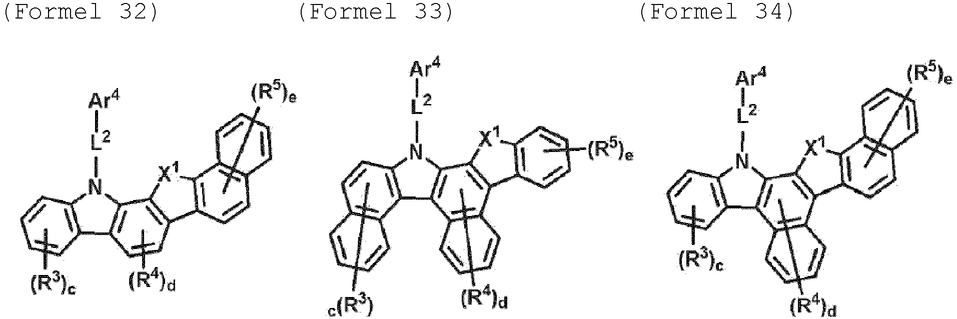 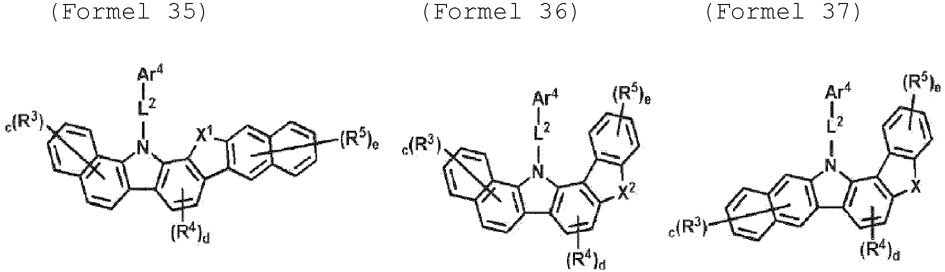 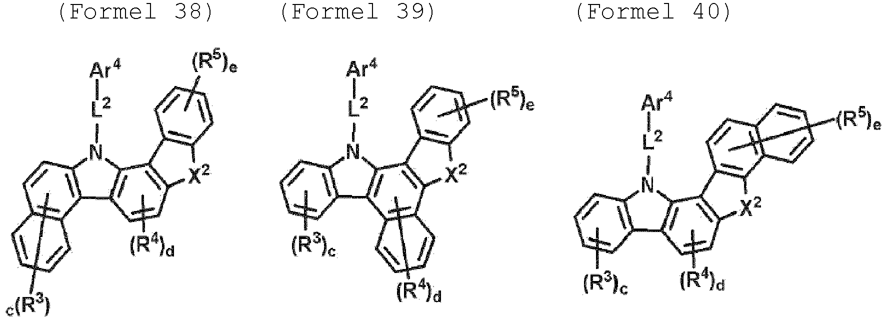 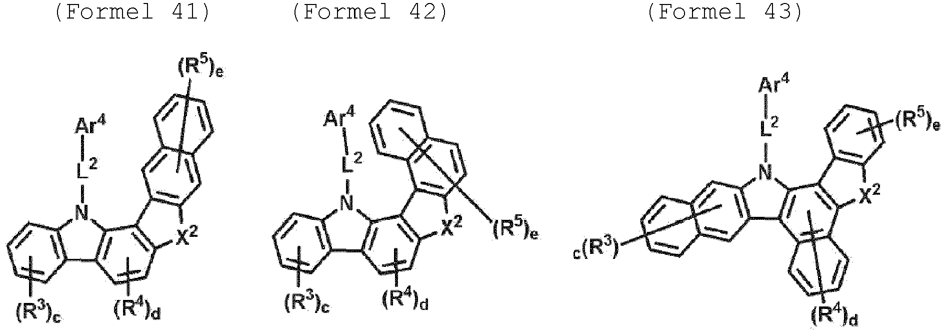 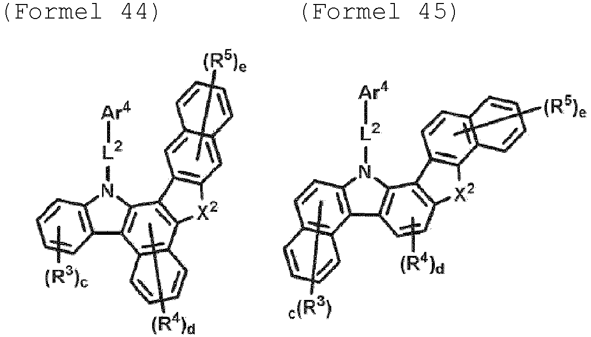 wobei
Ar⁴, X¹, X², R³, R⁴, R⁵, L², c, d und e gleich wie in Anspruch 1 definiert sind.

10. Organisches elektrisches Element nach Anspruch 1, wobei die zweite Hostverbindung, die durch die Formel 2 dargestellt wird, eine beliebige der Verbindungen umfasst, die durch die Formeln 46 bis 53 dargestellt werden, 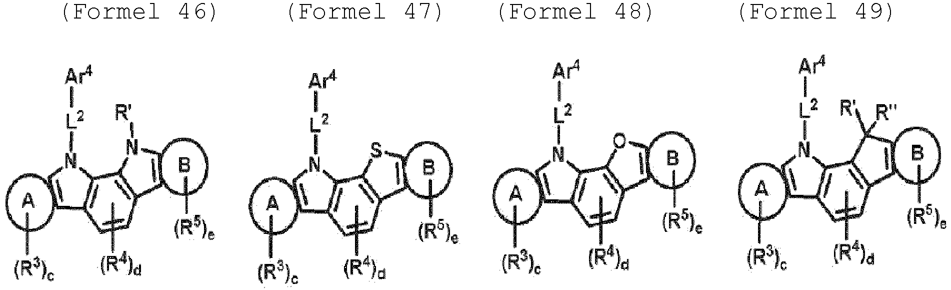 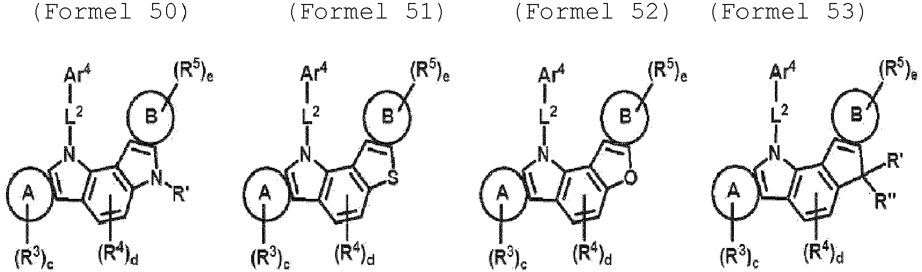 wobei
R³, R⁴, R⁵, Ar⁴, L², c, d, e, A, B, R' und R" gleich wie in Anspruch 1 definiert sind.

11. Organisches elektrisches Element nach Anspruch 1, wobei die erste Hostverbindung, die durch Formel 1 dargestellt wird, eine beliebige der folgenden Verbindungen 1-1 bis 1-68 und Verbindungen 2-1 bis 2-68 ist:

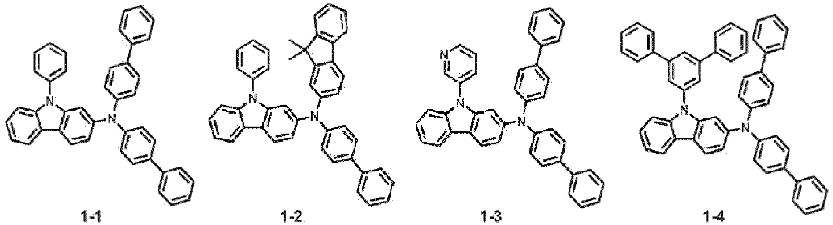

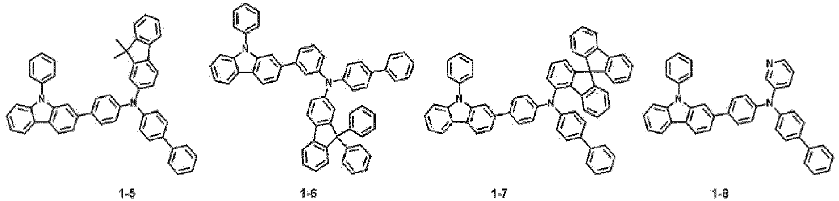

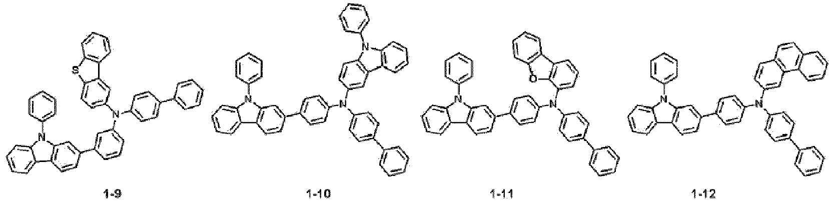

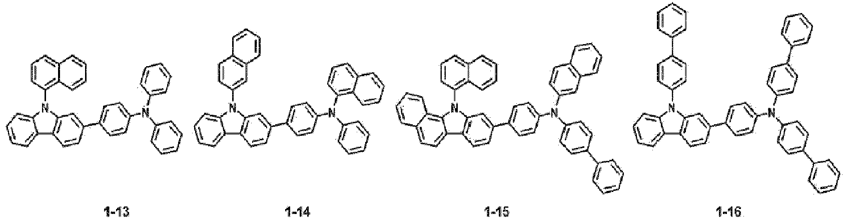

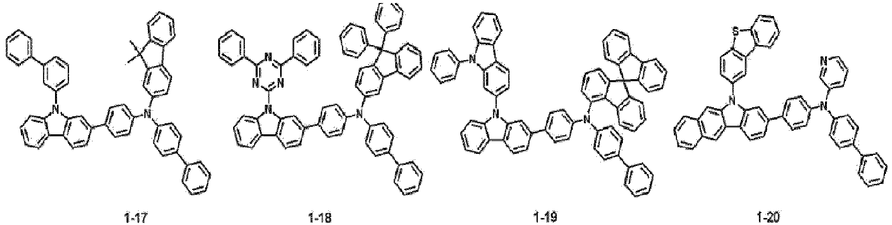

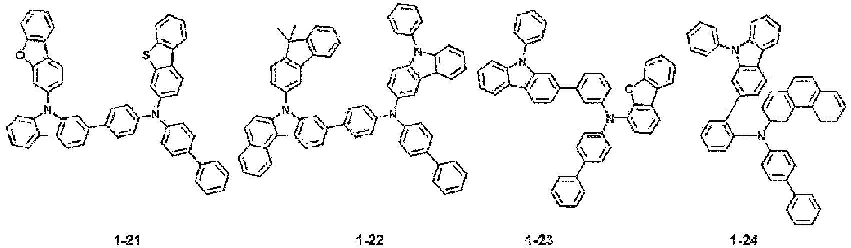

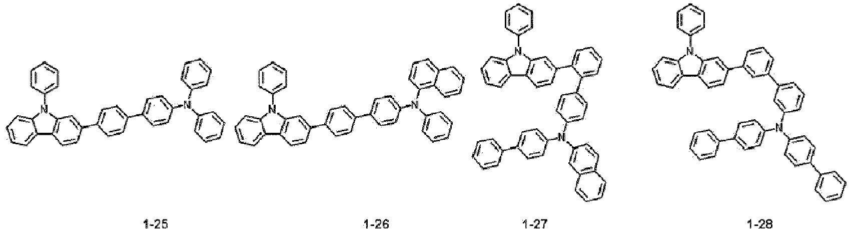

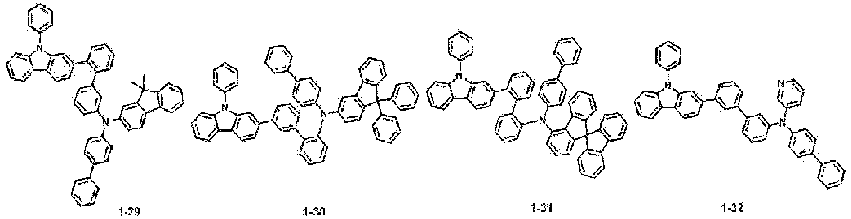

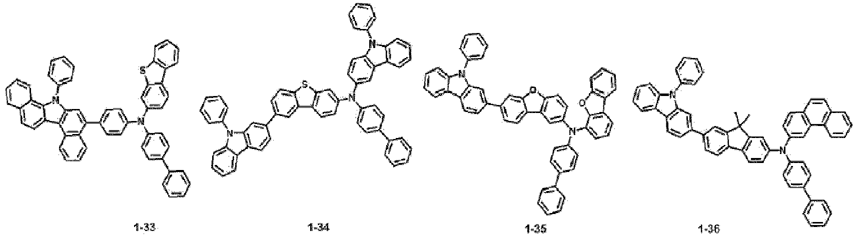

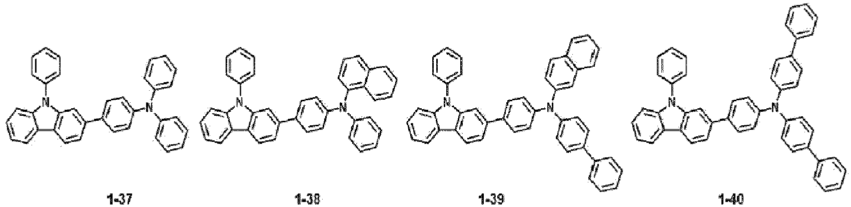

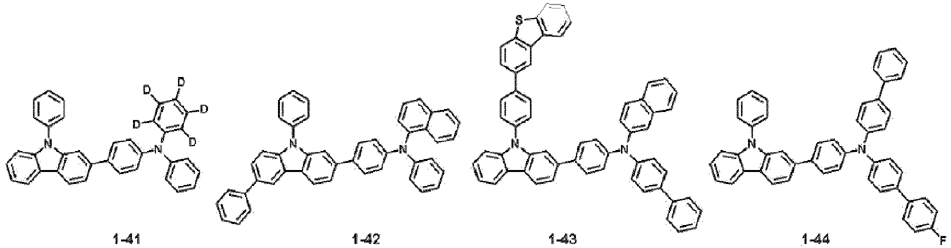

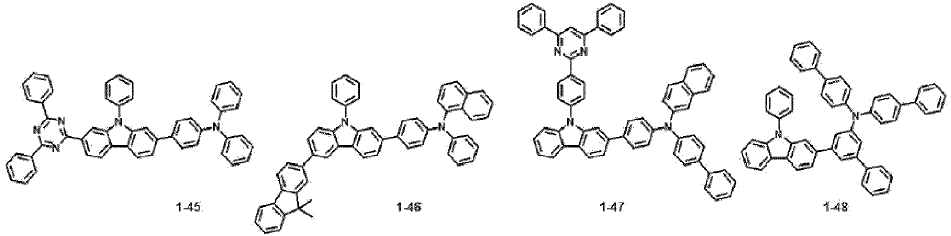

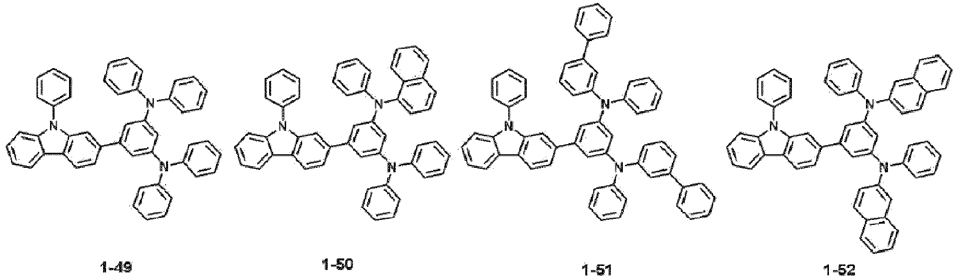

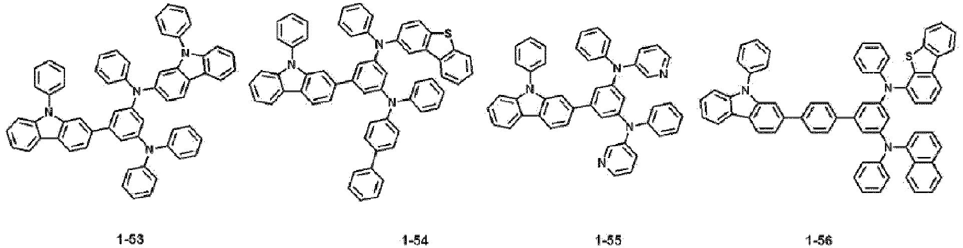

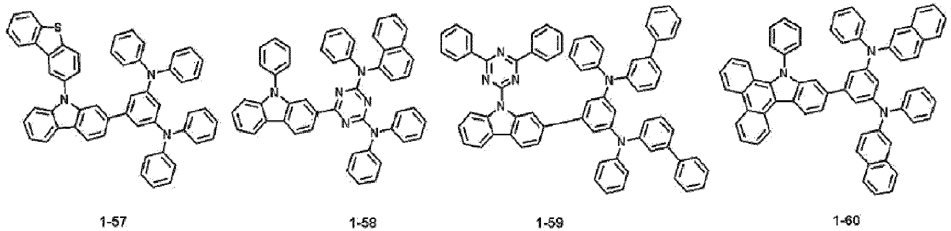

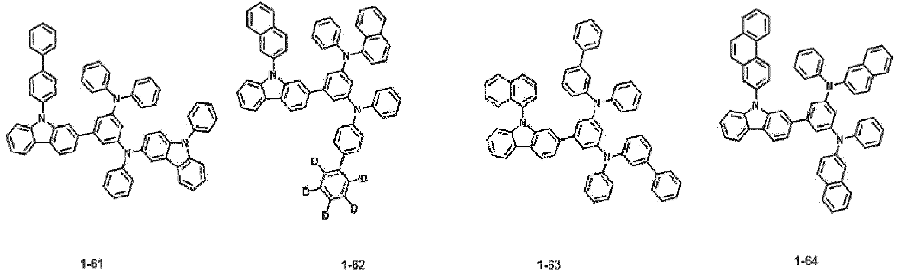

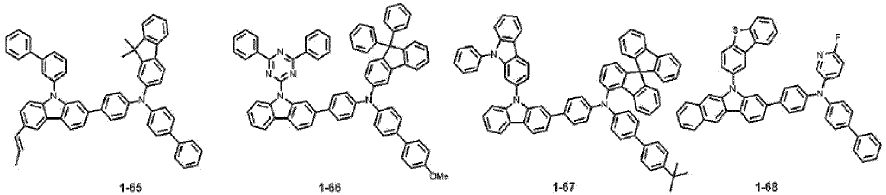

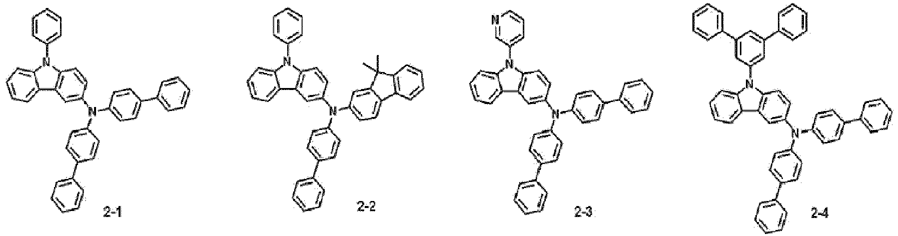

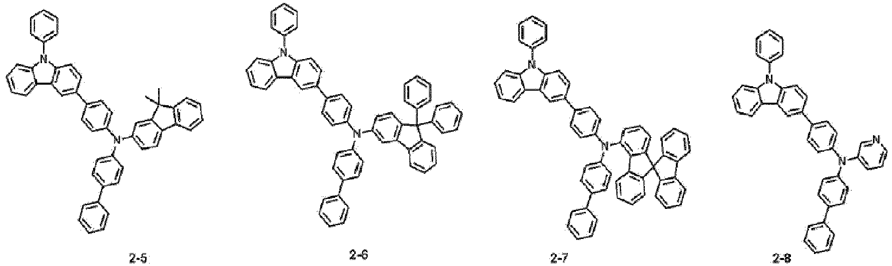

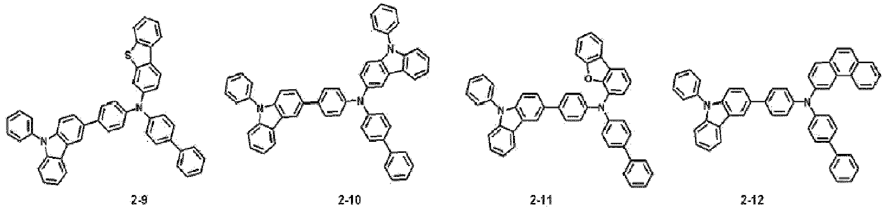

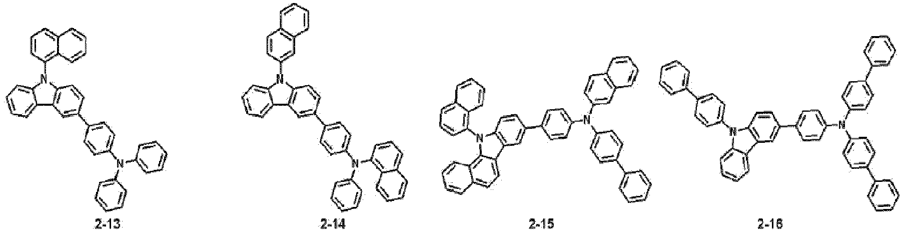

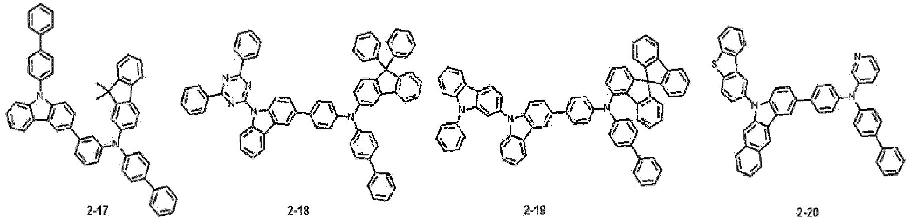

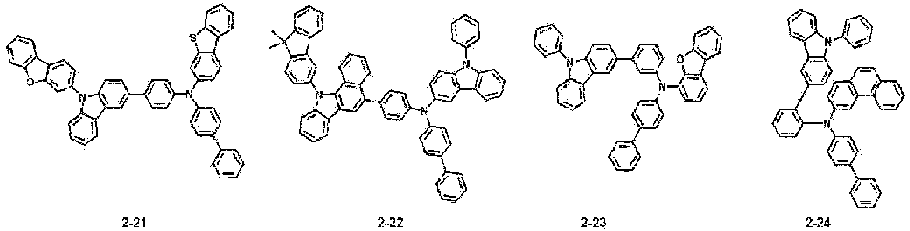

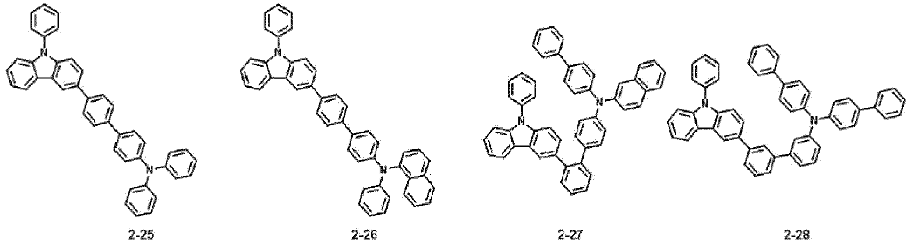

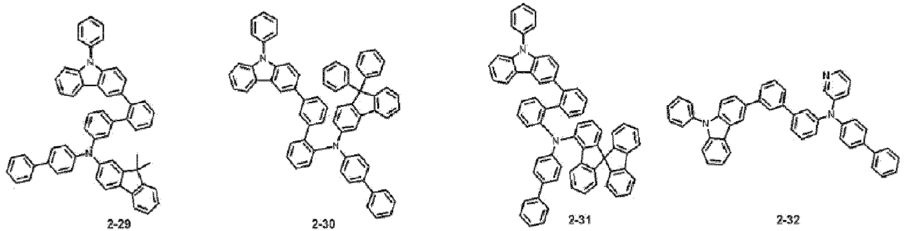

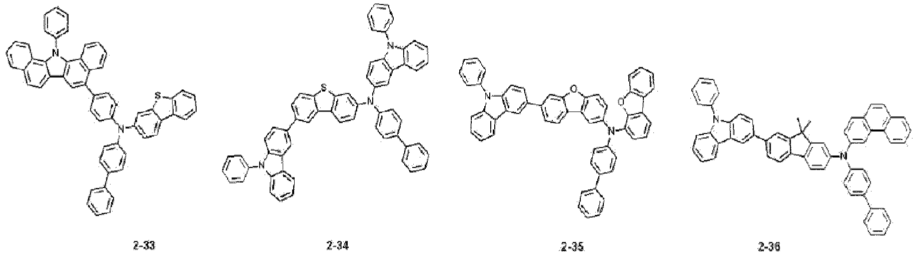

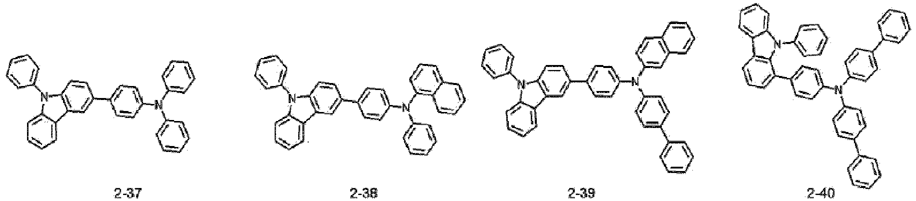

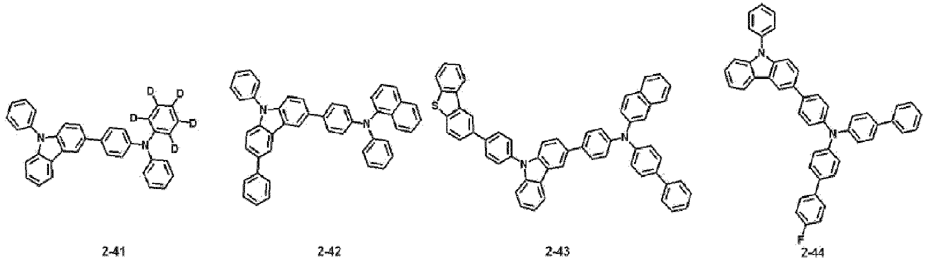

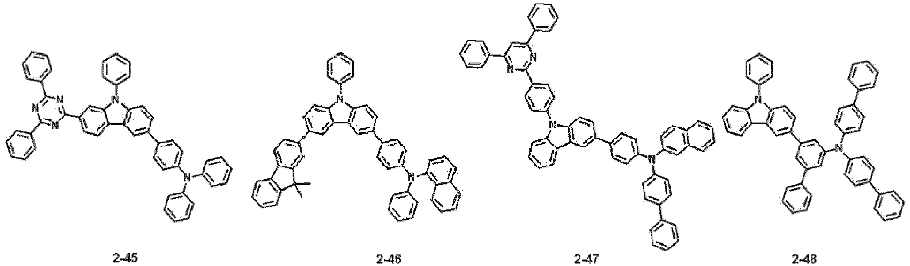

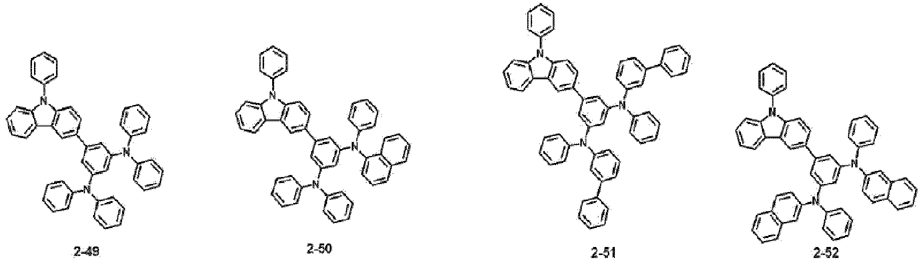

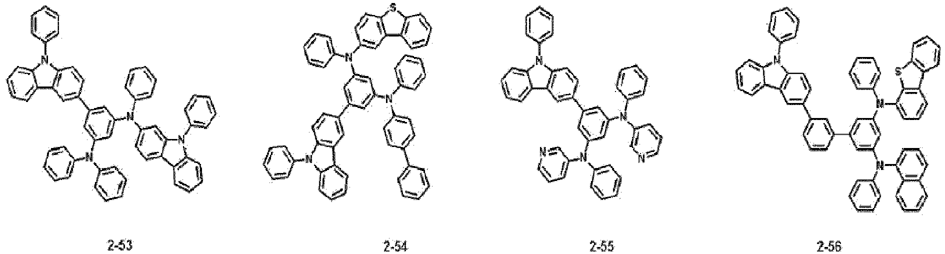

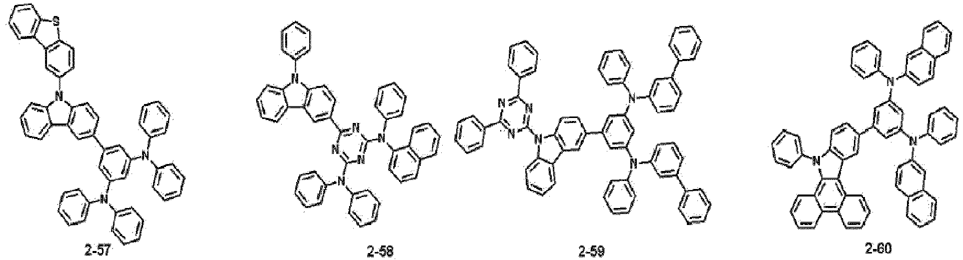

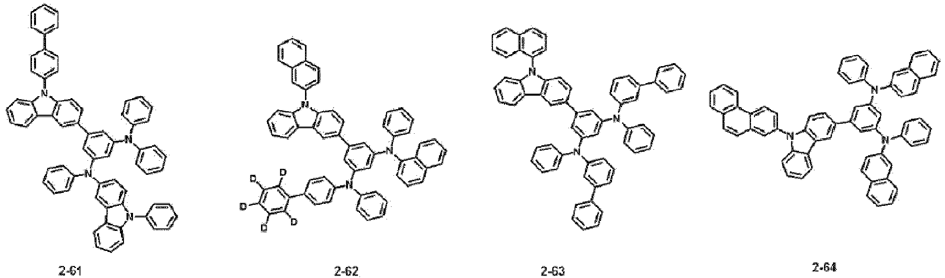

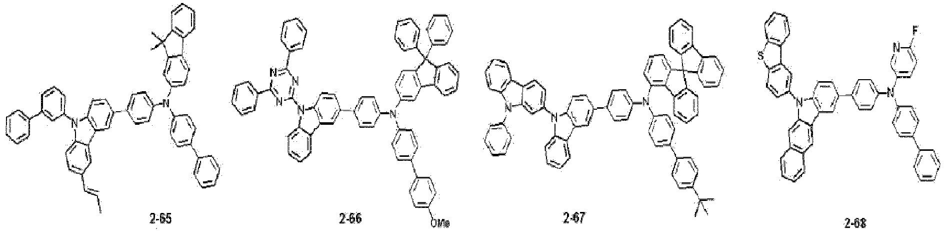

12. Organisches elektrisches Element nach Anspruch 1, wobei die zweite Hostverbindung, die durch Formel 2 dargestellt wird, eine beliebige der folgenden Verbindungen 3-1 bis 3-88 umfasst:

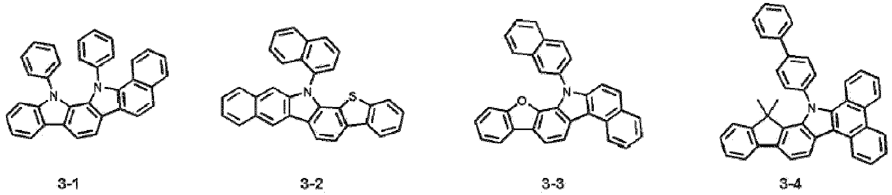

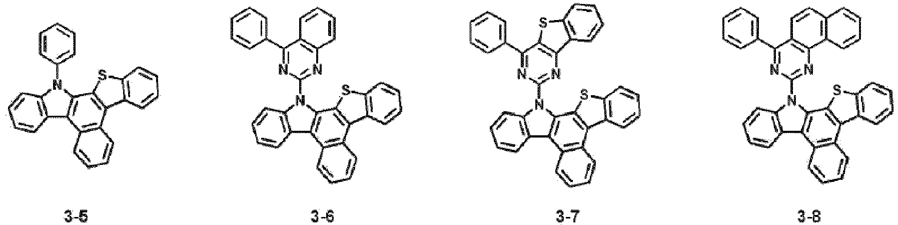

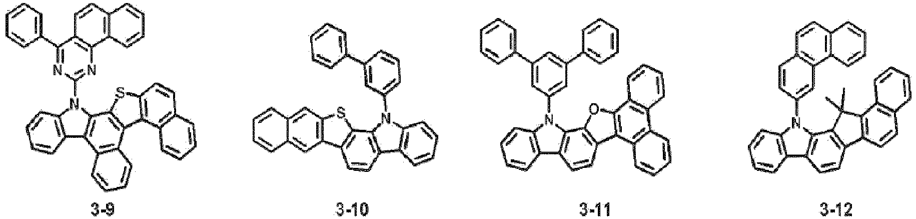

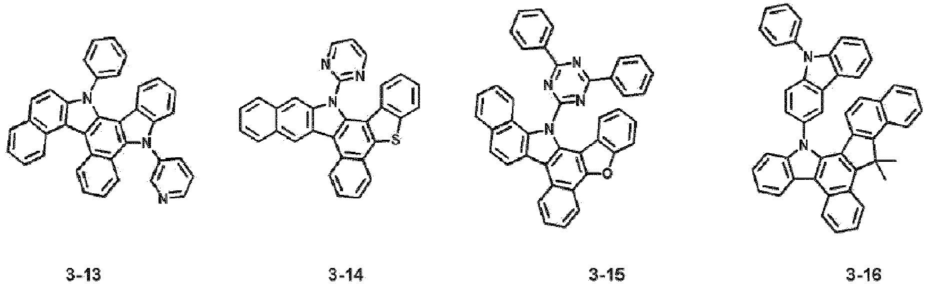

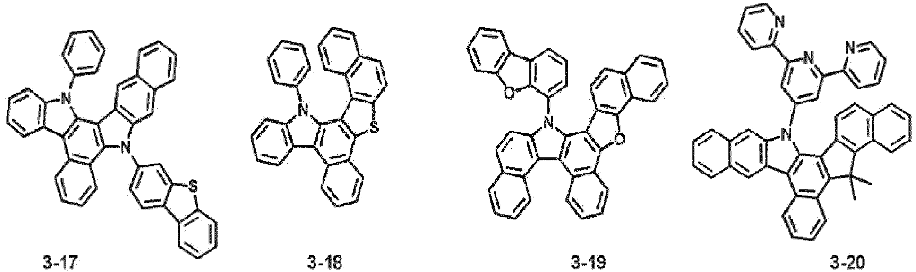

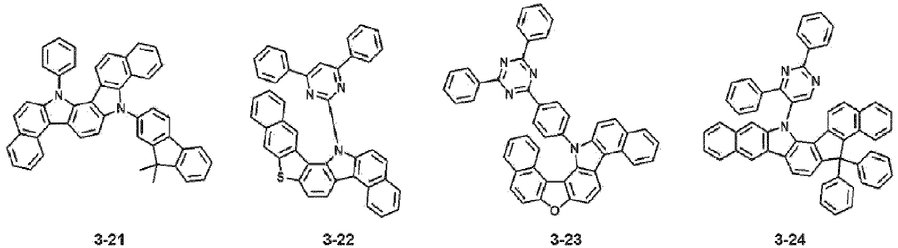

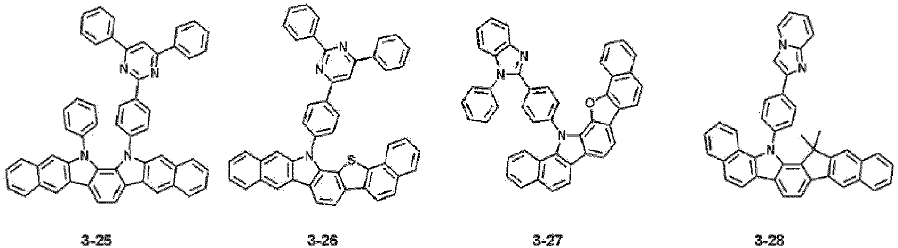

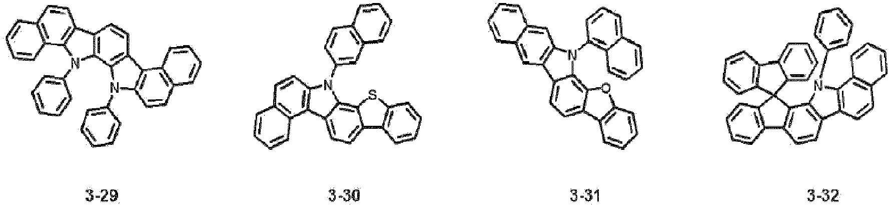

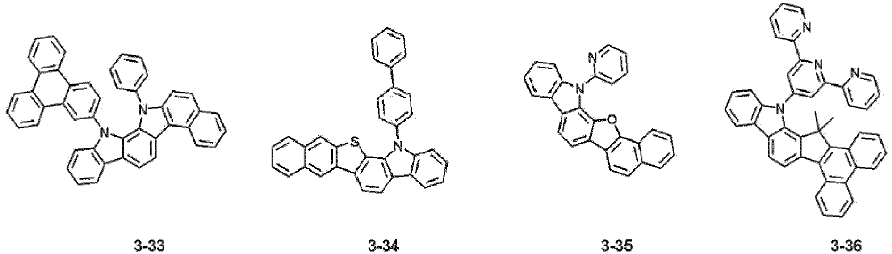

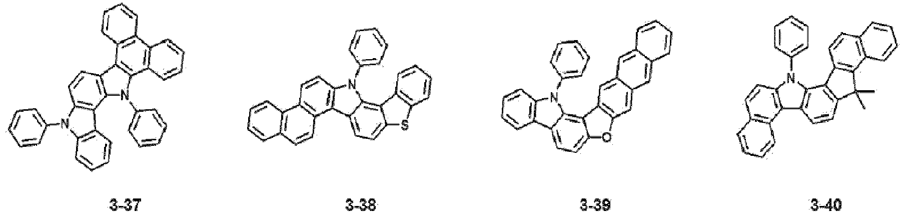

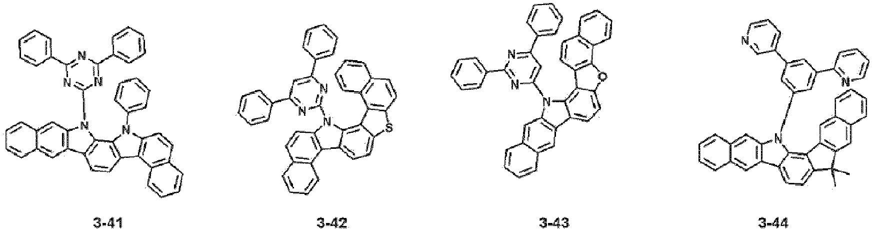

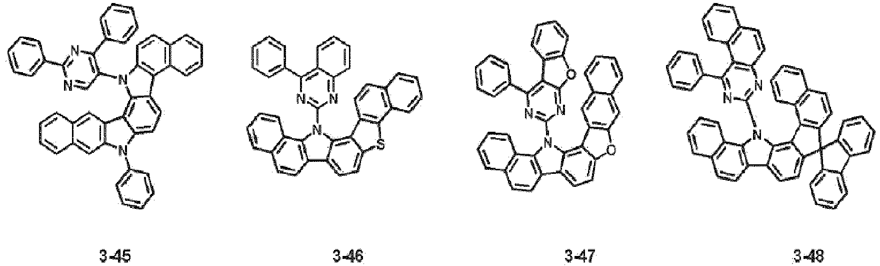

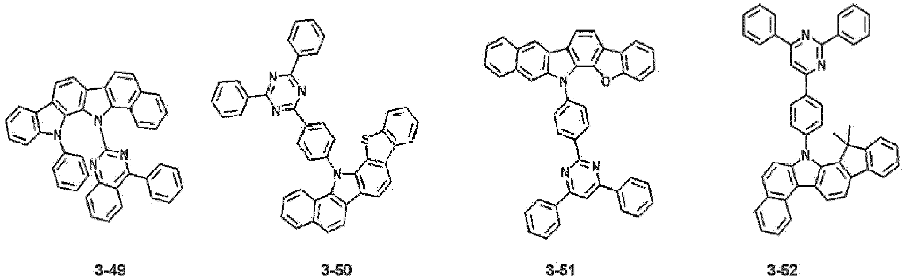

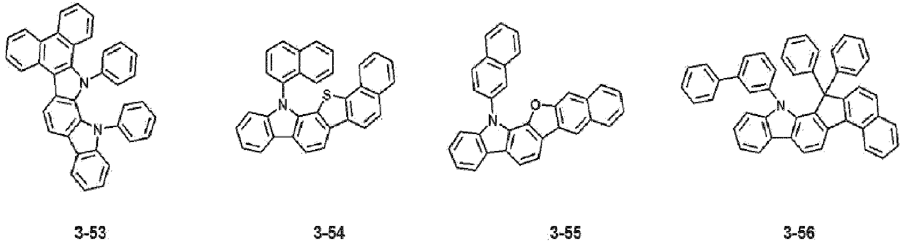

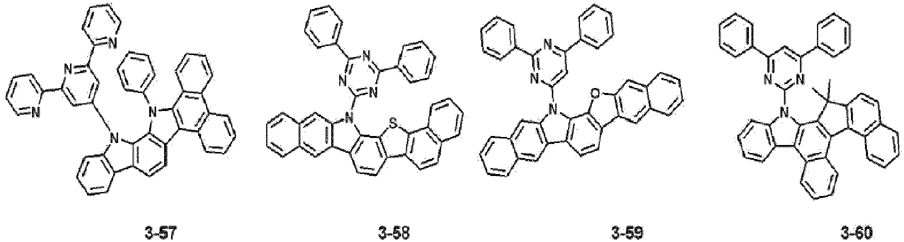

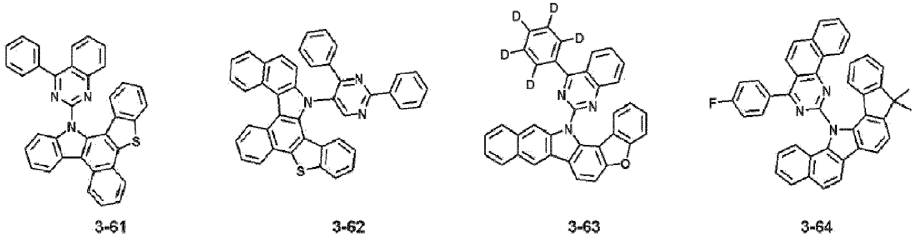

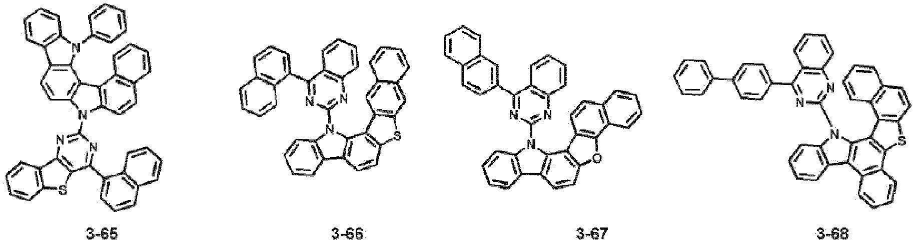

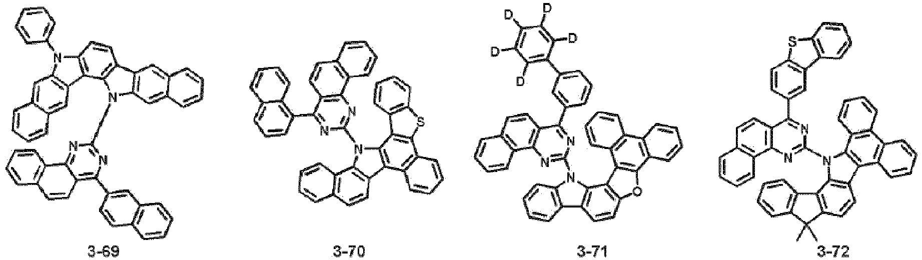

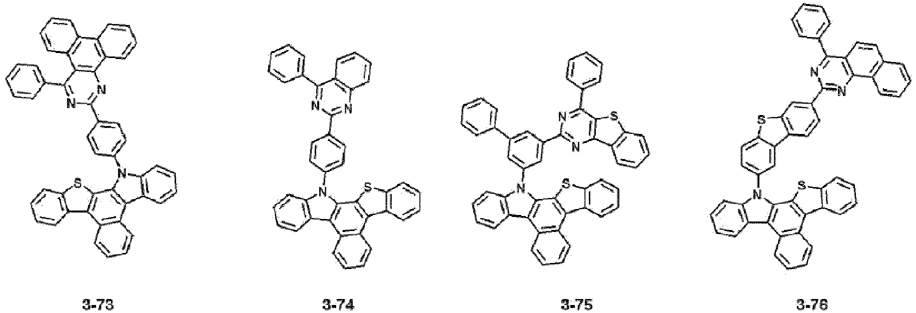

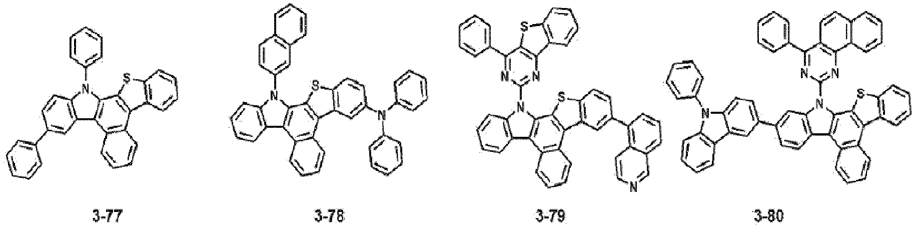

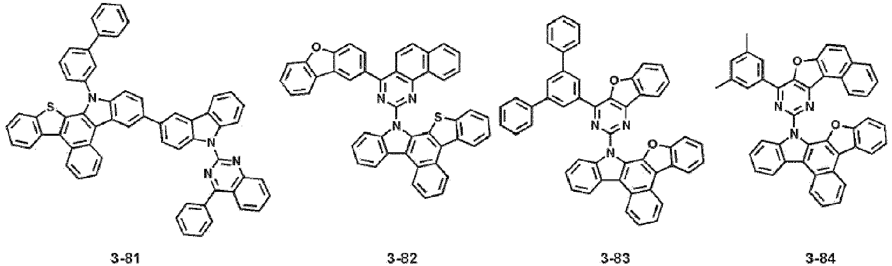

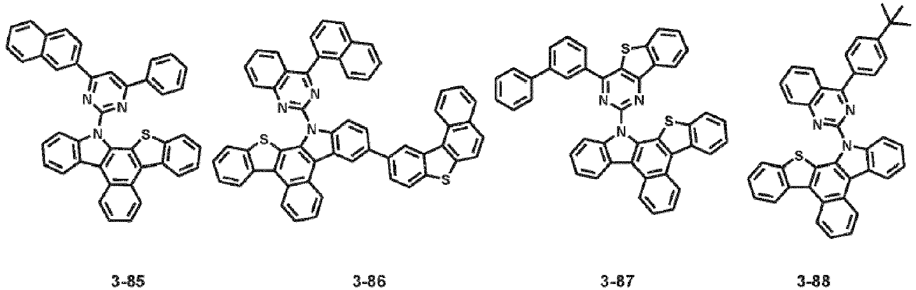

13. Organisches elektrisches Element nach Anspruch 1, wobei die Verbindungen, die durch Formel 1 und Formel 2 dargestellt werden, in einem Verhältnis von 1:9 bis 9:1 gemischt werden, um in die emittierende Schicht aufgenommen zu werden.

14. Organisches elektrisches Element nach Anspruch 1, wobei die Verbindungen, die durch Formel 1 und Formel 2 dargestellt werden, in einem Verhältnis von 1:9 bis 5:5 gemischt werden, um in die emittierende Schicht aufgenommen zu werden.

15. Organisches elektrisches Element nach Anspruch 1, wobei die Verbindungen, die durch Formel 1 und Formel 2 dargestellt werden, in einem Verhältnis von 2:8 oder 3:7 gemischt werden, um in die emittierende Schicht aufgenommen zu werden.

16. Elektronische Vorrichtung, umfassend eine Anzeigevorrichtung, die das organische elektrische Element nach Anspruch 1 und eine Steuereinheit zum Ansteuern der Anzeigevorrichtung beinhaltet.

## Revendications

1. Elément électrique organique (100) comprenant :
une première électrode (120), une seconde électrode (180) et une couche de matériau organique formée entre la première électrode et la seconde électrode, dans lequel la couche de matériau organique comprend une couche émettrice (150), dans laquelle la couche émettrice comprend un premier composé hôte représenté par la formule 1 en tant que couche émettrice de lumière phosphorescente et un second composé représenté par la formule 2, 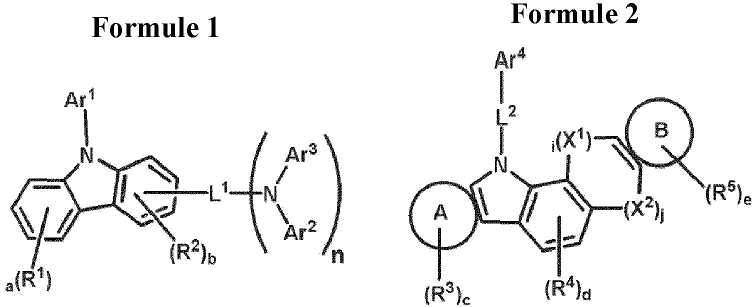
dans laquelle
1) Ar¹, Ar², Ar³ et Ar⁴ sont chacun indépendamment choisis dans le groupe constitué par un groupe C₆-C₆₀ aryle ; un groupe fluorényle ; un groupe C₂-C₆₀ hétérocyclique comprenant au moins un hétéroatome parmi O, N, S, Si et P ; un groupe à noyau fusionné parmi un noyau C₃-C₆₀ aliphatique et un noyau C₆-C₆₀ aromatique ; un groupe C₁-C₅₀ alkyle ; un groupe C₂-C₂₀ alcényle ; un groupe C₂-C₂₀ alcynyle ; un groupe C₁-C₃₀ alcoxy ; un groupe C₆-C₃₀ aryloxy ;
2) a représente un entier de 0 à 4, et b représente un entier de 0 à 3, et c et e représentent un entier de 0 à 10, et d représente un entier de 0 à 2,
3) R¹, R², R³, R⁴ et R⁵ peuvent être identiques ou différents, et sont chacun indépendamment choisis dans le groupe constitué par un deutérium ; un halogène ; un groupe C₆-C₆₀ aryle ; un groupe fluorényle ; un groupe C₂-C₆₀ hétérocyclique comprenant au moins un hétéroatome parmi O, N, S, Si ou P ; un groupe à noyau fusionné parmi un noyau C₃-C₆₀ aliphatique et un noyau C₆-C₆₀ aromatique ; un groupe C₁-C₅₀ alkyle ; un groupe C₂-C₂₀ alcényle ; un groupe C₂-C₂₀ alcynyle ; un groupe C₁-C₃₀ alcoxy ; un groupe C₆-C₃₀ aryloxy ; ou dans le cas où a, b, c et e valent 2 ou plus, et d vaut 2, R¹, R², R³, R⁴ et R⁵ sont chacun au pluriel et sont identiques ou différents, et une pluralité de R¹ ou une pluralité de R² ou une pluralité de R³ ou une pluralité de R⁴ ou une pluralité de R⁵ peuvent être liés les uns aux autres pour former un noyau,
4) L¹ et L² sont chacun indépendamment choisis dans le groupe constitué par une liaison simple, un groupe C₆-C₆₀ arylène et un groupe fluorénylène ; un groupe à noyau fusionné parmi un noyau C₃-C₆₀ aliphatique et un noyau C₆-C₆₀ aromatique ; et un groupe C₂-C₆₀ hétérocyclique,
5) A et B représentent chacun indépendamment un groupe C₆-C₆₀ aryle ou un groupe C₂-C₆₀ hétérocyclique, A et B représentant tous les deux un groupe C₆ aryle (groupe phényle) substitué ou non substitué, d vaut 2, et les groupes R⁴ sont liés les uns aux autres pour former un noyau pour former un noyau aromatique ou hétéro,
6) i et j valent indépendamment 0 ou 1, à condition que i + j vaille 1 ou plus, et lorsque i ou j vaut 0, il désigne une liaison directe,
7) X¹ et X² représentent indépendamment NR', O, S ou CR'R", dans lesquels R' et R" représentent indépendamment un hydrogène ; un groupe C₆-C₆₀ aryle ; un groupe fluorényle ; un groupe C₃-C₆₀ hétérocylique ; ou un groupe C₁-C₅₀ alkyle ; dans lesquels R' et R" peuvent être liés l'un à l'autre pour former un spiro, et
8) n représente un entier de 1 ou 2, lorsque n vaut 2, deux groupes Ar² sont identiques ou différents, et deux groupes Ar³ sont identiques ou différents,
et dans lesquels le groupe aryle, le groupe fluorényle, le groupe arylène, le groupe hétérocyclique, le groupe à noyau fusionné, le groupe alkyle, le groupe alcényle, le groupe alcoxy et le groupe aryloxy peuvent être substitués par un ou plusieurs substituants choisis dans le groupe constitué par un deutérium ; un halogène ; un groupe C₁-C₂₀ alkyle ; un groupe C₂-C₂₀ alcényle ; un groupe C₂-C₂₀ alcynyle ; un groupe C₆-C₂₀ aryle ; un groupe C₆-C₂₀ aryle substitué par un deutérium ; un groupe fluorényle ; un groupe C₂-C₂₀ hétérocyclique ; dans lesquels les substituants peuvent se combiner les uns aux autres et former un noyau saturé ou insaturé, dans lesquels le terme « noyau » désigne un noyau C₃-C₆₀ aliphatique ou un noyau C₆-C₆₀ aromatique ou un noyau C₂-C₆₀ hétérocyclique ou un noyau fusionné formé par la combinaison de ceux-ci.

2. Elément électrique organique selon la revendication 1, dans lequel le premier composé hôte représenté par la formule 1 comprend un composé représenté par la formule 3 ou la formule 4, la formule 21 ou la formule 22 ci-dessous, 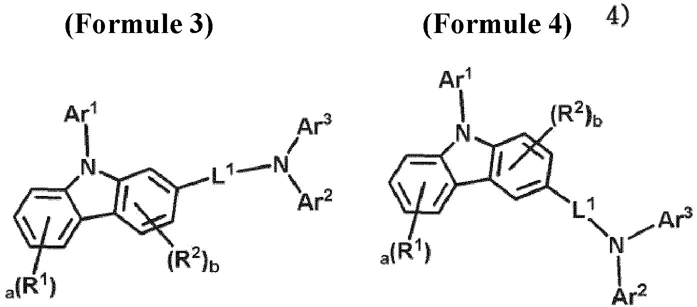 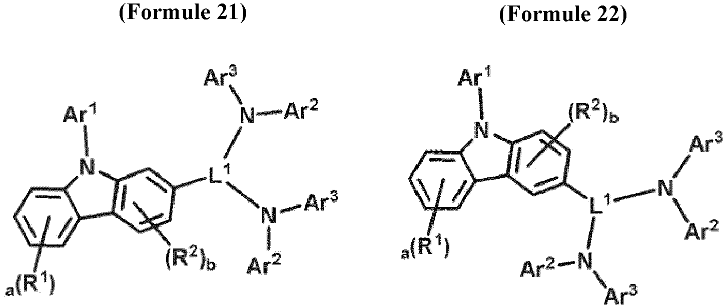 dans lesquelles R¹, R², L¹, Ar¹, Ar², Ar³, a et b sont les mêmes que ceux définis dans la revendication 1.

3. Elément électrique organique selon la revendication 1, dans lequel L¹ et L² sont choisis dans le groupe constitué par les formules (A-1) à (A-12) suivantes, 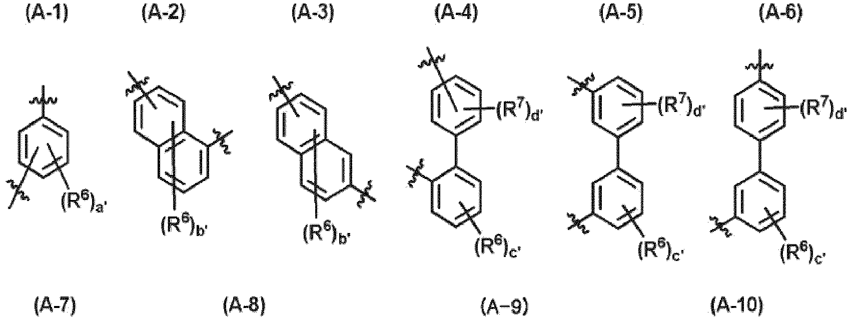 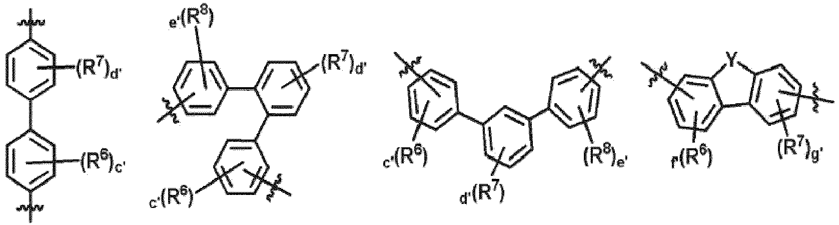 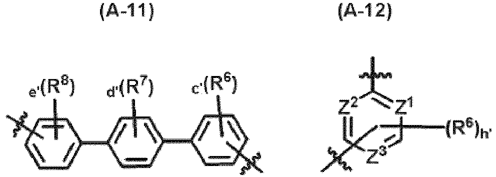 dans lesquelles
1) a', c', d' et e' représentent un entier de 0 à 4, et b' représente un entier de 0 à 5, et f' et g' représentent un entier de 0 à 3, et h' représente un entier de 0 à 1,
2) R⁶, R⁷ et R⁸ sont identiques ou différents, et sont chacun indépendamment choisis dans le groupe constitué par un deutérium ; un halogène ; un groupe C₆-C₆₀ aryle ; un groupe fluorényle ; un groupe C₂-C₆₀ hétérocyclique comprenant au moins un hétéroatome parmi O, N, S, Si ou P ; un groupe à noyau fusionné parmi un noyau C₃-C₆₀ aliphatique et un noyau C₆-C₆₀ aromatique ; un groupe C₁-C₅₀ alkyle ; un groupe C₂-C₂₀ alcényle ; un groupe C₂-C₂₀ alcynyle ; un groupe C₁-C₃₀ alcoxy ; un groupe C₆-C₃₀ aryloxy ;
ou lorsque f' et g' valent deux ou plus, ils sont identiques l'un à l'autre ou différents l'un de l'autre, et une pluralité de R⁶ ou une pluralité de R⁷ ou une pluralité de R⁸ peuvent être liés les uns aux autres pour former un noyau aromatique ou hétéroaromatique, deux groupes adjacents R⁶ et R⁷, ou R⁷ et R⁸ peuvent être liés pour former un noyau aromatique ou hétéroaromatique,
3) Y représente NR', O, S ou CR'R"
4) R' et R" sont les mêmes que ceux définis dans la revendication 1, et
5) Z¹, Z² et Z³ représentent indépendamment les uns des autres CR' ou N et au moins l'un représente N.

4. Elément électrique organique selon la revendication 1, dans lequel le premier composé hôte représenté par la formule 1 est représenté par l'une quelconque des formules 5 à 20 suivantes, 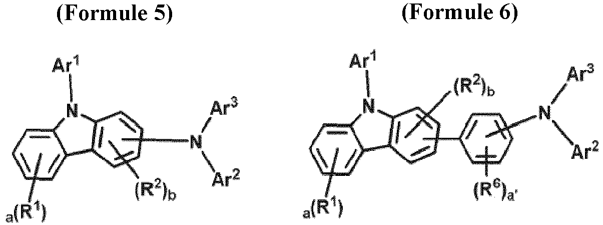 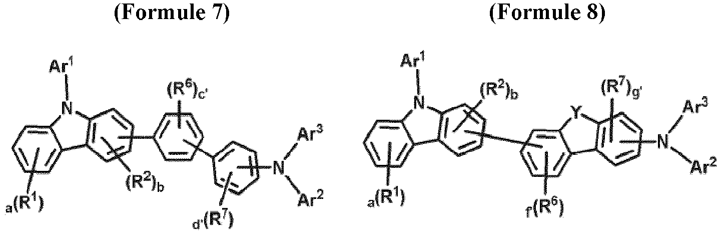 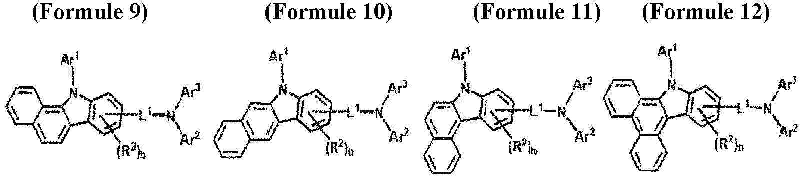 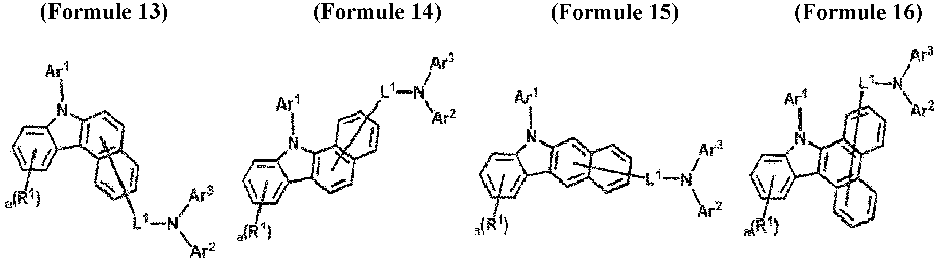 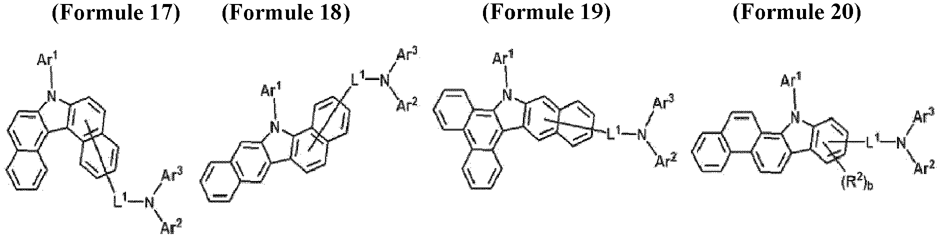 dans lesquelles
1) R¹, R², Ar¹, Ar², Ar³, a, b, R' et R" sont les mêmes que ceux définis dans la revendication 1,
2) R⁶ et R⁷ sont identiques ou différents, et sont chacun indépendamment choisis dans le groupe constitué par un deutérium ; un halogène ; un groupe C₆-C₆₀ aryle ; un groupe fluorényle ; un groupe C₂-C₆₀ hétérocyclique comprenant au moins un hétéroatome parmi O, N, S, Si ou P ; un groupe à noyau fusionné parmi un noyau C₃-C₆₀ aliphatique et un noyau C₆-C₆₀ aromatique ; un groupe C₁-C₅₀ alkyle ; un groupe C₂-C₂₀ alcényle ; un groupe C₂-C₂₀ alcynyle ; un groupe C₁-C₃₀ alcoxy ; un groupe C₆-C₃₀ aryloxy ; ou dans le cas où f' et g' valent 2 ou plus, chacun sous forme de pluralité sont identiques les uns aux autres ou différents les uns des autres, et une pluralité de R⁶ ou une pluralité de R⁷ ou une pluralité de R⁸ ou les groupes R⁶ et R⁷ adjacents peuvent être liés les uns aux autres pour former un noyau aromatique ou hétéroaromatique,
3) a', c' et d' représentent chacun un entier de 0 à 4, et f' et g' représentent chacun un entier de 0 à 3,
4) Y représente NR', O, S ou CR'R".

5. Elément électrique organique selon la revendication 1, dans lequel le premier composé hôte représenté par la formule 1 comprend un composé représenté par la formule 23 ci-dessous, 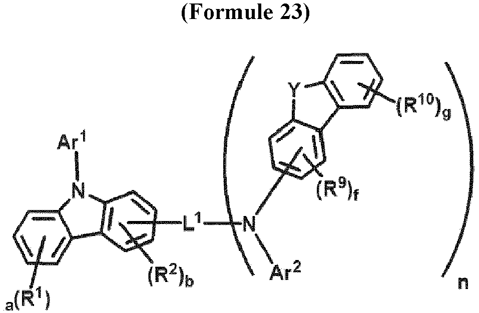 dans laquelle
1) R¹, R², L¹, Ar¹, Ar², a, b et n sont les mêmes que ceux définis dans la revendication 1,
2) f représente un entier de 0 à 3, et g représente un entier de 0 à 4,
3) R⁹ et R¹⁰ sont identiques ou différents, et sont chacun indépendamment choisis dans le groupe constitué par un deutérium ; un halogène ; un groupe C₆-C₆₀ aryle ; un groupe fluorényle ; un groupe C₂-C₆₀ hétérocyclique comprenant au moins un hétéroatome parmi O, N, S, Si ou P ; un groupe à noyau fusionné parmi un noyau C₃-C₆₀ aliphatique et un noyau C₆-C₆₀ aromatique ; un groupe C₁-C₅₀ alkyle ; un groupe C₂-C₂₀ alcényle ; un groupe C₂-C₂₀ alcynyle ; un groupe C₁-C₃₀ alcoxy ; un groupe C₆-C₃₀ aryloxy ; ou dans le cas où f et g valent 2 ou plus, chacun sous forme de pluralité sont identiques les uns aux autres ou différents les uns des autres, et une pluralité de R⁹ ou une pluralité de R¹⁰ ou les groupes R⁹ et R¹⁰ adjacents peuvent être liés les uns aux autres pour former un noyau aromatique ou hétéroaromatique,
4) Y représente NR', O, S ou CR'R", et
5) R' et R" sont les mêmes que ceux définis dans la revendication 1.

6. Elément électrique organique selon la revendication 1, dans lequel le second composé hôte représenté par la formule 2 comprend un composé représenté par la formule 24 ou la formule 25 ci-dessous, 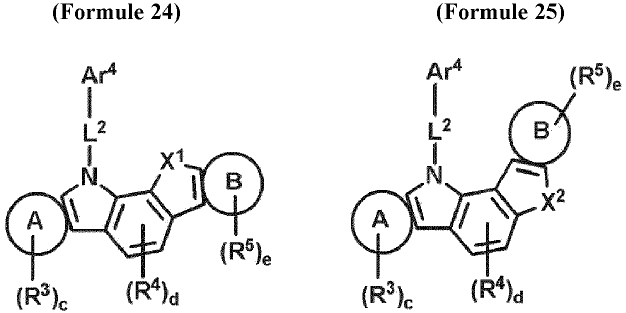 dans lesquelles R³, R⁴, R⁵, Ar⁴, L², c, d, e, A, B, X¹ et X² sont les mêmes que ceux définis dans la revendication 1.

7. Elément électrique organique selon la revendication 1, dans lequel A et B dans la formule 2 sont choisis dans le groupe constitué par les formules (B-1) à (B-7) suivantes, 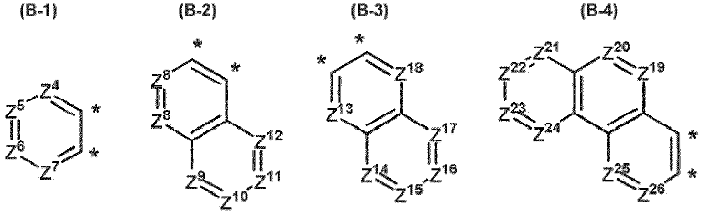 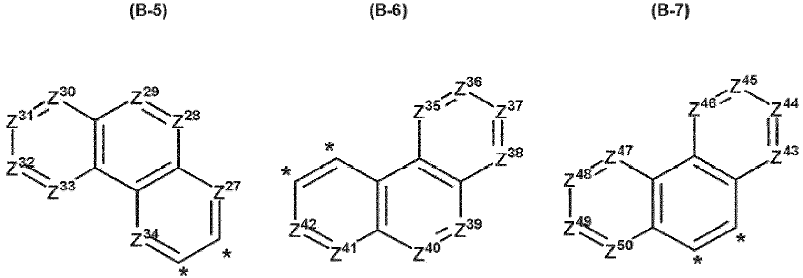 dans lesquelles
1) Z⁴ à Z⁵⁰ représentent CR' ou N,
2) R' est le même que celui défini dans la revendication 1, et
3) * indique la position devant être condensée.

8. Elément électrique organique selon la revendication 1, dans lequel R⁴ dans la formule 2 comprend un composé formant un noyau aromatique ou un noyau hétéroaromatique.

9. Elément électrique organique selon la revendication 1, dans lequel le second composé hôte représenté par la formule 2 comprend un composé représenté par l'une quelconque des formules 26 à 45 suivantes 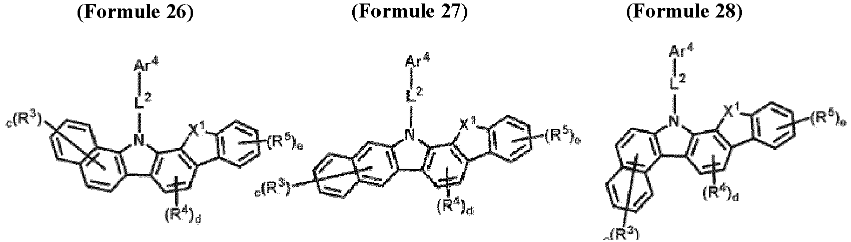 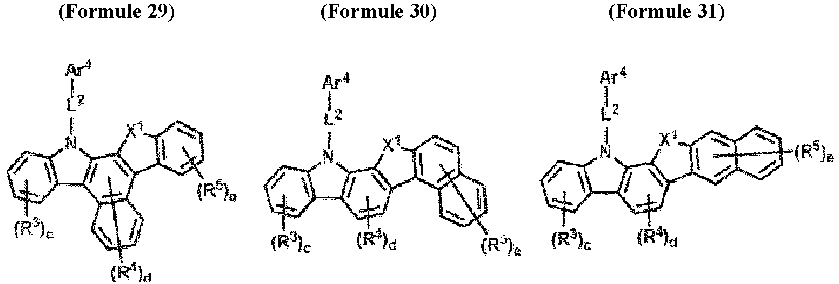 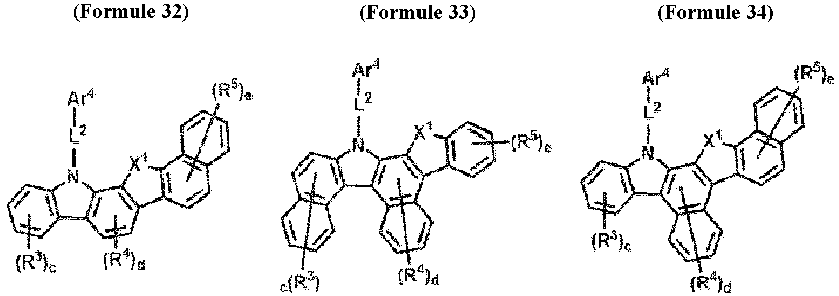 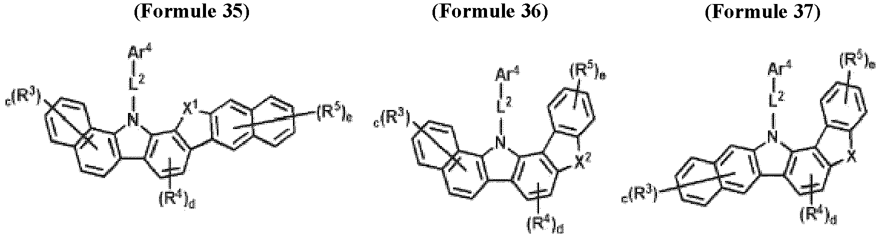 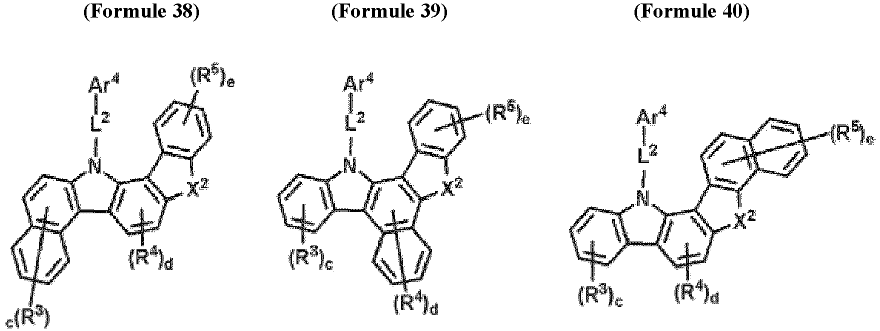 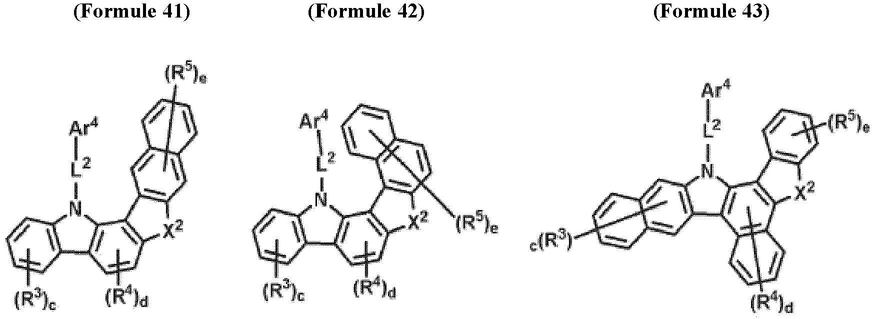 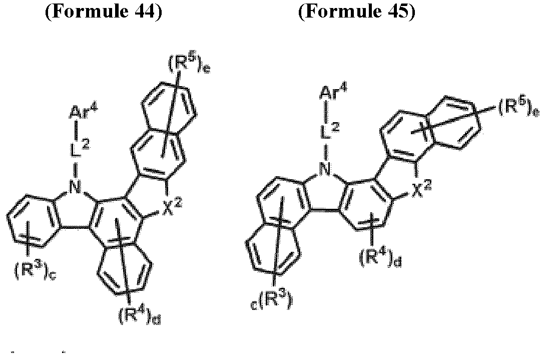 dans lesquelles Ar⁴, X¹, X², R³, R⁴, R⁵, L², c, d et e sont les mêmes que ceux définis dans la revendication 1.

10. Elément électrique organique selon la revendication 1, dans lequel le second composé hôte représenté par la formule 2 comprend l'un quelconque des composés représentés par les formules 46 à 53, 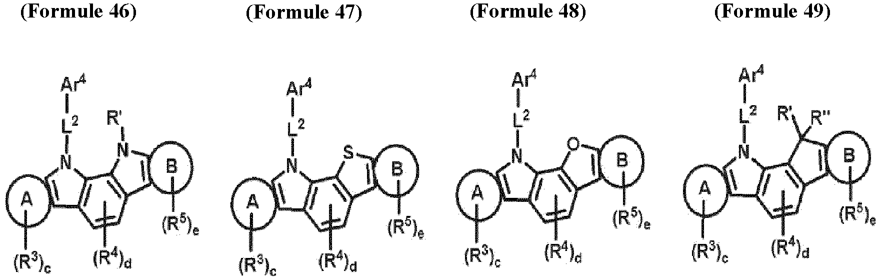 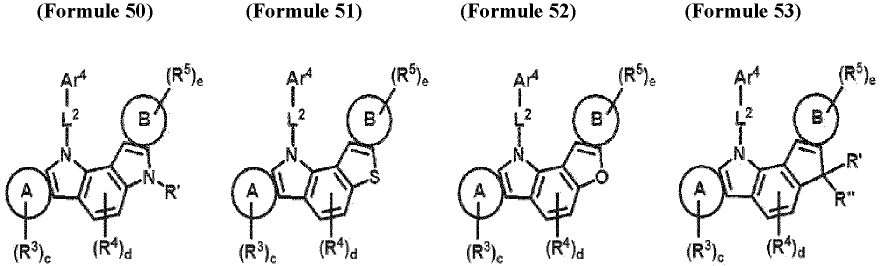 dans lesquelles
R³, R⁴, R⁵, Ar⁴, L², c, d, e, A, B, R' et R" sont les mêmes que ceux définis dans la revendication 1.

11. Elément électrique organique selon la revendication 1, dans lequel le premier composé hôte représenté par la formule 1 est l'un quelconque des composés 1-1 à 1-68 et des composés 2-1 à 2-68 suivants

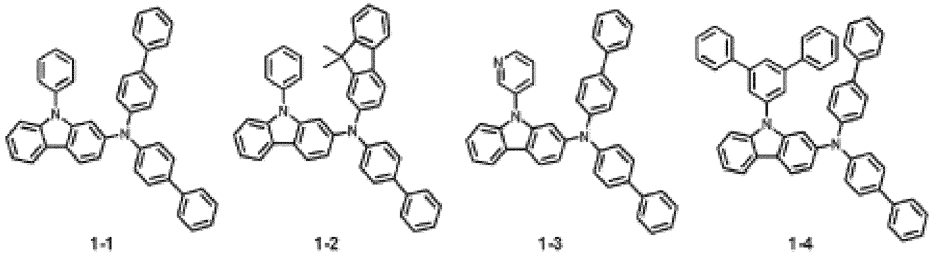

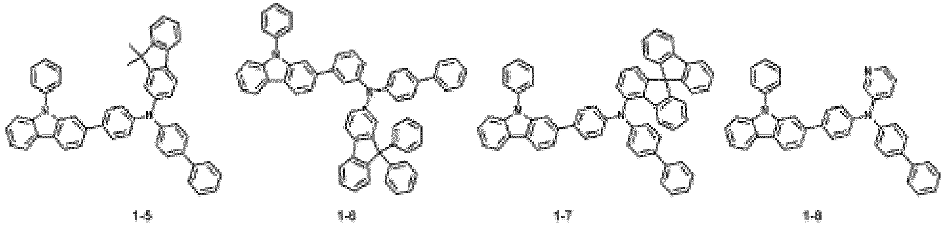

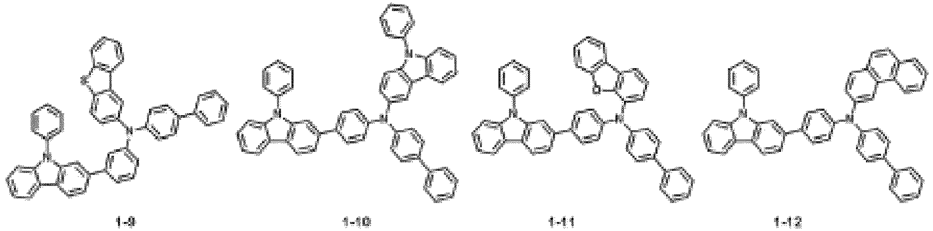

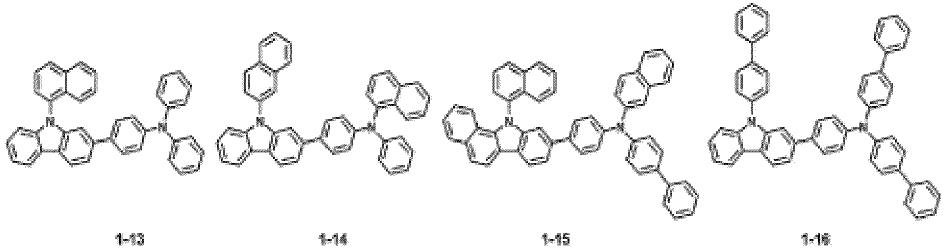

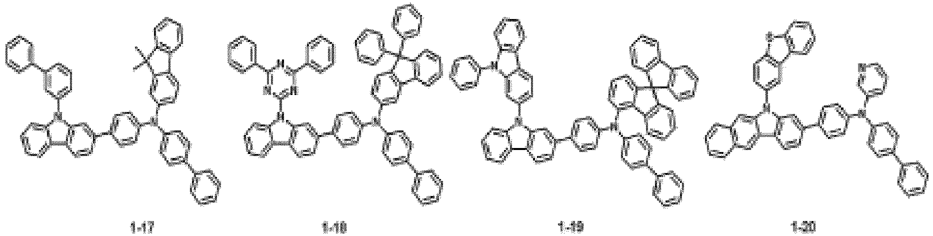

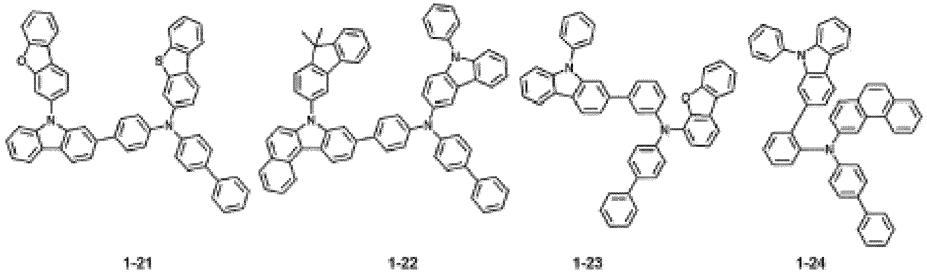

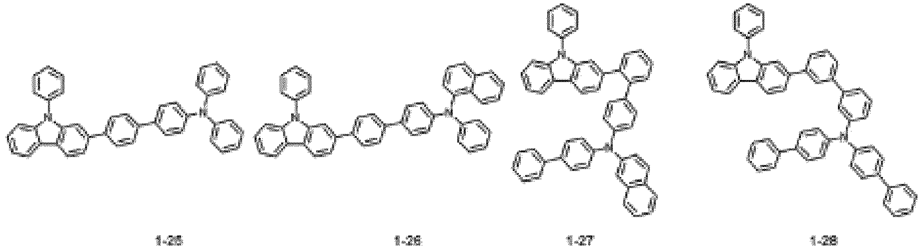

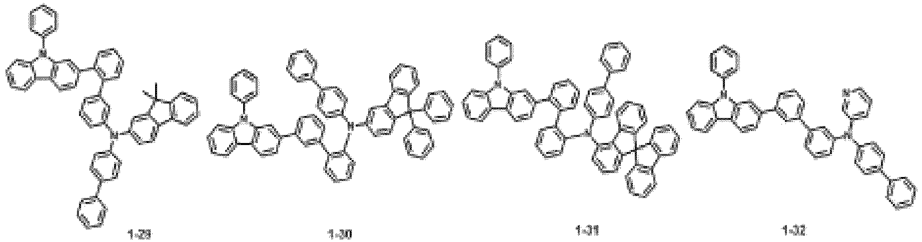

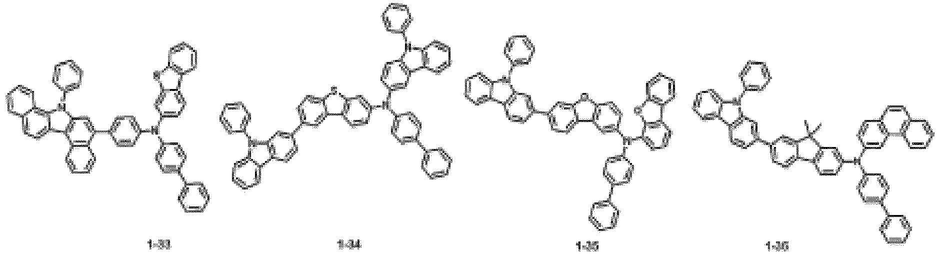

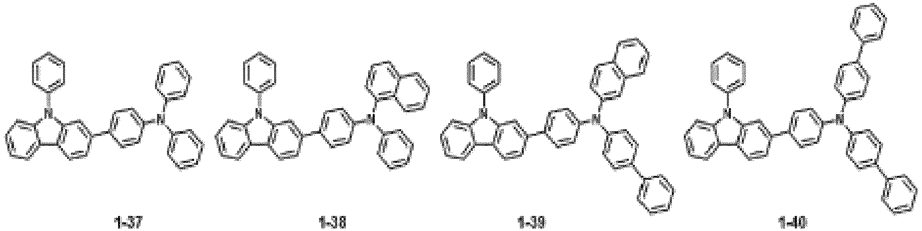

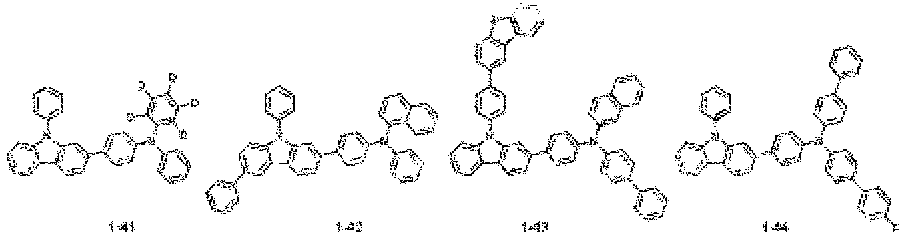

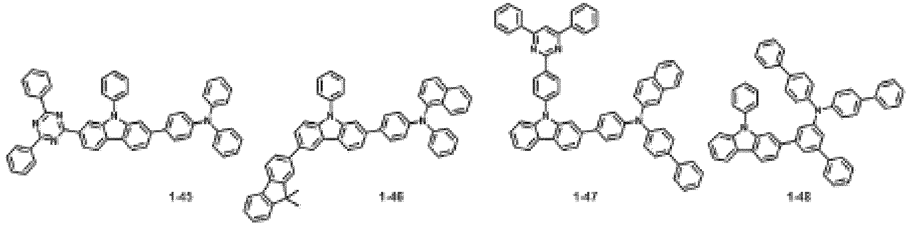

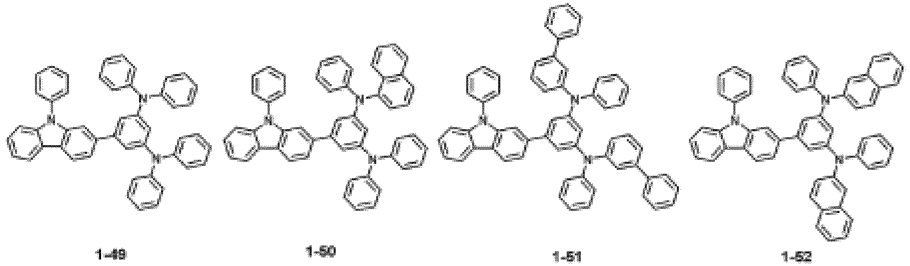

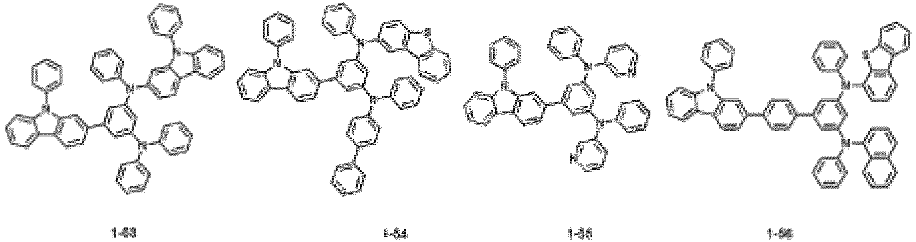

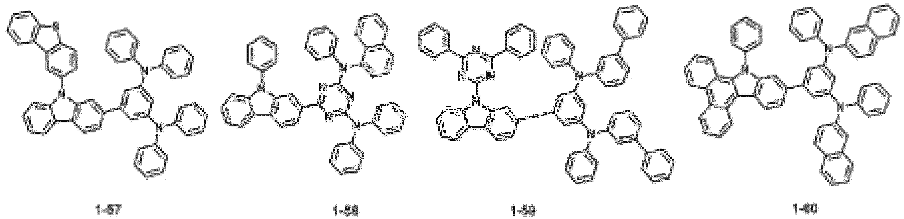

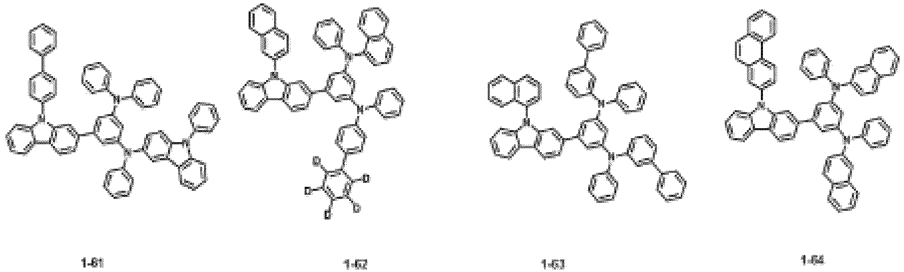

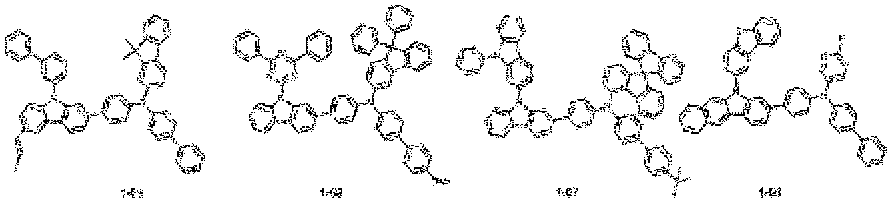

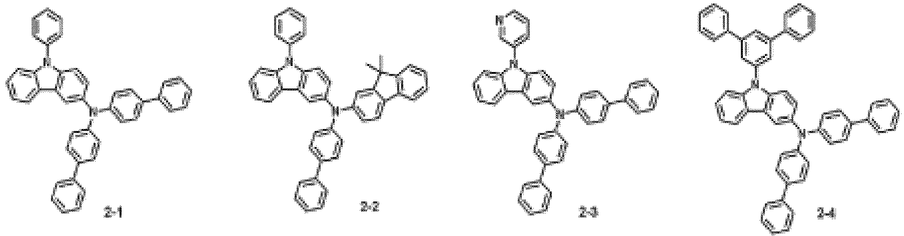

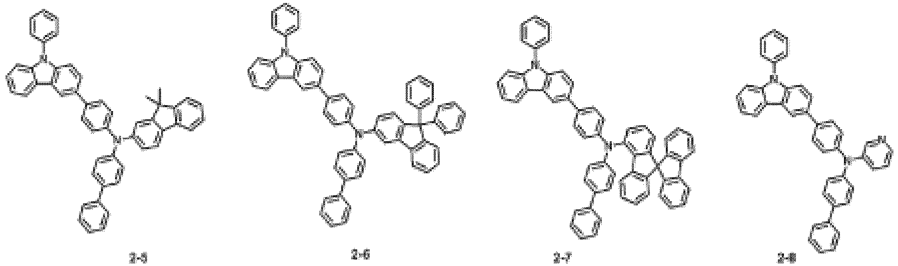

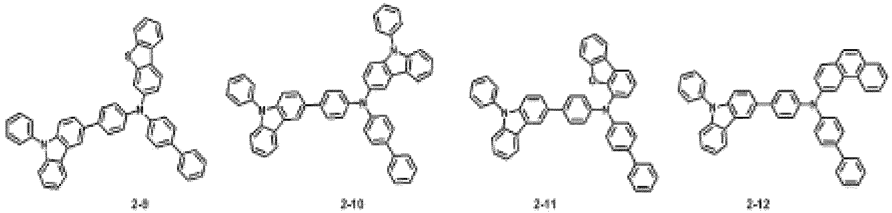

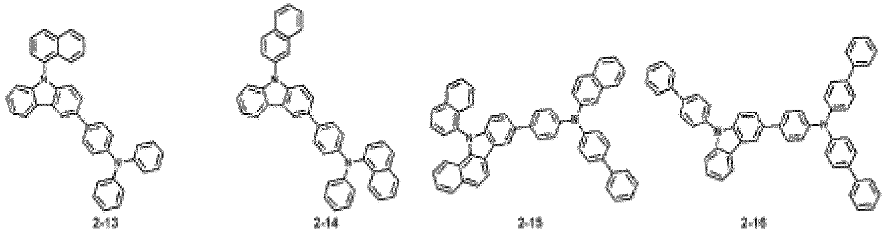

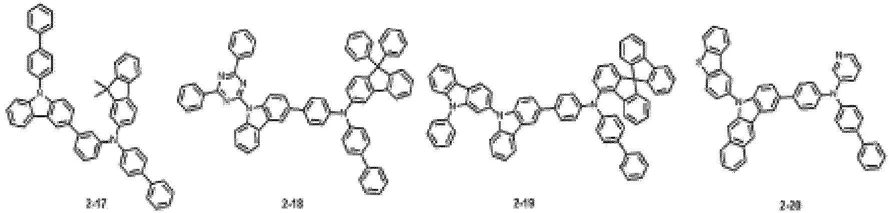

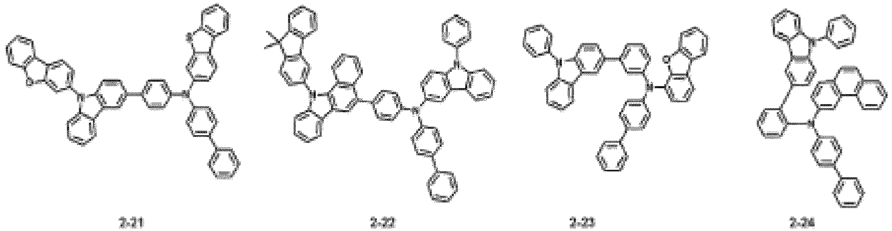

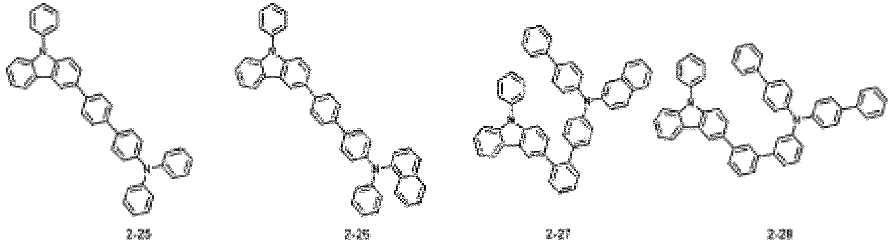

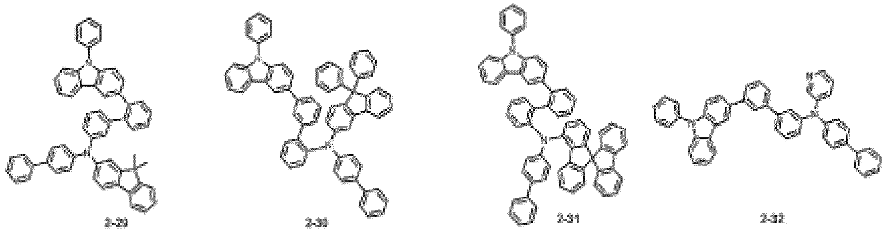

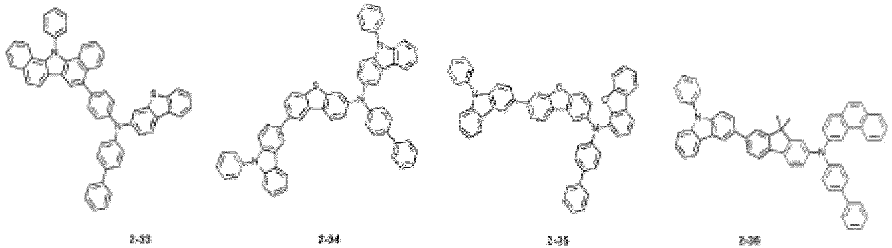

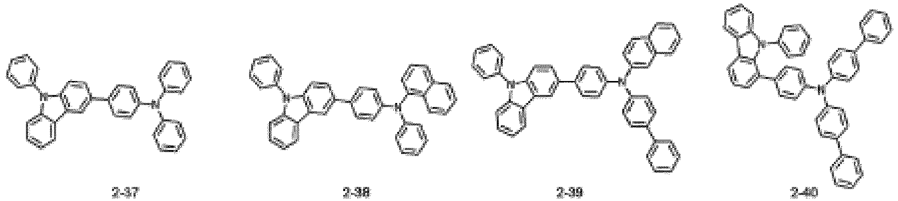

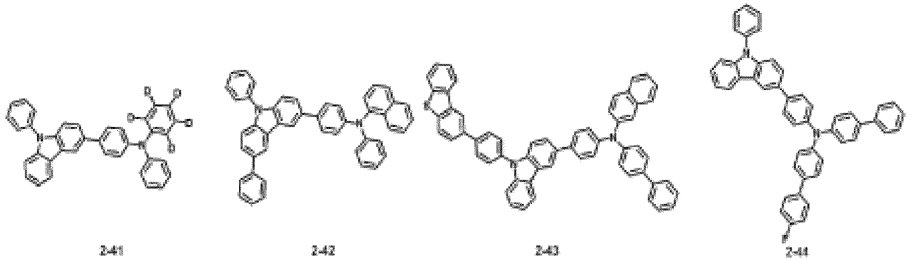

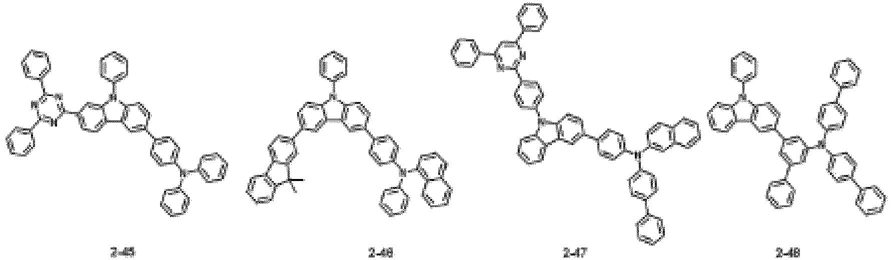

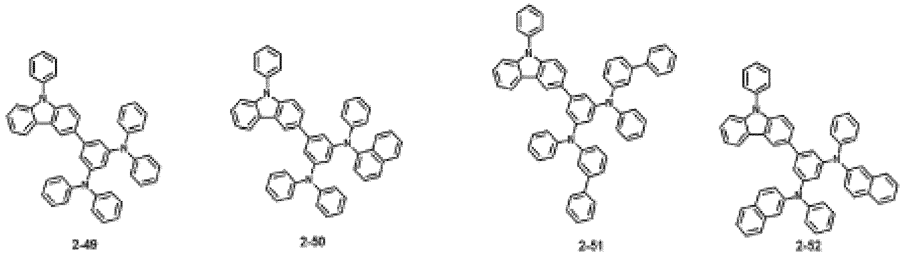

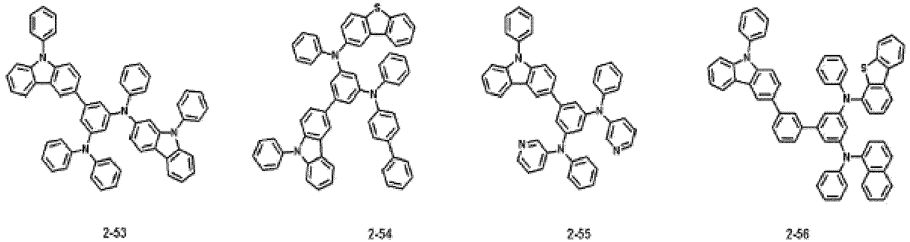

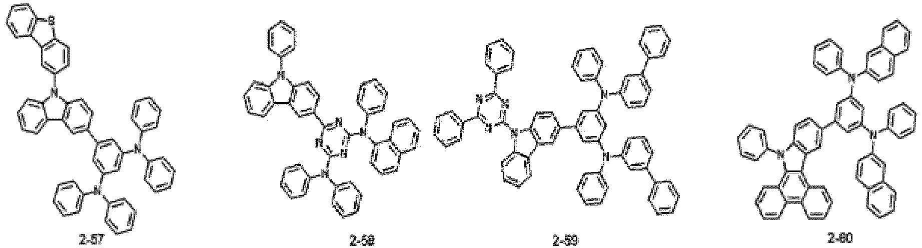

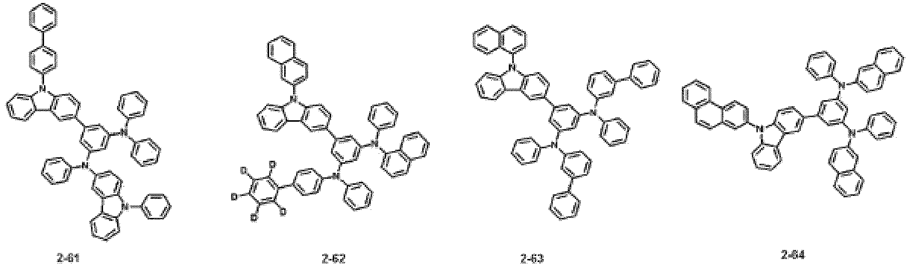

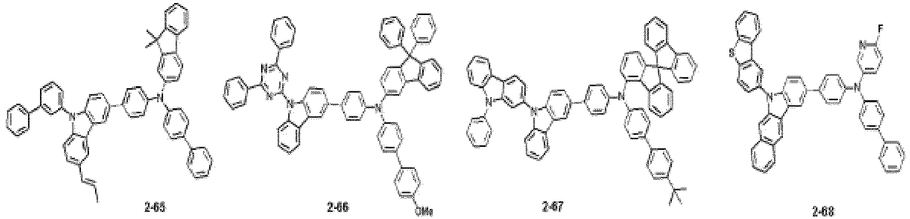

12. Elément électrique organique selon la revendication 1, dans lequel le second composé hôte représenté par la formule 2 comprend l'un quelconque des composés 3-1 à 3-88 suivants

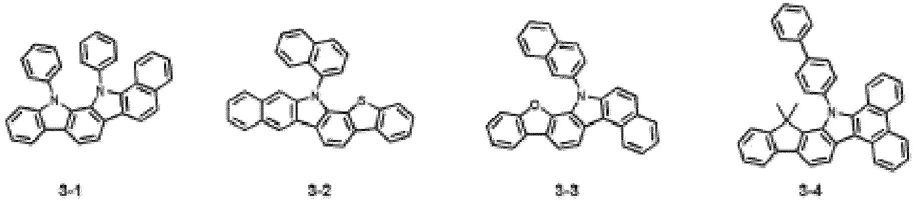

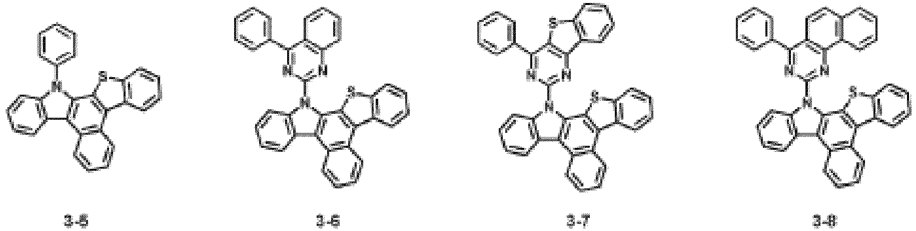

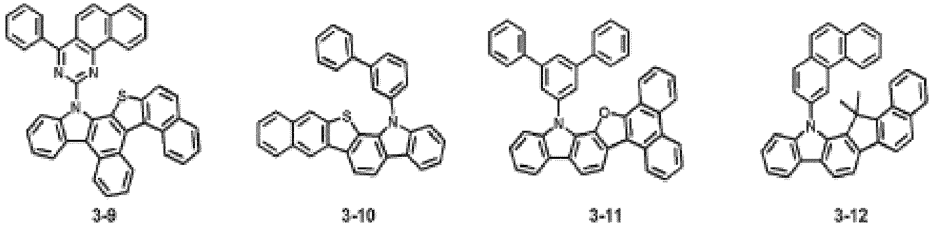

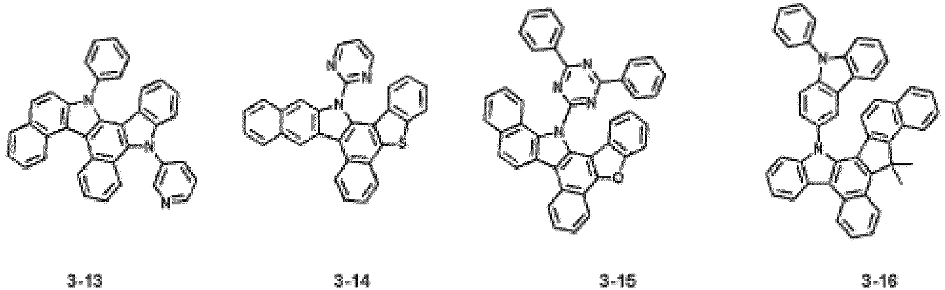

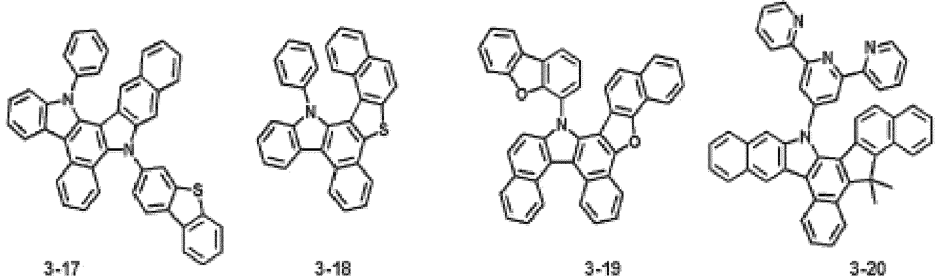

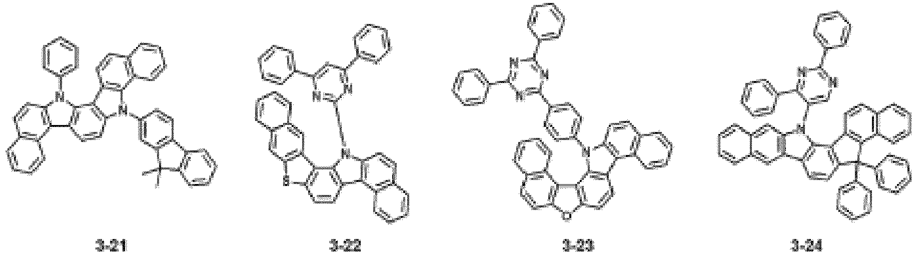

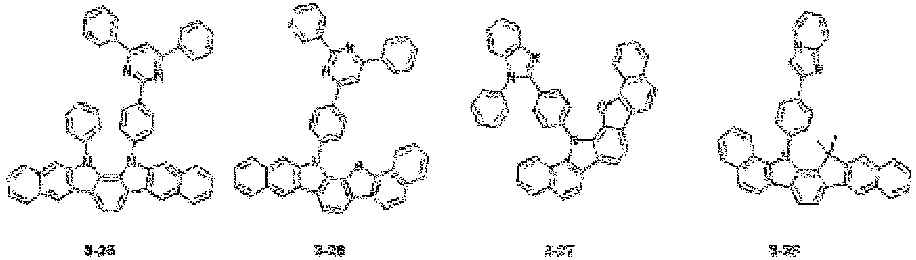

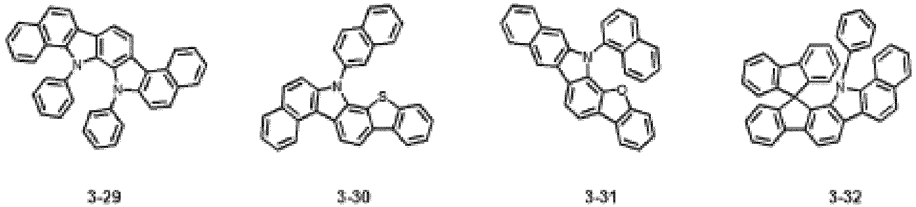

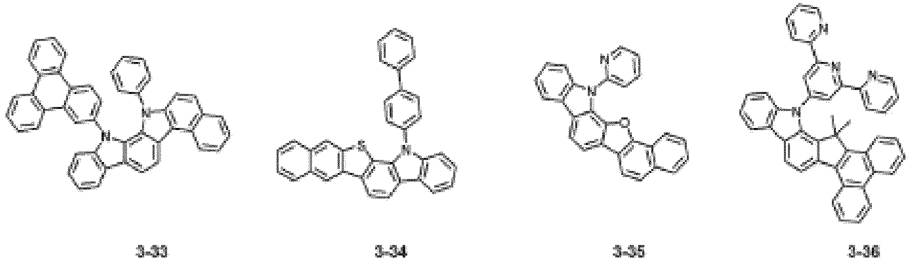

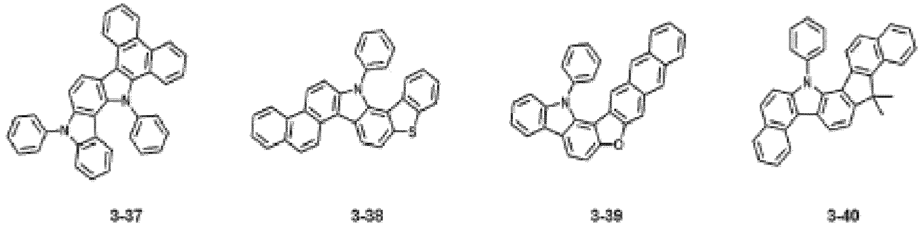

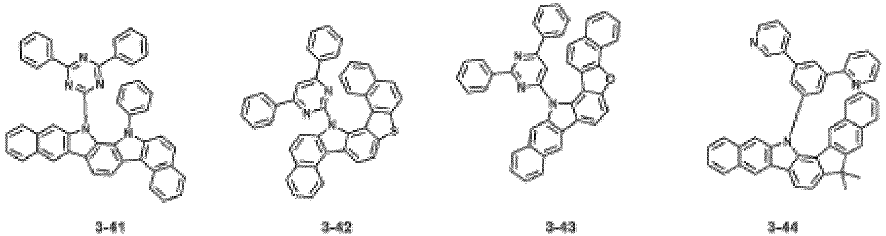

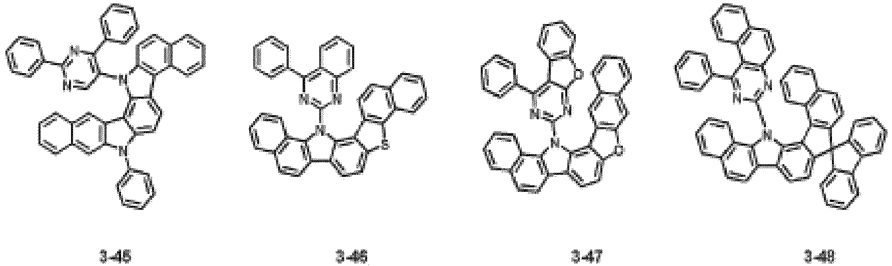

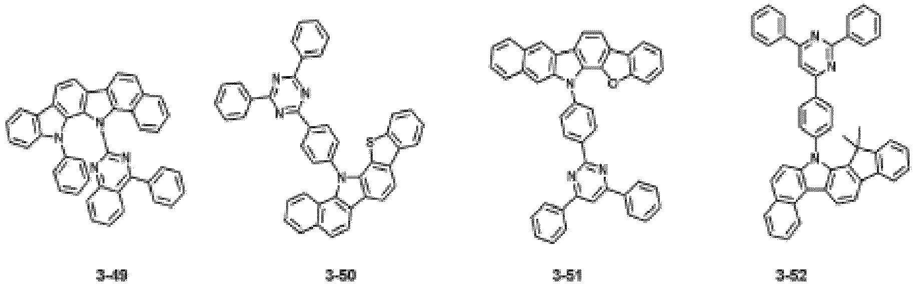

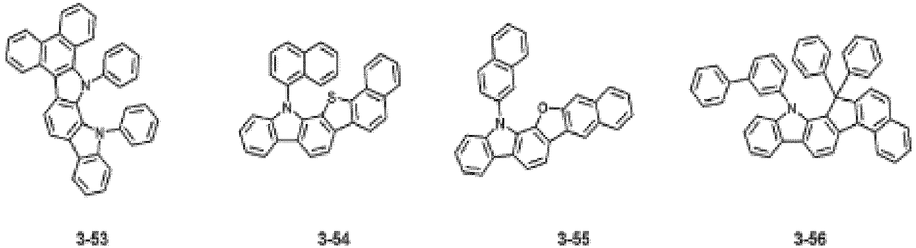

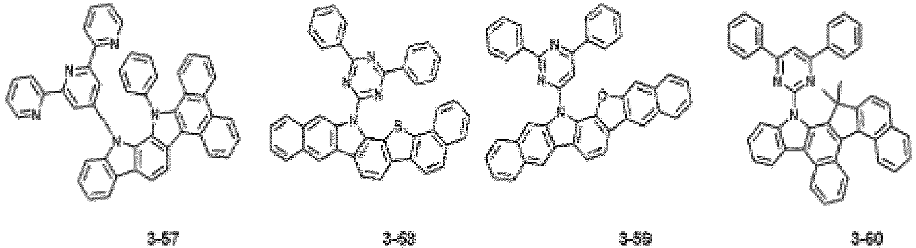

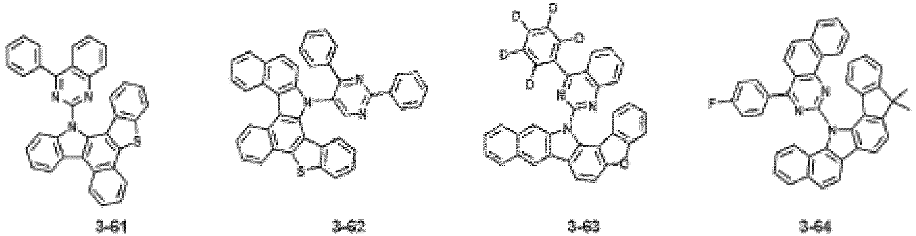

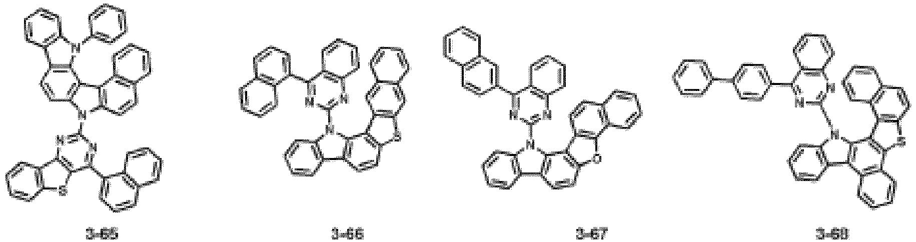

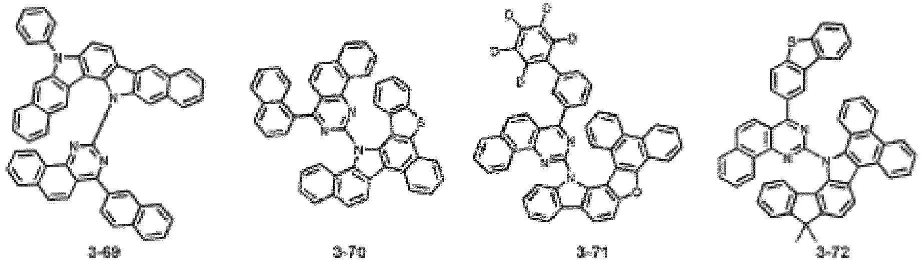

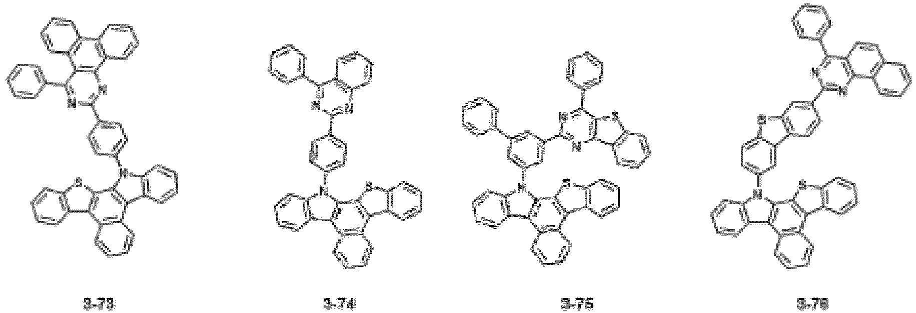

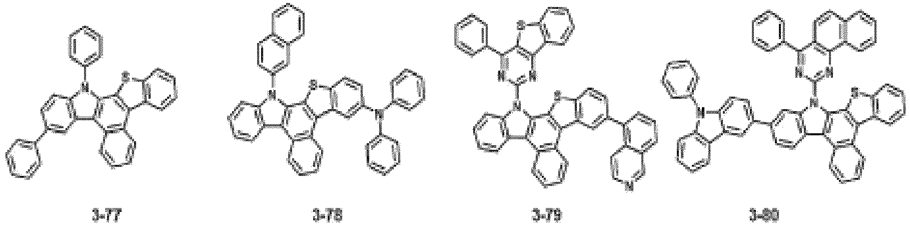

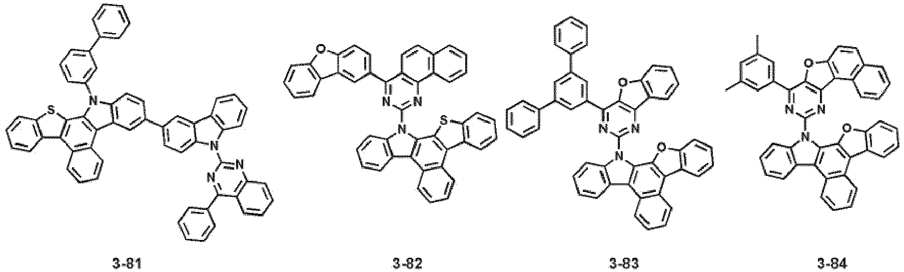

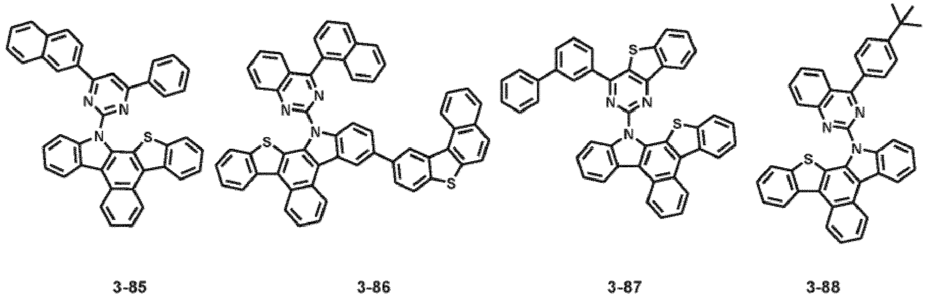

13. Elément électrique organique selon la revendication 1, dans lequel les composés représentés par la formule 1 et par la formule 2 sont mélangés dans un rapport de 1:9 à 9:1 pour être inclus dans la couche émettrice.

14. Elément électrique organique selon la revendication 1, dans lequel le composé représenté par la formule 1 et par la formule 2 sont mélangés dans un rapport de 1:9 à 5:5 pour être inclus dans la couche émettrice.

15. Elément électrique organique selon la revendication 1, dans lequel le composé représenté par la formule 1 et par la formule 2 sont mélangés dans un rapport de 2:8 ou 3:7 pour être inclus dans la couche émettrice.

16. Dispositif électronique comprenant un dispositif d'affichage incluant l'élément électrique organique selon la revendication 1 ; et une unité de commande pour commander le dispositif d'affichage.
